# EUROPEAN PATENT SPECIFICATION

(11) **EP 3 842 437 B1**
(45) Date of publication and mention of the grant of the patent: **10.04.2024**
(21) Application number: 19851013.3
(22) Date of filing: 21.08.2019
(51) Int. Cl.: C07D 498/06, C07D 471/06, C07D 487/06, A61K 31/4188, A61K 31/5517, A61P 35/00, A61P 37/06, A61P 37/00, A61P 31/12, A61P 25/28

(54) **HIGH ACTIVITY STING PROTEIN AGONIST**
STING-PROTEINAGONIST MIT HOHER AKTIVITÄT
AGONISTE DE PROTÉINE STING À HAUTE ACTIVITÉ

(30) Priority: 24.08.2018 CN 201810973172; 25.12.2018 CN 201811592949
(43) Date of publication of application: 30.06.2021
(73) Proprietor: Adlai Nortye Biopharma Co., Ltd., Yuhang District Hangzhou Zhejiang 311121 (CN)
(72) Inventor: CHEN, Yufeng, Hangzhou, Zhejiang 311121 (CN); CHEN, Kaixuan, Hangzhou, Zhejiang 311121 (CN); LI, Pan, Hangzhou, Zhejiang 311121 (CN); LIU, Canfeng, Hangzhou, Zhejiang 311121 (CN); WANG, Ji, Hangzhou, Zhejiang 311121 (CN); QIU, Qingchong, Hangzhou, Zhejiang 311121 (CN); LU, Yang, Hangzhou, Zhejiang 311121 (CN)
(74) Representative: Boult Wade Tennant LLP
(86) International application number: PCT/CN2019/101707
(87) International publication number: WO 2020/038387

(56) References cited:
- WO-A1-2010/144680
- WO-A1-2017/175147
- WO-A1-2017/175156
- WO-A1-2017/175156
- WO-A1-2020/006432
- CN-A- 1 384 835
- CN-A- 102 802 731
- CN-A- 104 903 321
- CN-A- 105 164 131
- HAAG, S.M. et al.: "Targeting STING with Covalent Small-Molecule Inhibitors", Nature, vol. 559, 12 July 2018 (2018-07-12), XP036553086, DOI: 20191108142218A

## Description

### TECHNICAL FIELD

The present invention relates to a heterocyclic compound, in particular to a high active STING protein agonist and use thereof.

### BACKGROUND

The positive response to immunotherapy generally depends on the interaction of tumor cells with immunoregulation within the tumor microenvironment (TME). Under these interactions, the tumor microenvironment plays an important role in suppressing or enhancing the immune response. Understanding the interaction between immunotherapy and TME is not only the key to analyze the mechanism of action, but also provides a new method to improve the efficacy of current immunotherapy. Cytokines are a broad class of proteins that can modulate immune responses and can directly activate immune effector cells or stimulate tumor stromal cells to produce chemokines and adhesion molecules for lymphocyte recruitment. These functions suggest that targeting cytokines may also be an effective approach for tumor immunotherapy, depending on different tumor microenvironments.

STING (interferon gene-stimulating protein) is currently the latest and hottest immunotherapy target in drug development in the field of tumor immunotherapy. Interferon gene-stimulating protein is a transmembrane protein, which is usually dimerized and self-inhibited in the region 152-173. Upon stimulation by a partial ligand, the molecular conformation changes and is activated, recruiting TANK-binding kinase 1 in the cytoplasm, mediating phosphorylation of IRF3 by TBK1, resulting in the formation of interferon-β and a variety of other cytokines. The production of IFNβ is a sign of STING activation. The signal transduction of innate immunity in the tumor microenvironment is a key step in the activation of tumor-specific T cells and infiltration of tumor-infiltrating lymphocytes. Where type I IFN plays a key role in tumor-activated T cell activation. Thus, STING not only induces the expression of type I interferon gene, but also plays an important role in innate immune signaling pathway; STING agonists may activate immunostimulatory cells including dendritic cells, alter the tumor microenvironment and induce the production of tumor-specific T cells. In murine experiments, DMXAA, a flavonoid vascular disrupting agent, induced the production of IFN-β and other natural cytokines by activating murine STING proteins, and effectively inhibited the growth of a variety of solid tumors. However, no significant effect was observed in a human non-small cell clinical trial in combination with standard chemotherapy. Later experiments demonstrated that although the similarity between human and murine STING proteins reached 81%, the former encoded 379 amino acids and the latter encoded 378 amino acids, DMXAA failed to activate human STING proteins. Cyclic dinucleotide is the only type of STING agonists discovered to date that activates both murine and human STING proteins directly. Direct injection of CDN into B16 melanoma, CT26 rectal cancer, and 4T1 breast cancer tumors not only resulted in significant inhibition until the tumor disappeared, but also induced systemic persistent antigen-specific T cell immunity, resulting in inhibition of tumor growth in other parts of the animal without drug injection. ML RR-S2 CDA causes changes in the microenvironment of a variety of solid tumors, activates effective tumor-induced CD8+T cells and has a long-lasting therapeutic effect. In recent years, a large number of study reports have demonstrated that STING pathway can effectively initiate the body's natural immune system, which is one of a few signaling pathways that have been proven to induce cytokine interferon production, and is very important in innate immunity. Sufficient infiltration of lymphocytes into tumor tissue is the key to successful immunotherapy. The activation of the target pathway also promotes the infiltration and response to effector T cells in the tumor microenvironment. Therefore, this target has gradually become an important target for anti-tumor therapy, especially immunotherapy. In a plurality of mouse inoculation models, the composition is effective for a plurality of refractory and metastatic solid tumors, not only is the tumor injected directly disappeared, but also the growth of tumor at other parts is obviously inhibited, and even the occurrence of the tumor can be prevented.

### SUMMARY

The present invention provides a compound having STING protein agonist activity.

The present invention is described in the appended claims.

An object of the present invention is to provide a compound having a structure of Formula (I), 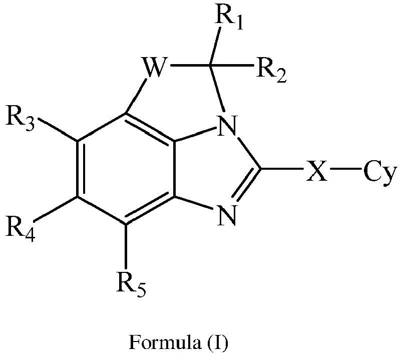
where W represents (CR^{a}R^{a'})ₘ, where any one CR^{a}R^{a'} is optionally substituted by 0, 1 or 2 O, S or NR^{b};
R₁ and R₂ are each independently selected from hydrogen, halogen, hydroxy, amino, mercapto, C₁-C₆ alkyl, C₁-C₆ alkoxy, C₁-C₆ alkylthio, C₁-C₆ alkylamino, di(C₁-C₆ alkyl) amino, C₂-C₆ alkenyl, C₂-C₆ alkynyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), and -(C₀-C₆ alkylene)-(5- to 12-membered heteroaryl), or R₁ and R₂ together with atoms adjacent thereto are cyclized to form a 3- to 6-membered ring optionally containing 0, 1 or 2 heteroatoms selected from O, N and S;
R₃, R₄, and R₅ are each independently selected from hydrogen, halogen, cyano, C₁-C₆ alkyl, C₂-C₆ alkenyl, C₂-C₆ alkynyl, -(C₀-C₆ alkylene)-(C₃-C₆ cycloalkyl), -(C₀-C₆ alkylene)-(4- to 7-membered heterocycloalkyl), -OR^{c}, -NR^{c}R^{c'}, -OC(O)R^{c'}, -C(O)R^{c}, -CO₂R^{c}, -CON(R^{c})(R^{c'}), -C(=NR^{c})N(R^{c'})(R^{c"}), -NHC(O)R^{c}, -NHS(O)₂R^{c}-, -NHS(O)R^{c}-, -SO₂R^{c}, -SO₂NR^{c}R^{c'}, -(C₀-C₆ alkylene)-(4-6 heterocycloalkyl), -(C₀-C₆ alkylene)-(6- to 12-membered aryl), and -(C₀-C₆ alkylene)-(5- to 12-membered heteroaryl);
or R₃ and R₄ are cyclized together to form a 5- to 8-membered ring optionally containing 0, 1, 2, 3 or 4 heteroatoms selected from O, S and N;
or R₄ and R₅ are cyclized together to form a 5- to 8-membered ring optionally containing 0, 1, 2, 3 or 4 heteroatoms selected from O, S and N;
X represents -NR^{d}C(O)-, or -NR^{d}C(=NR^{d'})-;
Cy represents 6- to 12-membered aryl or 5- to 12-membered heteroaryl;
m represents an integer of 1, 2 or 3;
R^{a} and R^{a'} each independently represent hydrogen, halogen, hydroxy, C₁-C₆ alkyl, C₁-C₆ alkylthio, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), -(C₀-C₆ alkylene)-(5- to 12-membered heteroaryl), -NR^{e}R^{e'}, -NR^{e}COR^{e'}, -NR^{e}SO₂R^{e'}, -OR^{e} or -OCOR^{e}, or R^{a} and R^{a'} together with atoms adjacent thereto, are cyclized into a 3- to 6-membered ring optionally containing 0, 1, or 2 heteroatoms selected from O, N and S; or any one CR^{a}R^{a'} is taken together to form -C=O;
R^{b} each independently represents hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene) -(6- to 12-membered aryl), -(C₀-C₆ alkylene)-(5- to 12-membered heteroaryl), -(C₀-C₆ alkylene) -(6- to 12-membered aryl), -C(O)R^{f}, -SO₂R^{f}, -SOR^{f}, -C(O)OR^{f}, or -C(O)NR^{f}R^{f'};
R^{c}, R^{c'}, R^{c"}, R^{d}, R^{d'}, R^{e}, R^{e'} , R^{f}, and R^{f'} each independently represent hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl) or -(C₀-C₆ alkylene)-(5- to 12-membered heteroaryl), or when the above-mentioned substituents are collectively bound to a single N atom, they are optionally cyclized with the bound N atom to form a 3- to 8-membered ring;
the above-mentioned alkyl, alkylene, aryl, heteroaryl, ring, cycloalkyl, heterocycloalkyl, alkenyl, alkynyl, and alkoxy are each optionally independently substituted by 0, 1, 2, 3, or 4 substituents selected from the following groups cosisting of: halogen, hydroxyl, cyano, carboxyl, C₁-C₆ alkyl, C₁-C₆ haloalkyl, sulfo, -OR^{g}, -SR^{g}, -NR^{g}R^{g'}, -NR^{g}COR^{g'}, -NR^{g}COOR^{g'}, -COR^{g}, -CO₂R^{g}, -SOR^{g}, -SO₂R^{g}, -OCONR^{g}R^{g'}-, -OCOR^{g}, -CONR^{g}R^{g'}, -NR^{g}SO₂R^{g'}, -SO₂NR^{g}R^{g'}, and -OP(O)(OR^{g}R^{g'})₂;
or for the aryl and heteroaryl, or when the number of substituents is 2, the adjacent two substituents are also optionally cyclized to each other into a 5- to 6-membered saturated or unsaturated carbocycle or heterocycle, the heterocycle being a ring containing 0, 1, 2, 3 or 4 heteroatoms selected from O, S and N;
where R^{g} and R^{g'} each independently consist ofhydrogen, or the following groups optionally substituted by 0, 1, 2, 3 or 4 groups selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino and di(C₁-C₆ alkyl) amino: C₁-C₆ alkyl, -(C₀-C₆ alkylene)-(C₃-C₆ cycloalkyl), -(C₀-C₆ alkylene)-O-(C₁-C₆ alkyl), -(C₀-C₆ alkylene)-O-CO (C₁-C₆ alkyl), -(C₀-C₆ alkylene)-C(O)O (C₁-C₆ alkyl), -(C₀-C₆ alkylene)-(6- to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆ alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl);
where the 6- to 12-membered aryl is preferably phenyl; the 5- to 12-membered heteroaryl is preferably pyridyl, imidazolyl, or pyrazolyl; or for the above-mentioned 6- to 12-membered aryl or 5- to 12-membered heteroaryl, when the number of substituents is 2, the adjacent two substituents may also be cyclized to each other into a 5- to 6-membered saturated or unsaturated carbocycle or heterocycle.

An object of the present invention is to provide a compound having a structure of Formula (II), 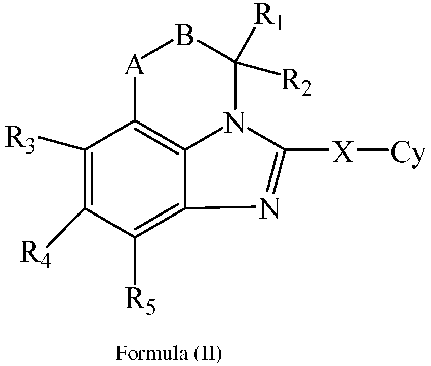 where A and B each independently represent CR^{a}R^{a'}, NR^{b}, O or S; R₁, R₂, R₃, R₄, R₅, X, Cy, R^{a}, R^{a'}, and R^{b} have the meaning as defined in Formula (I).

The following compounds are excluded from the compounds of Formula (I) and Formula (II):

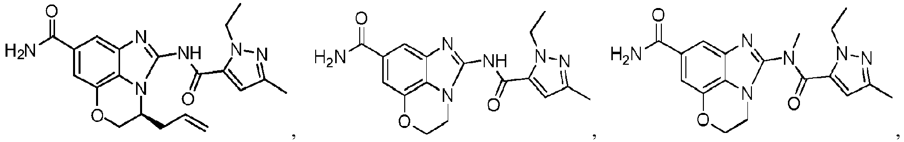

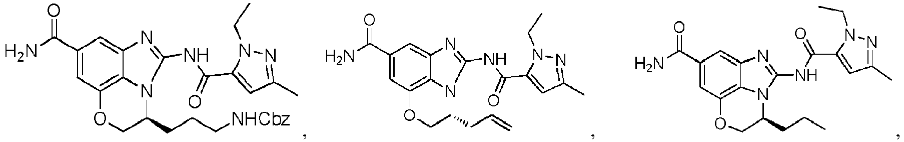

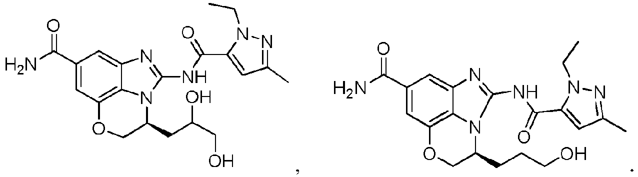

An object of the present invention is also to provide a compound having a structure of Formula (III), 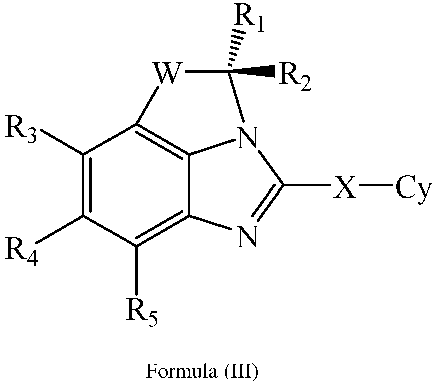 where R₁, R₃, R₄, R₅, W, X, and Cy have the meaning as defined in Formula (I); R₂ represents hydrogen or C₁-C₆ alkyl, and R₁ and R₂ represent different substituents.

An object of the present invention is to provide a compound having a structure of Formula (IV), 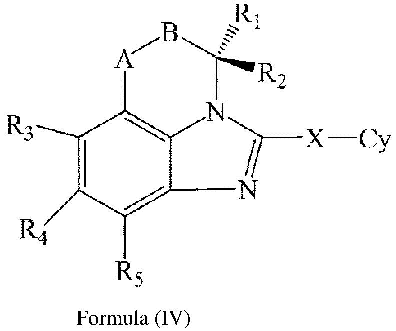 where R₁, R₃, R₄, R₅, X, Cy, A, and B have the meaning as defined in Formula (II), R₂ represents hydrogen or C₁-C₆ alkyl, and R₁ and R₂ represent different substituents.

In one embodiment, in the compounds of Formula (II) or Formula (IV) of the present invention, where A is preferably O, B is preferably CR^{a}R^{a'}.

In one embodiment, in the compounds of Formula (I) to (IV) of the present invention, where R₄ is preferably -CONR^{c}R^{c'}, and R^{c} and R^{c'} are independently preferably hydrogen or C₁-C₆ alkyl.

In one embodiment, in the compounds of Formulae (I) to (IV) of the present invention, where X is preferably -NR^{d}C(O)-, and R^{d} is preferably hydrogen or C₁-C₆ alkyl.

In one embodiment, in the compounds of Formulae (I) to (IV) of the present invention, where each Cy is independently selected from phenyl, pyridyl, pyrazolyl, pyrimidinyl, pyrazinyl, furanyl, thiazolyl, oxazolyl, imidazolyl, thienyl, triazolyl, and tetrazolyl; preferably pyrazolyl, imidazolyl, oxazolyl, triazolyl, and tetrazolyl; preferably imidazolyl; and Cy may each independently be substituted by 0, 1, 2, 3 or 4 substituents selected from the following groups consisting of: halogen, hydroxy, cyano, carboxy, C₁-C₆ alkyl, C₁-C₆ haloalkyl, sulfo, C₁-C₆ alkoxy, -amino, nitro, (C₁-C₆ alkyl) amino, and di(C₁-C₆ alkyl) amino.

In one embodiment, in the compounds of Formulae (I) to (IV) of the present invention, where R₁ is preferably C₁-C₆ alkyl, C₂-C₆ alkenyl, -(C₀-C₆ alkylene)-(C₃-C₆ cycloalkyl), -(C₀-C₆ alkylene)-(4- to 7-membered heterocycloalkyl), or -(C₀-C₆ alkylene)-(6- to 12-membered aryl); more preferably: C₁-C₆ alkyl, C₂-C₆ alkenyl, or -(C₀-C₆ alkylene)-(C₃-C₆ cycloalkyl); most preferably: C₁-C₆ alkyl, or C₂-C₆ alkenyl, and is optionally substituted by a substituent selected from: -NR^{g}COR^{g'}; and R^{g} is preferably hydrogen or C₁-C₆ alkyl; R^{g'} is preferably the following groups substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino: C₁-C₆ alkyl, -(C₀-C₆ alkylene)-(C₃-C₆ cycloalkyl), -(C₀-C₆ alkylene)-O-(C₁-C₆ alkyl), -(C₀-C₆ alkylene)-O-CO (C₁-C₆ alkyl), -(C₀-C₆ alkylene)-C ( O) O (C₁-C₆ alkyl), -(C₀-C₆ alkylene)-(6- to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆ alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl);
where the 6- to 12-membered aryl is preferably phenyl; the 5- to 12-membered heteroaryl is preferably pyridyl, imidazolyl, or pyrazolyl; or for the above-mentioned 6-to 12-membered aryl or 5-to 12-membered heteroaryl, when the number of substituents is 2, the adjacent two substituents may also be cyclized to each other into a 5-to 6-membered saturated or unsaturated carbocycle or heterocycle.

In another embodiment, in compounds of Formulae (I) to (IV) of the present invention, R¹ has the following structures: -(C₁-C₆ alkylene)-NR^{g}COR^{g'}, -(C₂-C₆ alkenylene)-NR^{g}COR^{g'}, where R^{g} is preferably hydrogen or C₁-C₆ alkyl; R^{g'} is preferably the following groups substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino: -(C₀-C₆ alkylene)-(6- to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆ alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl); where the 6- to 12-membered aryl is preferably phenyl; the 5- to 12-membered heteroaryl is preferably pyridyl; or for the above-mentioned 6-12-membered aryl or 5-12-membered heteroaryl, when the number of the substituents is 2, the two adjacent substituents may also be cyclized to each other into a 5-6-membered saturated or unsaturated carbocycle or heterocycle; more preferably -O-(C₁-C₆ alkylene)-phenyl, -O-(C₁-C₆ alkylene)-pyridyl, -(C₁-C₆ alkylene)-O-phenyl, -(C₁-C₆ alkylene)-O-pyridyl, -(C₁-C₆ alkylene)-phenyl, -(C₁-C₆ alkylene) -pyridinyl, -(C₂-C₆ alkenylene)-phenyl, or -(C₂-C₆ alkenylene)-pyridinyl, where the phenyl, and pyridinyl may be independently substituted by 0, 1, 2, 3 or 4 substituents selected from hydroxy, halogen, amino, sulfonyl, cyano, nitro, C₁-C₆ alkoxy, and C₁-C₆ haloalkyl.

In another embodiment, in the compounds of Formulae (I) to (IV) of the present invention, R² is preferably hydrogen or C₁-C₆ alkyl.

In another embodiment, in the compounds of Formulae (I) to (IV) of the present invention, R³ and R⁵ are each independently preferably hydrogen, halogen or C₁-C₆ alkyl.

In addition, the present invention also provides a compound having the Formula (V), 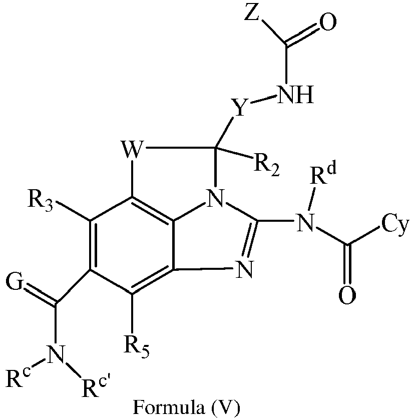
where W represents (CR^{a}R^{a'})ₘ, where any one CR^{a}R^{a} may be substituted by 0, 1 or 2 O, S or NRb;
R₂ independently represents hydrogen, halogen, hydroxy, amino, mercapto, C₁-C₆ alkyl, C₁-C₆ alkoxy, C₁-C₆ alkylthio, C₁-C₆ alkylamino, di(C₁-C₆ alkyl) amino, C₂-C₆ alkenyl, C₂-C₆ alkynyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), or -(C₀-C₆ alkylene)-(5- to 12-membered heteroaryl);
R₃ and R₅ are each independently selected from hydrogen, halogen, cyano, C₁-C₆ alkyl, C₂-C₆ alkenyl, C₂-C₆ alkynyl, -(C₀-C₆ alkylene)-(C₃-C₆ cycloalkyl), -OR^{c}, -NR^{c}R^{c'}, -OC(O)R^{c'}, -C(O)R^{c}, -CO₂R^{c}, -CON(R^{c})(R^{c'}), -C(=NR^{c})N(R^{c'})(R^{c"}), -NHC(O)R^{c}, -NHS(O)₂R^{c}-, -NHS(O)R^{c}-, -SO₂R^{c}, -SO₂NR^{c}R^{c'}, -(C₀-C₆ alkylene)-(4- to 7-membered heterocycloalkyl), -(C₀-C₆ alkylene)-(6- to 12-membered aryl), and -(C₀-C₆ alkylene)-(5- to 12-membered heteroaryl);
Cy represents 6- to 12-membered aryl, or 5- to 12-membered heteroaryl;
m represents an integer of 1, 2 or 3;
R^{a} and R^{a'} each independently represent hydrogen, halogen, hydroxy, C₁-C₆ alkyl, C₁-C₆ alkylthio, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), -(C₀-C₆ alkylene)-(5- to 12-membered heteroaryl), -NR^{e}R^{e'}, -NR^{e}COR^{e'}, -NR^{e}SO₂R^{e'}, -OR^{e} or -OCOR^{e}, or R^{a} and R^{a'} together with the atoms adjacent thereto, are cyclized into a 3- to 6-membered ring optionally containing 0, 1, or 2 heteroatoms selected from O, N and S; or any one CR^{a}R^{a'} may be taken together to form -C=O;
R^{b} each independently represents hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene) -(6- to 12-membered aryl), -(C₀-C₆ alkylene)-(5- to 12-membered heteroaryl), -C(O)R^{f}, -SO₂R^{f}, -SOR^{f}, -C(O)OR^{f} or -C(O)NR^{f}R^{f'};
G represents O or NR^{c};
R^{c}, R^{c'}, R^{c"}, R^{d}, R^{e}, R^{e'}, R^{f}, and R^{f'} each independently represent hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), or -(C₀-C₆ alkylene)-(5-to 12-membered heteroaryl), or when the above-mentioned substituents are collectively bound to a single N atom, they are able to be cyclized with the bound N atom to form a 3- to 8-membered ring;
the above-mentioned alkyl, alkylene, aryl, heteroaryl, ring, cycloalkyl, heterocycloalkyl, alkenyl, alkynyl, and alkoxy are optionally independently substituted by 0, 1, 2, 3, or 4 substituents selected from the following groups consisting of: halogen, oxo, hydroxy, cyano, carboxy, C₁-C₆ alkyl, C₁-C₆ haloalkyl, sulfo, C₁-C₆ alkoxy, -OR^{g}, -SR^{g}, -N(R^{g})(R^{g'}), -NR^{g}COR^{g'}, -NR^{g}COOR^{g'}, -COR^{g}, -CO₂R^{g}, -SOR^{g}, -SO₂R^{g}, -OCONR^{g}R^{g'}-, -OCOR^{g}, -CONR^{g}R^{g'}, -NR^{g}SO₂R^{g'}, -SO₂NR^{g}R^{g'}, and -OP(O)(OR^{g}R^{g'})₂;
or for the aryl and heteroaryl, or when the number of substituents is 2, the adjacent two substituents are also optionally cyclized to each other into a 5- to 6-membered saturated or unsaturated carbocycle or heterocycle, the heterocycle being a ring containing 0, 1, 2, 3 or 4 heteroatoms selected from O, S and N;
where R^{g} and R^{g'} each independently represent hydrogen, or the following groups optionally substituted by 0, 1, 2, 3 or 4 groups selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, di(C₁-C₆ alkyl) amino: C₁-C₆ alkyl, -(C₀-C₆ alkylene)-(C₃-C₆ cycloalkyl), halo (C₁-C₆ alkyl), -(C₀-C₆ alkyl)-OH, -(C₀-C₆ alkylene)-O-(C₁-C₆ alkyl), -C₀-C₆ alkylene)-O-CO (C₁-C₆ alkyl), -(C₀-C₆ alkylene)-C(O)O (C₁-C₆ alkyl), -(C₀-C₆ alkylene)-(6- to 12-membered aryl), -C₂-C₆ alkenylene-( 6- to 12-membered aryl), -(C₀-C₆ alkylene)-O-(6- to 12-membered aryl), -(C₀-C₆ alkylene)-O-C₁-C₆ alkyl, -O-(C₁-C₆ alkylene)-(6- to 12-membered aryl), -(C₀-C₆ alkylene)-(5- to 12-membered heteroaryl), -C₂-C₆ alkenylene-(5- to 12-membered heteroaryl), -(C₀-C₆ alkylene)-O-(5- to 12-membered heteroaryl), or -O-(C₀-C₆ alkylene)-(5- to 12-membered heteroaryl); or for the above-mentioned 6-to 12-membered aryl or 5-to 12-membered heteroaryl, when the number of substituents is 2, the adjacent two substituents are also cyclized to each other into a 5-to 6-membered saturated or unsaturated carbocycle or heterocycle;
Y represents the following groups optionally substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino: -C₁-C₆ alkylene-, -(C₀-C₆ alkylene)-(C₃-C₆ cycloalkyl)-(C₀-C₆ alkylene), -(C₀-C₆ alkylene)-(4- to 7-membered heterocycloalkyl)-(C₀-C₆ alkylene), (C₀-C₆ alkylene)-(6- to 12-membered aryl)-(C₀-C₆ alkylene), -(C₀-C₆ alkylene)-(5- to 12-membered heteroaryl)-(C₀-C₆ alkylene), or -C₂-C₆ alkenylene-;
Z represents the following groups optionally substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino: C₁-C₆ alkyl, -(C₀-C₆ alkylene)-(C₃-C₆ cycloalkyl), -(C₀-C₆ alkylene)-O-(C₁-C₆ alkyl), -(C₀-C₆ alkylene)-O-CO(C₁-C₆ alkyl), -(C₀-C₆ alkylene)-C(O)O(C₁-C₆ alkyl), -(C₀-C₆ alkylene)-(6- to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆ alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl); or for the above-mentioned 6-to 12-membered aryl or 5-to 12-membered heteroaryl, when the number of substituents is 2, the adjacent two substituents are also cyclized to each other into a 5-to 6-membered saturated or unsaturated carbocycle or heterocycle;
where the 6- to 12-membered aryl is preferably phenyl; the 5- to 12-membered heteroaryl is preferably pyridyl, imidazolyl, or pyrazolyl; or for the above-mentioned 6- to 12-membered aryl or 5- to 12-membered heteroaryl, when the number of substituents is 2, the adjacent two substituents may also be cyclized to each other into a 5- to 6-membered saturated or unsaturated carbocycle or heterocycle.

In addition, the present invention also provides a compound having Formula (VI), 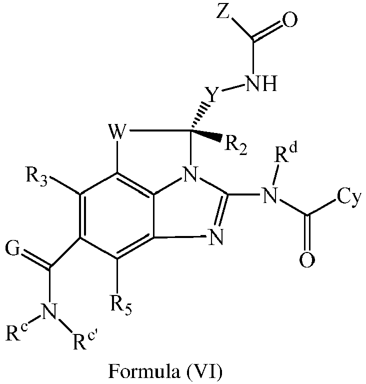 where R₂ is selected from hydrogen or C₁-C₆ alkyl, and W, R₃, R₅, R^{c}, R^{c'}, R^{d}, G, Z, Y, and Cy are as defined for Formula (V).

In the compounds of Formula (V) or (VI) of the present invention, G is preferably O or NH.

In the compounds of Formula (V) or (VI) of the present invention, where Y is preferably the following groups substituted by 0, 1, 2, 3 or 4 substituents selected from hydroxy, halogen and C₁-C₆ alkyl: -C₁-C₆ alkylene-, -C₂-C₆ alkenylene-, or -C₃-C₆ cycloalkyl-.

In the compounds of Formula (V) or (VI) of the present invention, where W is preferably -CR^{a}R^{a'}-O, -O- CR^{a}R^{a'}-, -C(O)-NR^{b}, or -NR^{b}-C(O)-, where R^{a}, R^{a'}, and R^{b} each independently represent hydrogen, C₁-C₆ alkyl, or C₃-C₆ cycloalkyl.

In the compound of Formula (V) or (VI) of the present invention, Z is preferably -O-(C₀-C₆ alkylene)-(6- to 12-membered aryl), -(C₀-C₆ alkylene)-(6- to 12-membered aryl), -(C₂-C₆ alkenylene) -(6- to 12-membered aryl), -(C₀-C₆ alkylene)-(5- to 12-membered heteroaryl), -(C₂-C₆ alkenylene)-(5- to 12-membered heteroaryl), -O-(C₂-C₆ alkenylene)-(6- to 12-membered aryl ), -(C₀-C₆ alkylene)-O-(6- to 12-membered aryl), -O-(C₀-C₆ alkylene)-(5- to 12-membered heteroaryl), -O-(C₂-C₆ alkenylene)-(5- to 12-membered heteroaryl), or - (C₀-C₆ alkylene)-O-(5-to 12-membered heteroaryl), and optionally the 6- to 12-membered aryl (preferably phenyl) or 5-to 12-membered heteroaryl (preferably pyridyl) is each independently substituted by 0, 1, 2, 3 or 4 substituents selected from the following groups consisting of: halo, hydroxy, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino.

In the compounds of Formula (V) or (VI) of the present invention, R² is preferably hydrogen or C₁-C₆ alkyl.

In the compound of Formula (V) or (VI) of the present invention, R³ and R⁵ are each independently preferably halogen, hydrogen, or C₁-C₆ alkyl.

In the compounds of Formula (V) or (VI) of the present invention, where R^{c} and R^{c'} are preferably hydrogen or C₁-C₆ alkyl.

In the compounds of Formula (V) or (VI) of the present invention, R^{d} is preferably hydrogen or C₁-C₆ alkyl.

In the compounds of Formula (V) or (VI) of the present invention, Cy is preferably pyrazolyl and may be optionally substituted by 0, 1, 2 or 3 C₁-C₆ alkyl groups.

More specifically, the present invention also provides a compound having the following structures,

| No. | Compound structure | No. | Compound structure |
|---|---|---|---|
| 1 | 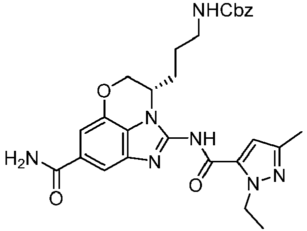 | 2 | |
| 3 | 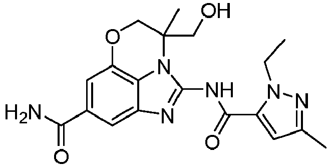 | 4 | 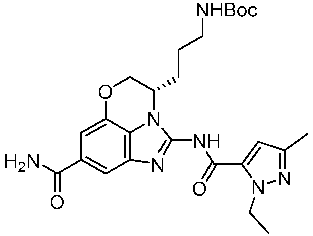 |
| 5 | 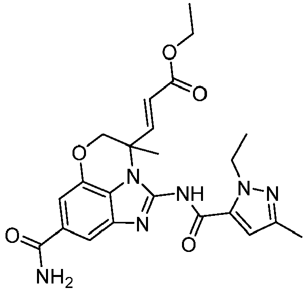 | 6 | 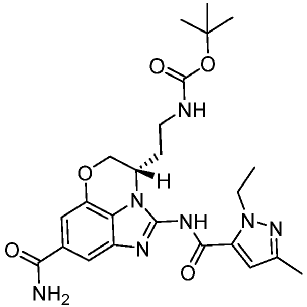 |
| 7 | 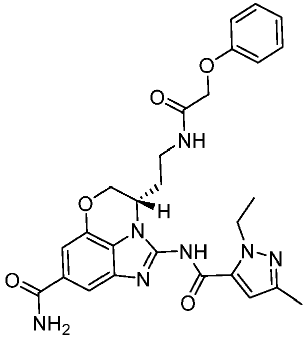 | 8 | 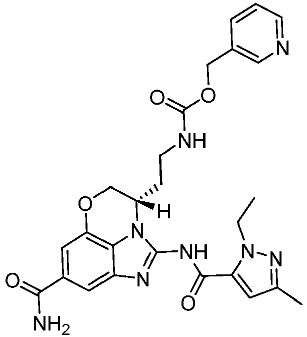 |
| 9 | 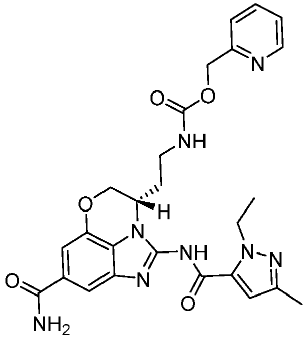 | 10 | 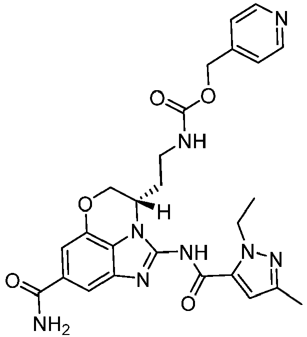 |
| 11 | 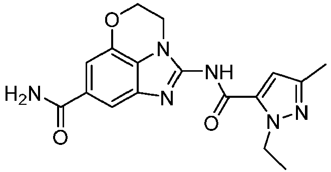 | 12 | 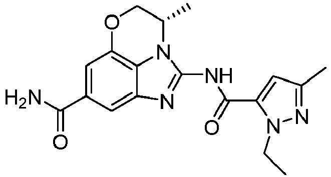 |
| 13 | 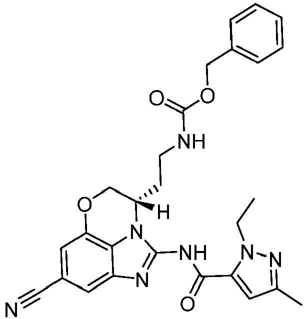 | 14 | 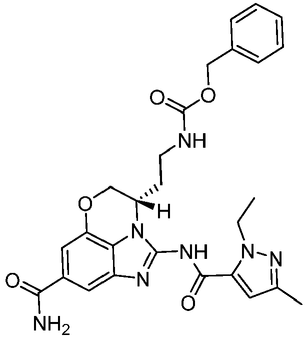 |
| 15 | 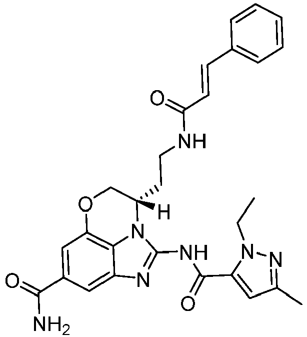 | 16 | 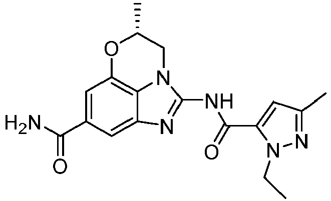 |
| 17 | 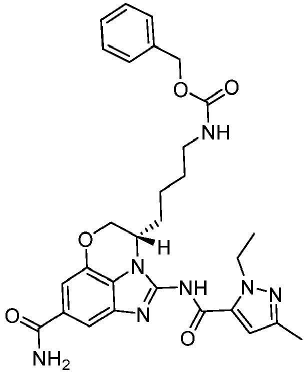 | 18 | 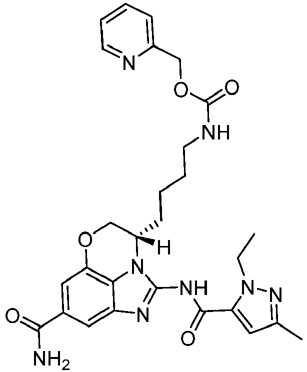 |
| 19 | 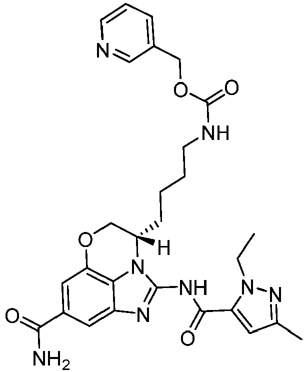 | 20 | 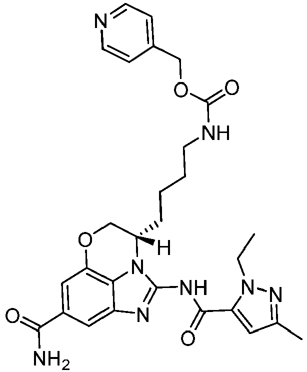 |
| 21 | 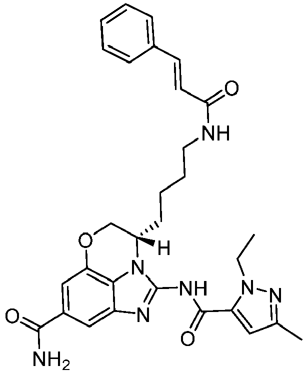 | 22 | 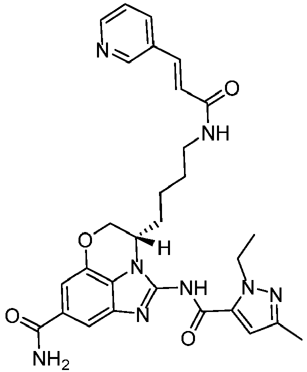 |
| 23 | 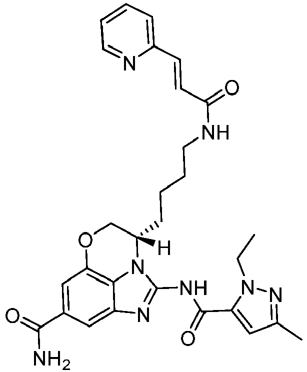 | 24 | 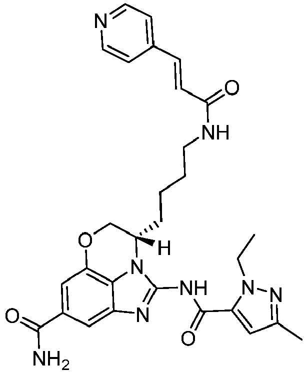 |
| 25 | 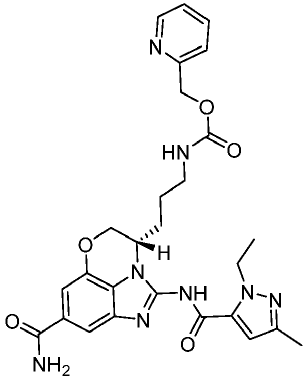 | 26 | 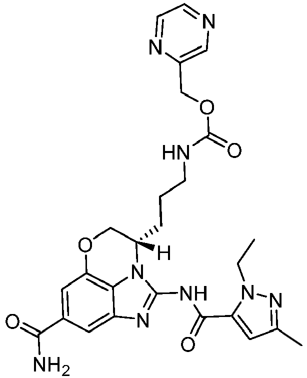 |
| 27 | 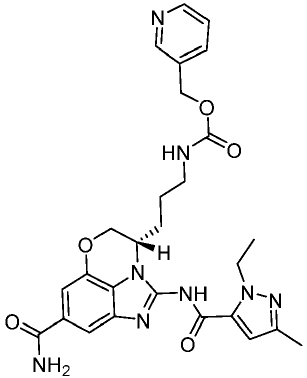 | 28 | 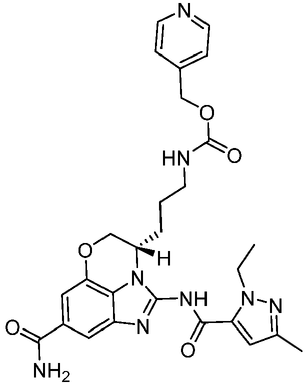 |
| 29 | 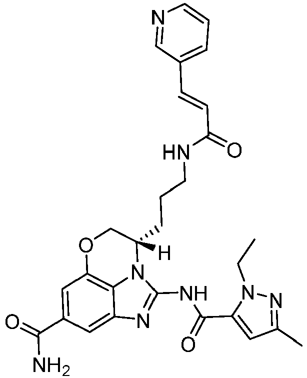 | 30 | 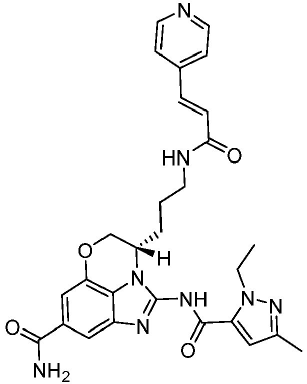 |
| 31 | 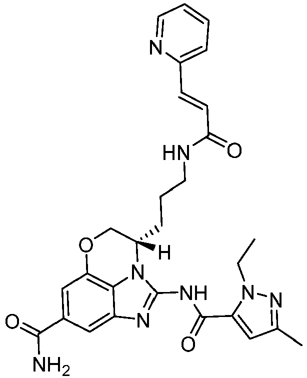 | 32 | 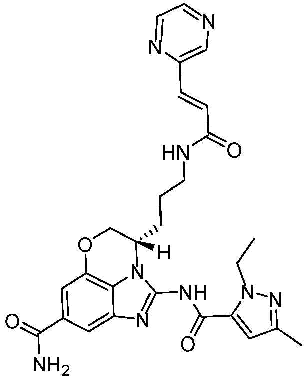 |
| 33 | 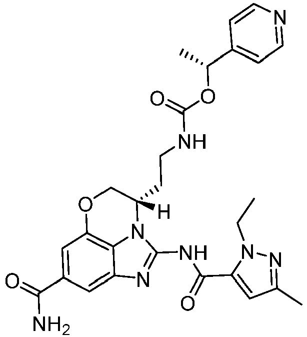 | 34 | 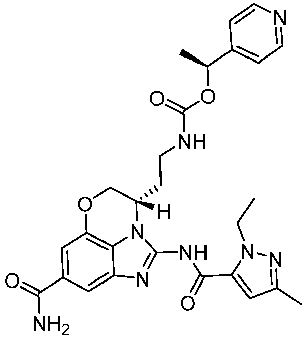 |
| 35 | 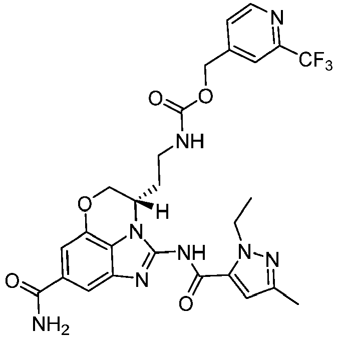 | 36 | 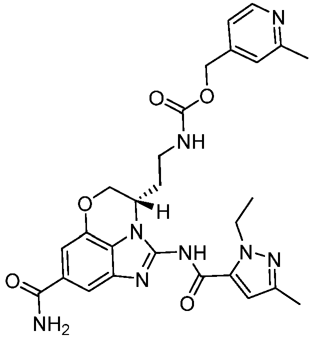 |
| 37 | 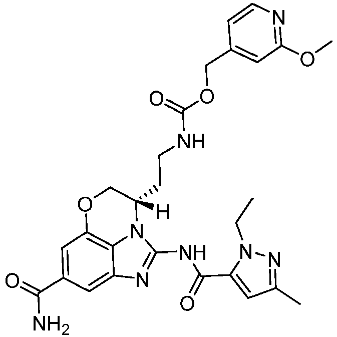 | 38 | 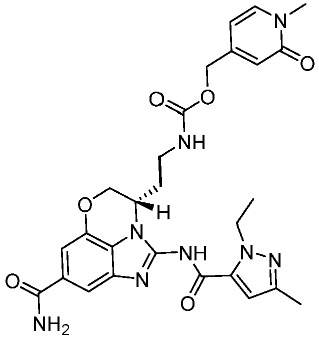 |
| 39 | 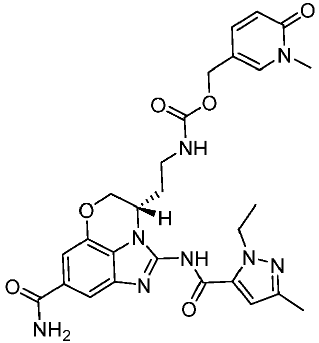 | 40 | 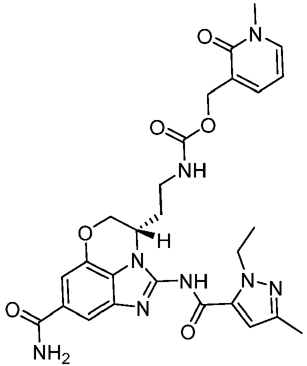 |
| 41 | 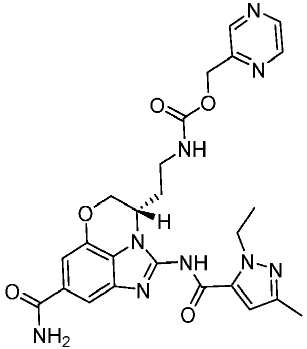 | 42 | 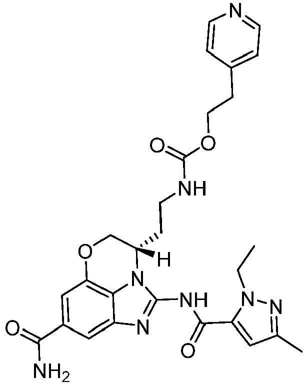 |
| 43 | 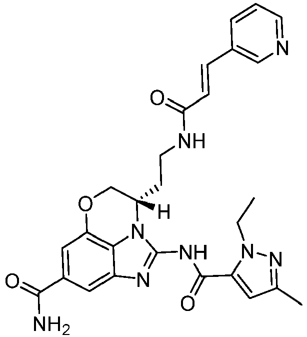 | 44 | 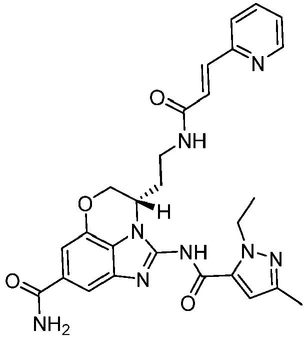 |
| 45 | 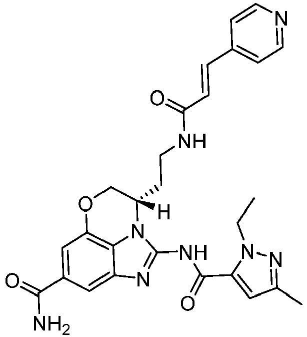 | 46 | 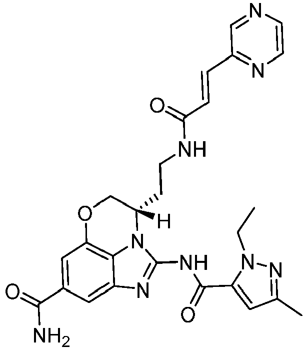 |
| 47 | 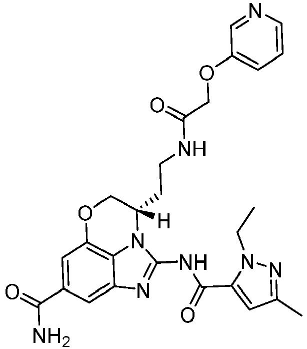 | 48 | 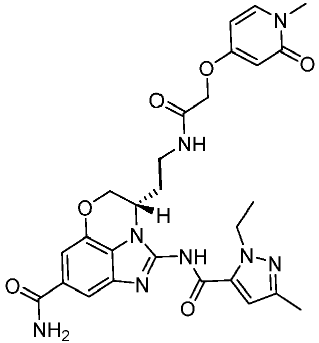 |
| 49 | 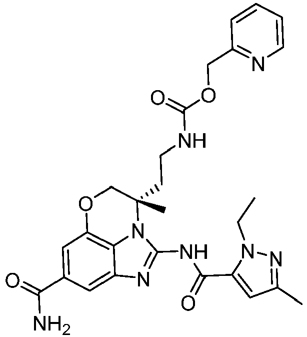 | 50 | 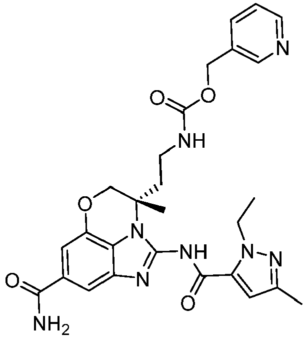 |
| 51 | 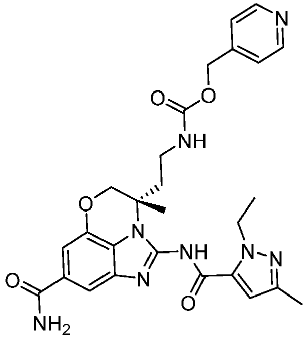 | 52 | 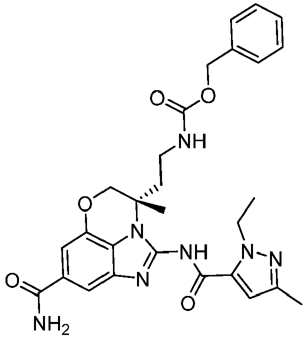 |
| 53 | 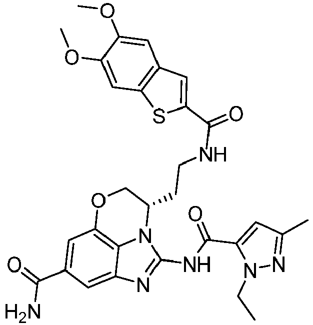 | 54 | 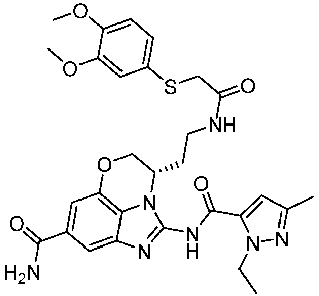 |
| 55 | 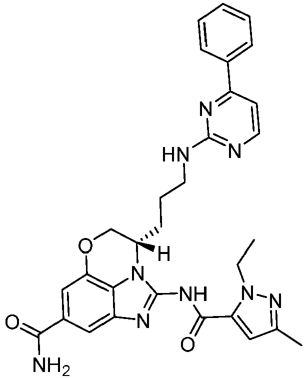 | 56 | 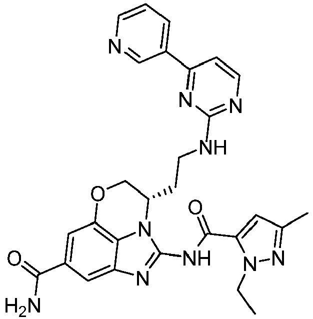 |
| 57 | 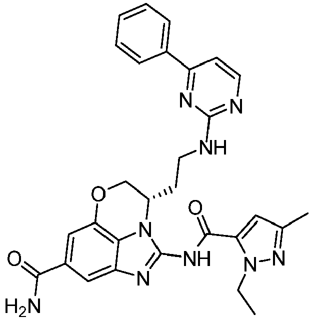 | 58 | 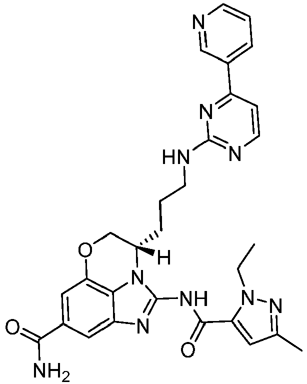 |
| 59 | 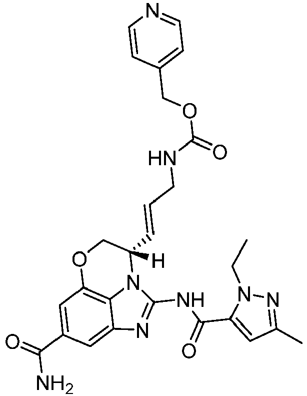 | 60 | 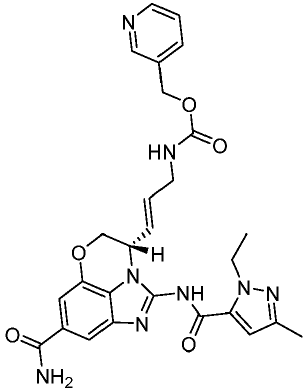 |
| 61 | 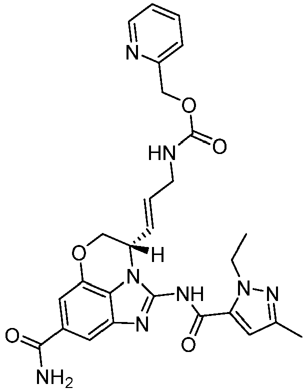 | 62 | 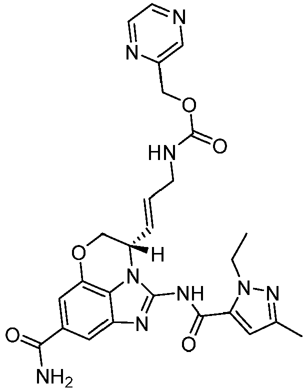 |
| 63 | 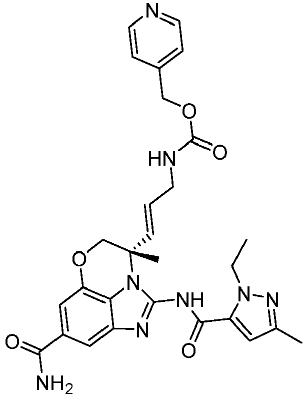 | 64 | 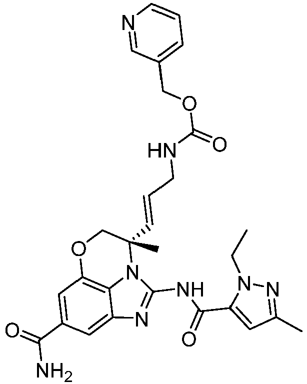 |
| 65 | 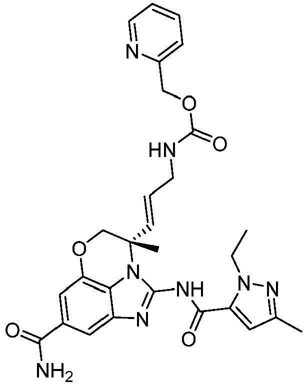 | 66 | 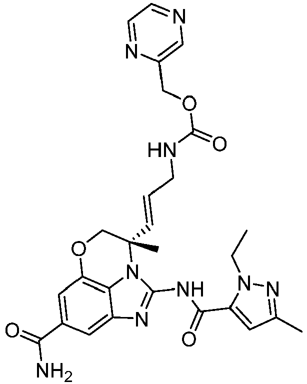 |
| 67 | 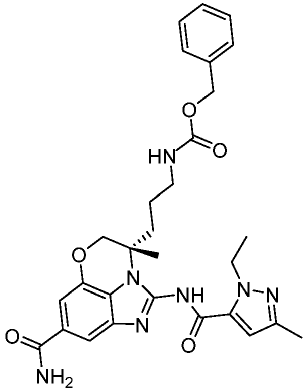 | 68 | 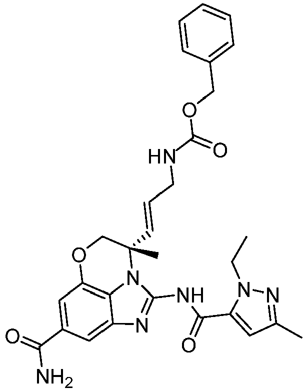 |
| 69 | 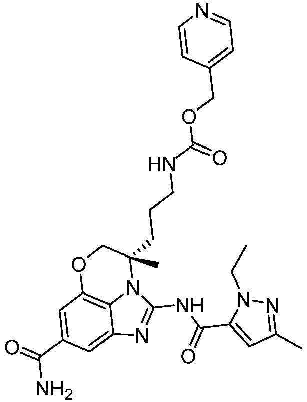 | 70 | 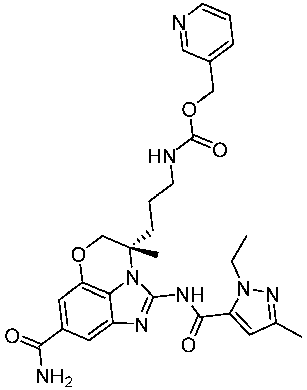 |
| 71 | 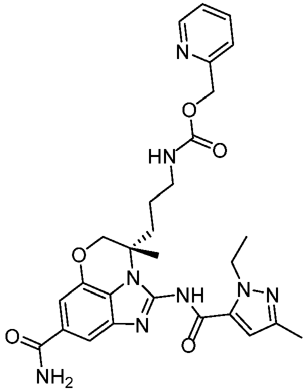 | 72 | 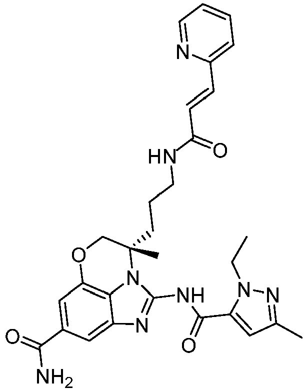 |
| 73 | 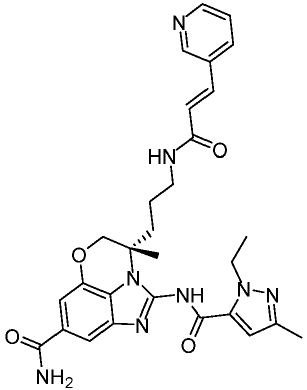 | 74 | 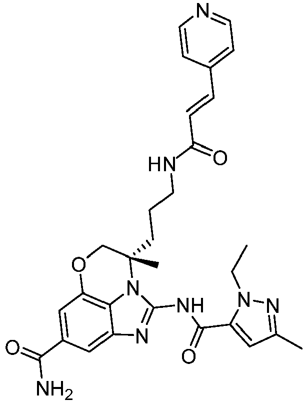 |
| 75 | 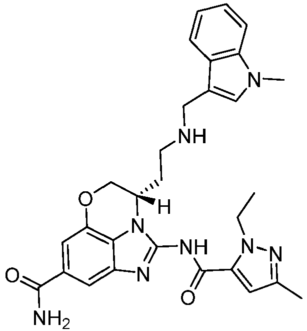 | 76 | 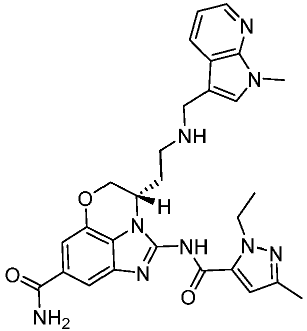 |
| 77 | 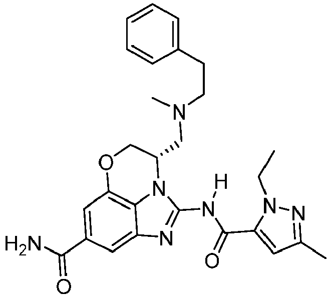 | 78 | 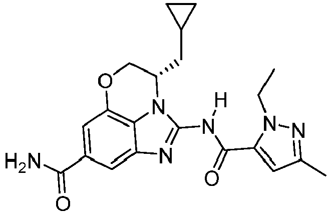 |
| 79 | 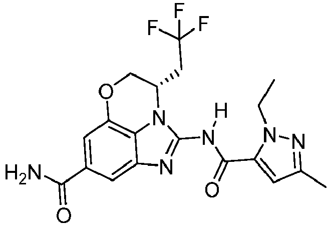 | 80 | 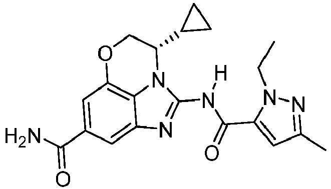 |
| 81 | 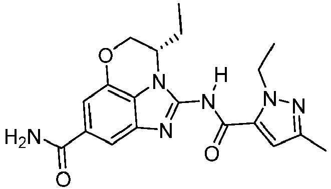 | 82 | 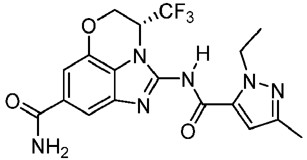 |
| 83 | 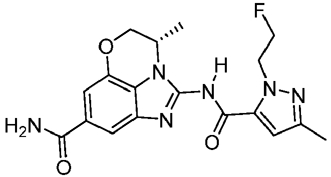 | 84 | 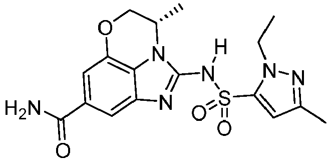 |
| 85 | 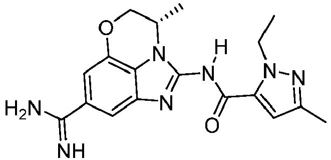 | 86 | 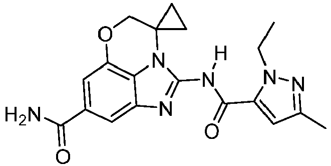 |
| 87 | 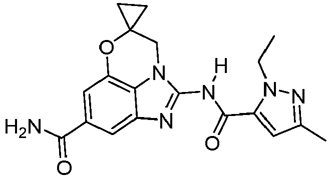 | 88 | 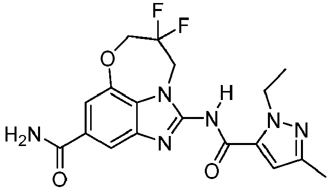 |
| 89 | 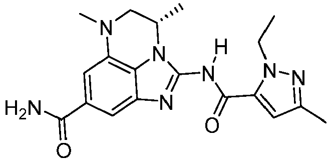 | 90 | 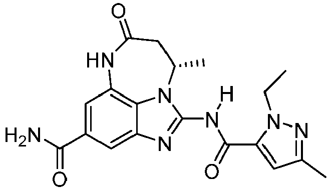 |
| 91 | 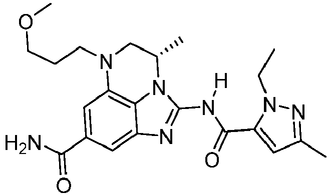 | 92 | 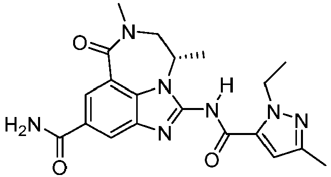 |
| 93 | 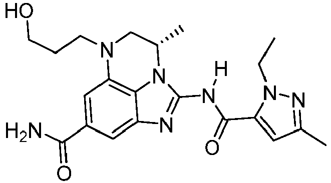 | 94 | 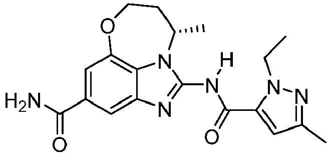 |
| 95 | 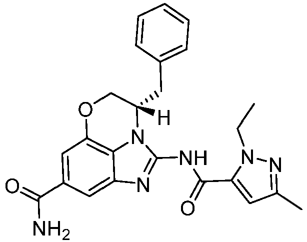 | 96 | 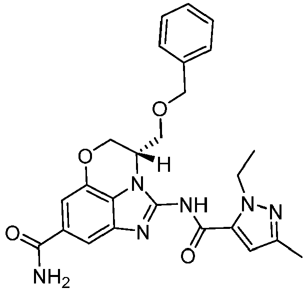 |
| 97 | 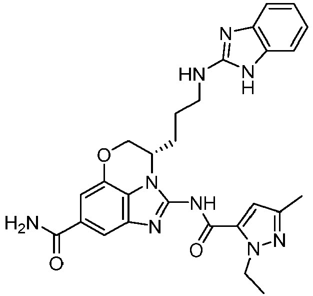 | 98 | 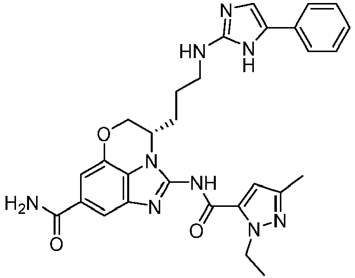 |
| 99 | 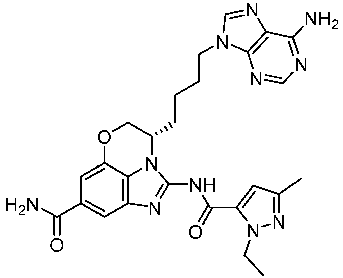 | 100 | 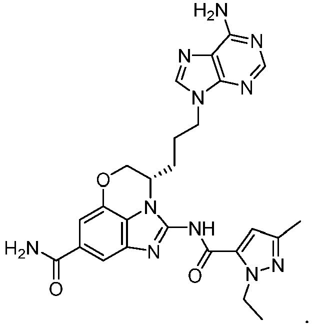 |
| 101 | 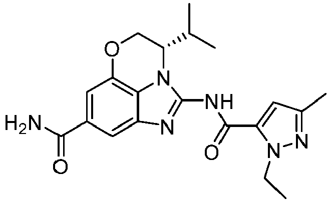 | 102 | 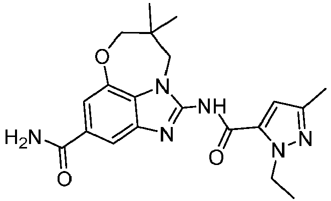 |
| 103 | 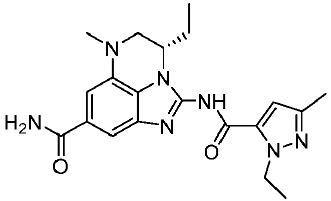 | 104 | 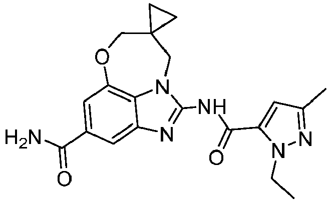 |
| 105 | 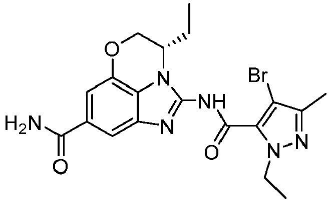 | 106 | 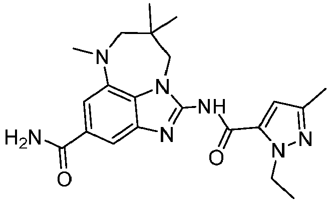 |
| 107 | 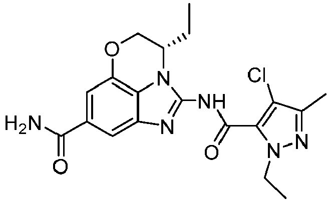 | 108 | 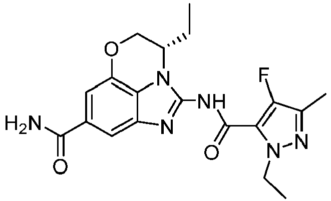 |
| 109 | 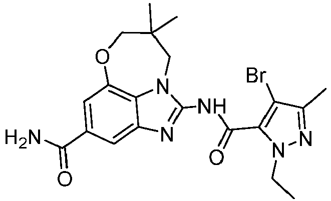 | 110 | 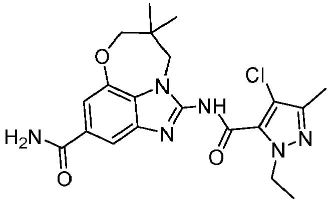 |
| 111 | 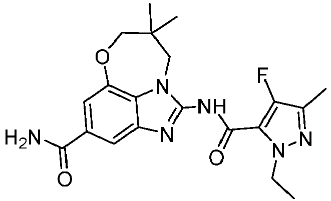 | 112 | 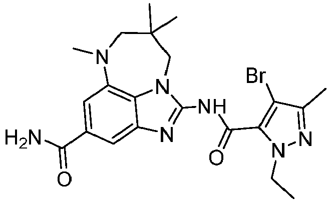 |
| 113 | 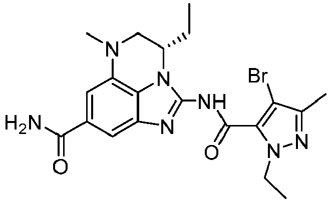 | 114 | 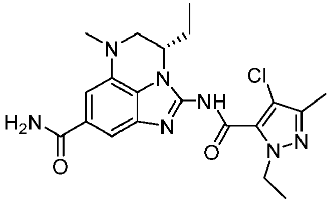 |
| 115 | 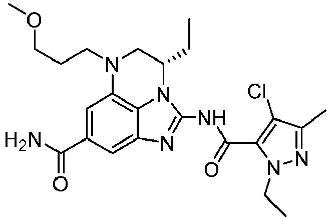 | 116 | 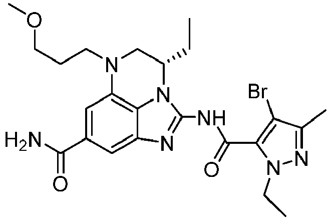 |
| 117 | 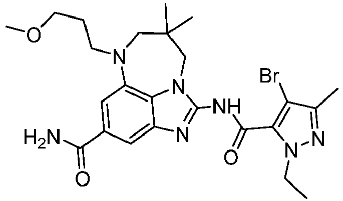 | 118 | 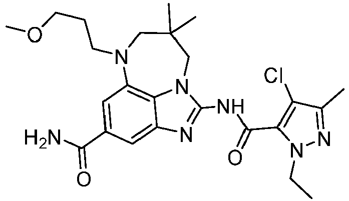 |
| 119 | 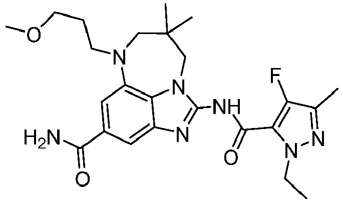 | 120 | 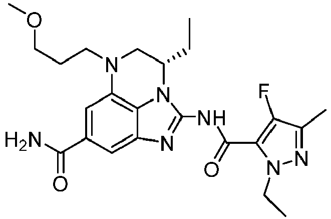 |
| 121 | 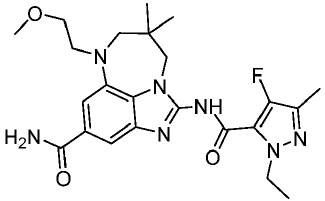 | 122 | 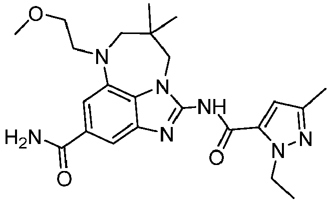 |
| 123 | 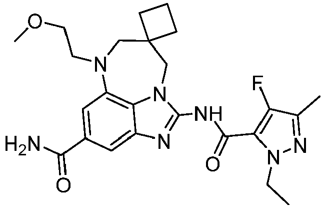 | 124 | 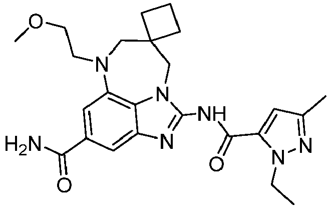 |
| 125 | 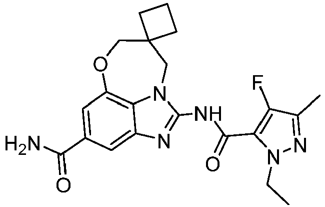 | 126 | 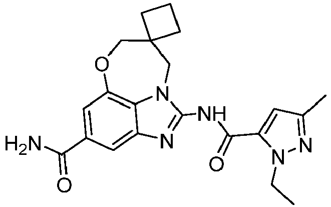 |
| 127 | 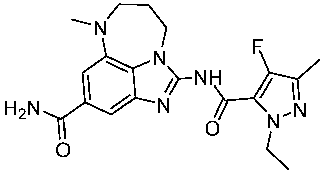 | 128 | 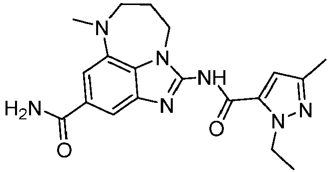 |
| 129 | 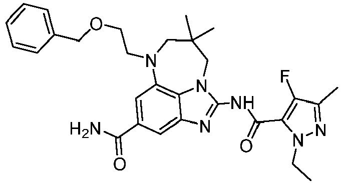 | 130 | 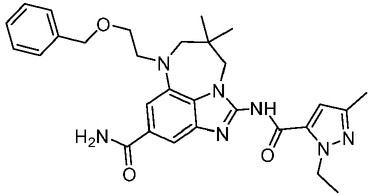 |
| 131 | 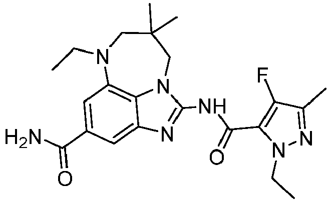 | 132 | 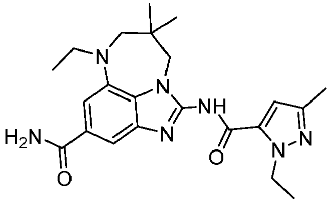 |
| 133 | 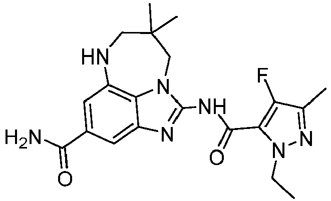 | 134 | 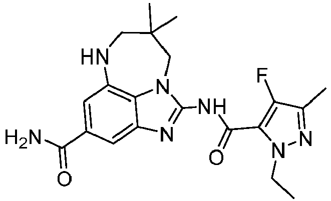 |
| 135 | 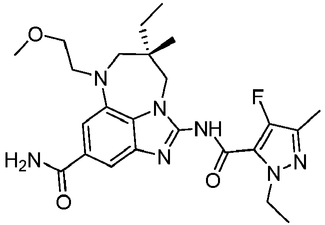 | 136 | 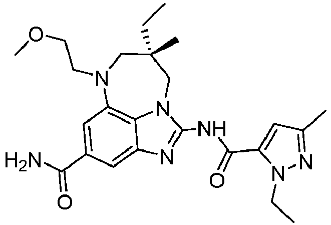 |
| 137 | 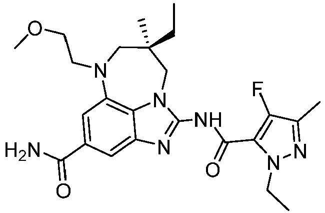 | 138 | 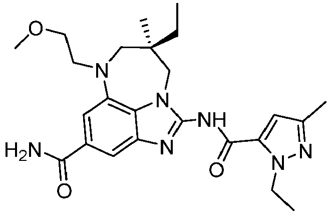 |
| 139 | 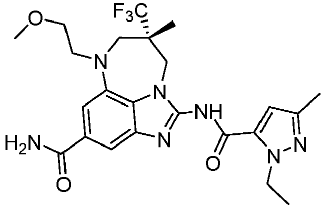 | 140 | 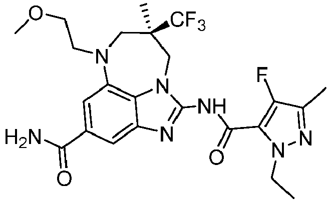 |
| 141 | 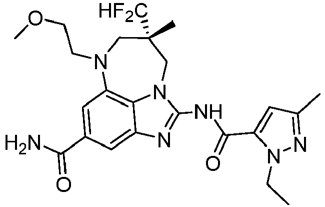 | 142 | 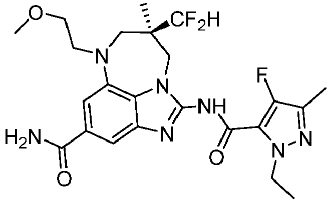 |
| 143 | 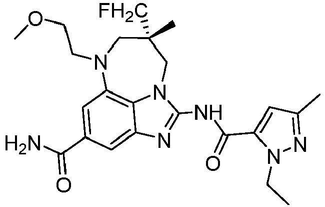 | 144 | 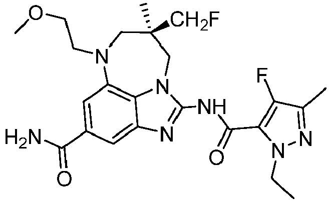 |
| 145 | 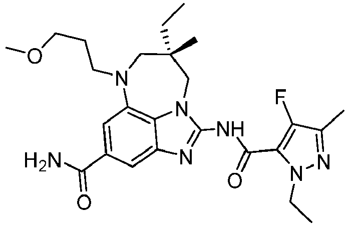 | 146 | 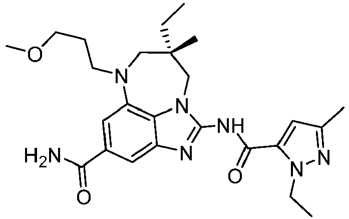 |
| 147 | 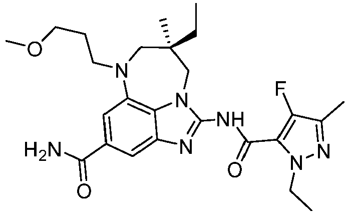 | 148 | 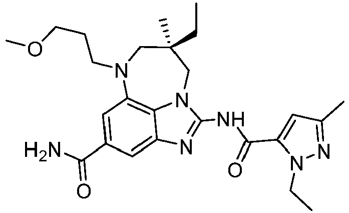 |
| 149 | 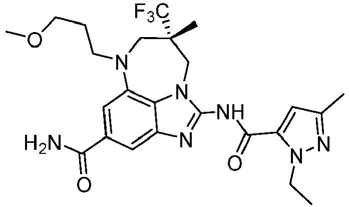 | 150 | 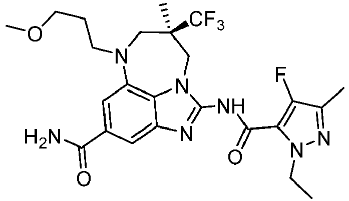 |
| 151 | 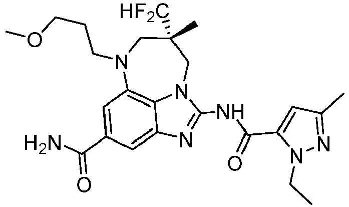 | 152 | 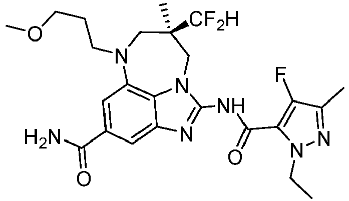 |
| 153 | 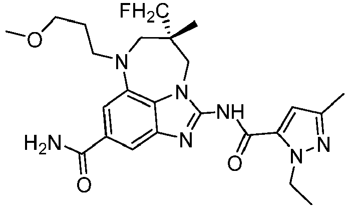 | 154 | 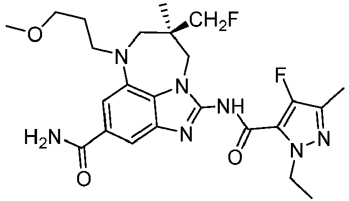 |
| 155 | 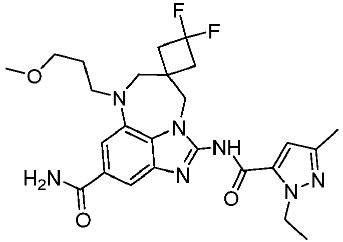 | 156 | 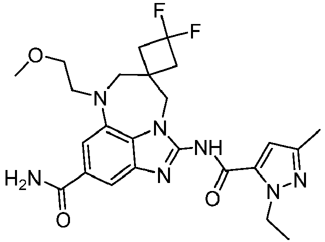 |
| 157 | 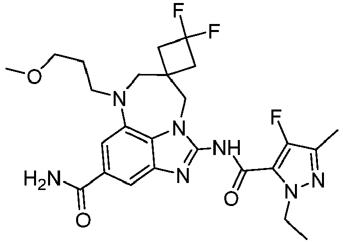 | 158 | 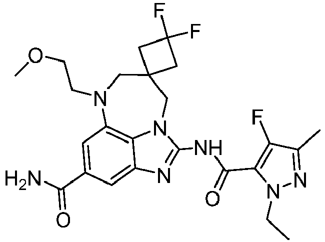 |

The present invention also provides the compound of the present invention for use in the prevention and/or treatment of cancer, tumors, viral infections, organ transplant rejection, neurodegenerative diseases, attention-related diseases, or autoimmune diseases.

In addition, the present invention provides a pharmaceutical composition for use in the prevention and/or treatment of cancer, tumors, viral infections, organ transplant rejection, neurodegenerative diseases, attention-related diseases, or autoimmune diseases.

The cancers or tumors is selected from the group consisting of skin cancer, bladder cancer, ovarian cancer, breast cancer, gastric cancer, pancreatic cancer, prostate cancer, colon cancer, lung cancer, bone cancer, brain cancer, neurocytoma, rectal cancer, colon cancer, familial adenomatous polyposis cancer, hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, renal cancer, carcinoma of renal parenchyma, ovarian cancer, cervical cancer, corpus carcinoma, endometrial cancer, choriocarcinoma, pancreatic cancer, prostate cancer, testicular cancer, carcinoma of urinary system, melanoma, brain tumors such as glioblastoma, astrocytoma, meningioma, medulloblastoma and peripheral neuroectodermal tumors, Hodgkin's lymphoma, non-Hodgkin's lymphoma, Burkitt's lymphoma, acute lymphoblastic leukemia (ALL), chronic lymphocytic leukemia (CLL), acute myelogenous leukemia (AML), chronic myelogenous leukemia (CML), adult T-cell leukemia lymphoma, diffuse large B-cell lymphoma (DLBCL), hepatocellular carcinoma, gallbladder carcinoma, bronchial carcinoma, small cell lung carcinoma, non-small cell lung carcinoma, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma, and plasmacytoma.

When a compound of the present invention or a pharmaceutically acceptable salt thereof is administered in combination with another anticancer agent or immune checkpoint inhibitor for the treatment of cancer or tumors, the compound of the present invention or a pharmaceutically acceptable salt thereof may provide an enhanced anticancer effect.

Representative examples of anti-cancer agents for treating a cancer or tumor may include cell signal transduction inhibitors, Chlorambucil, Melphalan, Cyclophosphamide, Ifosfamide, Busulfan, Carmustine, Lomustine, Streptozotocin, Cisplatin, Carboplatin, Oxaliplatin, Dacarbazine, Temozolomide, Procarbazine, Methotrexate, Fluorouracil, Cytarabine, Gemcitabine, Mercaptopurine, Fludarabine, Vinblastine, Vincristine, Vinorelbine, Paclitaxel, Docetaxel, Topotecan, Irinotecan, Etoposide, Trabectedin, Dactinomycin, Doxorubicin, Epirubicin, Daunorubicin, Mitoxantrone, Bleomycin, Mitomycin C, Ixabepilone, Tamoxifen, Flutamide, Gonadorelin Analogs, Megestrol, Prednisone, Dexamethasone, Methylprednisolone, Thalidomide, Interferon A, Calcium Folinate, Sirolimus, Sirolimus Lipide, Everolimus, Afatinib, Alisertib, Amuvatinib, Apatinib, Axitinib, Bortezomib, Bosutinib, Brivanib, Cabozantinib, Cediranib, Crenolanib, Crizotinib, Dabrafenib, Dacomitinib, Danusertib, Dasatinib, Dovitinib, Erlotinib, Foretinib, Ganetespib, Gefitinib, Ibrutinib, Icotinib, Imatinib, Iniparib, Lapatinib, Lenvatinib, Linifanib, Linsitinib, Masitinib, Momelotinib, Motesanib, Neratinib Nilotinib, Niraparib, Oprozomib, Olaparib, Pazopanib, Pictiliisib, Ponatinib, Quizartinib, Regorafenib, Rigosertib, Rucaparib, Ruxolitinib, Saracatinib, Saridegib, Sorafenib, Sunitinib, Telatinib, Tivantinib, Tivozanib, Tofacitinib, Trametinib, Vandetanib, Veliparib, Vemurafenib, Erivedge, Volasertib, Alemtuzumab, Bevacizumab, Brentuximab Vedotin, Catumaxomab, Cetuximab, Denosumab, Gemtuzumab, Ipilimumab, Nimotuzumab, Ofatumumab, Panitumumab, Rituximab, Tositumomab, Trastuzumab, PI3K inhibitors, CSF1R inhibitors, A2A and/or A2B receptor antagonists, IDO inhibitors, anti-PD-1 antibodies, anti-PD-L1 antibodies, LAG3 antibodies, TIM-3 antibodies, and anti-CTLA-4 antibodies, or any combination thereof.

When a compound of the present invention or a pharmaceutically acceptable salt thereof is administered in combination with another therapeutic agent for the treatment of inflammatory diseases, autoimmune diseases and immune-mediated diseases, the compound of the present invention or a pharmaceutically acceptable salt thereof may provide an enhanced therapeutic effect.

Representative examples of therapeutic agents for the treatment of inflammatory diseases, autoimmune diseases, and immune-mediated diseases may include steroidal drugs (e.g., prednisone, prednisolone, methylprednisolone, cortisone, hydroxycortisone, betamethasone, dexamethasone, etc.), methotrexate, leflunomide, anti-TNF α agents (e.g., etanercept, infliximab, adalimumab, etc.), calcineurin inhibitors (e.g., tacrolimus, pimecrolimus, etc.), and antihistamines (e.g., diphenhydramine, hydroxyzine, loratadine, ebastine, ketotifen, cetirizine, levocetirizine, fexofenadine, etc.), and at least one therapeutic agent selected therefrom may be included in the pharmaceutical compositions of the present invention.

The compound of the present invention or a pharmaceutically acceptable salt thereof can be administered orally or parenterally as an active ingredient in an effective amount ranging from 0.1 mg/kg body weight/day to 2,000 mg/kg body weight/day, preferably 1 mg/kg body weight/day to 1,000 mg/kg body weight/day in the case of mammals including humans (body weight about 70 kg), and administered in a single or four divided doses per day, or following/not following a predetermined time. The dosage of the active ingredient may be adjusted according to a number of relevant factors, such as the condition of the subject to be treated, the type and severity of the disease, the rate of administration and the opinion of the physician). In some cases, amounts less than the above doses may be suitable. If it does not cause harmful side effects, an amount larger than the above dose can be used and the amount can be administered in divided doses per day.

The pharmaceutical compositions of the present invention may be formulated into dosage forms, such as tablets, granules, powders, capsules, syrups, emulsions, microemulsions, solutions or suspensions, for oral or parenteral administration (including intramuscular, intravenous and subcutaneous routes, and intratumoral injection) according to any of the conventional methods.

The pharmaceutical compositions of the present invention for oral administration may be prepared by mixing the active ingredient with carriers such as: cellulose, calcium silicate, corn starch, lactose, sucrose, dextrose, calcium phosphate, stearic acid, magnesium stearate, calcium stearate, gelatin, talc, surfactants, suspending agents, emulsifying agents, and diluents. Examples of carriers employed in the injectable compositions of the present invention consist of water, saline solutions, dextrose solutions, glucose-like solutions, alcohols, glycols, ethers (e.g., polyethylene glycol 400), oils, fatty acids, fatty acid esters, glycerides, surfactants, suspending agents, and emulsifying agents.

The compounds of the present invention may be prepared in a variety of ways known to those skilled in the art of organic synthesis, and may be synthesized using the methods described below, as well as synthetic methods known in the art of organic synthetic chemistry, or by variations thereof known to those skilled in the art. Preferred methods include those described below. The reaction is carried out in a solvent or solvent mixture suitable for the kit materials used and for the transformations achieved. Those skilled in the art of organic synthesis will appreciate that the functionality present on the molecule is consistent with the proposed transformations. This sometimes requires judgment to modify the order of the synthetic steps or starting materials in order to obtain the desired compounds of the present invention.

### DETAILED DESCRIPTION

### Terms

Terms used in the present application, including the specification and claims, are defined as follows, unless otherwise indicated. It must be noted that, in the description and the appended claims, the singular forms "a", "an", and "the" include plural referents unless the context clearly dictates otherwise. If not stated otherwise, conventional methods of mass spectrometry, nuclear magnetic, HPLC, protein chemistry, biochemistry, recombinant DNA techniques and pharmacology are used. In this application, the use of "or" or "and" means "and/or" if not stated otherwise.

Throughout the specification and claims, a given chemical formula or name shall encompass all stereo and optical isomers and racemates in which such isomers exist. Cis- and trans-(or E- and Z-) geometric isomers of the compounds of the present invention are described herein and may be isolated as mixtures of isomers or as separated isomeric forms. The compounds of the present invention may be isolated in optically active or racemic forms. In preparing enantiomeric or diastereomeric products, they can be isolated by conventional methods, for example, by chromatography or fractional crystallization. Depending on the process conditions, the final products of the present invention are obtained in free (neutral) or salt form. Both the free forms and salts of these end products are within the scope of the present invention. If desired, one form of the compound may be converted to another form. The free base or acid may be converted to a salt; the salt may be converted to the free compound or another salt; mixtures of isomeric compounds of the present invention may be isolated into the individual isomers. The compounds, free forms and salts thereof of the present invention, may exist in a variety of tautomeric forms in which hydrogen atoms are transposed onto other parts of the molecule and the chemical bonds between the atoms of the molecule are thus rearranged.

Unless otherwise defined, the definitions of substituents of the present invention are each independent and not interrelated, e.g., for R^{a} (or R^{a}') in substituents, they are each independent in the definition of different substituents. Specifically, when a definition of R^{a} (or R^{a}') is selected in a substituent, it does not mean that R^{a} (or R^{a}') has the same definition in other substituents. More specifically, for example (a non-exhaustive list) for NR^{a}R^{a}', when the definition of R^{a} (or R^{a}') is selected from hydrogen, it does not mean that in -C(O)-NR^{a}R^{a}', R^{a} (or R^{a}') must be hydrogen.

Unless otherwise defined, when a substituent is labeled "optionally substituted", the substituent is selected from, for example, the following substituents consisting of alkyl, cycloalkyl, aryl, heterocyclyl, halogen, hydroxy, alkoxy, oxo, alkanoyl, aryloxy, alkanoyloxy, amino, alkylamino, arylamino, arylalkylamino, disubstituted amine group (in which two amino substituents are selected from alkyl, aryl or arylalkyl), alkanoylamino, aroylamino, aralkanoylamino, substituted alkanoylamino, substituted arylamino, substituted aralkanoylamino, thio, alkylthio, arylthio, arylalkylthio, arylthiocarbonyl, arylalkylthiocarbonyl, alkylsulfonyl, arylsulfonyl, arylalkylsulfonyl, sulfonamido such as -SO₂NH₂, substituted sulfonamido, nitro, cyano, carboxy, carbamoyl such as -CONH₂, substituted carbamoyl such as -CONH alkyl, -CONH aryl, -CONH arylalkyl or the case where there are two substituents selected from alkyl, aryl or arylalkyl on the nitrogen, alkoxycarbonyl, aryl, substituted aryl, guanidino, heterocyclyl such as indolyl, imidazolyl, furanyl, thienyl, thiazolyl, pyrrolidinyl, pyridyl, pyrimidinyl, pyrrolidinyl, piperidinyl, morpholinyl, piperazinyl, and homopiperazinyl, and substituted heterocyclyl.

As used herein, the term "alkyl" or "alkylene" is intended to include both branched and straight chain saturated aliphatic hydrocarbon groups having the specified number of carbon atoms. For example, "C1-C6 alkyl" denotes an alkyl group having 1 to 6 carbon atoms. Examples of alkyl groups include methyl (Me), ethyl (Et), propyl (e.g., n-propyl and isopropyl), butyl (e.g., n-butyl, isobutyl, tert-butyl), and pentyl (e.g., n-pentyl, isopentyl, neopentyl).

The term "alkenyl" denotes a straight or branched chain hydrocarbon group containing one or more double bonds and typically 2 to 20 carbon atoms in length. For example, "C2-C6 alkenyl" contains 2 to 6 carbon atoms. Alkenyl groups include, for example, ethenyl, propenyl, butenyl, and 1-methyl-2-buten-1-yl.

The term "alkynyl" denotes a straight or branched chain hydrocarbon group containing one or more triple bonds and typically 2 to 20 carbon atoms in length. For example, "C2-C6 alkynyl" contains 2 to 6 carbon atoms. Representative alkynyl groups include for example, ethynyl, 1-propynyl, and 1-butynyl.

The term "alkoxy" or "alkyloxy" refers to -O-alkyl. "C1-C6 alkoxy " (or alkyloxy) is intended to include C1, C2, C3, C4, C5, and C6 alkoxy. Examples of alkoxy groups include methoxy, ethoxy, propoxy (e.g., n-propoxy and isopropoxy), and t-butoxy. Similarly, "alkylthio" or "thioalkoxy" means an alkyl group, as defined above, with the specified number of carbon atoms linked via a sulfur bridge; for example, methyl-S- and ethyl-S-.

The term "carbonyl" refers to an organic functional group (C=O) composed of two carbon and oxygen atoms linked by a double bond.

The term "aryl", alone or as part of a larger moiety such as "aralkyl", "aralkoxy", or "aryloxyalkyl", refers to a monocyclic, bicyclic, or tricyclic ring system having a total of 5 to 12 ring members, where at least one ring in the system is aromatic and where each ring in the system contains 3 to 7 ring members. In certain embodiments of the present invention, "aryl" refers to an aromatic ring system including phenyl, biphenyl, indanyl, 1-naphthyl, 2-naphthyl, and tetrahydronaphthyl. The term "aralkyl" or "arylalkyl" refers to an alkyl residue attached to an aryl ring. Non-limiting examples include benzyl, and phenethyl. The fused aryl group may be attached to another group at a suitable position on the cycloalkyl ring or the aromatic ring. For example, a dashed line drawn from a ring system indicates that the bond may be attached to any suitable ring atom.

The term "cycloalkyl" refers to a monocyclic or bicyclic alkyl group. Monocyclic alkyl refers to C3-C8 cyclic alkyl including cyclopropyl, cyclobutyl, cyclopentyl, cyclohexyl, and norbornyl. Branched cycloalkyl such as 1-methylcyclopropyl and 2-methylcyclopropyl are included in the definition of "cycloalkyl". Bicyclic alkyl includes bridged, spiro, or fused cycloalkyl.

The term "cycloalkenyl" refers to a monocyclic or bicyclic alkenyl group. Monocyclic alkenyl refers to C3-C8 cyclic alkenyl including cyclopropenyl, cyclobutenyl, cyclopentenyl, cyclohexenyl, and norbornenyl. Branched cycloalkenyl such as 1-methylcyclopropenyl and 2-methylcyclopropenyl are included in the definition of "cycloalkenyl". Bicyclic alkenyl includes bridged, spiro or fused cyclic alkenyl.

"Halo" or "halogen" includes fluoro, chloro, bromo and iodo. "Haloalkyl" is intended to include both branched and straight-chain saturated aliphatic hydrocarbon groups having the specified number of carbon atoms and substituted with one or more halogens. Examples of haloalkyl include fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" groups intended to include branched and straight chain saturated aliphatic hydrocarbon groups having the specified number of carbon atoms and substituted with one or more fluorine atoms.

"Haloalkoxy" or "haloalkyloxy" denotes a haloalkyl group, as defined above, having the indicated number of carbon atoms linked via an oxygen bridge. For example, "C1-C6 haloalkoxy" is intended to include C1, C2, C3, C4, C5, and C6 haloalkoxy. Examples of haloalkoxy include trifluoromethoxy, 2,2,2-trifluoroethoxy, and pentafluoroethoxy. Similarly, "haloalkylthio" or "thiohaloalkoxy" denotes a haloalkyl group, as defined above, having the indicated number of carbon atoms linked via a sulfur bridge; for example, trifluoromethyl-S- and pentafluoroethyl-S-.

In the present disclosure, the expression Cxl-Cx2 is used when referring to some substituent groups, which means that the number of carbon atoms in the substituent group may be x1 to x2. For example, C0-C8 means that the group contains 0, 1, 2, 3, 4, 5, 6, 7 or 8 carbon atoms, C1-C8 means that the group contains 1, 2, 3, 4, 5, 6, 7 or 8 carbon atoms, C2-C8 means that the group contains 2, 3, 4, 5, 6, 7 or 8 carbon atoms, C3-C8 means that the group contains 3, 4, 5, 6, 7 or 8 carbon atoms, C4 -C8 means that the group contains 4, 5, 6, 7 or 8 carbon atoms, C0-C6 means that the group contains 0, 1, 2, 3, 4, 5 or 6 carbon atoms, C1-C6 means that the group contains 1, 2, 3, 4, 5 or 6 carbon atoms, C2-C6 means that the group contains 2, 3, 4, 5 or 6 carbon atoms, and C3-C6 means that the group contains 3, 4, 5 or 6 carbon atoms.

In the present disclosure, the expression "x1-x2 membered ring" is used when referring to cyclic groups such as aryl, heteroaryl, cycloalkyl and heterocycloalkyl, which means that the number of ring atoms of the group may be x1 to x2. For example, the 3- to 12-membered cyclic group may be a 3, 4, 5, 6, 7, 8, 9, 10, 11, or 12 membered ring, the number of ring atoms of which may be 3, 4, 5, 6, 7, 8, 9, 10, 11, or 12; the 3- to 6-membered ring means that the cyclic group may be a 3, 4, 5 or 6 membered ring, the number of ring atoms of which may be 3, 4, 5 or 6; the 3- to 8-membered ring means that the cyclic group may be a 3, 4, 5, 6, 7 or 8 membered ring, the number of ring atoms of which may be 3, 4, 5, 6, 7 or 8; the 3- to 9-membered ring means that the cyclic group may be a 3, 4, 5, 6, 7, 8 or 9 membered ring, the number of ring atoms of which may be 3, 4, 5, 6, 7, 8 or 9; the 4- to 7-membered ring means that the cyclic group may be a 4, 5, 6 or 7 membered ring, the number of ring atoms of which may be 4, 5, 6 or 7; the 5- to 8-membered ring means that the cyclic group may be a 5, 6, 7 or 8 membered ring, the number of ring atoms of which may be 5, 6, 7 or 8; the 5- to 12-membered ring means that the cyclic group may be a 5, 6, 7, 8, 9, 10, 11 or 12 membered ring, the number of ring atoms of which may be 5, 6, 7, 8, 9, 10, 11 or 12; and the 6- to 12-membered ring means that the cyclic group may be a 6, 7, 8, 9, 10, 11 or 12 membered ring, the number of ring atoms of which may be 6, 7, 8, 9, 10, 11 or 12. The ring atom may be a carbon atom or a heteroatom, for example, a heteroatom selected from N, O and S. When the ring is a heterocycle, the heterocycle may contain 1, 2, 3, 4, 5, 6, 7, 8, 9, 10 or more ring heteroatoms, for example, a heteroatom selected from N, O and S.

In the present disclosure, one or more halogens may each independently be selected from fluorine, chlorine, bromine, and iodine.

The term "heteroaryl" means a stable 3-, 4-, 5-, 6-, or 7-membered aromatic monocyclic or aromatic bicyclic or 7-, 8-, 9-, 10-, 11-, 12-membered aromatic polycyclic heterocycle, which is fully unsaturated, partially unsaturated, and contains carbon atoms and 1, 2, 3 or 4 heteroatoms independently selected from N, O and S; and includes any polycyclic group in which any heterocycle defined above is fused to a benzene ring. The nitrogen and sulfur heteroatoms may optionally be oxidized. The nitrogen atom is substituted or unsubstituted (i.e., N or NR, where R is H or another substituent if defined). The heterocycle may be attached to its pendant group at any heteroatom or carbon atom that results in a stable structure. If the resulting compound is stable, the heterocyclyl groups described herein may be substituted on a carbon or nitrogen atom.

The nitrogen in the heterocycle may be optionally quaternized. Preferably, when the total number of S and O atoms in the heterocycle exceeds 1, then these heteroatoms are not adjacent to each other. Preferably, the total number of S and O atoms in the heterocycle is not greater than 1. When the term "heterocycle" is used, it is intended to include heteroaryl. Examples of heteroaryls include acridinyl, azetidinyl, azocinyl, benzimidazolyl, benzofuranyl, benzothiofuranyl, benzothienyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H, 6H-1,5,2-dithiazinyl, dihydrofuro[2,3-b]tetrahydrofuranyl, furanyl, furazanyl, imidazolidinyl, imidazolinyl, imidazolyl, 1H-indazolyl, imidazopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinyl, perimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2H-pyrrolyl, pyrrolyl, quinazolinyl, quinolinyl, 4H-quinolizinyl, quinoxalinyl, quinuclidinyl, tetrazolyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridyl, thienothiazolyl, thienooxazolyl, thienoimidazolyl, thienyl, triazinyl, 1,2,3-triazolyl, 1,2,4-triazolyl, 1,2,5-triazolyl, 1,3,4-triazolyl and xanthenyl, quinolinyl, isoquinolinyl, phthalazinyl, quinazolinyl, indolyl, isoindolyl, indolinyl, 1H-indazolyl, benzimidazolyl, 1,2,3,4-tetrahydroquinolinyl, 1,2,3,4-tetrahydroisoquinolinyl, 5,6,7,8-tetrahydro-quinolinyl, 2,3-dihydro-benzofuranyl, chromanyl, 1,2,3,4-tetrahydro-quinoxalinyl and 1,2,3,4-tetrahydro-quinazolinyl. The term "heteroaryl" may also include biaryl structures formed from "aryl" and monocyclic "heteroaryl" as defined above, for example, "-phenylbipyridyl-", "-phenylbipyrimidinyl ", "-pyridylbiphenyl ", "-pyridylbipyrimidinyl-", "-pyrimidinylbiphenyl-"; where the present invention also includes fused and spiro compounds containing, for example, the above heterocycles.

As used herein, the term "heterocycloalkyl" refers to a monocyclic heterocycloalkyl system, or a bicyclic heterocycloalkyl system, and also includes spiroheterocycles or bridged heterocycloalkyl groups. The monocyclic heterocycloalkyl refers to a saturated or unsaturated but not aromatic 3-to 8-membered cyclic alkyl system containing at least one atom selected from O, N, S, and P. The bicyclic heterocycloalkyl system refers to a heterocycloalkyl fused with a phenyl, or a cycloalkyl, or a cycloalkenyl, or a heterocycloalkyl, or a heteroaryl.

As used herein, the term "bridged cycloalkyl" refers to polycyclic compounds that share two or more carbon atoms, including bicyclic bridged cyclic hydrocarbons and polycyclic bridged cyclic hydrocarbons. The former are composed of two alicyclic rings sharing more than two carbon atoms; the latter are a bridged cyclic hydrocarbons consisting of more than three rings.

As used herein, the term "spirocycloalkyl" refers to polycyclic hydrocarbons that share one carbon atom (referred to as a spiro atom) between single rings.

As used herein, the term "bridged cycloheteryl" refers to polycyclic compounds that share two or more carbon atoms, and contain at least one atom selected from O, N, S, including bicyclic bridged heterocycles and polycyclic bridged heterocycles.

As used herein, the term "heterospirocyclyl" refers to polycyclic hydrocarbons that share one carbon atom (referred to as a spiro atom) between single rings, and contain at least one atom selected from O, N, S.

As used herein, the term "substituted" means that at least one hydrogen atom is substituted with a non-hydrogen group, provided that normal valency is maintained and that the substitution results in a stable compound. As used herein, the ring double bond is a double bond (e.g., C=C, C=N, or N=N) formed between two adjacent ring atoms.

In the case where nitrogen atoms (e.g., amines) are present on the compounds of the present invention, these nitrogen atoms may be converted to N-oxides by treatment with an oxidizing agent (e.g., mCPBA and/or hydrogen peroxide) to obtain other compounds of the present invention. Thus, the nitrogen atoms shown and claimed are considered to encompass both the nitrogen shown and its N-oxides to obtain the derivatives of the present invention.

When any variable occurs more than once in any composition or formula of a compound, its definition at each occurrence is independent of its definition at every other occurrence. Thus, for example, if a group is shown to be substituted with 0-3 R, the group may be optionally substituted with up to three R groups, and at each occurrence R is independently selected from the definition of R. Furthermore, combinations of substituents and/or variables are permissible only if such combinations result in stable compounds.

As used herein, the term "patient" refers to an organism treated by compounds for use in the methods of the present invention. Such organisms preferably include mammals (e.g., murine, ape/monkey, equine, bovine, swine, canine, feline, etc.) and most preferably refer to humans.

As used herein, the term "effective amount" means an amount of a drug or pharmaceutical agent (i.e., a compound of the present invention) that will elicit the biological or medical response of a tissue, system, animal or human that is being sought, for example, by a researcher or clinician. Furthermore, the term "therapeutically effective amount" means an amount results in an improved treatment, cure, prevention or alleviation of a disease, disorder or side effect, or a reduction in the rate of progression of a disease or disorder, as compared to a corresponding subject not receiving such an amount. An effective amount can be administered in one or more dosing, administrations, or dosages and is not intended to be limited by the particular formulation or route of administration. The term also includes an amount effective that enhances normal physiological function within its scope.

As used herein, the term "treating" includes any effect that results in amelioration of a condition, disease, or disorder, for example, alleviation, reduction, modulation, amelioration or elimination, or amelioration of a symptom thereof.

The term "pharmaceutically acceptable" is used herein to refer to those compounds, materials, compositions, and/or dosage forms as follows: within the scope of sound medical judgment, suitable for use in contact with the tissues of humans and animals without undue toxicity, irritation, allergic response, and/or other problems or complications, commensurate with a reasonable benefit/risk ratio.

As used herein, the phrase "pharmaceutically acceptable carrier" means a pharmaceutical material, composition or vehicle, such as a liquid or solid filler, diluent, excipient, manufacturing adjuvant (e.g., lubricant, talc, magnesium stearate, calcium stearate or zinc stearate or stearic acid), or solvent encapsulating material, which refers to carrying or transporting the subject compound from one organ or portion of the body to another organ or portion of the body. Each carrier must be "acceptable" in the sense of being compatible with the other ingredients of the formulation and not deleterious to the patient.

The term "pharmaceutical composition" means a composition including a compound of the present invention and at least one other pharmaceutically acceptable carrier. "Pharmaceutically acceptable carrier" means a medium generally accepted in the art for the delivery of a biologically active agent to an animal, particularly a mammal, and includes, i.e., adjuvants, excipients, or vehicles such as diluents, preservatives, fillers, flow regulating agents, disintegrating agents, wetting agents, emulsifying agents, suspending agents, sweetening agents, flavoring agents, perfuming agents, antibacterial agents, antifungal agents, lubricating agents, and dispersing agents, depending on the mode of administration and the nature of the dosage form.

### Specific pharmaceutical and medical terms

The term "acceptable", as used herein, refers to a prescription component or active ingredient that does not unduly adversely affect the health of the general therapeutic target.

The term "cancer", as used herein, refers to an uncontrolled abnormal growth of cells and is capable of metastasis (transmission) under certain conditions. This type of cancer includes solid tumors (e.g., bladder, bowel, brain, chest, uterus, heart, kidney, lung, lymphoid tissue (lymphoma), ovary, pancreas, or other endocrine organs (e.g., thyroid), prostate, skin (melanoma), or hematological tumors (e.g., aleukemic leukemia).

The term "administered in combination" or similar terms, as used herein, refers to the administration of several selected therapeutic agents to a patient in the same or different modes of administration at the same or different times.

The term "enhance" or "can enhance", as used herein, means that the desired result can be increased or prolonged in potency or duration. Thus, in enhancing the therapeutic effect of a drug, the term "enhance" refers to the ability of the drug to increase or prolong potency or duration in the system. "Synergistic value", as used herein, refers to the ability to maximize the ability of another therapeutic agent in an ideal system.

The term "immunological disease" refers to a disease or condition that responds adversely or deleteriously to endogenous or exogenous antigens. The result is often a dysfunction of the cells, or thus destruction and dysfunction, or destruction of organs or tissues that may produce immune symptoms.

The term "kit" is synonymous with "product package".

The term "subject" or "patient" includes mammals and non-mammals. Mammals include mammals: human, non-human primates such as chimpanzees, apes and monkeys; agricultural animals such as bovines, equines, goats, sheep, and swines; domestic animals such as rabbits, and canines; experimental animals include rodents, such as rats, mice, and guinea pigs. Non-mammalian animals include birds, and fish. In a preferred embodiment, the selected mammal is a human.

The terms "treatment", "treatment process", or "therapy", as used herein, include alleviating, inhibiting, or ameliorating the symptoms or conditions of a disease; inhibiting the generation of complications; ameliorating or preventing potential metabolic syndrome; inhibiting the development of a disease or condition, such as controlling the development of a disease or condition; alleviating a disease or condition; reducing the disease or symptoms; alleviating complications resulting from the disease or condition, or preventing and/or treating symptoms resulting from the disease or condition.

As used herein, a compound or pharmaceutical composition, upon administration, may result in amelioration of a disease, symptom, or condition, particularly amelioration of the severity, delay of the onset, alleviation of the progression, or reduction of the duration of the condition. Regardless of fixed administration or temporary administration, continuous administration or intermittent administration, it may be attributed to or related to the administration.

### Route of administration

Suitable routes of administration include oral, intravenous, rectal, aerosol, parenteral, ophthalmic, pulmonary, transdermal, vaginal, aural, nasal, and topical administration. In addition, by way of example only, parenteral administration includes intramuscular, subcutaneous, intravenous, intramedullary, ventricular, intraperitoneal, intralymphatic, and intranasal injections.

In one aspect, the compounds described herein are administered locally rather than systemically. In particular embodiments, the prolonged action preparation is administered by implantation (e.g., subcutaneously or intramuscularly) or by intramuscular injection. Further, in another embodiment, the drug is administered by a targeted drug delivery system, for example, liposomes encapsulated by organ-specific antibodies. In this particular embodiment, the liposomes are selectively targeted to specific organs and absorbed.

### Pharmaceutical compositions and dosages

The present invention also provides pharmaceutical compositions including a therapeutically effective amount of one or more compounds of the present invention formulated together with one or more pharmaceutically acceptable carriers (additives) and/or diluents, and optionally one or more of the other therapeutic agents described above. The compounds of the present invention may be administered for any of the above uses by any suitable means, for example by orally, such as in the form of tablets, pills, powders, granules, elixirs, tinctures, suspensions (including nanosuspensions, microsuspensions, spray-dried dispersions), syrups and emulsions; by sublingually; by buccally; by parenterally, such as by subcutaneous, intravenous, intramuscular or intrasternal injection or infusion techniques (e.g., in the form of sterile injectable aqueous or nonaqueous solutions or suspensions); by nasally, including administration to the nasal mask, such as by inhalation spray; by topically, such as in the form of a cream or ointment; or by rectally, such as in the form of suppositories; or by intratumoral injection. They may be administered alone, but are generally administered using pharmaceutical acceptable carriers selected based on the chosen route of administration and standard pharmaceutical practice.

The pharmaceutical acceptable carriers are formulated according to a number of factors within the knowledge of those skilled in the art. These factors include: types and properties of the formulated active agents; a subject to be administered the composition containing the active agent; the intended route of administration of the composition; and targeted therapeutic indications. The pharmaceutically acceptable carriers include aqueous and non-aqueous liquid media and various solid and semi-solid dosage forms.

The above-mentioned carrier may include many different ingredients and additives in addition to the active agent, and the above-mentioned other ingredients, for example, stabilizing active agent and binder, are included in the formulation for various reasons known to those skilled in the art. For a description of suitable pharmaceutical acceptable carriers and factors involved in the selection of carrier, see a number of readily available sources, such as Allen L.V.Jr. et al. Remington: The Science and Practice of Pharmacy (2 Volumes), 22nd Edition (2012), Pharmaceutical Press.

The dosage regimen for the compounds of the present invention will, of course, vary depending upon known factors such as the pharmacodynamic characteristics of the particular agent and its mode and route of administration; species, age, sex, health, medical condition and weight of the recipient; the nature and extent of symptoms; kind of concurrent treatment; treatment frequency; routes of administration, renal and hepatic function and desired effects in patients. According to general guidelines, when used for a given effect, the daily oral dosage of each active ingredient should be from about 0.001 mg/day to about 10-5000 mg/day, preferably from about 0.01 mg/day to about 1000 mg/day, and most preferably from about 0.1 mg/day to about 250 mg/day. During constant infusion, the most preferred intravenous dose should be from about 0.01 mg/kg/min to about 10 mg/kg/min. The compounds of the present invention may be administered in a single daily dose, or the total daily dose may be administered in divided doses of two, three or four times daily.

The compounds are generally administered in the form of a mixture of suitable pharmaceutical diluents, excipients, or carriers (collectively referred to herein as pharmaceutical acceptable carriers) suitably selected with respect to the intended form of administration (e.g., oral tablets, capsules, elixirs, and syrups) and consistent with conventional pharmaceutical practice.

Dosage forms (pharmaceutical compositions) suitable for administration may contain from about 1 mg to about 2000 mg of active ingredient per dosage unit. In these pharmaceutical compositions, the active ingredient will generally be present in an amount of about 0.1-95% by weight, based on a total weight of the composition.

Typical capsules for oral administration contain at least one compound of the present invention (250 mg), lactose (75 mg) and magnesium stearate (15 mg). The mixture was processed through a 60 meshes screen and packaged into No.1 gelatin capsules.

A typical injectable formulation may be prepared as follows: at least one compound of the present invention (250 mg) was placed in a vial in a sterile manner, and lyophilized and sealed in a sterile manner. For use, the contents in the vial were mixed with 2 mL of normal saline to produce an injectable formulation.

The scope of the present invention includes (alone or in combination with a pharmaceutical acceptable carrier) pharmaceutical compositions containing a therapeutically effective amount of at least one compound of the present invention as an active ingredient. Optionally, the compounds of the present invention may be used alone, in combination with other compounds of the present invention, or in combination with one or more other therapeutic agents (e.g., anticancer agents or other pharmaceutically active agents).

Regardless of the selected route of administration, the compounds of the present invention (which may be used in suitable hydrated forms) and/or the pharmaceutical compositions of the present invention are formulated into pharmaceutically acceptable dosage forms by conventional methods known to those skilled in the art.

The actual dosage level of the active ingredient in the pharmaceutical compositions of the present invention may be varied so as to obtain an amount of the active ingredient that is effective to achieve the desired therapeutic response, composition, and mode of administration for a particular patient without being toxic to the patient.

The selected dosage level will depend upon a variety of factors, including the factors well known in the medical field such as the activity of the employed specific compound of the present invention, or an ester, salt or amide thereof; routes of administration; administration time; the discharge rate of the employed specific compound; the absorption rate and extent; duration of treatment; other drugs, compounds and/or substances used in combination with the employed specific compounds; the age, sex, weight, condition, general health and prior medical history of the patient being treated.

A physician or veterinarian having ordinary skill in the art can readily determine and prescribe an effective amount of the desired pharmaceutical composition. For example, to achieve the desired therapeutic effect, the physician or veterinarian may start a relatively small amount of the compound of the present invention used in the pharmaceutical composition below the desired level and gradually increase the dosage until the desired effect is achieved. In general, a suitable daily dose of a compound of the present invention will be that amount of the compound that is the lowest dose effective to produce a therapeutic effect. Such an effective dose will generally depend on such factors. In general, oral, intravenous, intracerebroventricular, and subcutaneous doses of a compound of the present invention for a patient range from about 0.01 to about 50 mg/kg body weight/day. If desired, an effective daily dose of the active compound may be administered in two, three, four, five, six or more sub-doses respectively at appropriate intervals throughout the day, optionally in unit dosage form. In certain aspects of the present invention, the medication is administered once a day.

Although the compound of the present invention may be administered alone, it is preferably administered in the form of a pharmaceutical preparation (composition).

### Kit/Product package

Kits/product packages are also described herein for the treatment of the above indications. These kits may be composed of a conveyor, a medicine pack or a container box. The container box can be divided into multiple compartments to accommodate one or more containers, such as vials, and test tubes, where each container contains all a single component in the method. Suitable containers consist of bottles, vials, syringes, and test tubes. The container is made of an acceptable glass or plastic material.

For example, the container may contain one or more of the compounds described herein; the compound may exist either in the form of a pharmaceutical composition or may exist as a mixture with other ingredients described herein. The container may have a sterile outlet (e.g., the container may be an intravenous infusion bag or bottle and the stopper may be pierced by a hypodermic needle). Such kits may contain a compound and descriptions, labels or instructions for the method of use described herein.

A typical kit may include one or more containers, each containing one or more materials (e.g., reagents, concentrated stock solutions, and/or equipment) to accommodate commercial promotions and the needs of the user for the use of compounds. Such materials include buffers, diluents, filters, needles, syringes, conveyors, bags, containers, bottles, and/or tubes, with a list of contents and/or instructions for use, and with a build-in package. The entire set of instructions must be included.

The label may be displayed on or closely related to the container. The appearance of the label on the container means that the label letters, numbers or other features are pasted, molded, or engraved on the container; the label can also appear in the container box or shipping box containing a variety of containers, such as in the product insert. A label may be used to indicate a particular therapeutic use of the contents. The label may also indicate directions for the use of contents, such as described in the methods described above.

The units in weight-volume percent in the present invention are well known to those skilled in the art and refer, for example, to the weight of solute in a 100 milliliters solution. Unless otherwise defined, all professional and scientific terms used in the text have the same meaning as those familiar to those skilled in the art.

In preferred embodiments of the present invention, the following compounds are provided:

| No. | Compound structure | No. | Compound structure |
|---|---|---|---|
| 1 | 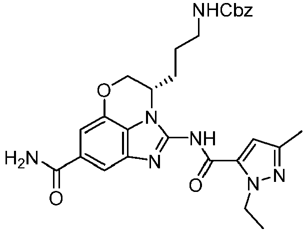 | 2 | |
| 3 | 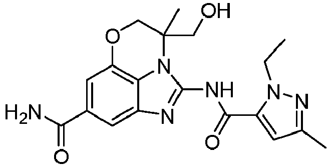 | 4 | 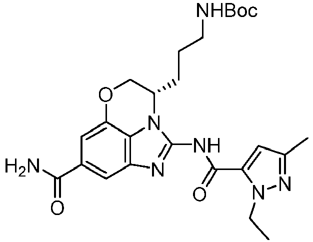 |
| 5 | 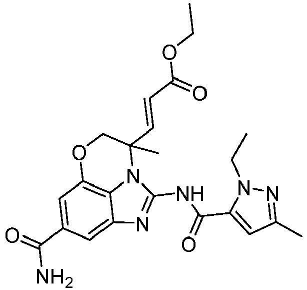 | 6 | 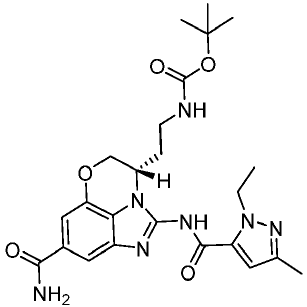 |
| 7 | 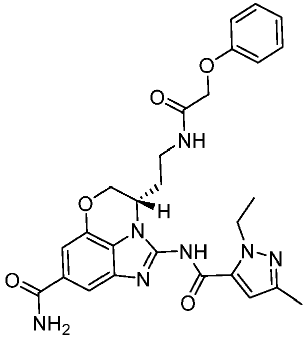 | 8 | 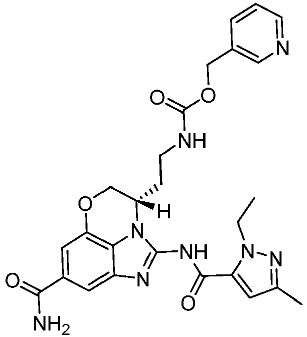 |
| 9 | 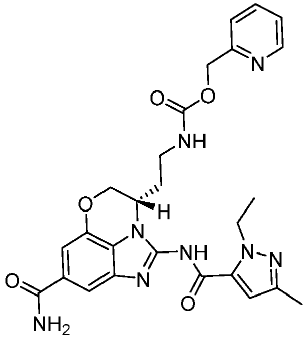 | 10 | 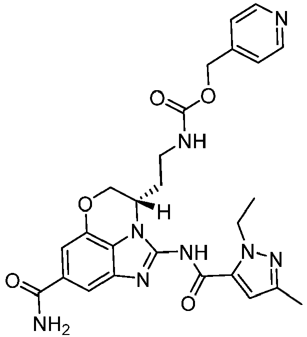 |
| 11 | 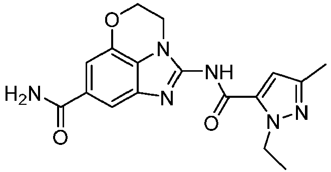 | 12 | 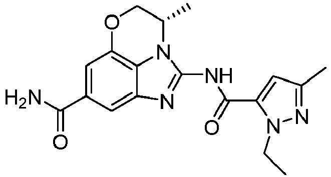 |
| 13 | 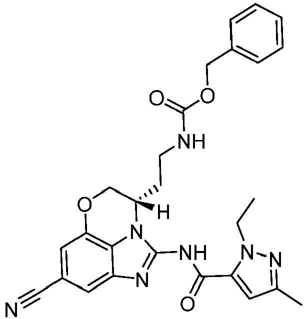 | 14 | 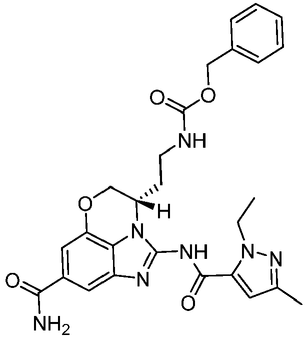 |
| 15 | 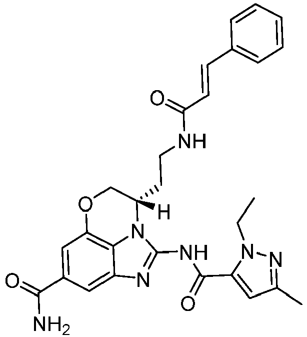 | 16 | 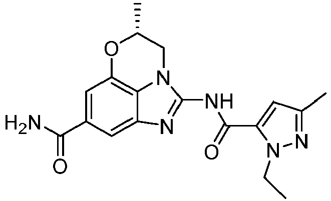 |
| 17 | 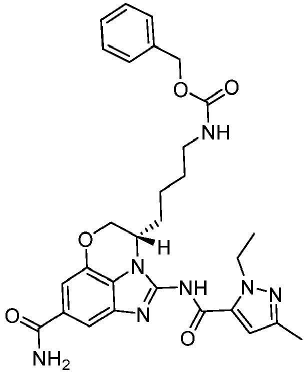 | 18 | 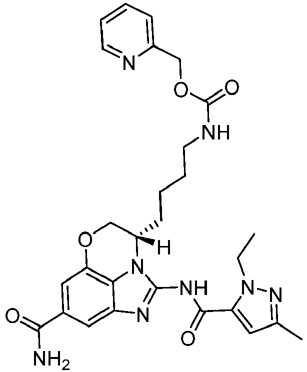 |
| 19 | 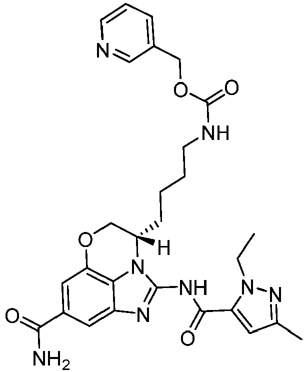 | 20 | 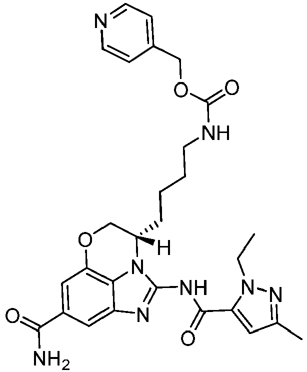 |
| 21 | 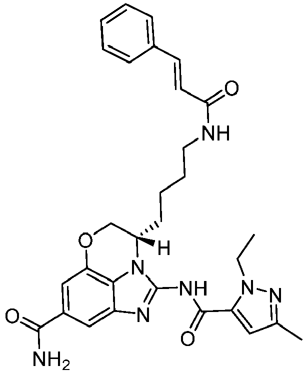 | 22 | 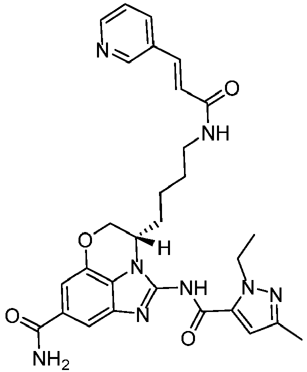 |
| 23 | 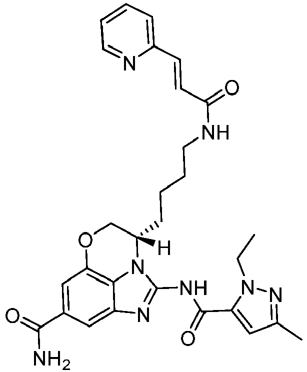 | 24 | 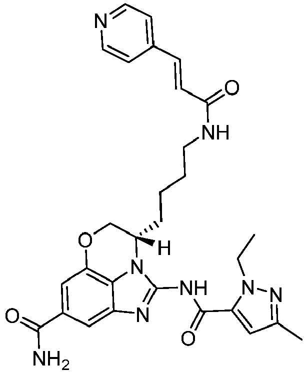 |
| 25 | 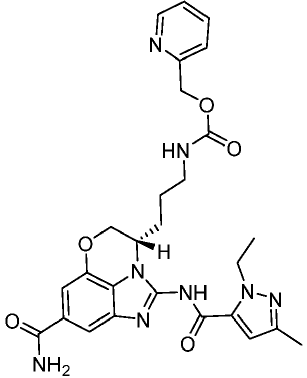 | 26 | 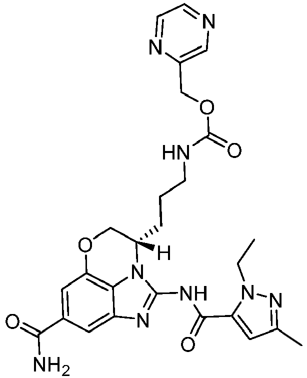 |
| 27 | 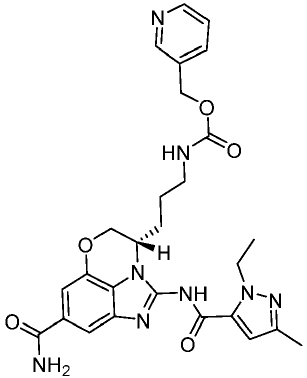 | 28 | 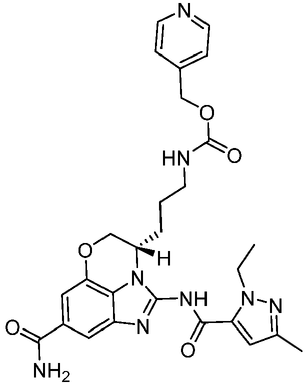 |
| 29 | 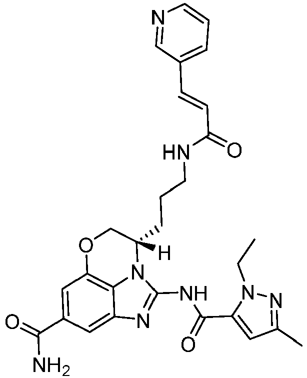 | 30 | 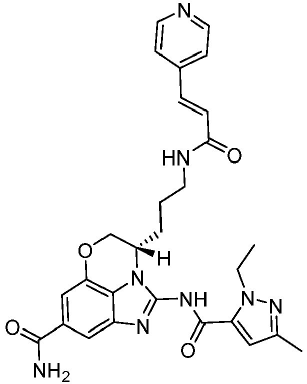 |
| 31 | 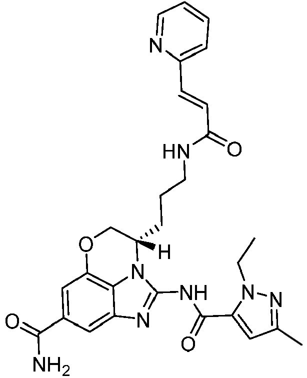 | 32 | 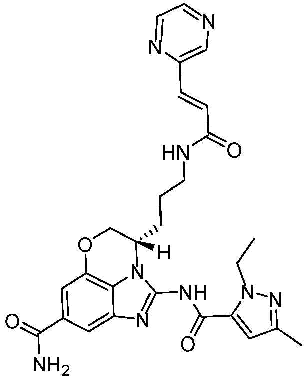 |
| 33 | 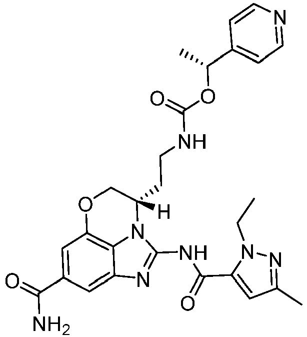 | 34 | 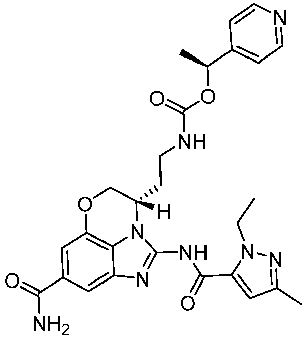 |
| 35 | 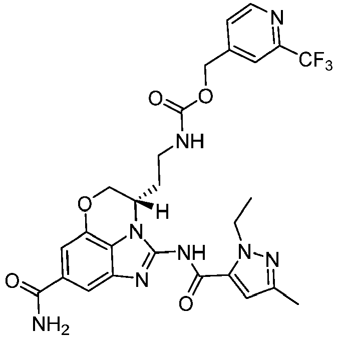 | 36 | 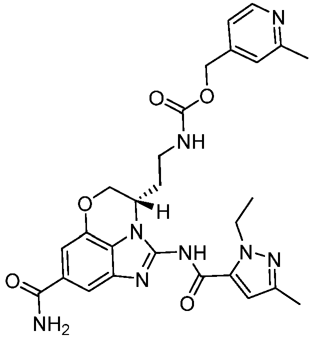 |
| 37 | 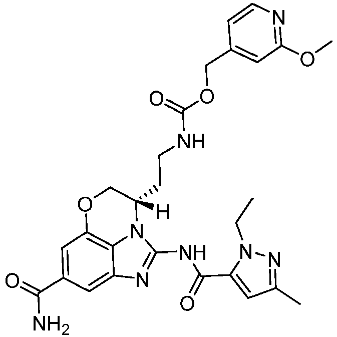 | 38 | 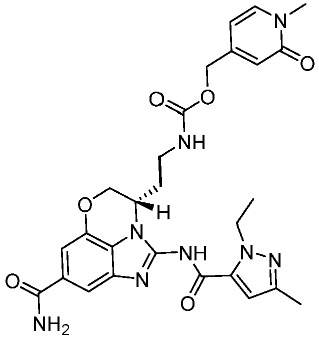 |
| 39 | 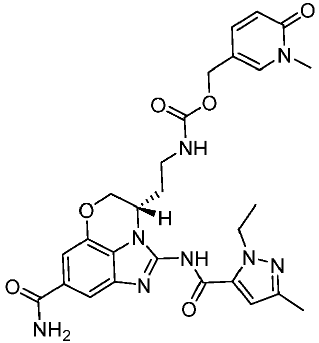 | 40 | 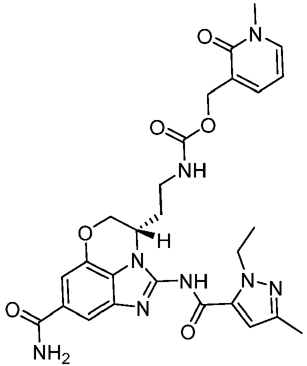 |
| 41 | 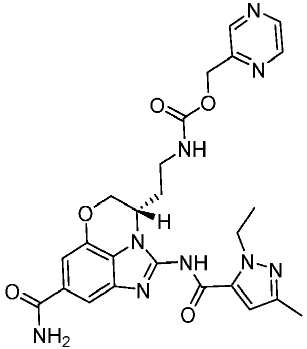 | 42 | 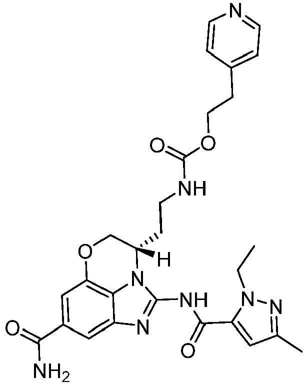 |
| 43 | 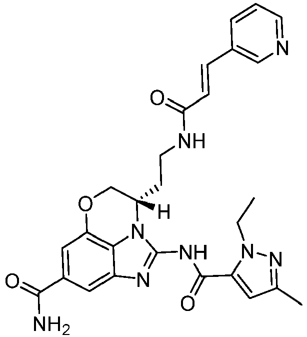 | 44 | 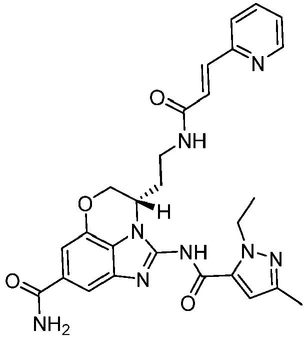 |
| 45 | 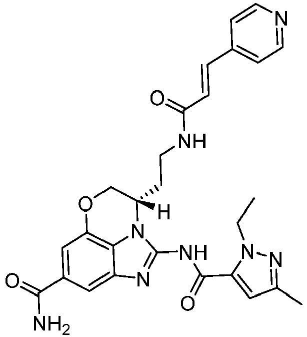 | 46 | 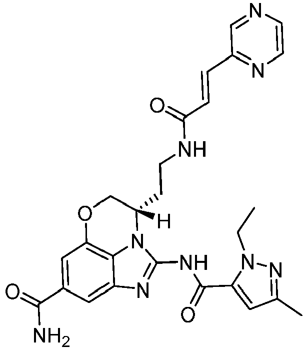 |
| 47 | 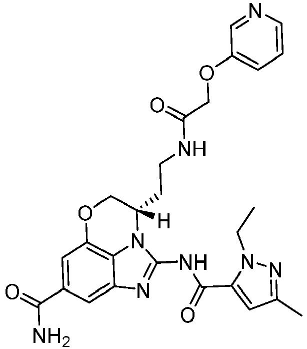 | 48 | 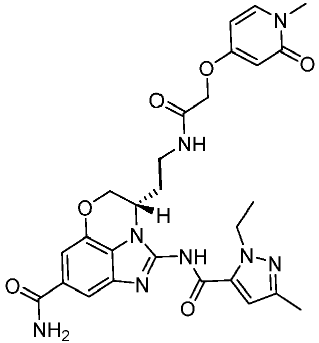 |
| 49 | 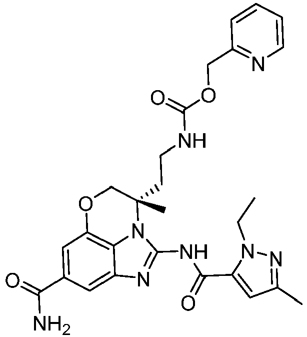 | 50 | 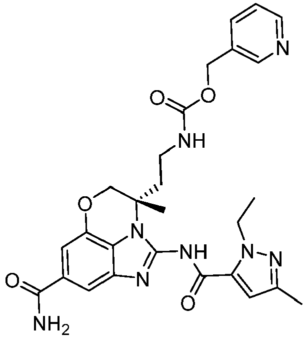 |
| 51 | 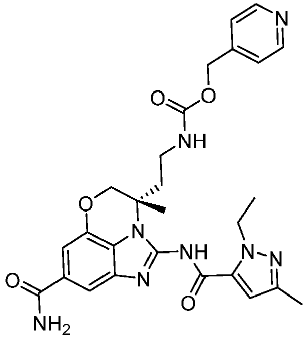 | 52 | 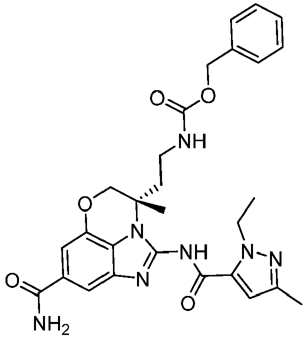 |
| 53 | 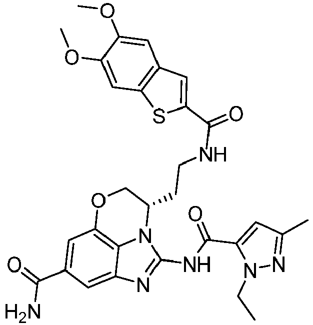 | 54 | 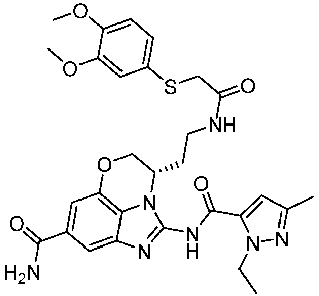 |
| 55 | 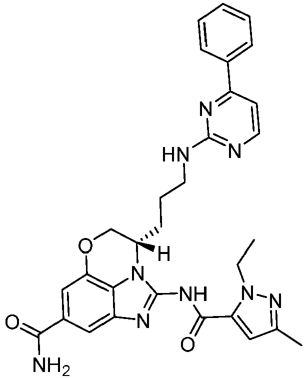 | 56 | 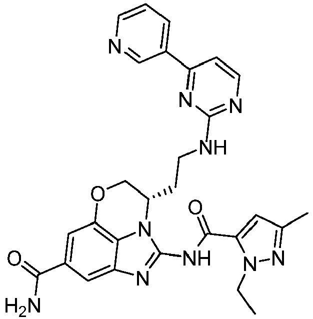 |
| 57 | 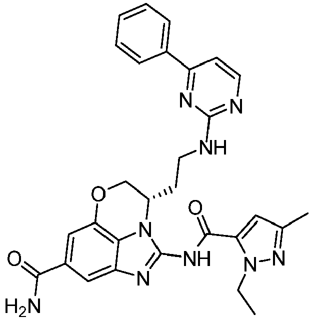 | 58 | 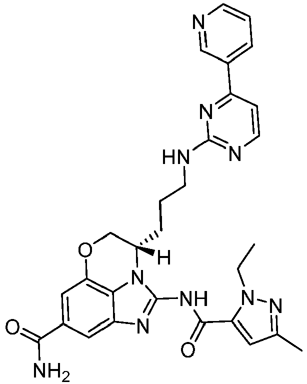 |
| 59 | 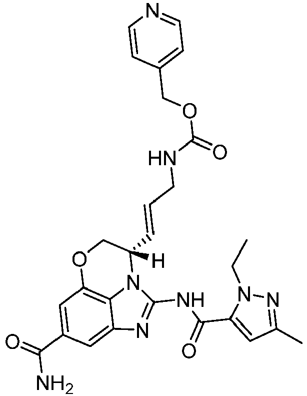 | 60 | 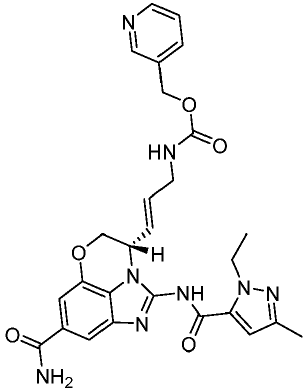 |
| 61 | 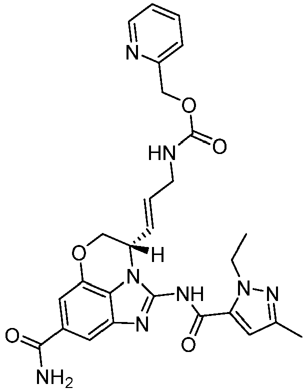 | 62 | 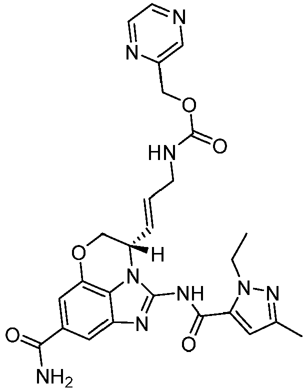 |
| 63 | 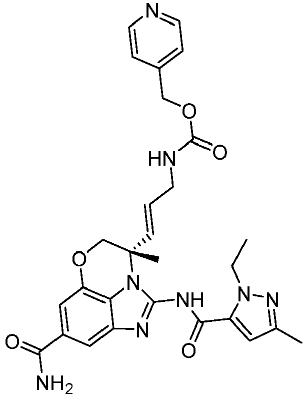 | 64 | 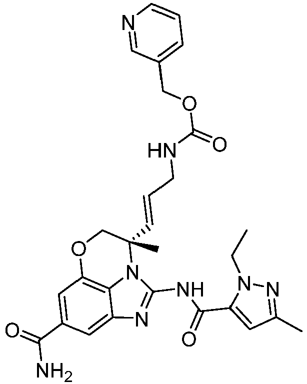 |
| 65 | 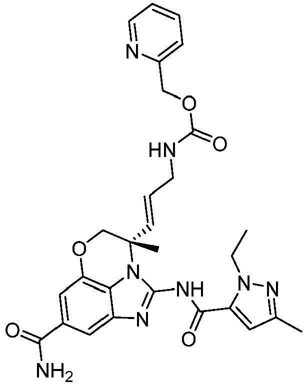 | 66 | 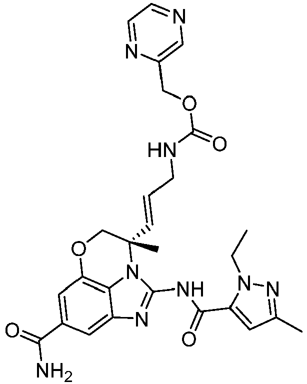 |
| 67 | 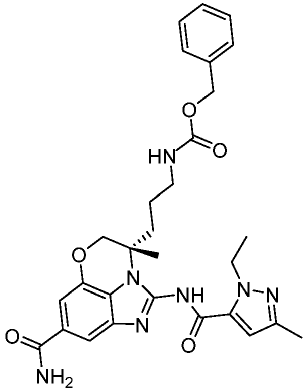 | 68 | 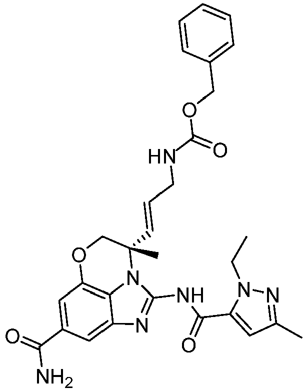 |
| 69 | 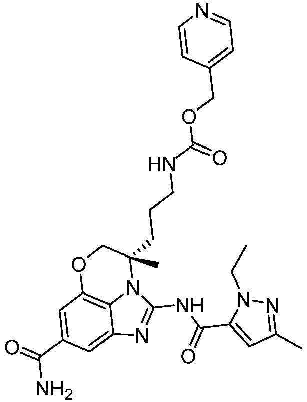 | 70 | 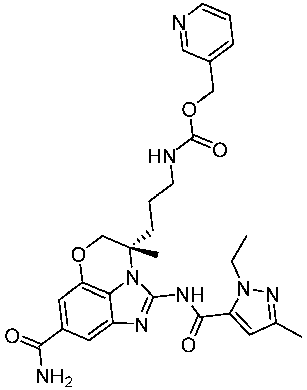 |
| 71 | 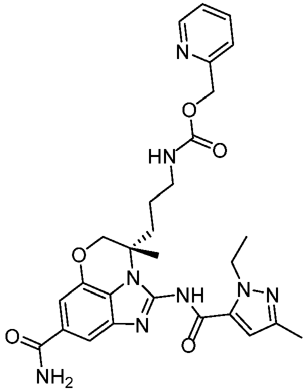 | 72 | 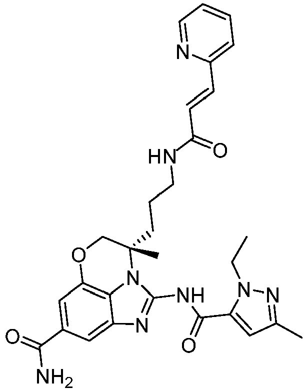 |
| 73 | 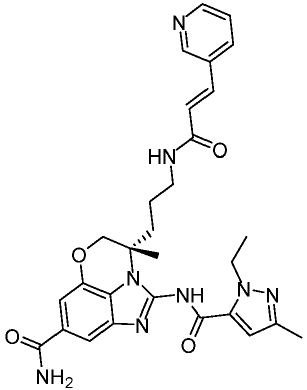 | 74 | 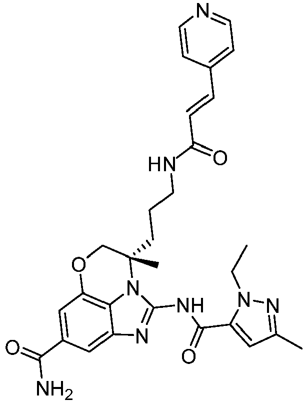 |
| 75 | 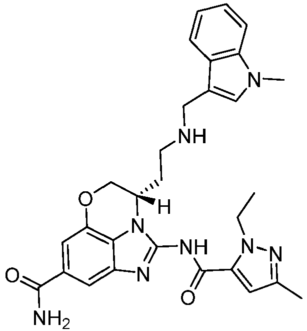 | 76 | 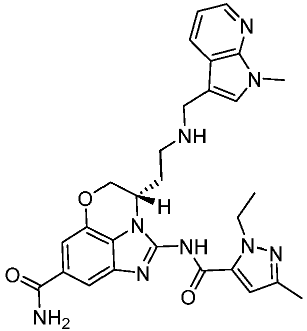 |
| 77 | 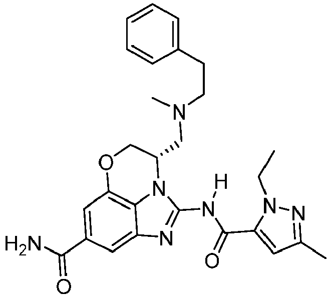 | 78 | 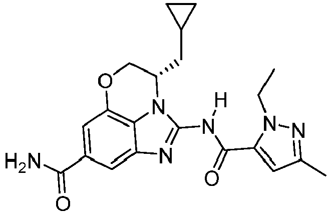 |
| 79 | 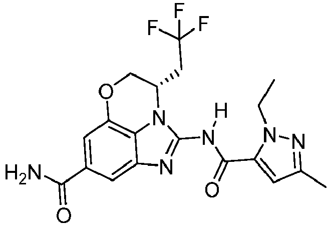 | 80 | 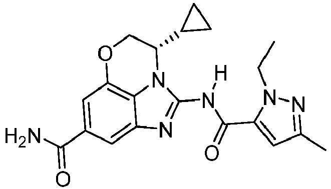 |
| 81 | 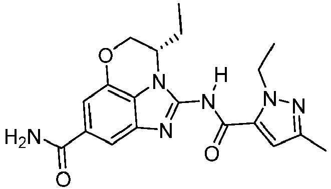 | 82 | 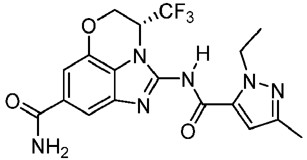 |
| 83 | 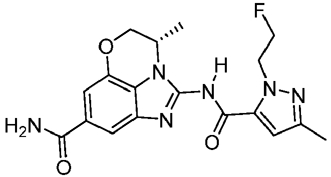 | 84 | 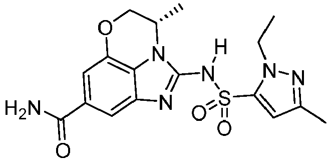 |
| 85 | 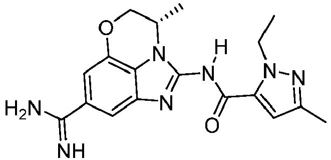 | 86 | 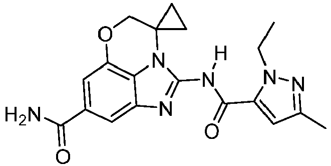 |
| 87 | 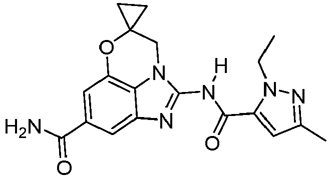 | 88 | 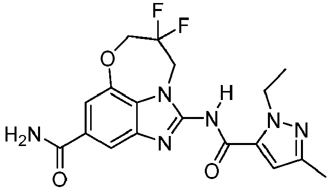 |
| 89 | 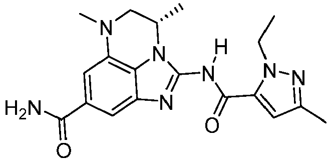 | 90 | 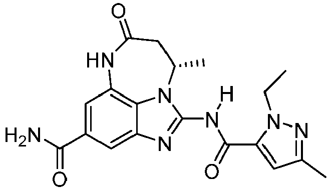 |
| 91 | 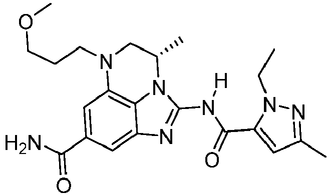 | 92 | 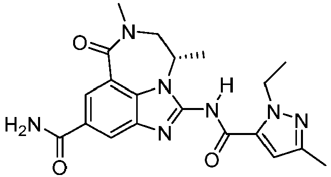 |
| 93 | 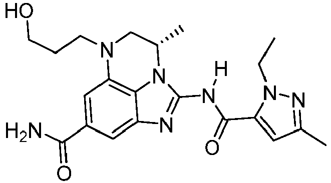 | 94 | 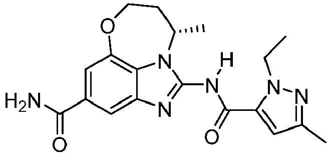 |
| 95 | 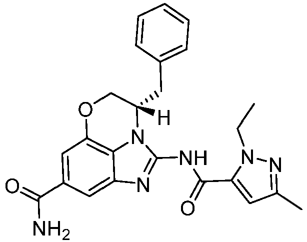 | 96 | 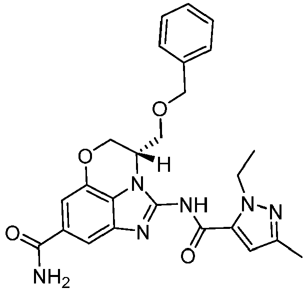 |
| 97 | 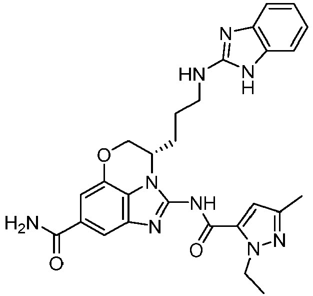 | 98 | 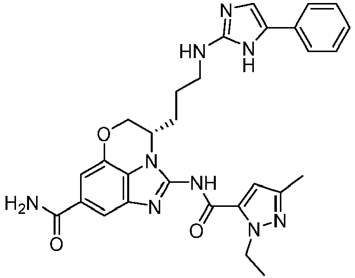 |
| 99 | 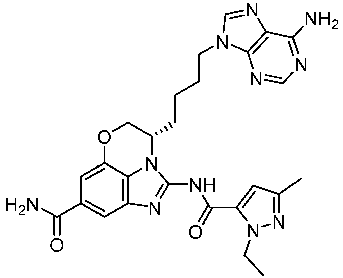 | 100 | 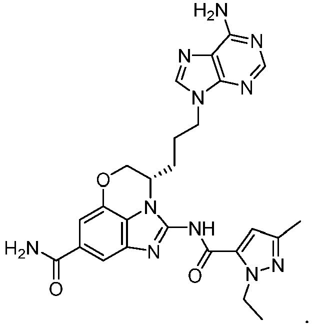 |
| 101 | 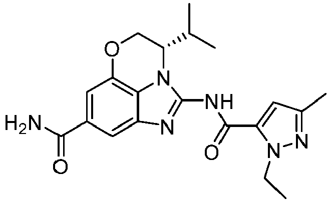 | 102 | 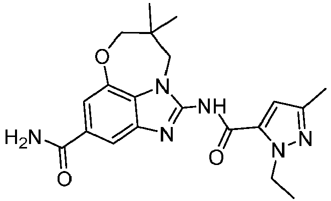 |
| 103 | 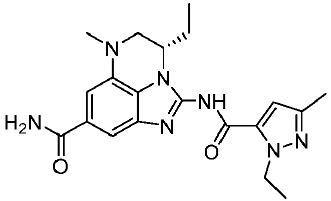 | 104 | 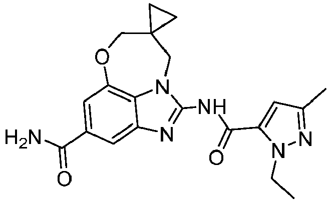 |
| 105 | 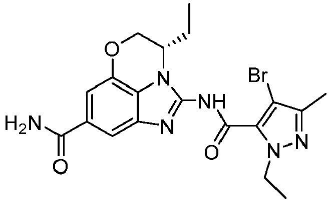 | 106 | 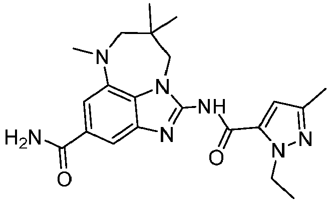 |
| 107 | 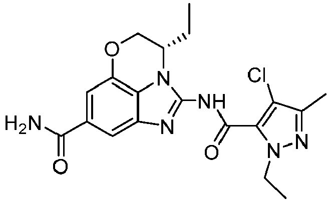 | 108 | 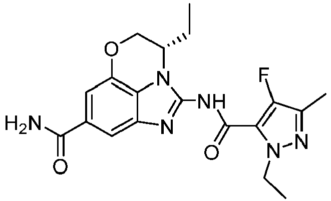 |
| 109 | 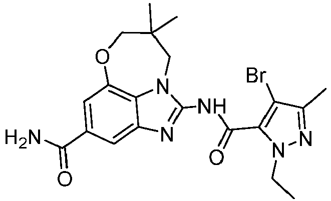 | 110 | 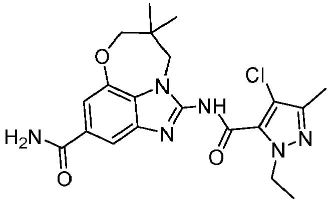 |
| 111 | 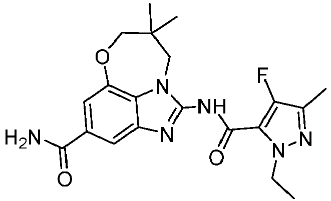 | 112 | 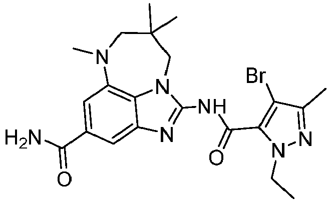 |
| 113 | 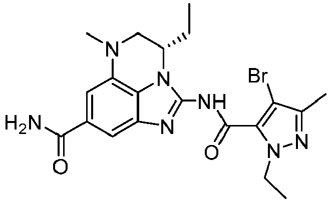 | 114 | 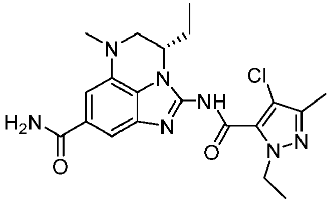 |
| 115 | 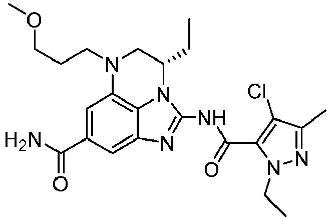 | 116 | 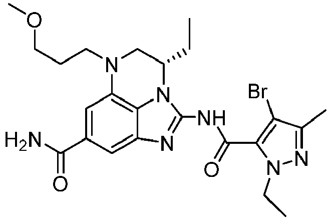 |
| 117 | 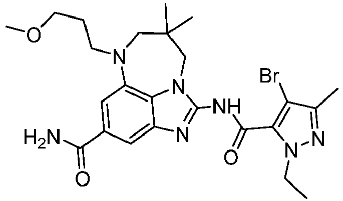 | 118 | 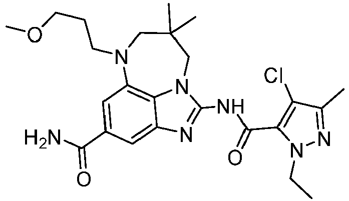 |
| 119 | 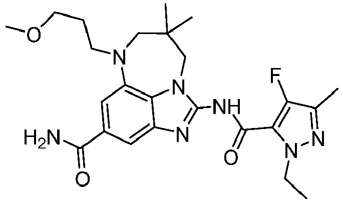 | 120 | 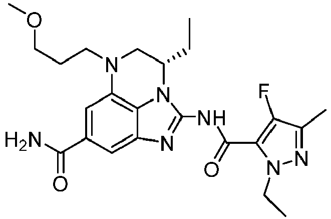 |
| 121 | 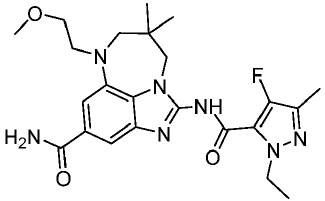 | 122 | 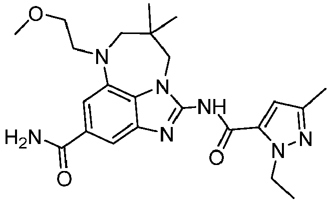 |
| 123 | 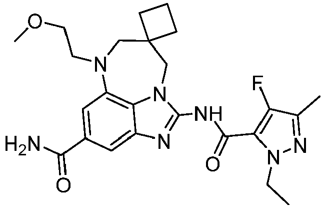 | 124 | 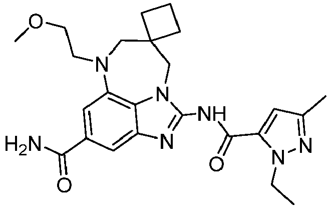 |
| 125 | 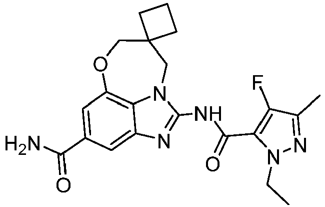 | 126 | 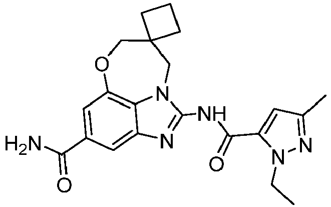 |
| 127 | 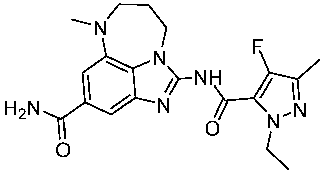 | 128 | 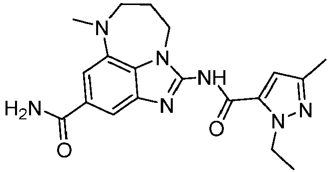 |
| 129 | 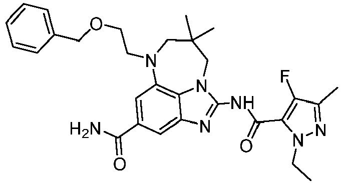 | 130 | 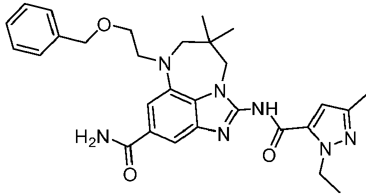 |
| 131 | 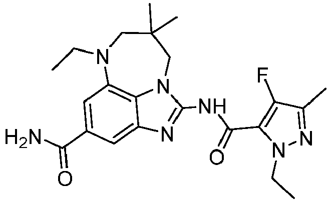 | 132 | 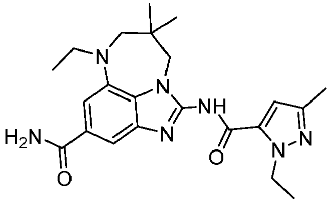 |
| 133 | 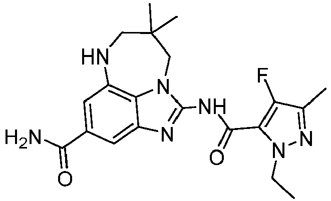 | 134 | 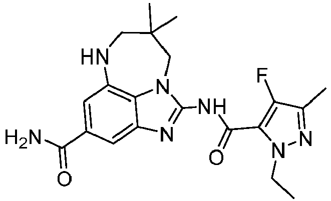 |
| 135 | 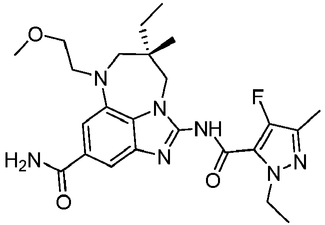 | 136 | 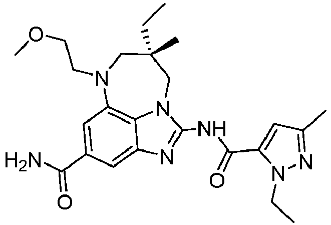 |
| 137 | 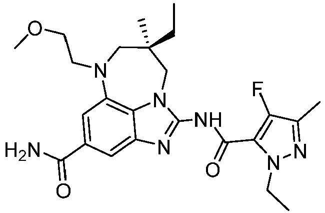 | 138 | 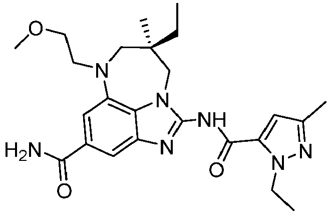 |
| 139 | 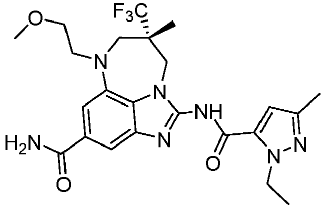 | 140 | 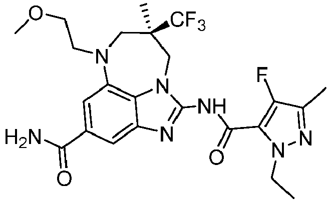 |
| 141 | 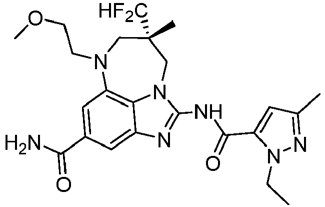 | 142 | 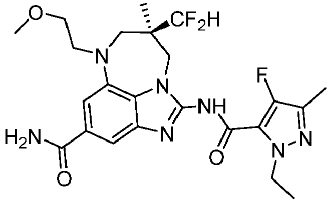 |
| 143 | 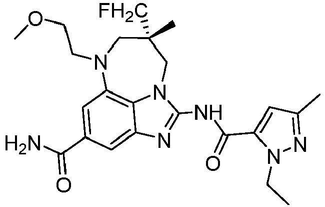 | 144 | 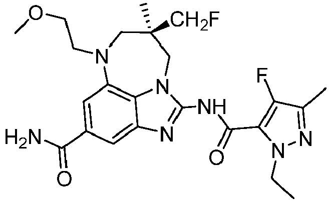 |
| 145 | 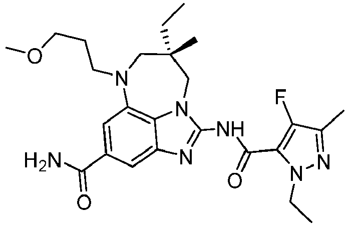 | 146 | 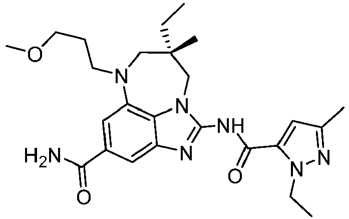 |
| 147 | 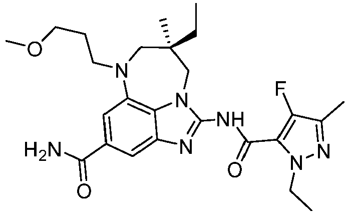 | 148 | 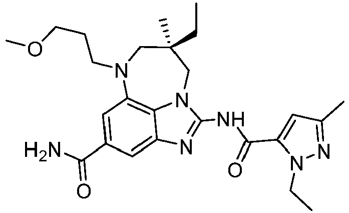 |
| 149 | 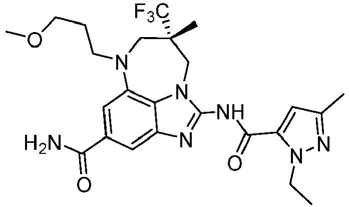 | 150 | 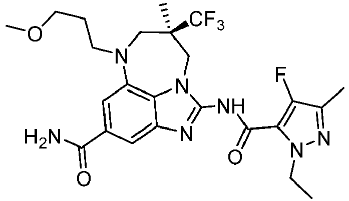 |
| 151 | 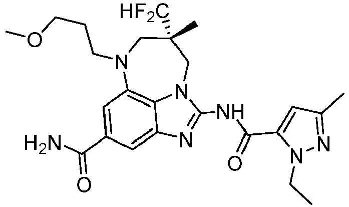 | 152 | 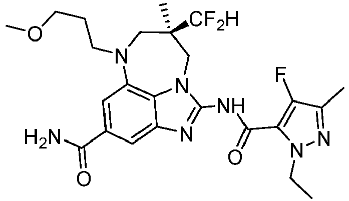 |
| 153 | 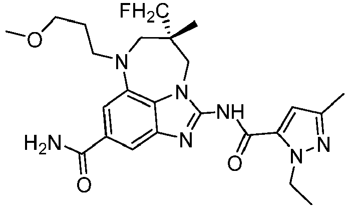 | 154 | 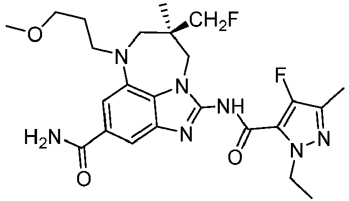 |
| 155 | 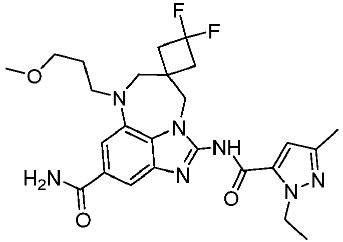 | 156 | 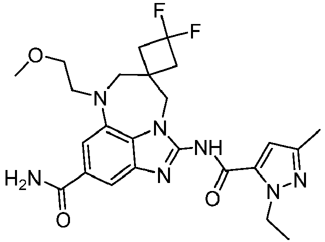 |
| 157 | 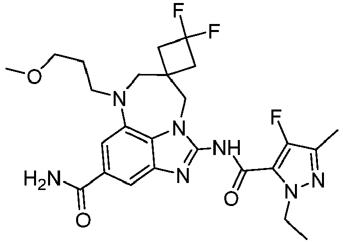 | 158 | 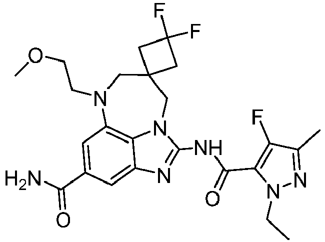 |

### Specific Examples

When no preparative route is included, related intermediates are commercially available (e.g. from Sigma Aldrich, Alfa).

### General Procedure

Commercial reagents were used without further purification. Room temperature refers to 20°C to 27°C. 1 H-NMR spectra were recorded on a Bruker instrument at 500 MHz. Chemical shift values are expressed in parts per million, i.e. δ value. The following abbreviations are used for the multiplicity of NMR signals: s = singlet, brs = broad, d = doublet, t = triplet, m = multiplet. Coupling constants were listed as J values, measured in Hz. NMR and mass spectrum results were corrected for background peaks. Chromatography refers to column chromatography performed using 100 meshes silica gel and completed under nitrogen pressure (flash chromatography). TLC used to monitor the reaction refers to TLC performed using a specific mobile phase and silica gel F254 from Merck as stationary phase.

### Example 1

### Benzyl (S)-(3-(7-carbamoyl-2-(1-ethyl-3-methyl-1H-pyrazole-5-carboxamido)-3,4-dihydro-5-oxa-1, 2a-diazaacenaphthylen-3-yl)propyl)carbamate

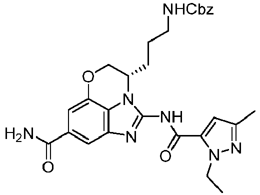

### Synthetic scheme:

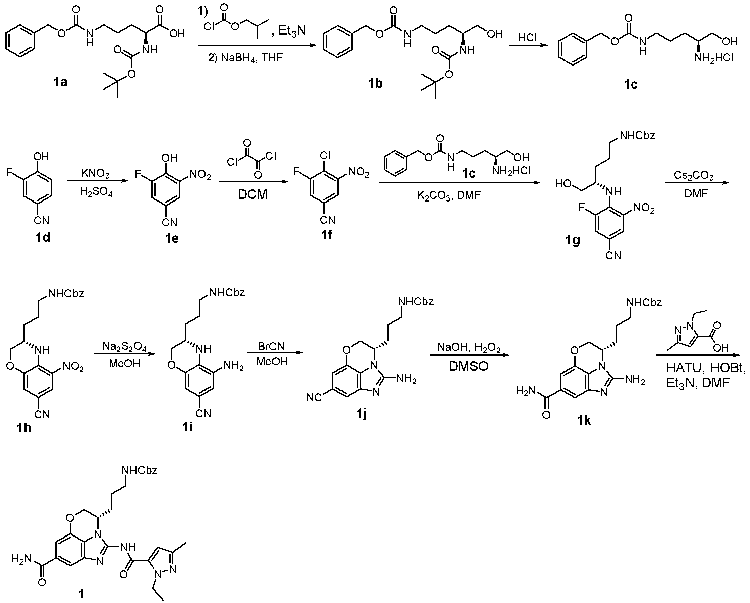

Step 1: To a stirring solution of (S)-5-(((benzyloxy)carbonyl)amino)-2-((tert-butoxycarbonyl)amino)pentanoic acid **1a** (25 g, 69 mmol) and triethylamne (11.5 mL, 81.9 mmol) in THF (100 mL) was dropwise added isobutyl chloroformate (10 mL, 79 mmol) at 0 °C. The mixture was stirred at 0° C for 30 minutes, then sodium borohydride (7.8 g, 205 mmol) was added, followed by drop-wise addition of water (3 mL) to the reaction. The mixture was kept stirring at 0 °C for 2 hours. After the reaction was complete, water (150 mL) was added to the reaction mixture, which was extracted with ethyl acetate (150 mL × 3). The combined organic layers were dried over Na₂SO₄, filtered and concentrated under reduced pressure to give the compound **1b** (20 g, 83% yield) as colorless oil. ESI-MS (m/z): 353.6 [M+H]⁺; ¹H NMR (500 MHz, DMSO-*d6*) δ ppm 7.39-7.25 (m, 5H), 7.19 (t, *J* = 5.2 Hz, 1H), 6.43 (d, *J* = 8.3 Hz, 1H), 4.98 (s, 2H), 4.53 (t, *J* = 5.4 Hz, 1H), 3.30-3.07 (m, 2H), 2.94 (dd, *J* = 12.4, 6.4 Hz, 2H), 1.57-1.39 (m, 2H), 1.35 (s, 9H), 1.23-1.12 (m, 2H).

Step 2: To a stirring solution of compound **1b** (20 g, 56 mmol) in dichloromethane (200 mL) was added 4M HCl in dioxane (70 mL, 280 mmol) at room temperature. The mixture was stirred overnight at room temperature and LCMS indicated the product was formed. The mixture was concentrated under reduced pressure to give compound 1c (13 g, 93% yiled) as colorless oil. ESI-MS (m/z): 253.6 [M+H]⁺.

Step 3: Fuming HNO₃ (50 mL) was added dropwise to a solution of 3-fluoro-4-hydroxybenzonitrile **1d** (13.7 g, 100 mmol) in conc. H₂SO₄ (200 mL) at 0 °C. The mixture was stirred at 0 °C for 3 hours. LCMS showed that the starting material was consumed. The reaction mixture was poured into ice slowly and extracted with ethyl acetate (300 mL × 3). The combined organic layers were dried over Na₂SO₄, filtered and concentrated under reduced pressure to give compound **1e** (15 g, 82% yield) as brown solid. ¹H NMR (500 MHz, DMSO-d6) δ ppm 8.29 (s, 1H), 8.12 (d, *J* = 10.4 Hz, 1H).

Step 4: Oxalyl chloride (13.7 mL, 163 mmol) was added dropwise to a solution of compound **1e** (15 g, 82 mmol) in DCM (100 mL) at 0 °C. After stirring at 0 °C for 30 minutes, the reaction was heated to 80 °C for 2 hours. The reaction mixture was allowed to cool to room temperature, poured into ice water and extracted with ethyl acetate (250 mL × 3). The combined organic layers were dried over Na₂SO₄, filtered and concentrated under reduced pressure to give compound **1f** (11.3 g, 69% yield) as yellow solid. ¹H NMR (500 MHz, DMSO-76) δ ppm 8.62 (s, 1H), 8.50 (dd, *J* = 8.8, 1.6 Hz, 1H).

Step 5: To a stirring solution of compound **1f** (5 g, 25 mmol) and compound **1c** (13 g, 51 mmol) in DMF (20 mL) was added K₂CO₃ (6.9 g, 50 mmol), and the resulting mixture was heated at 60 °C for 24 hours. The reaction mixture was cooled to room temperature, diluted with water (150 mL) and extracted with ethyl acetate (150 mL × 3). The combined organic layers were dried over Na₂SO₄, filtered and concentrated under reduced pressure, the residue was purified by silica gel chromatography to give compound **1g** (6.1 g, 57% yield) as yellow oil. ESI-MS (m/z): 417.6 [M+H]⁺.

Step 6: To a stirring solution of compound **1g** (6 g, 14 mmol) in DMF (15 mL) was added Cs₂CO₃ (9.2 g, 28 mmol), and the reaction mixture was heated at 60 °C for 2 hours. The reaction was cooled to room temperature, diluted with water (100 mL) and extracted with ethyl acetate (150 mL × 3). The combined organic layers were dried over Na₂SO₄, filtered and concentrated under reduced pressure, the residue was purified by silica gel chromatography to give compound **1h** (4.7 g, 82% yield) as yellow solid. ESI-MS (m/z): 397.7 [M+H]⁺; ¹H NMR (500 MHz, DMSO-76) δ ppm 8.88 (s, 1H), 8.12 (s, 1H), 7.38 (s, 1H), 7.36-7.19 (m, 6H), 4.97 (s, 2H), 4.10 (dd, *J* = 23.9, 10.9 Hz, 2H), 3.72 (s, 1H), 3.01 (s, 2H), 1.67-1.52 (m, 4H).

Step 7: Compound **1h** (4.7 g, 11.8 mmol) was dissolved in a mixture of MeOH (100 mL) and concentrated ammonium hydroxide (20 mL). And sodium dithionite (10 g, 57 mmol) was dissolved in water (20 mL), the resultant solution was added to the reaction mixture at room temperature. Stirring was continued at room temperature for half an hour. The reaction mixture was diluted with water (200 mL) and extracted with ethyl acetate (200 mL × 3). The combined organic layers were dried over Na₂SO₄, filtered and concentrated under reduced pressure to give compound **1i** (3.8 g, 87% yield) as light yellow solid. ESI-MS (m/z): 367.7 [M+H]⁺; ¹H NMR (500 MHz, DMSO-76) δ ppm 7.42-7.23 (m, 6H), 6.45 (dd, *J =* 11.9, 1.8 Hz, 2H), 5.45 (d, *J* = 1.6 Hz, 1H), 5.01 (s, 2H), 4.99 (s, 2H), 4.07 (dd, *J* = 10.5, 2.5 Hz, 1H), 3.71 (dd, *J* = 10.5, 6.3 Hz, 1H), 3.34 (dd, *J* = 5.9, 2.8 Hz, 1H), 3.01 (dd, *J* = 12.2, 6.2 Hz, 2H), 1.63-1.29 (m, 4H).

Step 8: To a stirring solution of compound **1i** (3.8 g, 10 mmol) in MeOH (60 mL) was added cyanogen bromide (5.4 g, 51 mmol). The resulting mixture was stirred at room temperature overnight. The mixture was concentrated in vacuo to remove the solvent, the residue was suspended in saturated aqueous Na₂CO₃ solution (150 mL) and extracted with ethyl acetate (150 mL × 3). The combined organic layers were dried over Na₂SO₄, filtered and concentrated under reduced pressure to yield compound **Ij** (3.5 g, 86% yield) as light yellow solid. ESI-MS (m/z): 392.6 [M+H]⁺.

Step 9: To a stirring solution of compound **Ij** (3.5 g, 9 mmol) in DMSO (20 mL) at 0°C was added solid NaOH (1g, 25 mmol), followed by the addition of hydrogen peroxide (30 wt. %, 12 mL). The reaction was warmed to room temperature and stirred for half an hour. The mixture was diluted with water (100 mL) and extracted with EtOAc (150 mL × 3). The combined organic layers were dried over Na₂SO₄, filtered and concentrated under reduced pressure to give compound **1k** (2.8 g, 76% yield) as yellow solid. ESI-MS (m/z): 410.5 [M+H]⁺.

Step 10: To a stirring solution of 1-ethyl-3-methyl-1H-pyrazole-5-carboxylic acid (1.6 g, 10.3 mmol) in DMF (8 mL) was added HATU (3.9 g, 10.3 mmol), HOBt (700 mg, 5.2 mmol) and triethylamine (2.8 mL, 20 mmol). The mixture was stirred at room temperature for half an hour, then compound **1k** (2.8 g, 6.8 mmol) was added. The resulting mixture was heated to 60°C for 5 hours. After cooled down to toom temperature, the mixture diluted with water (40 mL) and extracted with ethyl acetate (100 mL × 3). The combined organic layers were dried over Na₂SO₄, filtered and concentrated under reduced pressure. The residue was purified by silica gel chromatography to give compound **1l** (2.6 g, 70% yield) as white solid. ESI-MS (m/z): 546.4 [M+H]⁺; ¹H NMR (500 MHz, DMSO-*d6*) δ ppm 12.71 (s, 1H), 7.90 (s, 1H), 7.57 (s, 1H), 7.34 - 7.21 (m, 8H), 6.62 (s, 1H), 4.95 (s, 2H), 4.66 - 4.53 (m, 4H), 4.23 (d, *J* = 9.5 Hz, 1H), 3.08 - 2.96 (m, 2H), 2.15 (s, 3H), 1.81 - 1.72 (m, 2H), 1.62 - 1.52 (m, 2H), 1.34 (t, *J* = 7.1 Hz, 3H).

### Example 2

### Benzyl (R)-(3-(7-carbamoyl-2-(1-ethyl-3-methyl-1H-pyrazole-5-carboxamido)-3,4-dihydro-5-oxa-1, 2a-diazaacenaphthylen-3-yl)propyl)carbamate

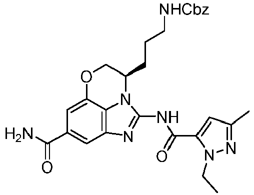

### Synthetic scheme:

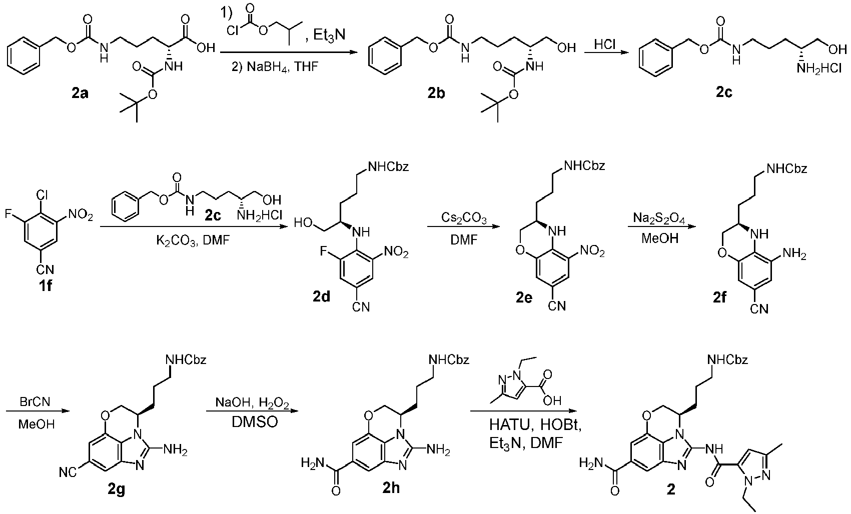

**Step 1:** To a stirring solution of (R)-5-(((benzyloxy)carbonyl)amino)-2-((tert-butoxycarbonyl)amino)pentanoic acid **2a** (25 g, 68 mmol) and triethylamne (11.5 mL, 81.9 mmol) in THF (100 mL) was dropwise added isobutyl carbonochloridate (10 mL, 79 mmol) at 0°C. The mixture was stirred at 0°C for 30 minutes, then sodium borohydride (7.8 g, 205 mmol) was added, followed by drop-wise addition of water (3 mL) to the reaction. The mixture was kept stirring at 0 °C for 2 hours. After the reaction was complete, water (150 mL) was added to the reaction mixture, which was extracted with ethyl acetate (150 mL × 3). The combined organic layers were dried over Na₂SO₄, filtered and concentrated under reduced pressure to give the compound **2b** (22 g, 91% yield) as colorless oil. ESI-MS (m/z): 353.6 [M+H]⁺.

**Step 2:** To a stirring solution of compound **1b** (22 g, 56 mmol) in dichloromethane (200 mL) was added 4M HCl in dioxane (75 mL, 300 mmol) at room temperature. The mixture was stirred overnight at rom temperature and LCMS indicated the product was formed. The mixture was concentrated under reduced pressure to give compound **2c** (13.5 g, 86% yiled) as colorless oil. ESI-MS (m/z): 253.6 [M+H]⁺.

**Step 3:** To a stirring solution of compound **If** (5.3 g, 26.5 mmol) and compound **2c** (13.4 g, 53 mmol) in DMF (20 mL) was added K₂CO₃ (7.4 g, 54 mmol), and the resulting mixture was heated at 60°C overnight. The reaction mixture was cooled to room temperature, diluted with water (150 mL) and extracted with ethyl acetate (150 mL × 3). The combined organic layers were dried over Na₂SO₄, filtered and concentrated under reduced pressure, the residue was purified by silica gel chromatography to give compound **2d** (6.9 g, 63% yield) as yellow oil. ESI-MS (m/z): 417.6 [M+H]⁺.

**Step 4:** To a stirring solution of compound **2d** (6.9 g, 16.5 mmol) in DMF (15 mL) was added Cs₂CO₃ (10.8 g, 33 mmol), and the reaction mixture was heated at 60 °C for 2 hours. The reaction was cooled to room temperature, diluted with water (150 mL) and extracted with ethyl acetate (150 mL × 3). The combined organic layers were dried over Na₂SO₄, filtered and concentrated under reduced pressure, the residue was purified by silica gel chromatography to give compound **2e** (4.4 g, 67 % yield) as yellow solid. ESI-MS (m/z): 397.7 [M+H]⁺.

**Step 5:** Compound **2e** (4.4 g, 11.1 mmol) was dissolved in a mixture of MeOH (100 mL) and concentrated ammonium hydroxide (20 mL). And sodium dithionite (9.6 g, 55 mmol) was dissolved in water (20 mL), the resultant solution was added to the reaction mixture at room temperature. Stirring was continued at room temperature for half an hour. The reaction mixture was diluted with water (200 mL) and extracted with ethyl acetate (200 mL × 3). The combined organic layers were dried over Na₂SO₄, filtered and concentrated under reduced pressure to give compound **2f** (3.5 g, 86% yield) as light yellow solid. ESI-MS (m/z): 367.7 [M+H]⁺.

**Step 6:** To a stirring solution of compound **2f** (3.5 g, 9.5 mmol) in MeOH (60 mL) was added cyanogen bromide (5.05 g, 47.6 mmol). The resulting mixture was stirred at 60 °C overnight. The mixture was concentrated in vacuo to remove the solvent, the residue was suspended in saturated aqueous Na₂CO₃ solution (150 mL) and extracted with ethyl acetate (150 mL × 3). The combined organic layers were dried over Na₂SO₄, filtered and concentrated under reduced pressure to yield compound **2g** (3.4 g, 91 % yield) as light yellow solid. ESI-MS (m/z): 392.6 [M+H]⁺.

**Step 7:** To a stirring solution of compound **2g** (3.4 g, 8.7 mmol) in DMSO (20 mL) at 0°C was added solid NaOH (1g, 25 mmol), followed by the addition of hydrogen peroxide (30 wt. %, 12 mL). The reaction was warmed to room temperature and stirred for half an hour. The mixture was diluted with water (100 mL) and extracted with EA (150 mL × 3). The combined organic layers were dried over Na₂SO₄, filtered and concentrated under reduced pressure to give compound **2h** (2.7 g, 76% yield) as yellow solid. ESI-MS (m/z): 410.5 [M+H]⁺.

**Step 8:** To a stirring solution of 1-ethyl-3-methyl-1H-pyrazole-5-carboxylic acid (1.5 g, 9.7 mmol) in DMF (8 mL) was added HATU (3.8 g, 10 mmol), HOBt (670 mg, 5 mmol) and triethylamine (2.8 mL, 20 mmol). The mixture was stirred at room temperature for half an hour, then compound **2h** (2.7 g, 6.6 mmol) was added. The resulting mixture was heated to 60°C for 5 hours. After cooled down to toom temperature, the mixture diluted with water (35 mL) and extracted with ethyl acetate (100 mL × 3). The combined organic layers were dried over Na₂SO₄, filtered and concentrated under reduced pressure. The residue was purified by silica gel chromatography to give compound **2** (2.8 g, 77% yield) as white solid. ESI-MS (m/z): 546.4 [M+H]⁺; ¹H NMR (500 MHz, DMSO-d6) δ 12.66 (s, 1H), 7.92 (s, 1H), 7.60 (s, 1H), 7.38-7.21 (m, 8H), 6.63 (s,1H), 4.96 (s, 2H), 4.62 (dd, *J* = 17.3, 8.8 Hz, 4H), 4.30-4.19 (m, 1H), 3.11-2.96 (m, 2H), 2.17 (s, 3H),1.83-1.74 (m, 2H), 1.59-1.52 (m, 2H), 1.35 (t, *J* = 7.0 Hz, 3H).

### Example 3

### 2-(1-Ethyl-3-methyl-1H-pyrazole-5-carboxamido)-3-(hydroxymethyl)-3-methyl-3,4-dihydro -5-oxa-1,2a-diazaacenaphthylene-7-carboxamide

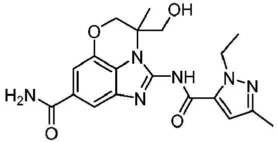

### Synthetic scheme:

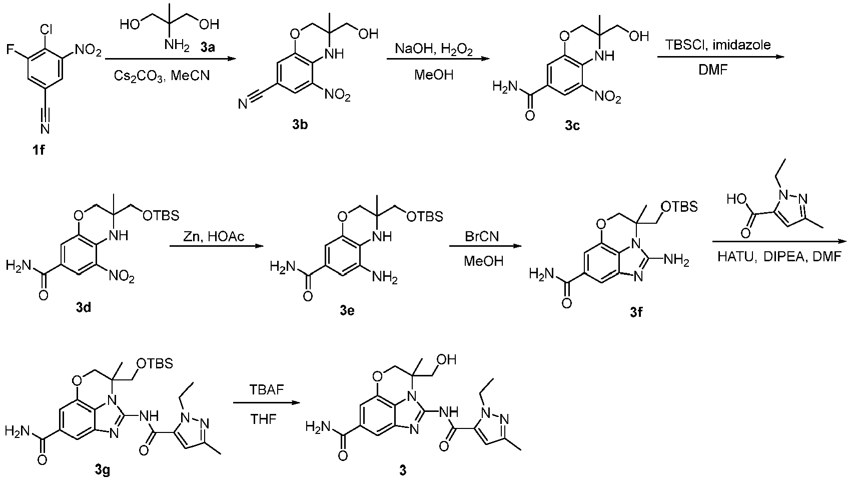

Step 1: To a stirring solution of compound **1f** (10.0 g, 49.86 mmol) and compound **3a** (26.21 g, 249.31 mmol) in acetonitrile (100 mL) was added Cs₂CO₃ (24.37 g, 74.79 mmol). The reaction mixture was heated at 70°C for 7 hours. The reaction mixture was allowed to cool to room temperature, diluted with water (400 mL) and extracted with ethyl acetate (300 mL × 3). The combined organic layers were washed with brine (100 mL × 3), dried over Na₂SO₄, filtered and concentrated under reduced pressure. The residue was purified by silica gel chromatography to give compound **3b** (8 g, 64% yield) as yellow solid. ESI-MS (m/z): 499.3 [2M+H]⁺; ¹H NMR (500 MHz, DMSO-*d6*) δ ppm 8.43(s, 1H), 8.13 (s, 1H), 7.43 (s, 1H), 5.27 (s, 1H), 4.11 (d, *J* = 10.8 Hz, 1H), 3.87 (d, *J* = 10.8 Hz, 1H), 3.45 (d, *J* = 10.6 Hz, 1H), 3.42-3.35 (m, 1H), 1.25 (s, 3H).

Step 2: To a stirring solution of compound **3b** (8 g, 31.20 mmol) in MeOH (80 mL) was added solid NaOH (3.85 g, 96.30 mmol). The reaction mixture was heated at 50 °C, and hydrogen peroxide (30 wt. %, 5.46 g, 48.15 mmol) was added dropwise into the reaction mixture. The mixture was stirred at 50 °C for 2 hours, then cooled to room temperature. The reaction mixture was diluted with water (100 mL) and extracted with EtOAc (200 mL × 3). The combined organic layers were washed with brine (100 mL × 3), dried over Na₂SO₄, filtered and concentrated. The residue was purified by silica gel chromatography to give compound **3c** (8 g, 89% yield) as yellow solid. ESI-MS (m/z): 268.5 [M+H]⁺; ¹H NMR (500 MHz, CDCl₃) δ 8.28 (d, *J* = 2.0 Hz, 1H), 8.18 (s, 1H), 7.95 (s, 1H), 7.55 (d, *J* = 1.6 Hz, 1H), 7.28 (s, 1H), 5.23 (t, *J* = 5.5 Hz, 1H), 4.10 (d, *J* = 10.8 Hz, 1H), 3.85 (d, *J* = 10.8 Hz, 1H), 3.45 (dd, *J* = 10.7, 5.1 Hz, 1H), 3.38 (dd, *J* = 10.7, 5.4 Hz, 1H), 1.25 (s, 3H).

Step 3: To a solution of compound **3c** (8 g, 29.94 mmol) in DMF (80 mL) at 0 °C was added imidazole (8.15 g, 119.74 mmol) and TBSCl (13.54 g, 89.81 mmol). The reaction mixture was stirred at 0 °C for 2 hours, then diluted with water (300 mL), extracted with EtOAc (200 mL × 3). The combined organic layers were washed with brine (100 mL × 3), dried over Na₂SO₄, filtered and concentrated. The residue was purified by silica gel colomn chromatography to give compound **3d** (5.6 g, 49% yield) as yellow solid. ESI-MS (m/z): 382.5 [M+H]⁺; ¹H NMR (500 MHz, DMSO-*d6*) δ 8.27 (d, *J* = 2.0 Hz, 1H), 8.21 (s, 1H), 7.95 (s, 1H), 7.55 (d, *J* = 1.9 Hz, 1H), 7.30 (s, 1H), 4.09 (d, *J* = 10.8 Hz, 1H), 3.85 (d, *J* = 10.8 Hz, 1H), 3.60 (d, *J* = 9.8 Hz, 1H), 3.54 (d, *J* = 9.8 Hz, 1H), 0.84 (s, 9H), 0.01 (s, 3H), 0.00 (s, 3H).

Step 4: Compound **3d** (2 g, 5.24 mmol) was dissolved in acetic acid (20 mL), and Zn powder (2.74 g, 14.94 mmol) was added by portions at room temperature. The reaction mixture was heated at 50 °C for 1 hour, then cooled to room temperature. The mixture was filtered through a pad of celite, and washed with EtOAc (50 mL × 3). The filtrate was concentrated, the adjusted to pH 7 with NaHCO3 solution. The resulting mixture was extracted with EtOAc (100 × 3), the combined organic layers were washed with brine (50 mL × 3), dried over Na₂SO₄, filtered and concentrated to give crude 3e (1.8 g) as yellow solid, which was used directly without further purification. ESI-MS (m/z): 352.5 [M+H]⁺.

Step 5: Compound **3e** (1.8 g, from step 4) was dissolved in MeOH (20 mL), and cyanogen bromide (2.78 g, 26.20 mmol) was added by portions. The resulting mixture was heated at 50 °C for 2 hours, then cooled to room temperature. The mixture was concentrated in vacuo to remove the solvent. The residue was suspended in water (100 mL), and adjusted to pH 7 with saturated NaHCO₃ solution, then extracted with EtOAc (100 mL × 3). The combined organic layers were washed with brine (50 mL × 3), dried over Na₂SO₄, filtered and concentrated. The residue was purified by silica gel chromatography to give compound **3f** (1.7 g, 86% yield for 2 steps) as brown solid. ESI-MS (m/z): 377.5 [M+H]⁺; ¹H NMR (500 MHz, DMSO-*d6*) δ ppm 7.70 (s, 1H), 7.35 (s, 1H), 6.99 (d, *J* = 12.1 Hz, 2H), 6.37 (s, 2H), 4.24 (d, *J* = 11.5 Hz, 1H), 4.00 (dd, *J* = 14.3, 7.1 Hz, 2H), 3.92 (d, *J* = 11.5 Hz, 1H), 3.75 (s, 2H), 1.47 (d, *J* = 11.4 Hz, 4H), 0.77 (d, *J* = 18.5 Hz, 10H), 0.02 - -0.03 (m, 4H), -0.06 (s, 3H).

Step 6: To a stirring solution of **3f** (1.5 g, 4.02 mmol) and 1-ethyl-3-methyl-1H-pyrazole-5-carboxylic acid (990.53 mg, 6.43 mmol) in DMF (8 mL) was added HATU (3.05 g, 8.03 mmol) and DEPEA (1.56 g, 12.05 mmol). The mixture was heated at 60 °C for 2 hours, then cooled to room temperature. The mixture diluted with water (20 mL) and extracted with EtOAc (50 mL × 3). The combined organic layers were washed with brine (50 mL × 3), dried over Na₂SO₄, filtered and concentrated under reduced pressure. The residue was purified by silica gel chromatography to give compound **3g** (1.75 g, 85% yield) as white solid. ESI-MS (m/z): 513.4 [M+H]⁺.

Step 7: To a solution of compound **3g** (990 mg, 1.93 mmol) in THF (15 mL) at 0 °C was added TBAF (1.43 g, 6.36 mmol). The reaction mixture was stirred at 0 °C for 3 hours. The reaction mixture was diluted with water (20 mL) and extracted with EtOAc (50 mL × 3). The combined organic layers were washed with brine (50 mL x3), dried over Na₂SO₄, filtered and concentrated. The residue was purified by silica gel colomn chromatography to give product **3** (693 mg, 90% yield) as white solid. ESI-MS (m/z): 398.5 [M+H]⁺; ¹H NMR (500 MHz, DMSO-d6) δ ppm 12.77 (s, 1H), 7.89 (s, 1H), 7.55 (d, *J* = 1.0 Hz, 1H), 7.28 (t, *J* = 2.5 Hz,2H), 6.56 (s, 1H), 5.60 (t, *J* = 6.0 Hz, 1H), 4.56 (q, *J* = 7.1 Hz, 2H), 4.37 (d, J = 11.6 Hz, 1H), 4.17 (d, *J* =11.6 Hz, 1H), 3.90 (dd, *J* = 11.3, 6.7 Hz, 1H), 3.78 (dd, *J* = 11.3, 5.4 Hz, 1H), 2.16 (s, 3H), 1.60 (s, 3H),1.32 (t, *J* = 7.1 Hz, 3H).

### Example 4

### tert-Butyl (S)-(3-(7-carbamoyl-2-(1-ethyl-3-methyl-1H-pyrazole-5-carboxamido)-3,4-dihydro-5-oxa-1, 2a-diazaacenaphthylen-3-yl)propyl)carbamate

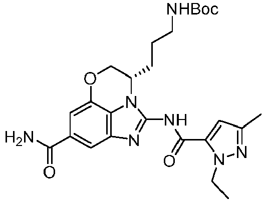

### Synthetic scheme:

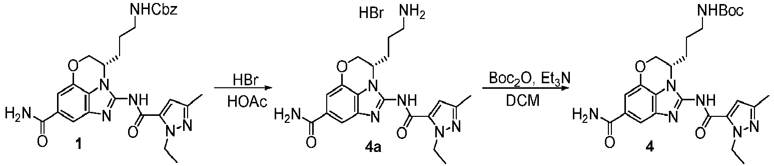

**Step 1:** Compound **1** (2.5 g, 4.6 mmol) was dissolved in hydrobromic acid in acetic acid solution (33wt. %, 50 mL), the resulting mixture was stirred at room temperature for half an hour. LCMS indicated the reaction was complete. Diethyl ether (50 mL) was added to the reaction mixture, the formed solid was collected by filtration. The solid was washed by diethyl ether (30 mL × 3) and dried in vacuo to give compound **4a** (1.8 g, 96% yield) as a white solid. ESI-MS (m/z): 412.6 [M+H]⁺.

Step 2: To a solution of compound **4a** (50 mg, 0.12 mmol) in DCM (10 mL) was added triethylamine (25 mg, 0.25 mmol) and Boc₂O (53 mg, 0.24 mmol), the solution was stirred at room temperature for 2 hours. LCMS indicated the starting material was consumed. The reaction mixture was concentrated, the residue was purified by reversed phase preparative HPLC to give compound **4** (40 mg, 64% yield) as a white solid. ESI-MS (m/z): 512.6 [M+H]⁺; ¹H NMR (500 MHz, DMSO-76) δ ppm 12.76 (s, 1H), 7.93 (s, 1H), 7.59 (s, 1H), 7.34 (s, 1H), 7.32 (s, 1H), 6.80(s, 1H), 6.65 (s, 1H), 4.68-4.54 (m, 4H), 4.29-4.23 (m, 1H), 3.01-2.84 (m, 2H), 2.18 (s, 3H), 1.83-1.71 (m, 2H), 1.53 (d, *J* = 6.5 Hz, 2H), 1.36 (t, *J* = 7.1 Hz, 3H), 1.32 (s, 9H).

### Example 5

### Ethyl (E)-3-(7-carbamoyl-2-(1-ethyl-3-methyl-1H-pyrazole-5-carboxamido)-3-methyl-3,4-dihydro -5-oxa-1,2a-diazaacenaphthylen-3-yl)acrylate

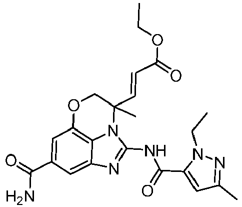

### Synthetic scheme:

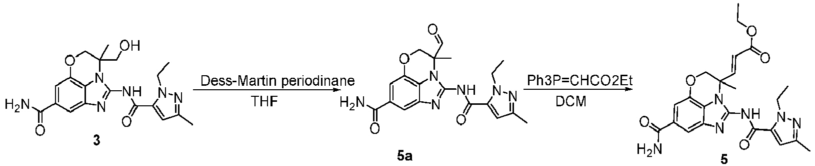

Step 1: To a solution of compound **3** (100 mg, 0.25 mmol) in THF (5 mL) at 0 °C was added Dess-Martin periodinane (212 mg, 0.5 mmol) by portions. The mixture was stirred at room temperature for 2 hours, then diluted with water (20 mL) and extracted with EtOAc (50 mL × 3). The combined organic layers were dried over Na₂SO₄, filtered and concentrated to give crude compound **5a,** which was used directly without further purification

Step 2: To a solution of compound **5a** (crude sample from step 1) in DCM (5 mL) at 0 °C was added ethyl (triphenylphosphoranylidene) acetate (131 mg, 0.37mmol) by portions. The mixture was stirred at room temperature for 12 hours, then diluted with water (10 mL) and extracted with EA (50 mL × 3). The combined organic layers were dried over Na₂SO₄, filtered and concentratedand. The residue was purified by reversed phase preparative HPLC to give the compound **5** (13 mg, 11% yield for 2 steps) as white solid.ESI-MS (m/z): 467.5 [M+H]⁺; ¹H NMR (500 MHz, DMSO-76) δ ppm 12.85 (s, 1H), 7.94 (s, 1H), 7.61 (s, 1H), 7.34 (s, 2H), 7.25 (dd, *J* = 15.9,3.0 Hz, 1H), 6.52 (s, 1H), 5.65 (dd, *J* = 15.9, 3.0 Hz, 1H), 4.67 - 4.48 (m, 3H), 4.27 (d, *J* = 11.5 Hz, 1H),4.11 (dd, *J* = 14.0, 7.0 Hz, 2H), 2.15 (s, 3H), 1.89 (s, 3H), 1.32 (t, *J* = 7.0 Hz, 3H), 1.18 (t, *J* = 7.0 Hz, 3H).

### Example 6

### tert-Butyl (S)-(2-(7-carbamoyl-2-(1-ethyl-3-methyl-1H-pyrazole-5-carboxamido)-3,4-dihydro-5-oxa-1, 2a-diazaacenaphthylen-3-yl)ethyl)carbamate

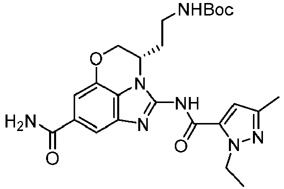

### Synthetic scheme:

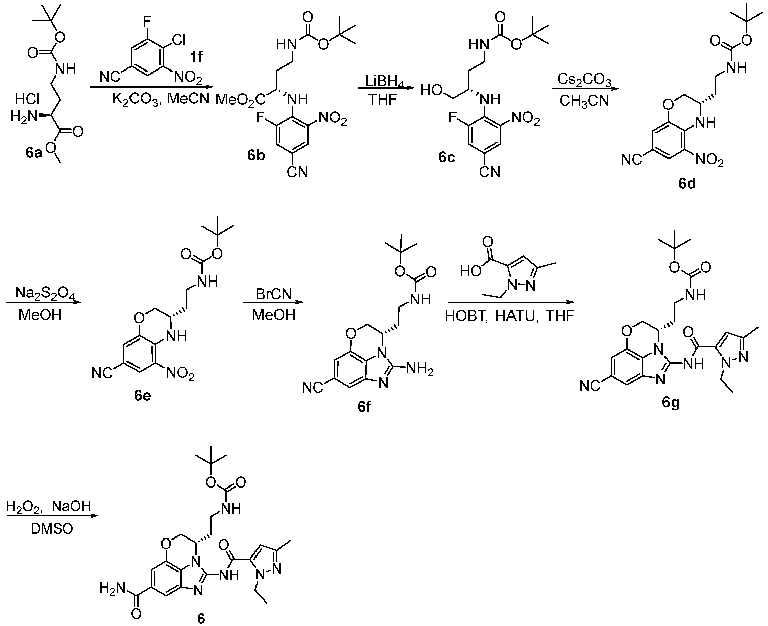

**Step 1:** To a stirring solution of compound **1f** (4 g, 20 mmol) and compound **6a** (10 g, 37.21 mmol) in acetonitrile (150 mL) was added K₂CO₃ (8.3 g, 60 mmol), and the resulting mixture was heated at 70°C for 24 hours. TLC indicated the starting material was consumed. The reaction mixture was cooled to room temperature, filtered and washed with dichloromethane (100 mL), then concentrated under reduced pressure. The residue was purified by silica gel chromatography to give compound **6b** (4.3 g, 54.4% yield) as yellow oil. ESI-MS (m/z): 397.5 [M+H]⁺.

**Step 2:** To a solution of compound **6b** (4.30 g, 10.85 mmol) in anhydrous THF (40 mL) at 0 °C was added by portion lithium borohydride (354 mg, 16.27 mmol), and the resulting solution was stirred at room temperature for half an hour. TLC indicated the starting material was consumed. The reaction was slowly quenched with aqueous NHaCl (10 mL), diluted with water (50 mL), and extracted with ethyl acetate (50 mL × 3). The combined organic layers were washed with brine, dried over Na₂SO₄, filtered and concentrated. The residue was purified by silica gel chromatography to give the compound **6c** (2.3 g, 57.5% yield). ESI-MS (m/z): 369.5[M+H]⁺.

**Step 3:** To a stirring solution of compound **6c** (2.3 g, 6.24 mmol) in acetonitrile (20 mL) was added Cs₂CO₃ (4 g, 12.31 mmol), and the reaction mixture was heated at 70 °C overnight. LCMS indicated the starting material was consumed. The reaction mixture was cooled to room temperature, filtered and washed with dichloromethane, then concentrated under reduced pressure to give compound **6d** (2 g, 90% yield) as a brown oil. ESI-LC-MS (m/z): 349.4 [M+H]⁺.

**Step 4:** Compound **6d** (2 g, 5.75 mmol) was dissolved in a mixture of MeOH (20 mL) and concentrated ammonium hydroxide (5 mL). Sodium dithionite (5.7 g, 32.74 mmol) was dissolved in water (2 mL) and added to the reaction mixture at room temperature. Stirring was continued at room temperature for half an hour, LCMS indicated the product was formed. The reaction mixture was diluted with water (50 mL) and extracted with ethyl acetate (50 mL × 3). The combined organic layers were washed with brine, dried over Na₂SO₄, filtered and concentrated under reduced pressure to give compound **6e** (530 mg, 30% yield) as brown oil. ESI-MS (m/z): 319.6 [M+H]⁺.

**Step 5:** Compound **7e** (530 mg. 1.67 mmol) was dissolved in MeOH (10 mL), and cyanogen bromide (550 mg, 5 mmol) was added. The resulting mixture was stirred at room temperature overnight. LCMS indicated the reaction was complete. The mixture was concentrated in vacuo to remove the solvent. The residue was purified by silica gel chromatography to give the compound **6f** (550 mg, 96.2 % yield) as brown solid. ESI-MS (m/z): 344.5 [M+H]⁺; ¹H NMR (500 MHz, DMSO-*d6*) δ 7.18 (d, *J* = 1.2 Hz, 1H), 7.01 (s, 2H), 6.88 (t, *J* = 5.7 Hz, 1H), 6.86 (d, *J* = 1.2 Hz, 1H), 4.65-4.55(m,2H),4.18-4.12(m, 1H),3.10-3.00 (m, 2H), 1.79-1.68 (m, 2H), 1.37 (s, 9H).

**Step 6:** To a stirring solution of 1-ethyl-3-methyl-1H-pyrazole-5-carboxylic acid (250 mg, 1.60 mmol) and compound **6f** (550 mg, 1.60 mmol) in THF (10 mL) was added HATU (617 mg, 1.60 mmol), HOBt (219 mg, 1.60 mmol) and triethylamine (0.67 mL, 4.86 mmol). After stirred at room temperature for half an hour, compound **6f** (550 mg, 1.60 mmol) was added to the reaction. The resulting mixture was stirred at room temperature overnight, LCMS indicated the product was formed. The mixture was diluted with water (20 mL) and extracted with ethyl acetate (15 mL × 3). The combined organic layers were washed with brine, dried over Na₂SO₄ and concentrated to give compound **7g** (700 mg, 90% yield) as brown oil. ESI-MS (m/z): 480.6[M+H]⁺.

**Step 7:** To a stirring solution of compound **6g** (20 mg, 41.71 umol) in DMSO (2 mL) at 0°C was added solid NaOH (5 mg, 125.12 umol), followed by the addition of hydrogen peroxide (30 wt. %, 0.2 mL). The reaction was warmed to room temperature and stirred for half an hour, LCMS indicated the product was formed. The reaction mixture was purified directly by reversed phase preparative HPLC to give compound **6** (6.8 mg, 32.8% yield) as white solid. ESI-MS (m/z): 498.6 [M+H]⁺; ¹H NMR (500 MHz, DMSO-*d6*) δ ppm 12.69 (s, 1H), 7.92 (s, 1H), 7.59 (s, 1H), 7.34 (s, 1H), 7.30 (s, 1H), 6.94 (s, 1H), 6.68 (s, 1H), 4.63 (dt, *J* = 14.0, 8.7 Hz, 4H), 4.26 (d, *J* = 9.9 Hz, 1H), 3.21-2.96 (m, 2H), 2.18 (s, 3H), 1.90 (d, *J* = 7.0 Hz, 2H), 1.36 (d, *J* = 8.1 Hz, 9H), 1.35 (d, *J* = 7.1 Hz, 3H).

### Example 7

### (S)-2-(1-Ethyl-3-methyl-1H-pyrazole-5-carboxamido)-3-(2-(2-phenoxyacetamido)ethyl)-3,4-dihydro-5-oxa-1,2a-diazaacenaphthylene-7-carboxamide

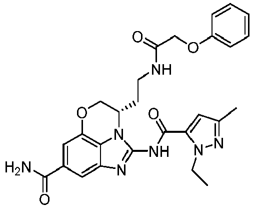

### Synthetic scheme:

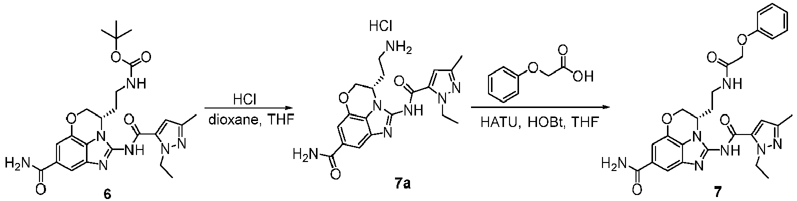

Step 1: To a stirring solution of compound **6** (530 mg, 1.07 mmol) in THF (10 mL) was added 4M HCl in dioxane (10 mL, 40 mmol) at room temperature. The mixture was stirred overnight at rom temperature and LCMS indicated the product was formed. The mixture was concentrated under reduced pressure to give compound **7a** (430 mg, 93% yiled) as yellow solid, which was used directly withour further purification. ESI-MS (m/z): 396.6 [M+H]⁺.

Step 2: To a stirring solution of **7a** (50 mg, 115.24 umol) and 2-phenoxyacetic acid (17.5 mg, 115.24 umol) in DMF (60 mL) was added HATU (48.2 mg, 126.76 umol), HOBt (17.1 mg, 126.76 umol) and triethylamine (35 mg, 345.71 umol). The mixture was stirred at room temperature overnight, then purified directly by reversed phase preparative HPLC to give compound **7** (13.8 mg, 22.53% yield) as white solid. ESI-LC-MS (m/z): 532.5 [M+H]⁺; ¹HNMR (500MHz, DMSO-*d6*) δ ppm 12.69 (s, 1H), 8.23 (t, *J* = 5.8Hz,1H), 7.92 (s, 1H), 7.34 (s,1H), 7.32-7.25 (m, 3H), 6.95 (t, *J* = 7.8 Hz, 3H), 6.68 (s, 1H), 4.70-4.56 (m, 4H), 4.45 (s, 2H), 4.26 (d, *J* = 9.7Hz, 1H), 3.38 (d, *J* = 7.2Hz, 2H) ,2.09 (s, 3H), 1.96 (dt, *J* = 14.4, 7.2 Hz, 2H), 1.34 (t, *J* = 7.1 Hz, 3H).

### Example 8

### Pyridin-3-ylmethyl (S)-(2-(7-carbamoyl-2-(1-ethyl-3-methyl-1H-pyrazole-5-carboxamido)-3,4-dihydro-5-oxa-1, 2a-diazaacenaphthylen-3-yl)ethyl)carbamate

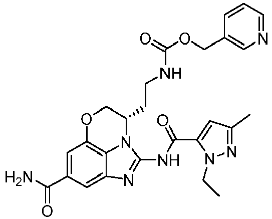

### Synthetic scheme:

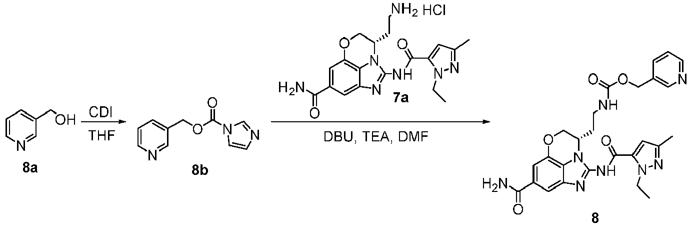

**Step 1:** To a solution of CDI (1.5 g, 9.25 mmol) in dry THF (15 mL) was added dropwise compound **8a** (1 g, 9.17 mmol) in THF (2 mL), the mixture was then stirred at room temperature for 2 hours. TLC indicated the reaction was complete. The reaction mixture was poured into water (30 mL), extracted with DCM (15 mL × 2). The combined organic layers were washed with brine, dried over Na₂SO₄, filtered and concentrated. The residue was purified by silica gel column chromatography to give compound **8b** (1.2 g, 64% yield) as white solid. ESI-LC-MS (m/z): 204.6 [M+H]⁺.

**Step 2:** To a solution of compound **7a** (30 mg, 69.14 umol) in DMF (3 mL) was added triethylamine (21 mg, 207.43 umol), DBU (21 mg, 138.28 umol) and compound **8b** (28.1 mg, 138.28 umol), the reaction mixture was heated at 50 °C overnight, LCMS indicated the reaction was complete. The reaction mixture was purified directly by reversed phase preparative HPLC to give compound **8** (12.6 mg, 34.22 % yield) as a white solid. ESI-LC-MS (m/z): 533.5 [M+H]⁺; ¹H NMR (500 MHz, DMSO-d6) δ ppm 12.73 (s, 1H), 8.56 (s,1H), 8.52 (d, *J* = 3.6 Hz, 1H), 7.92 (s, 1H), 7.74 (d, *J* = 7.9 Hz, 1H), 7.59 (s, 1H), 7.39(dd, *J* = 12.7, 7.4 Hz, 2H), 7.34 (s, 1H), 7.31 (s, 1H), 6.67 (s,1H), 5.06 (q, *J* = 12.8 Hz, 2H), 4.73-4.64 (m, 2H), 4.63-4.54 (m,2H), 4.27 (d, *J* = 10.9 Hz, 1H), 3.27-3.20 (m, 1H), 3.20-3.08 (m, 1H), 2.09 (s, 3H), 1.94 (d, *J* = 6.4 Hz, 2H), 1.34 (t, *J* = 7.1 Hz, 3H).

### Example 9

### Pyridin-2-ylmethyl (S)-(2-(7-carbamoyl-2-(1-ethyl-3-methyl-1H-pyrazole-5-carboxamido)-3,4-dihydro-5-oxa-1, 2a-diazaacenaphthylen-3-yl)ethyl)carbamate

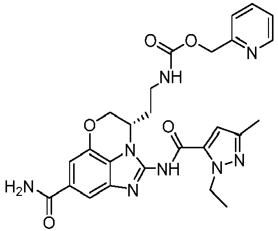

### Synthetic scheme:

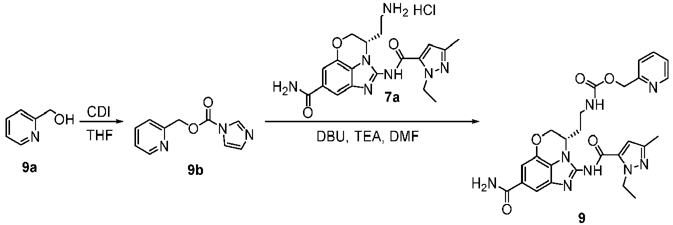

Step 1: To a solution of CDI (750 mg, 4.63 mmol) in dry THF (10 mL) was added dropwise compound **9a** (500 mg, 4.6 mmol) in THF (1 mL), the mixture was then stirred at room temperature for 2 hours. TLC indicated the reaction was complete. The reaction mixture was poured into water (20 mL), extracted with DCM (10 mL × 2). The combined organic layers were washed with brine, dried over Na₂SO₄, filtered and concentrated. The residue was purified by silica gel column chromatography to give compound **9b** (560 mg, 60% yield) as light yellow oil. ESI-LCMS (m/z): 204.6 [M+H]⁺.

Step 2: To a solution of compound **7a** (40 mg, 92.19 umol) in DMF (3 mL) was added triethylamine (28 mg, 276.57 umol), DBU (28 mg, 184.38 umol) and compound **9b** (37.5 mg, 184.38 umol) in DMF (0.5 mL). The reaction mixture was heated at 50 °C overnight, LCMS indicated the reaction was complete. The reaction mixture was purified directly by reversed phase preparative HPLC to give compound **9** (7.5 mg, 15% yield) as white solid. ESI-LC-MS (m/z): 533.3 [M+H]⁺; ¹H NMR (500 MHz, DMSO-*d6*) δ ppm 12.75 (s, 1H), 8.52 (d, *J* = 4.2 Hz, 1H), 7.92 (s, 1H), 7.78(t, *J* = 7.6 Hz, 1H), 7.60 (s, 1H), 7.50 (s, 1H), 7.33 (dd, *J* = 14.6, 6.3 Hz, 4H), 6.69 (s,1H), 5.08 (q, *J* = 13.6 Hz, 2H), 4.74-4.64 (m, 2H), 4.60 (d, *J* = 10.0 Hz, 2H), 4.28 (d, *J* = 10.0 Hz, 1H), 3.26 (s, 1H), 3.20-3.11 (m, 1H), 2.11 (d, *J* = 16.5 Hz, 3H), 2.00-1.90 (m, 2H), 1.34 (t, *J* = 7.1 Hz, 3H).

### Example 10

### Pyridin-4-ylmethyl (S)-(2-(7-carbamoyl-2-(1-ethyl-3-methyl-1H-pyrazole-5-carboxamido)-3,4-dihydro-5-oxa-1, 2a-diazaacenaphthylen-3-yl)ethyl)carbamate

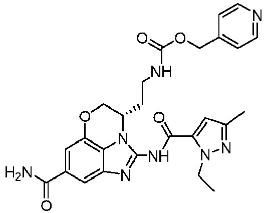

### Synthetic scheme:

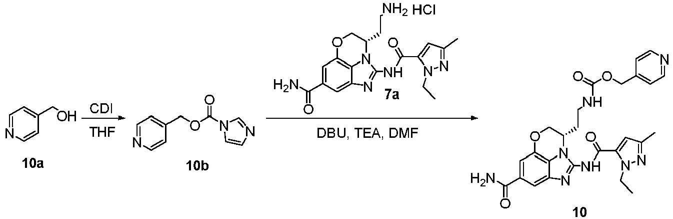

**Step 1:** To a solution of CDI (750 mg, 4.63 mmol) in dry THF (10 mL) was added dropwise compound **10a** (500 mg, 4.6 mmol) in THF (1 mL), the mixture was then stirred at room temperature for 2 hours. TLC indicated the reaction was complete. The reaction mixture was poured into water (20 mL), extracted with DCM (10 mL × 2). The combined organic layers were washed with brine, dried over Na₂SO₄, filtered and concentrated. The residue was purified by silica gel column chromatography to give compound **10b** (510 g, 54.6% yield) as white solid. ESI-LCMS (m/z): 204.6 [M+H]⁺.

**Step 2:** To a solution of compound **7a** (40 mg, 92.19 umol) in DMF (3 mL) was added triethylamine (28 mg, 276.57 umol), DBU (28 mg, 184.38 umol) and compound **10b** (37.5 mg, 184.38 umol) in DMF (0.5 mL). The reaction mixture was heated at 50 °C overnight, LCMS indicated the reaction was complete. The reaction mixture was purified directly by reversed phase preparative HPLC to give compound **10** (13 mg, 26.5 % yield) as a white solid. ESI-LC-MS (m/z): 533.4[M+H]⁺; ¹H NMR (500 MHz, DMSO-*d6*)δ 12.75 (s, 1H), 8.50 (dd, *J* = 26.0, 15.3 Hz, 2H), 7.93 (s, 1H), 7.60 (s, 1H), 7.53 (t, *J* = 5.7 Hz, 1H), 7.35 (s, 1H), 7.26 (dd, *J* =35.7, 18.7 Hz 3H), 6.69 (s, 1H), 5.07 (q, *J* = 14.4 Hz, 2H), 4.69 (dd, *J* = 24.1, 11.5 Hz, 2H), 4.60 (dd, *J* = 14.0, 7.0 Hz, 2H), 4.28 (d, *J* = 9.8 Hz, 1H), 3.25 (s, 1H), 3.20-3.11 (m, 1H), 2.09 (s, 3H), 1.96 (d, *J* = 6.9 Hz, 2H), 1.35 (t, *J* = 7.1 Hz, 3H).

### Example 11

### 2-(1-Ethyl-3-methyl-1H-pyrazole-5-carboxamido)-3,4-dihydro-5-oxa-1,2a-diazaacenaphthyl ene-7-carboxamide

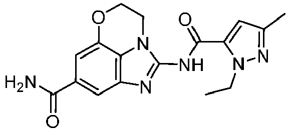

### Synthetic scheme:

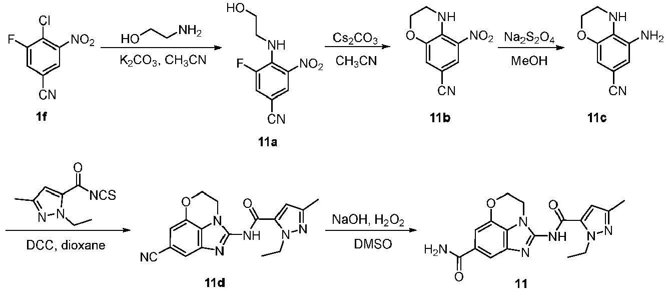

Step 1: To a stirring solution of compound **If** (500 mg, 2.49 mmol) and 2-aminoethanol (304.6 mg, 4.99 mmol) in acetonitrile (20 mL) was added K₂CO₃ (861.4 mg, 6.23 mmol). The reaction mixture was heated at 70 °C for 2 hours, and TLC indicated the starting material was consumed. The reaction mixture was allowed to cool to room temperature, filtered through a pad of celite. The filtrated was concentrated, and the residue was purified by silica gel chromatography to give compound **11a** (520 mg, 92.8% yield) as yellow solid. ¹H NMR (500 MHz, DMSO-*d6*) δ ppm 8.47 (s, 1H), 8.40 (d, *J* = 1.3 Hz, 1H), 7.95 (dd, *J* = 14.3, 1.5 Hz, 1H), 5.03 (t, *J* = 5.0 Hz, 1H), 3.66 (dd, *J* = 9.7, 4.8 Hz, 2H), 3.64-3.56 (m, 2H).

Step 2: To a stirring solution of compound **11a** (520 mg, 2.31mmol) in acetonitrile (10 mL) was added Cs₂CO₃ (1.5 g, 4.62 mmol). The reaction mixture was heated at 70 °C overnight, and TLC indicated some starting material was left. The reaction mixture was allowed to cool to room temperature, filtered through a pad of celite. The filtrated was concentrated, and the residue was purified by silica gel chromatography to give compound **11b** (170 mg, 36% yield) as yellow solid.

Step 3: Compound **11b** (170 mg, 0.83 mmol) was dissolved in a mixture of MeOH/DCM (5/1, 12 mL) and concentrated ammonium hydroxide (3 mL). Sodium dithionite (433 mg, 2.49 mmol) was dissolved in water (1 mL) and added to the reaction mixture at room temperature. Stirring was continued at room temperature for half an hour, and TLC indicated the starting material was consumed. The reaction mixture was poured into brine (20 mL), and extracted with EtOAc (10 mL × 5). The combined organic layers were dried over Na₂SO₄, filtered and concentrated to give compound **11c** (108 mg, 74% yield) as light red solid. ESI-LC-MS (m/z): 176.4 [M+H]⁺.

Step 4: Compound **11c** (108 mg, 616.48 umol) was dissolved in dioxane (5 mL), and then 1-ethyl-3-methyl-1H-pyrazole-5-carbonyl isothiocyanate (0.4M in dioxane, 1.7 mL, 678.13 umol) was added. The reaction mixture was stirred at room temperature for 1 hour, LCMS indicated the starting material was consumed. DCC (140 mg, 678.12 umol) was added, and the mixture was heated at 80 °C for 1 hour, LCMS indicated the product was formed. The reaction mixture was concentrated to give crude compound **11d** (200 mg) as red oil, which was used directly without further purification. ESI-LC-MS (m/z): 337.5 [M+H]⁺.

Step 5: To a stirring solution of crude compound **11d** (200 mg, from step 4) and NaOH (71.4 mg, 1.78 mmol) in DMSO (10 mL) at 0 °C was added hydrogen peroxide (30 wt. %, 1 mL) dropwise. The reaction was then heated at 60 °C for 30 minutes, LCMS indicated the reaction was complete. The reaction mixture was cooled to room temperature, carefully quenched with saturated Na₂SO₃ solution (3 mL), then poured into brine (15 mL). The mixture was extracted with EtOAc (10 mL × 5), the combined organic layers were dried over Na₂SO₄, filtered and concentrated. The residue was purified by reversed phase preparative HPLC to give compound **11** (50 mg) as pink solid. ESI-LC-MS (m/z): 355.4 [M+H]⁺; ¹H NMR (500 MHz, DMSO-*d6*) δ ppm 12.63 (s, 1H), 7.91 (s, 1H), 7.58 (s, 1H), 7.32 (s, 1H), 7.29 (s, 1H), 6.65 (s, 1H), 4.61 (dd, *J* = 14.0, 6.9 Hz, 2H), 4.56-4.49 (m, 2H), 4.22 (d, *J* = 4.4 Hz, 2H), 2.17 (s, 3H), 1.35 (t, *J* = 7.1 Hz, 3H).

### Example 12

### (E)-2-(1-Ethyl-3-methyl-4-(3-phenylprop-1-en-1-yl)-1H-pyrazole-5-carboxamido)-7-methox y-1-methyl-1H-benzo[d]imidazole-5-carboxamide

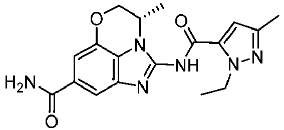

### Synthetic scheme:

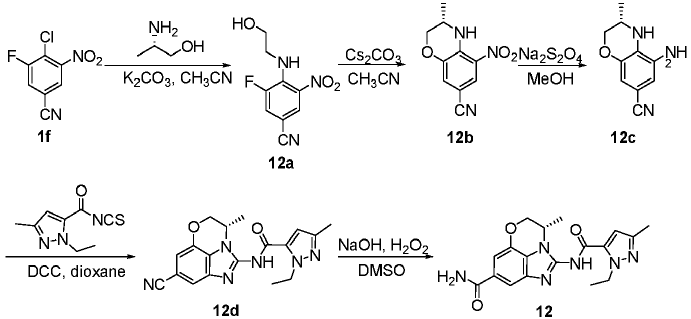

**Step 1:** To a stirring solution of compound **1f** (500 mg, 2.49 mmol) and S-(+)-2-amino-1-propanol (374.5 mg, 4.99 mmol) in acetonitrile (20 mL) was added K₂CO₃ (861.4 mg, 6.23 mmol). The reaction mixture was heated at 70 °C overnight, and TLC indicated the starting material was consumed. The reaction mixture was allowed to cool to room temperature, filtered through a pad of celite. The filtrated was concentrated, and the residue was purified by silica gel chromatography to give compound **12a** (550 mg, 92 % yield) as a yellow solid.

**Step 2:** To a stirring solution of compound **12a** (550 mg, 2.3 mmol) in acetonitrile (10 mL) was added Cs₂CO₃ (1.5 g, 4.62 mmol). The reaction mixture was heated at 70 °C for 2 hours, and TLC indicated the reaction was complete. The reaction mixture was allowed to cool to room temperature, filtered through a pad of celite. The filtrated was concentrated, and the residue was purified by silica gel chromatography to give compound **12b** (390 mg, 77.38 % yield) as a yellow solid. ¹H NMR (500 MHz, DMSO-*d6*) δ ppm 8.82 (s, 1H), 8.13 (s, 1H), 7.39 (s, 1H), 4.30-4.09 (m, 1H), 3.97 (dd, *J* = 10.6, 4.3 Hz, 1H), 3.85 (s, 1H), 1.26 (d, *J* = 6.3 Hz, 3H).

**Step 3:** Compound **12b** (390 mg, 1.78 mmol) was dissolved in a mixture of MeOH/DCM (5/1, 24 mL) and concentrated ammonium hydroxide (8 mL). Sodium dithionite (929 mg, 5.34 mmol) was dissolved in water (2 mL) and added to the reaction mixture at room temperature. Stirring was continued at room temperature for half an hour, and TLC indicated the starting material was consumed. The reaction mixture was poured into brine (30 mL), and extracted with EtOAc (15 mL × 5). The combined organic layers were dried over Na₂SO₄, filtered and concentrated to give compound **12c** (200 mg, 59.4% yield) as pink solid. ESI-LC-MS (m/z): 190.7 [M+H]⁺.

**Step 4:** Compound **12c** (200 mg, 1.06 mmol) was dissolved in dioxane (10 mL), and then 1-ethyl-3-methyl-1H-pyrazole-5-carbonyl isothiocyanate (0.4M in dioxane, 3 mL, 1.17 mmol) was added. The reaction mixture was stirred at room temperature for 1 hour, LCMS indicated the starting material was consumed. DCC (240 mg, 1.17 mmol) was added, and the mixture was heated at 80 °C for 1 hour, LCMS indicated the product was formed. The reaction mixture was concentrated to give crude compound **12d** (350 mg) as red oil, which was used directly without further purification. ESI-LC-MS (m/z): 351.6 [M+H]⁺.

**Step 5:** To a stirring solution of crude compound **12d** (350 mg, from step 4) and NaOH (102.7 mg, 2.57 mmol) in DMSO (2 mL) at 0 °C was added hydrogen peroxide (30 wt. %, 2 mL) dropwise. The reaction was then heated at 60 °C for 30 minutes, LCMS indicated the reaction was complete. The reaction mixture was cooled to room temperature, carefully quenched with saturated Na₂SO₃ solution (5 mL), then poured into brine (15 mL). The mixture was extracted with EtOAc (10 mL × 5), the combined organic layers were dried over Na₂SO₄, filtered and concentrated. The residue was purified by reversed phase preparative HPLC to give compound **12** (130 mg, 41.21%) as pink solid. ESI-LC-MS (m/z): 355.4 [M+H]⁺; ¹H NMR (500 MHz, DMSO-*d6*) δ ppm 12.67 (s, 1H), 7.92 (s, 1H), 7.59 (s, 1H), 7.34 (d, *J* = 0.9 Hz, 1H), 7.30 (s, 1H), 6.64 (s, 1H), 4.73-4.66 (m, 1H), 4.62 (qd, *J* = 13.1, 7.0 Hz, 2H), 4.46 (dd, *J* = 11.6, 2.2 Hz, 1H), 4.36 (dd, *J* = 11.5, 2.4 Hz,1H), 2.18 (s, 3H), 1.46 (d, *J* = 6.7 Hz, 3H), 1.36 (t, *J* = 7.1 Hz, 3H).

### Example 13

### Benzyl (S)-(2-(7-cyano-2-(1-ethyl-3-methyl-1H-pyrazole-5-carboxamido)-3,4-dihydro-5-oxa-1,2a-di azaacenaphthylen-3-yl)ethyl)carbamate

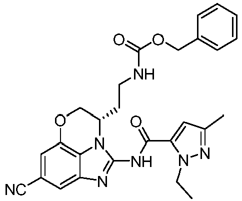

### Synthetic scheme:

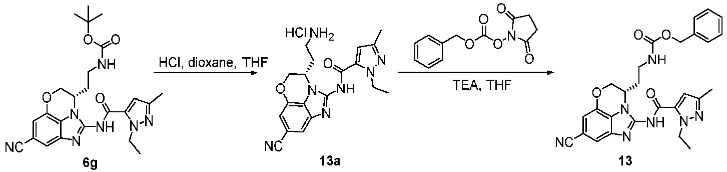

Step 1: To a stirring solution of compound **6g** (700 mg,1.46 mmol) in THF (10 mL) was added 4M HCl in dioxane (10 mL, 40 mmol) at room temperature. The mixture was stirred overnight at rom temperature and LCMS indicated the product was formed. The mixture was concentrated under reduced pressure to give compound **13a** (610 mg, 100% yiled) as yellow solid. ESI-LC-MS (m/z): 380.6 [M+H]⁺.

Step 2: To a solution of compound **13a** (300 mg, 721.37 umol) in THF (10 mL) was added N-(benzyloxycarbonyloxy)succinimide (198 mg, 793.51 umol) and triethylamine (219 mg, 2.16 mmol). The mixture was stirred at room temperature for 2 hours, LCMS indicated the reaction was complete. The reaction was concentrated, and the residue was purified by silica gel colomn chromatography to give compound **13** (210 mg, 56.7% yield) as light yellow solid. ESI-LC-MS (m/z): 514.4 [M+H]⁺; ¹H NMR (500 MHz, DMSO-*d6*) δ ppm 12.85 (s, 1H), 7.38 (s, 1H), 7.33 (t, *J* = 7.0 Hz, 7H), 6.70 (s, 1H), 5.02 (q, *J* = 12.8 Hz, 2H), 4.70 (d, *J* = 10.9 Hz, 2H), 4.59 (d, *J* = 7.0 Hz, 2H), 4.31 (d, *J* = 11.0 Hz, 1H), 3.25 (dd, *J* = 13.6, 6.7 Hz, 1H), 3.16 (s, 1H), 2.12(d, *J* = 28.6 Hz, 3H), 1.94 (d, *J* = 7.7 Hz, 2H), 1.34 (t, *J* = 7.0 Hz, 3H).

### Example 14

### Benzyl (S)-(2-(7-carbamoyl-2-(1-ethyl-3-methyl-1H-pyrazole-5-carboxamido)-3,4-dihydro-5-oxa-1, 2a-diazaacenaphthylen-3-yl)ethyl)carbamate

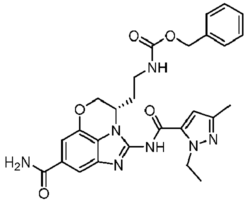

### Synthetic scheme:

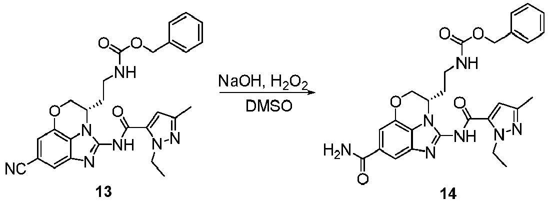

Step 1: To a stirring solution of compound **13** (40 mg, 77.89 umol) and NaOH (9 mg, 233.57 umol) in DMSO (2 mL) at 0 °C was added hydrogen peroxide (30 wt. %, 0.6 mL) dropwise. The reaction was stirred at 0 °C for 1 hour, LCMS indicated the reaction was complete. The reaction mixture was purified directly by reversed phase preparative HPLC to give compound **14** (14 mg, 34 % yield) as a white solid. ESI-LC-MS (m/z): 532.4 [M+H]⁺; ¹H NMR (500 MHz, DMSO-d(5) δ ppm 12.74 (s, 1H), 7.92 (s, 1H), 7.60 (s, 1H), 7.43-7.24 (m, 8H), 6.69 (s, 1H), 5.02 (q, *J* = 12.5 Hz, 2H), 4.72-4.64 (m, 2H), 4.60 (dd, *J* = 13.9, 6.9 Hz, 2H), 4.27 (d, *J* = 10.1 Hz, 1H), 3.25 (dd, *J* = 14.0, 7.0 Hz, 1H), 3.14 (d, *J* = 6.6 Hz, 1H), 2.13 (d, *J* = 32.1 Hz, 3H), 1.94 (s, 2H), 1.35 (t, *J* = 7.1 Hz, 3H).

### Example 15

### (S)-3-(2-Cinnamamidoethyl)-2-(1-ethyl-3-methyl-1H-pyrazole-5-carboxamido)-3,4-dih ydro-5-oxa-1,2a-diazaacenaphthylene-7-carboxamide

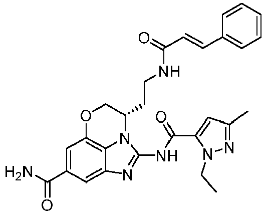

### Synthetic scheme:

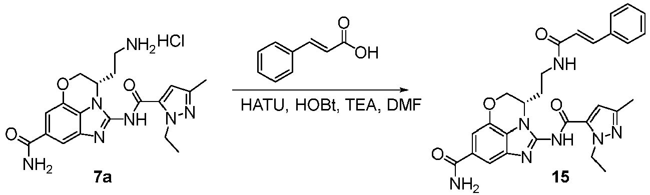

Step 1: To a solution of cinnamic acid (18 mg, 125.8 umol) in DMF (2 mL) was added HATU (47.8 mg, 125.8 umol), HOBt (17 mg, 125.8 umol) and TEA (38.1 mg, 377 umol). The mixture was stirred at room temperature for 1 hour, then compound **7a** (50 mg, 125.8 umol) was added to the reaction. The reaction mixture was stirred at room temperature overnight. LCMS indicated the reaction was complete. The mixture was purified directly by reversed phase preparative HPLC to give compound **15** (6.8 mg, 7 % yield) as white solid. ESI-LC-MS (m/z): 528.4 [M+H]⁺; ¹HNMR (500 MHz, DMSO-*d6*) δ ppm 12.71 (s, 1H), 8.31 (t, J = 5.9 Hz, 1H), 7.93 (s, 1H), 7.63-7.52 (m, 3H), 7.49-7.34 (m, 6H), 6.66 (s, 1H), 6.62 (d, *J* = 15.7 Hz, 1H), 4.81-4.66 (m, 2H), 4.58( m, 2H), 4.31 (m, 1H), 3.49-3.44 (m, 2H), 2.05 (s, 3H), 1.97 (m, 2H), 1.33 (t, *J* = 7.1 Hz, 3H).

### Example 16

### (R)-2-(1-Ethyl-3-methyl-1H-pyrazole-5-carboxamido)-4-methyl-3,4-dihydro-5-oxa-1,2a-diazaacenaphthylene-7-carboxamide

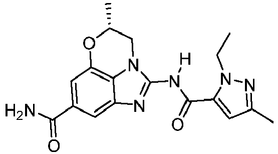

### Synthetic scheme:

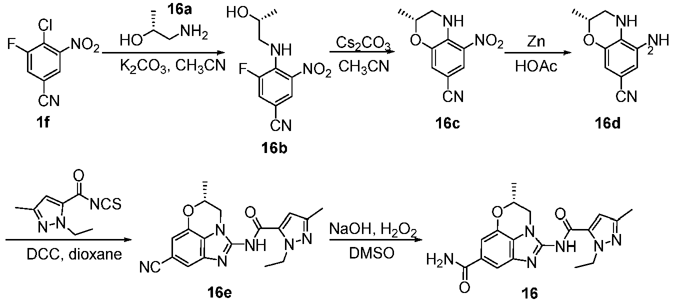

**Step 1:** To a stirring solution of compound **1f** (500 mg, 2.49 mmol) and **16a** (374 mg, 5 mmol) in acetonitrile (10 mL) was added K₂CO₃ (1.03 g, 7.5 mmol). The reaction mixture was heated at 70 °C for 6 hours, and TLC indicated the starting material was consumed. The reaction mixture was allowed to cool to room temperature, filtered through a pad of celite, and washed with EtOAc (10 mL × 3). The filtrated was concentrated to give crude compound **16b** (520 mg, purity: 86%), which was used directly without further purification. ESI-LC-MS (m/z): 240.1 [M+H]⁺.

**Step 2:** To a stirring solution of compound **16b** (520 mg, from step 1) in acetonitrile (10 mL) was added Cs₂CO₃ (2.12g, 6.52 mmol). The reaction mixture was heated at 70 °C for 6 hours, then cooled to room temperature. The mixture was filtered through a pad of celite, and washed with EtOAc (10 mL × 3). The filtrated was concentrated to give crude compound **16c** (440 mg, purity: 77%), which was used directly without further purification. ESI-LC-MS (m/z): 220.4 [M+H]⁺.

Step 3: Compound **16c** (440 mg, from step 2) was dissolved in acetic acid (10 mL), and Zn powder (652 mg, 10.04 mmol) was added by portions at room temperature. The reaction mixture was stirred at room temperature for 1 hour. The mixture was filtered through a pad of celite, and the cake was rinsed with EtOAc (10 mL × 3). The filtrate was concentrated and the residue was purified by silica gel chromatography to give the compound **16d** (200 mg, purity: 95%, 42% yield for three steps). ESI-LC-MS (m/z): 190.5 [M+H]⁺.

**Step 4:** Compound **16d** (200 mg, 1.06 mmol) was dissolved in dioxane (10 mL), and then 1-ethyl-3-methyl-1H-pyrazole-5-carbonyl isothiocyanate (0.4M in dioxane, 3 mL, 1.17 mmol) was added. The reaction mixture was stirred at room temperature for 1 hour, then DCC (218.09 mg, 1.06 mmol) was added, and the mixture was heated at 70 °C overnight, LCMS indicated the product was formed. The mixture was filtered through a pad of celite, and the cake was rinsed with EtOAc (10 mL × 3). The filtrate was concentrated to give crude compound **16e** (240 mg, purity: 82%), which was used directly without further purification. ESI-LC-MS (m/z): 351.2 [M+H]⁺.

**Step 5:** To a stirring solution of compound **16e** (120 mg, from step 4) and NaOH (40 mg, 1 mmol) in DMSO (3 mL) was added hydrogen peroxide (30 wt. %, 0.5 mL) dropwise. The reaction was stirred at room temperature for 30 minutes, LCMS indicated the reaction was complete. The reaction mixture was purified directly by reversed phase preparative HPLC to give compound **16** (48 mg, 24 % yield for 2 steps). ESI-LC-MS (m/z): 369.5 [M+H]⁺; ¹HNMR (500 MHz, DMSO-*d6*) δ ppm 12.64 (s, 1H), 7.89 (s, 1H), 7.57 (s, 1H), 7.30 (m, 2H), 6.67 (s, 1H), 4.60 (q, *J* = 7.1 Hz, 2H), 4.51 (m, 1H), 4.41 (m, 1H), 3.77 (m, 1H), 2.17 (s, 3H), 1.51 (d, J = 6.3 Hz, 3H), 1.35 (t, *J* = 7.1 Hz, 3H).

### Example 17

### Benzyl (S)-(4-(7-carbamoyl-2-(1-ethyl-3-methyl-1H-pyrazole-5-carboxamido)-3,4-dihydro-5-oxa-1, 2a-diazaacenaphthylen-3-yl)butyl)carbamate

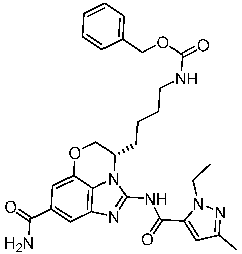

### Synthetic scheme:

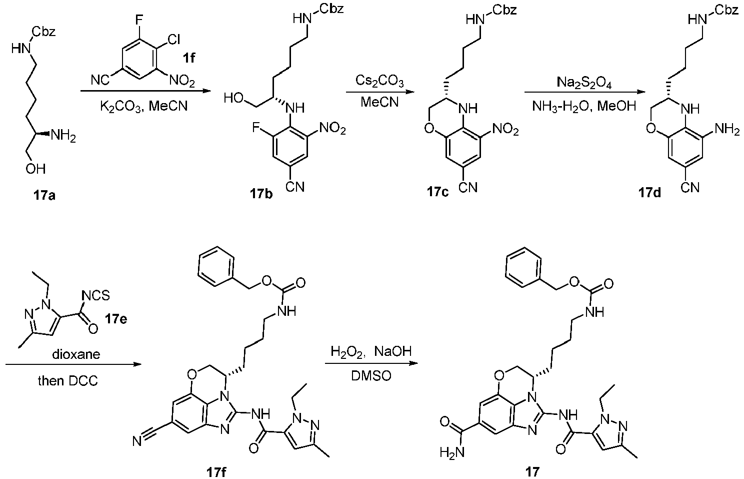

Step 1: To a stirring solution of compound **1f** (6.0 g, 30 mmol) and compound **17a** (16 g, 60 mmol) in acetonitrile (150 mL) was added K₂CO₃ (12.4 g, 90 mmol), and the resulting mixture was heated at 70 °C for 24 hours, TLC indicated the product was formed. The reaction mixture was cooled to room temperature, the solid was removed by filtration and washed with DCM (100 mL), the filtrate was concentrated. The residue was purified by silica gel chromatography to give compound **17b** (8.2 g, 63% yield) as a yellow oil. ESI-MS (m/z): 431.2 [M+H]⁺.

Step 2: To a stirring solution of compound **17b** (4.0 g, 9.29 mmol) in acetonitrile (150 mL) was added Cs₂CO₃ (9.08 g, 27.88 mmol), and the reaction mixture was heated at 70°C for 6 hours, TLC indicated the conversion was complete. The reaction mixture was cooled to room temperature, filtered through a pad of silica gel. The filtrate was concentrated under reduced pressure to give compound **8c** (3.90 g, 68% purity). The sample was used directly without further purification. ESI-MS (m/): 411.6 [M+H]⁺.

Step 3: Compound **17c** (3.90 g, from step 2) was dissolved in a mixture of MeOH (30 mL) and concentrated ammonium hydroxide (5 mL). Sodium dithionite (5.5 g, 31.59 mmol) was dissolved in water (2 mL) and added to the reaction mixture at room temperature. Stirring was continued at room temperature for half an hour, and LCMS indicated the product was formed. The reaction mixture was diluted with water (50 mL) and extracted with ethyl acetate (50 mL × 3). The combined organic layers were washed with brine, dried over Na₂SO₄, filtered and concentrated under reduced pressure. The residue was purified by silica gel chromatography to give compound **17d** (1.2 g, yield 34% for two steps) as yellow solid. ESI-MS (m/): 381.6 [M+H]⁺.

Step 4: Compound **17d** (700 mg, 1.84 mmol) was dissolved in dioxane (10 mL), and then compound **17e** (0.4M in dioxane, 4.6 mL, 1.84 mmol) was added. The reaction mixture was stirred at room temperature for 1 hour, then DCC (379 mg, 1.84 mmol) was added. The mixture was heated at 80°C for 6 hours, LCMS indicated the product was formed. The reaction mixture was cooled to room temperature, filtered through a pad of silica gel and washed with ethyl acetate (10 mL × 3). The filtrate was concentrated to give compound **8e** (970 mg, 61% purity), which was used directly without further purification.

Step 5: To a stirring solution of compound **17f** (700 mg, from step 4) and NaOH (200 mg, 5 mmol) in DMSO (5 mL) at 0°C was added hydrogen peroxide (30 wt. %, 0.5 mL) dropwise. The reaction was stirred at room temperature for 30 minutes, LCMS indicated the reaction was complete. The reaction mixture was purified directly by reversed phase preparative HPLC to give **17** (400 mg). ESI-MS (m/z): 560.4 [M+H]⁺; ¹H NMR (500 MHz, DMSO-*d*₆) δ 12.72 (s, 1H), 7.92 (s, 1H), 7.58 (s, 1H), 7.38-7.27 (m, 7H), 7.21 (s, 1H), 6.63 (s, 1H), 4.96 (s, 2H), 4.68-4.55 (m, 4H), 4.30-4.21 (m, 1H), 3.04-2.93 (m, 2H), 2.17 (s, 3H), 1.86-1.73 (m, 2H), 1.52-1.40 (m, 4H), 1.35 (t, *J* = 7.0 Hz, 3H).

### Example 18

### Pyridin-3-ylmethyl (S)-(4-(7-carbamoyl-2-(1-ethyl-3-methyl-1H-pyrazole-5-carboxamido)-3,4-dihydro-5-oxa-1, 2a-diazaacenaphthylen-3-yl)butyl)carbamate

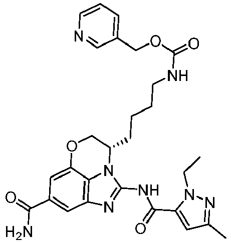

### Synthetic scheme:

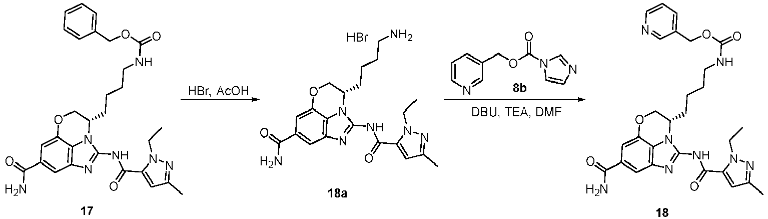

**Step 1:** Compound **17** (400 mg, 0.73 mmol) was dissolved in acetic acid (5 mL), and hydrobromic acid in acetic acid solution (33wt. %, 1 mL) was added. The resulting mixture was stirred at room temperature for 30 minutes, LCMS indicated the starting material was consumed. Diethyl ether was added to the reaction mixture, the formed white solid was collected by filtration and dried in vacuo to give compound **18a** (320 mg). ESI-MS (m/z): 526.3 [M+H]⁺.

**Step 2:** To a solution of compound **18a** (80 mg) in DMF (2 mL) was added compound **8b** (76 mg, 0.38mol), DBU (57 mg, 0.38 mol) and triethylamine (57 mg, 0.56mmol), the reaction mixture was heated at 50 °C overnight. LCMS indicated the reaction was complete. The reaction mixture was purified directly by reversed phase preparative HPLC to give compound **18** (29 mg) as a white solid. ESI-MS (m/z): 561.5 [M+H]⁺; ¹H NMR (500 MHz, DMSO-*d*₆) δ 12.60 (br s, 1H), 8.55 (d, *J* =2.0 Hz, 1H), 8.52 (dd, *J* = 4.5 and 1.5 Hz, 1H), 7.92 (s, 1H), 7.73 (d, *J* = 7.5 Hz, 1H), 7.59 (s, 1H), 7.41-7.38 (m, 1H), 7.33 (s, 2H), 7.30 (s, 1H), 7.28-7.25 (m, 1H), 6.64 (s, 1H), 5.00 (s, 2H), 4.67- 4.52 (m, 4H), 4.31- 4.23 (m, 1H), 3.10-2.95 (m, 2H), 2.16 (s, 3H), 1.86-1.75 (m, 2H), 1.52-1.41 (m, 4H), 1.35 (t, *J* = 6.9 Hz, 3H).

### Example 19

### Pyridin-2-ylmethyl (S)-(4-(7-carbamoyl-2-(1-ethyl-3-methyl-1H-pyrazole-5-carboxamido)-3,4-dihydro-5-oxa-1, 2a-diazaacenaphthylen-3-yl)butyl)carbamate

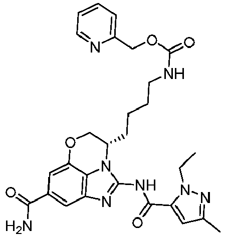

### Synthetic scheme:

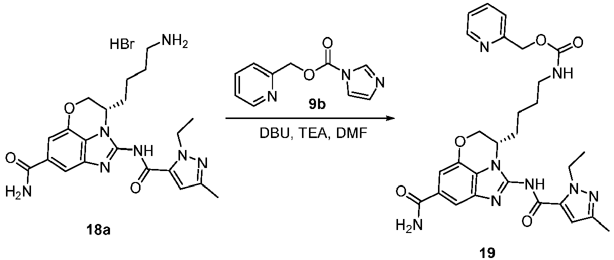

Step 1: To a solution of compound **18a** (80 mg) in DMF (2 mL) was added compound **9b** (76 mg, 0.37 mmol), DBU (57 mg, 0.38 mol) and triethylamine (57 mg, 0.56 mmol), the reaction mixture was heated at 50 °C overnight. LCMS indicated the reaction was complete. The reaction mixture was purified directly by reversed phase preparative HPLC to give compound **19** (26 mg) as a white solid. ESI-MS (m/z): 561.4 [M+H]⁺; ¹H NMR (500 MHz, DMSO-*d*₆) δ 12.69 (br s, 1H), 8.51 (d, *J* = 5.0 Hz, 1H), 7.93 (s, 1H), 7.79 (t, *J* = 7.5 Hz, 1H), 7.60 (s, 1H), 7.44-7.22 (m, 5H), 6.64 (s, 1H), 5.03 (s, 2H), 4.71-4.53 (m, 4H), 4.33-4.18 (m, 1H), 3.06-2.93 (m, 2H), 2.17 (s, 3H), 1.88-1.70 (m, 2H), 1.54-1.40 (m, 4H), 1.35 (t, *J* = 7.1 Hz, 3H).

### Example 20

### Pyridin-4-ylmethyl (S)-(4-(7-carbamoyl-2-(1-ethyl-3-methyl-1H-pyrazole-5-carboxamido)-3,4-dihydro-5-oxa-1, 2a-diazaacenaphthylen-3-yl)butyl)carbamate

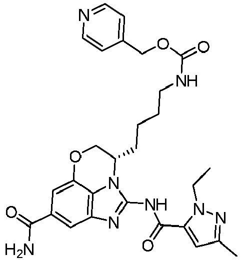

### Synthetic scheme:

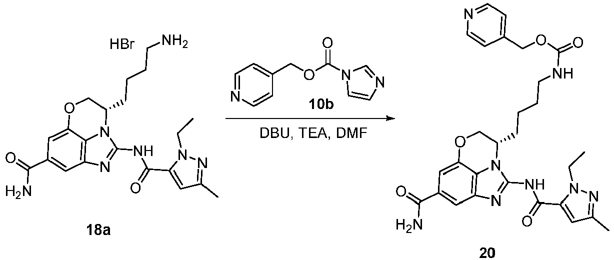

**Step 1:** To a solution of compound **18a** (80 mg) in DMF (2 mL) was added compound **10b** (76 mg, 0.38mol), DBU (57 mg, 0.38 mol) and triethylamine (57 mg, 0.56 mmol), the reaction mixture was heated at 50 °C overnight. LCMS indicated the reaction was complete. The reaction mixture was purified directly by reversed phase preparative HPLC to give compound **20** (38 mg) as a white solid. ESI-MS (m/z): 561.4 [M+H]⁺; ¹H NMR (500 MHz, DMSO-*d*₆) δ 12.70 (br s, 1H), 8.53 (d, *J* = 6.0 Hz, 1H), 7.93 (s, 1H), 7.60 (s, 1H), 7.42-7.23 (m, 5H), 6.64 (s, 1H), 5.02 (s, 2H), 4.69-4.56 (m, 4H), 4.31-4.22 (m, 1H), 3.06-2.95 (m, 2H), 2.16 (s, 3H), 1.87-1.74 (m, 2H), 1.53-1.42 (m, 4H), 1.35 (t, *J* = 7.1 Hz, 3H).

### Example 21

### 2-(1-Ethyl-3-methyl-1H-pyrazole-5-carboxamido)-3,3-dimethyl-3,4-dihydro-5-oxa-1,2a-diaz aacenaphthylene-7-carboxamide

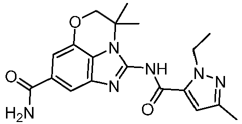

### Synthetic scheme:

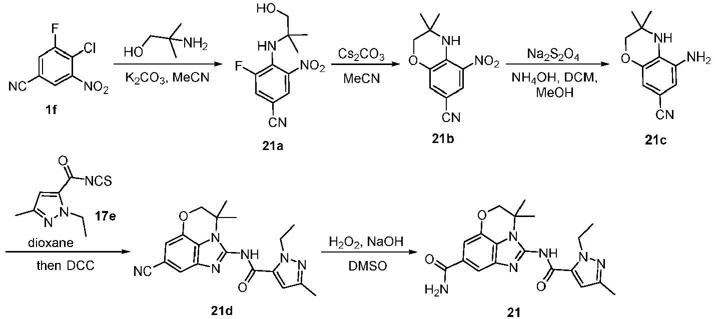

Step 1: To a stirring solution of compound **1f** (1 g, 4.99 mmol) and 2-amino-2-methylpropan-1-ol (889 mg, 9.97 mmol) in acetonitrile (20 mL) was added K₂CO₃ (2.07 g, 14.96 mmol). The reaction mixture was heated at 70 °C overnight, and TLC indicated the starting material was consumed. The reaction mixture was allowed to cool to room temperature, filtered through a pad of celite. The filtrated was concentrated, and the residue was purified by silica gel chromatography to give compound **21a** (1.2 g, 95% yield).

Step 2: To a stirring solution of compound **21a** (1.2 g, 4.74 mmol) in acetonitrile (20 mL) was added Cs₂CO₃ (4.63 g,14.22 mmol). The reaction mixture was heated at 70 °C for 2 hours, and TLC indicated the reaction was complete. The reaction mixture was allowed to cool to room temperature, filtered through a pad of celite. The filtrated was concentrated, and the residue was purified by silica gel chromatography to give compound **21b** (1.0 g, 90% yield). ESI-MS (m/z): 234.2 [M+H]⁺.

Step 3: Compound **21b** (1.0 g, 4.29 mmol) was dissolved in a mixture of MeOH/DCM (5/1, 24 mL) and concentrated ammonium hydroxide (5 mL). Sodium dithionite (4.1 g, 23.5 mmol) was dissolved in water (10 mL) and added to the reaction mixture at room temperature. Stirring was continued at room temperature for half an hour, and TLC indicated the starting material was consumed. The reaction mixture was poured into brine (30 mL), and extracted with EtOAc (15 mL × 5). The combined organic layers were dried over Na₂SO₄, filtered and concentrated to give compound **21c** (360 mg, 37% yield) as white solid. ESI-MS (m/z): 204.2 [M+H]⁺.

Step 4: Compound **21c** (360 mg, 1.77 mmol) was dissolved in dioxane (10 mL), and then compound **17e** (0.4M in dioxane, 4.42 mL,1.77 mmol) was added. The reaction mixture was stirred at room temperature for 1 hour, LCMS indicated the starting material was consumed. DCC (365 mg, 1.77 mmol) was added, and the mixture was heated at 80 °C for 1 hour, LCMS indicated the product was formed. The reaction mixture was concentrated, and the residue was purified by silica gel chromatography to give compound **21d** (360 mg, 55% yield). ESI-MS (m/z): 365.4 [M+H]⁺.

Step 5: To a stirring solution of compound **21d** (360 mg, 0.98 mmol) and NaOH (102 mg, 2.57 mmol) in DMSO (10 mL) at 0 °C was added hydrogen peroxide (30 wt. %, 2 mL) dropwise. The reaction was then heated at 60 °C for 30 minutes, LCMS indicated the reaction was complete. The reaction mixture was cooled to room temperature, carefully quenched with saturated Na₂SO₃ solution (5 mL), then poured into brine (15 mL). The mixture was extracted with EtOAc (10 mL × 5), the combined organic layers were dried over Na₂SO₄, filtered and concentrated. The residue was purified by reversed phase preparative HPLC to give compound **21** (72 mg, 19% yield) as white solid. ESI-MS (m/z): 383.6 [M+H]⁺; ¹H NMR (500 MHz, DMSO-*d*₆) δ 12.74 (s, 1H), 8.15 (s, 1H), 7.92 (s, 1H), 7.59 (d, *J* = 3.2 Hz, 1H), 7.38-7.21 (m, 2H), 6.62 (s, 1H), 4.60 (q, *J* = 7.1 Hz, 2H), 4.25 (s, 2H), 2.19 (s, 3H), 1.68 (s, 6H), 1.35 (t, *J* = 7.1 Hz, 3H).

### Example 22

### (S)-1-(1-Ethyl-3-methyl-1H-pyrazole-5-carboxamido)-9-methyl-8,9-dihydro-7H-6-oxa-2,9a-diazabenzo[cd]azulene-4-carboxamide

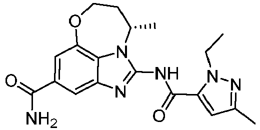

### Synthetic scheme:

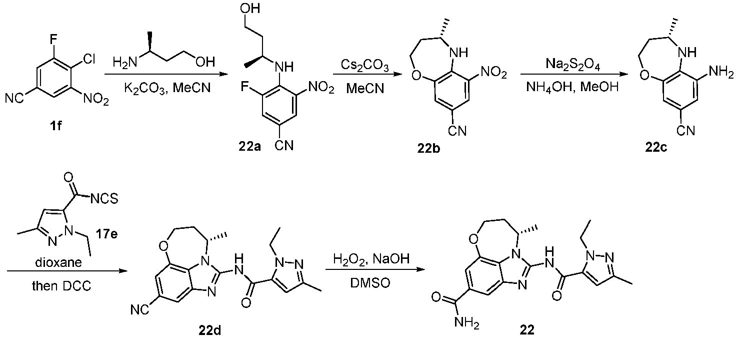

**Step 1:** To a stirring solution of compound **1f** (1 g, 4.99 mmol) and (S)-3-aminobutan-1-ol (889 mg, 9.97 mmol) in acetonitrile (20 mL) was added K₂CO₃ (2.07 g, 14.96 mmol). The reaction mixture was heated at 70 °C overnight, and TLC indicated the starting material was consumed. The reaction mixture was allowed to cool to room temperature, filtered through a pad of celite. The filtrated was concentrated, and the residue was purified by silica gel chromatography to give compound **22a** (1.2 g, 95% yield).

**Step 2:** To a stirring solution of compound **21a** (1.2 g, 4.74 mmol) in acetonitrile (20 mL) was added Cs₂CO₃ (4.63 g,14.22 mmol). The reaction mixture was heated at 70 °C for 2 hours, and TLC indicated the reaction was complete. The reaction mixture was allowed to cool to room temperature, filtered through a pad of celite. The filtrated was concentrated, and the residue was purified by silica gel chromatography to give compound **22b** (1.1g, 99% yield). ESI-MS (m/z): 234.2 [M+H]⁺.

**Step 3:** Compound **21b** (1.1 g, 4.72 mmol) was dissolved in MeOH (5 mL) and concentrated ammonium hydroxide (5 mL). Sodium dithionite (4.1 g, 23.5 mmol) was dissolved in water (10 mL) and added to the reaction mixture at room temperature. Stirring was continued at room temperature for half an hour, and TLC indicated the starting material was consumed. The reaction mixture was poured into brine (30 mL), and extracted with EtOAc (15 mL × 5). The combined organic layers were dried over Na₂SO₄, filtered and concentrated to give compound **22c** (200 mg, 21% yield) as a white solid. ESI-MS (m/z): 204.2 [M+H]⁺.

**Step 4:** Compound **22c** (200 mg, 0.98 mmol) was dissolved in dioxane (10 mL), and then compound **17e** (0.4M in dioxane, 2.45 mL, 0.98 mmol) was added. The reaction mixture was stirred at room temperature for 1 hour, LCMS indicated the starting material was consumed. DCC (203 mg, 0.98 mmol) was added, and the mixture was heated at 80 °C for 1 hour, LCMS indicated the product was formed. The reaction mixture was concentrated, and the residue was purified by silica gel chromatography to give compound **22d** (200 mg, 56% yield). ESI-MS (m/z): 365.4 [M+H]⁺.

**Step 5:** To a stirring solution of compound **22d** (200 mg, 0.55mmol) and NaOH (105 mg, 2.75 mmol) in DMSO (10 mL) at 0 °C was added hydrogen peroxide (30 wt. %, 2 mL) dropwise. The reaction was then heated at 60 °C for 30 minutes, LCMS indicated the reaction was complete. The reaction mixture was cooled to room temperature, carefully quenched with saturated Na₂SO₃ solution (5 mL), then poured into brine (15 mL). The mixture was extracted with EtOAc (10 mL × 5), the combined organic layers were dried over Na₂SO₄, filtered and concentrated. The residue was purified by reversed phase preparative HPLC to give compound 22 (64 mg, 30% yield ) as a white solid. ESI-MS (m/z): 383.5 [M+H]⁺; ¹H NMR (500 MHz, DMSO-*d*₆) δ 12.85 (s, 1H), 7.90 (s, 1H), 7.65 (s, 1H), 7.30 (s, 1H), 6.66 (s, 1H), 5.03-4.92 (m, 1H), 4.70-4.52 (m, 3H), 4.48-4.39 (m, 1H), 2.60-2.56 (m, 1H), 2.36-2.27 (m, 1H), 2.18 (s, 3H), 1.46 (d, *J* = 6.6 Hz, 3H), 1.36 (t, *J* = 7.1 Hz, 4H).

### Example 23

### (S)-2-(1-Ethyl-3-methyl-1H-pyrazole-5-carboxamido)-3-propyl-3,4-dihydro-5-oxa-1,2a-diaz aacenaphthylene-7-carboxamide

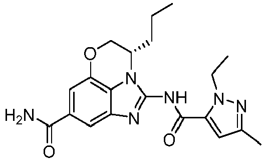

### Synthetic scheme:

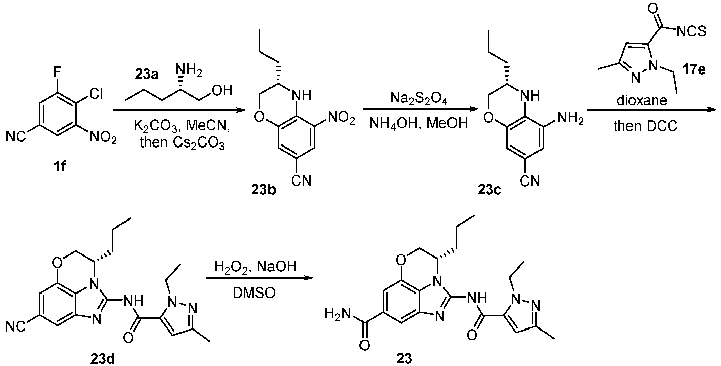

Step 1: To a stirring solution of compound **1f** (500 mg,2.49 mmol) and (S)-2-aminopentan-1-ol (514 mg,4.99 mmol) in acetonitrile (20 mL) was added K₂CO₃ (1.03 g, 7.48 mmol). The reaction mixture was heated at 70 °C, and monitored by TLC. After the starting material was consumed, Cs₂CO₃ (2.43 g,7.48 mmol) was added to the reaction. Stirring was continued at 70 °C for about 2 hours, and LCMS indicated the product was formed. The reaction mixture was allowed to cool to room temperature, filtered through a pad of celite. The filtrated was concentrated, and the residue was purified by silica gel chromatography to give compound **23b** (517 mg, 84% yield) as red solid.

Step 2: Compound **23b** (517 mg,2.09 mmol) was dissolved in MeOH (30 mL) and concentrated ammonium hydroxide (3 mL). Sodium dithionite (1.82 g,10.46 mmol) was dissolved in water (3 mL) and added to the reaction mixture at room temperature. Stirring was continued at room temperature for half an hour, and TLC indicated the starting material was consumed. The reaction mixture was concentrated, the residue was mixed with water (20 mL), and extracted with EtOAc (15 mL × 3). The combined organic layers were washed with bribe, dried over Na₂SO₄, filtered and concentrated to give compound **23c** (414 mg, 91% yield) as yellow solid. ESI-MS (m/z): 296.7 [M+H]⁺.

Step 3: Compound **23c** (414 mg,1.91 mmol) was dissolved in dioxane (5 mL), and then compound **17e** (0.4M in dioxane, 4.8 mL,1.91 mmol) was added. The reaction mixture was stirred at room temperature for 1 hour, LCMS indicated the starting material was consumed. DCC (394 mg,1.91 mmol) was added, and the mixture was heated at 80 °C for 2 hours, LCMS indicated the product was formed. The reaction mixture was concentrated to give crude compound **23d** (990 mg) as brown oil, which was used directly without further purification. ESI-MS (m/z): 492.6 [M+MeOH]⁺.

Step 4: To a stirring solution of compound **23d** (155 mg, from step 3) and NaOH (60 mg, 1.51 mmol) in DMSO (3 mL) at room temperature was added hydrogen peroxide (30 wt. %, 0.6 mL) dropwise. The reaction was then heated at 50 °C for 30 minutes, LCMS indicated the reaction was complete. The reaction mixture was cooled to room temperature and filtered. The filtrate was purified directly by reversed phase preparative HPLC to give compound **23** (38 mg) as a white solid. ESI-MS (m/z): 397.5 [M+H]⁺; ¹H NMR (500 MHz, DMSO-*d*₆) δ 12.70 (br s, 1H), 7.93 (s, 1H), 7.60 (s, 1H), 7.34 (s, 1H), 7.31 (s, 1H), 6.64 (s, 1H), 4.74-4.54 (m, 4H), 4.32-4.25 (m, 1H), 2.19 (s, 3H), 1.86-1.69 (m, 2H), 1.55-1.41 (m, 2H), 1.36 (t, *J* = 7.1 Hz, 3H), 0.93 (t, *J* = 7.3 Hz, 3H).

### Example 24

### (S)-3-Benzyl-2-(1-ethyl-3-methyl-1H-pyrazole-5-carboxamido)-3,4-dihydro-5-oxa-1,2a-diaz aacenaphthylene-7-carboxamide

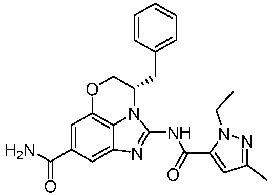

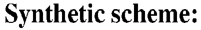

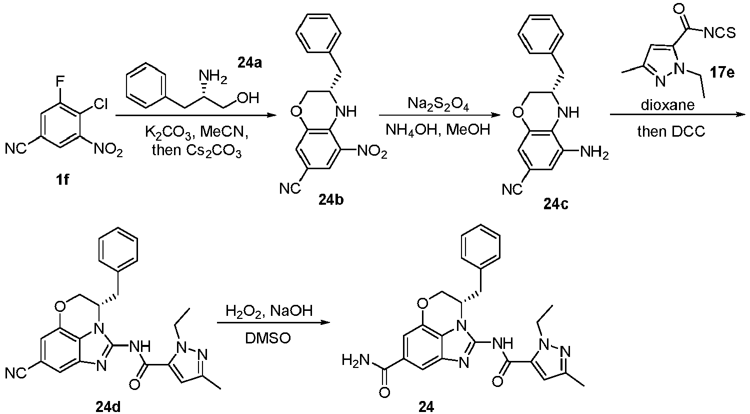

Step 1: To a stirring solution of compound **1f** (500 mg,2.49 mmol) and compound **24a** (754 mg, 4.99 mmol) in acetonitrile (10 mL) was added K₂CO₃ (1.03 g, 7.48 mmol). The reaction mixture was heated at 70 °C, and monitored by TLC. After the starting material was consumed, Cs₂CO₃ (2.43 g, 7.48 mmol) was added to the reaction. Stirring was continued at 70 °C for about 2 hours, and LCMS indicated the product was formed. The reaction mixture was allowed to cool to room temperature, filtered through a pad of celite, and rinsed with DCM. The filtrated was concentrated, and the residue was purified by silica gel chromatography to give compound **24b** (570 mg, 73% yield) as a red solid.

**Step 2:** Compound **24b** (570 mg, 1.93 mmol) was dissolved in MeOH (30 mL) and concentrated ammonium hydroxide (3 mL). Sodium dithionite (1.68 g, 9.65 mmol) was dissolved in water (3 mL) and added to the reaction mixture at room temperature. Stirring was continued at room temperature for half an hour, and TLC indicated the starting material was consumed. The reaction mixture was concentrated, the residue was mixed with water (20 mL), and extracted with EtOAc (15 mL × 3). The combined organic layers were washed with bribe, dried over Na₂SO₄, filtered and concentrated to give compound **24c** (431 mg, 85% yield) as a light-yellow solid.

**Step 3:** Compound **24c** (431 mg, 1.62 mmol) was dissolved in dioxane (5 mL), and then compound **17e** (0.4M in dioxane, 4.0 mL,1.62 mmol) was added. The reaction mixture was stirred at room temperature for 1 hour, LCMS indicated the starting material was consumed. DCC (335 mg, 1.62 mmol) was added, and the mixture was heated at 80 °C for 2 hours, LCMS indicated the product was formed. The reaction mixture was concentrated to give crude compound **23d** (820 mg) as brown oil, which was used directly without further purification.

**Step 4:** To a stirring solution of compound **24d** (118 mg, from step 3) and NaOH (55 mg, 1.38 mmol) in DMSO (3 mL) at 50 °C was added hydrogen peroxide (30 wt. %, 0.6 mL) dropwise. The reaction was then heated at 50 °C for 30 minutes, LCMS indicated the reaction was complete. The reaction mixture was cooled to room temperature and filtered. The filtrate was purified directly by reversed phase preparative HPLC to give compound **24** (22 mg, 21% yield for 2 steps) as a white solid. ESI-MS (m/z): 445.5 [M+H]⁺; ¹H NMR (500 MHz, DMSO-*d*₆) δ 12.66 (br s, 1H), 8.39 (s, 1H), 7.93 (s, 1H), 7.60 (s, 1H), 7.42-7.28 (m, 4H), 7.27-7.19 (m, 3H), 6.63 (s, 1H), 4.86 (br s, 1H), 4.73-4.54 (m, 2H), 4.44 (d, *J* = 11.9 Hz, 1H), 4.25 (d, *J* = 11.8 Hz, 1H), 3.24-3.19 (m, 1H), 3.05-2.96 (m, 1H), 2.20 (s, 3H), 1.37 (t, *J* = 7.1 Hz, 3H).

### Example 25

### (S)-2-(1-Ethyl-3-methyl-1H-pyrazole-5-carboxamido)-3-isopropyl-3,4-dihydro-5-oxa-1,2a-di azaacenaphthylene-7-carboxamide

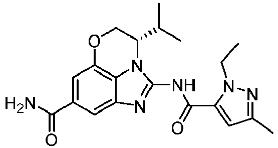

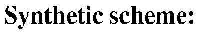

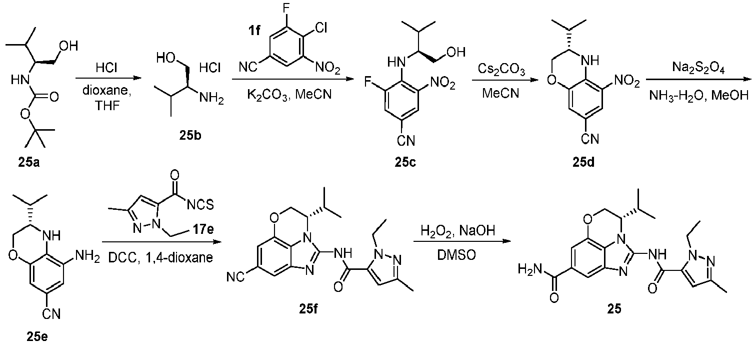

Step 1: To a stirring solution of compound **25a** (4 g, 19.68 mmol) in THF (20 mL) was added 4M HCl in dioxane (10 mL) at room temperature. The mixture was stirred at room temperature for 2 hours. The mixture was concentrated to remove the solvent, and the residue was triturated with petroleum ether (20 mL). The formed solid was collected by filtration and dried in vacuo to give compound **25b** (1.8 g, 65% yiled) as white solid.

Step 2: To a stirring solution of compound **1f** (1.3 g, 6.48 mmol) and compound **25b** (1.8 g,12.96 mmol) in acetonitrile (15 mL) was added K₂CO₃ (2.69 g,19.45 mmol). The reaction mixture was heated at 70 °C overnight, and TLC indicated the starting material was consumed. The reaction mixture was allowed to cool to room temperature, filtered through a pad of celite and rinsed with DCM. The filtrated was concentrated, and the residue was purified by silica gel chromatography to give compound **25c** (1.6 g, 92% yield) as yellow solid.

Step 3: To a stirring solution of compound **25c** (1.6 g, 5.99 mmol) in acetonitrile (20 mL) was added Cs₂CO₃ (3.9 g, 11.97 mmol). The reaction mixture was heated at 70 °C for 3 hours, and TLC indicated the reaction was complete. The reaction mixture was allowed to cool to room temperature, filtered through a pad of celite. The filtrated was concentrated, and the residue was purified by silica gel chromatography to give compound **25d** (1.3 g, 88% yield) as yellow solid.

Step 4: Compound **25d** (700 mg, 2.83 mmol) was dissolved in MeOH (20 mL) and concentrated ammonium hydroxide (5 mL). Sodium dithionite (1.48 g, 8.49 mmol) was dissolved in water (2 mL) and added to the reaction mixture at room temperature. Stirring was continued at room temperature for half an hour, the color of the mixture turned to pink from yellow, and TLC indicated the starting material was consumed. The reaction mixture was diluted with water (20 mL), extracted with EtOAc (15 mL × 3). The combined organic layers were washed with brine, dried over Na₂SO₄, filtered and concentrated to give compound **25e** (510 mg, 83% yield) as red solid. ESI-MS (m/z): 218.6 [M+H]⁺.

Step 5: Compound **25e** (200 mg, 0.92 mmol) was dissolved in dioxane (5 mL), and then compound **17e** (0.4M in dioxane, 2.5 mL,1.01 mmol) was added. The reaction mixture was stirred at room temperature for 10 minutes, LCMS indicated the starting material was consumed. DCC (209 mg, 1.01 mmol) was added, and the mixture was heated at 80 °C for 2 hours, LCMS indicated the product was formed. The reaction mixture was concentrated to give crude compound **25f** (300 mg) as red oil, which was used directly without further purification. ESI-MS (m/z): 379.5 [M+H]⁺.

Step 6: To a stirring solution of compound **25f** (300 mg, from step 5) and NaOH (87 mg, 2.19 mmol) in DMSO (4 mL) at room temperature was added hydrogen peroxide (30 wt. %, 1.5 mL) dropwise. The reaction was then stirred at room temperature for 30 minutes, LCMS indicated the reaction was complete. The reaction mixture was filtered, and the filtrate was purified directly by reversed phase preparative HPLC to give compound **25** (117 mg, 32% yield for 2 steps) as white solid. ESI-MS (m/z): 397.5 [M+H]⁺; ¹H NMR (500 MHz, DMSO-*d6*) δ 12.75 (s, 1H), 8.15 (s, 1H), 7.91 (s, 1H), 7.60 (s, 1H), 7.32 (s, 1H), 7.30 (s, 1H), 6.63 (s, 1H), 4.80 (d, *J* = 12.0 Hz, 1H), 4.68-4.54 (m, 2H), 4.47-4.40 (m, 1H), 4.18 (dd, *J* = 12.5 and 3.5 Hz, 1H), 2.35-2.24 (m, 1H), 2.18 (s, 3H), 1.35 (t, *J* = 7.1 Hz, 3H), 1.01 (d, *J* = 6.9 Hz, 3H), 0.94 (d, *J* = 6.9 Hz, 3H).

### Example 26

### (S)-3-Ethyl-2-(1-ethyl-3-methyl-1H-pyrazole-5-carboxamido)-3,4-dihydro-5-oxa-1,2a-diazaa cenaphthylene-7-carboxamide

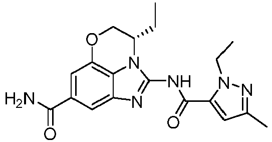

### Synthetic scheme:

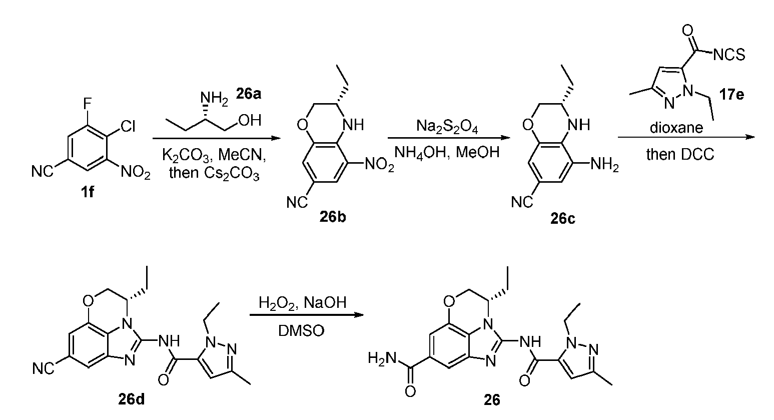

**Step 1:** To a stirring solution of compound **1f** (500 mg, 2.49 mmol) and compound **26a** (444 mg, 4.99 mmol) in acetonitrile (10 mL) was added K₂CO₃ (861 mg, 6.23 mmol). The reaction mixture was heated at 70 °C for 2 hurs, TLC indicated the starting material was consumed. Cs₂CO₃ (2.43 g, 7.48 mmol) was added to the reaction. Stirring was continued at 70 °C for about 2 hours, and TLC indicated the product was formed. The reaction mixture was allowed to cool to room temperature, filtered through a pad of celite, and rinsed with DCM. The filtrated was concentrated to give crude compound **26b** (500 mg) as a yellow solid, which was used directly without further purification.

**Step 2:** Compound **26b** (500 mg, from step 1) was dissolved in MeOH (20 mL) and concentrated ammonium hydroxide (5 mL). Sodium dithionite (1.49 g, 8.58 mmol) was dissolved in water (3 mL) and added to the reaction mixture at room temperature. Stirring was continued at room temperature for half an hour, the color of the mixture turned to pink from yellow, and TLC indicated the starting material was consumed. The reaction mixture was diluted with water (20 mL), extracted with EtOAc (15 mL × 3). The combined organic layers were washed with brine, dried over Na₂SO₄, filtered and concentrated to give crude compound **26c** (250 mg) as red solid, which was used directly without further purification. ESI-MS (m/z): 204.6 [M+H]⁺.

**Step 3:** Compound **26c** (250 mg, from step 2) was dissolved in dioxane (10 mL), and then compound **17e** (0.4M in dioxane, 3.4 mL, 1.35 mmol) was added. The reaction mixture was stirred at room temperature for 20 minutes, LCMS indicated the starting material was consumed. DCC (279 mg, 1.35 mmol) was added, and the mixture was heated at 80 °C for 2 hours, LCMS indicated the product was formed. The reaction mixture was concentrated to give crude compound **26d** (400 mg) as brown oil, which was used directly without further purification. ESI-MS (m/z): 365.5 [M+H]⁺.

**Step 4:** To a stirring solution of compound **26d** (400 mg, from step 3) and NaOH (131 mg, 3.29 mmol) in DMSO (5 mL) at room temperature was added hydrogen peroxide (30 wt. %, 2 mL) dropwise. The reaction was then stirred at room temperature for 30 minutes, LCMS indicated the reaction was complete. The reaction mixture was filtered, and the filtrate was purified directly by reversed phase preparative HPLC to give compound **26** (145 mg, 15% yield for four steps) as a white solid. ESI-MS (m/z): 383.5 [M+H]⁺; ¹H NMR (500 MHz, DMSO-*d*₆) δ 12.73 (s, 1H), 7.93 (s, 1H), 7.60 (s, 1H), 7.34 (s, 1H), 7.31 (s, 1H), 6.64 (s, 1H), 4.74-4.50 (m, 4H), 4.32-4.25 (m, 1H), 2.19 (s, 3H), 1.93-1.75 (m, 2H), 1.37 (t, *J* = 7.1 Hz, 3H), 1.00 (t, *J* = 7.5 Hz, 3H).

### Example 27

### 1-(1-Ethyl-3-methyl-1H-pyrazole-5-carboxamido)-8,8-dimethyl-8,9-dihydro-7H-6-oxa-2,9a-diazabenzo[cd]azulene-4-carboxamide

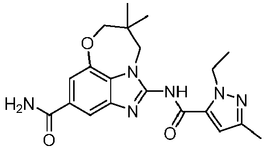

### Synthetic scheme:

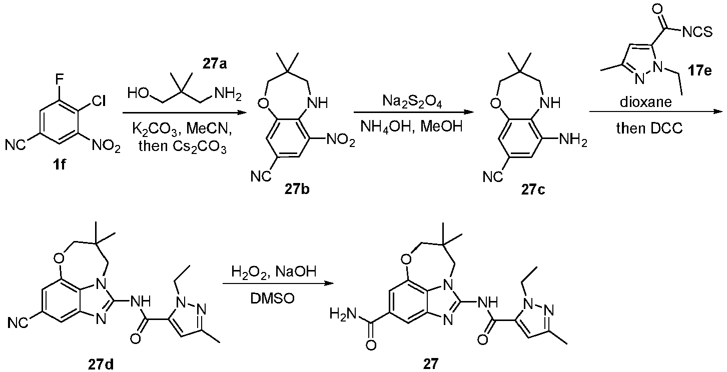

Step 1: To a stirring solution of compound **1f** (500 mg, 2.49 mmol) and compound **27a** (514 mg, 4.99 mmol) in acetonitrile (20 mL) was added K₂CO₃ (861 mg, 6.23 mmol). The reaction mixture was heated at 70 °C for 2 hurs, TLC indicated the starting material was consumed. Cs₂CO₃ (1.62 g, 4.99 mmol) was added to the reaction. Stirring was continued at 70 °C for about 2 hours, and TLC indicated the product was formed. The reaction mixture was allowed to cool to room temperature, filtered through a pad of celite, and rinsed with DCM. The filtrated was concentrated to give compound **27b** (550 mg, 89% yield) as yellow solid.

Step 2: Compound **27b** (550 mg, 2.22 mmol) was dissolved in MeOH (10 mL) and concentrated ammonium hydroxide (3 mL). Sodium dithionite (1.55 g, 8.9 mmol) was dissolved in water (2 mL) and added to the reaction mixture at room temperature. Stirring was continued at room temperature for half an hour, and TLC indicated the starting material was consumed. The reaction mixture was diluted with water (20 mL), extracted with EtOAc (15 mL × 3). The combined organic layers were washed with brine, dried over Na₂SO₄, filtered and concentrated to give crude compound **27c** (250 mg) as red solid, which was used directly without further purification. ESI-MS (m/z): 218.7 [M+H]⁺.

Step 3: Compound **27c** (250 mg, from step 2) was dissolved in dioxane (10 mL), and then compound **17e** (0.4M in dioxane, 3.2 mL, 1.28 mmol) was added. The reaction mixture was stirred at room temperature for 20 minutes, LCMS indicated the starting material was consumed. DCC (261 mg, 1.26 mmol) was added, and the mixture was heated at 80 °C for 2 hours, LCMS indicated the product was formed. The reaction mixture was concentrated to give crude compound **27d** (390 mg) as red oil, which was used directly without further purification. ESI-MS (m/z): 379.5 [M+H]⁺.

Step 4: To a stirring solution of compound **27d** (390 mg, from step 3) and NaOH (123 mg, 3.09 mmol) in DMSO (5 mL) at room temperature was added hydrogen peroxide (30 wt. %, 2 mL) dropwise. The reaction was then stirred at room temperature for 30 minutes, LCMS indicated the reaction was complete. The reaction mixture was filtered, and the filtrate was purified directly by reversed phase preparative HPLC to give compound **27** (100 mg, 12% yield for 3 steps) as a white solid. ESI-MS (m/z): 397.5 [M+H]⁺; ¹H NMR (500 MHz, DMSO-*d*₆) δ 12.87 (br s, 1H), 7.92 (s, 1H), 7.67 (s, 1H), 7.40-7.27 (m, 2H), 6.71 (s, 1H), 4.61 (q, *J* = 7.0 Hz, 2H), 4.15 (s, 2H), 3.97 (s, 2H), 2.19 (s, 3H), 1.36 (t, *J* = 7.1 Hz, 3H), 1.10 (s, 6H).

### Example 28

### 2-(1-Ethyl-3-methyl-1H-pyrazole-5-carboxamido)-3H-5-oxa-1,2a-diazaspiro[acenaphthylen e-4,1'-cyclopropan]-1,2a¹(5a),6,8-tetraene-7-carboxamide

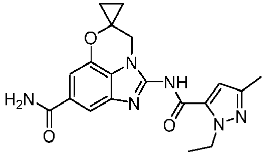

### Synthetic scheme:

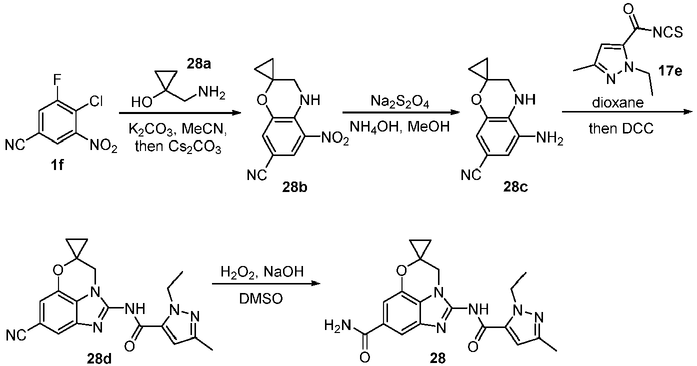

Step 1: To a stirring solution of compound **1f** (500 mg, 2.49 mmol) and compound **28a** (434 mg, 4.99 mmol) in acetonitrile (20 mL) was added K₂CO₃ (861 mg, 6.23 mmol). The reaction mixture was heated at 70 °C for 2 hurs, TLC indicated the starting material was consumed. Cs₂CO₃ (1.62 g, 4.99 mmol) was added to the reaction. Stirring was continued at 70 °C for 24 hours, and TLC indicated the product was formed. The reaction mixture was allowed to cool to room temperature, filtered through a pad of celite, and rinsed with DCM. The filtrated was concentrated to give compound **28b** (180 mg, 31% yield) as yellow solid.

Step 2: Compound **28b** (180 mg, 2.14 mmol) was dissolved in MeOH (10 mL) and concentrated ammonium hydroxide (3 mL). Sodium dithionite (407 mg, 2.34 mmol) was dissolved in water (2 mL) and added to the reaction mixture at room temperature. Stirring was continued at room temperature for half an hour, and TLC indicated the starting material was consumed. The reaction mixture was diluted with water (20 mL), extracted with EtOAc (15 mL × 3). The combined organic layers were washed with brine, dried over Na₂SO₄, filtered and concentrated to give crude compound **28c** (120 mg) as red solid, which was used directly without further purification. ESI-MS (m/z): 202.5 [M+H]⁺.

Step 3: Compound **28c** (120 mg, from step 2) was dissolved in dioxane (10 mL), and then compound **17e** (0.4M in dioxane, 1.6 mL, 0.65 mmol) was added. The reaction mixture was stirred at room temperature for 20 minutes, LCMS indicated the starting material was consumed. DCC (135 mg, 0.66 mmol) was added, and the mixture was heated at 80 °C for 2 hours, LCMS indicated the product was formed. The reaction mixture was concentrated to give crude compound **28d** (200 mg) as brown oil, which was used directly without further purification. ESI-MS (m/z): 363.4 [M+H]⁺.

Step 4: To a stirring solution of compound **28d** (200 mg, from step 3) and NaOH (66 mg, 1.66 mmol) in DMSO (4 mL) at room temperature was added hydrogen peroxide (30 wt. %, 1 mL) dropwise. The reaction was then stirred at room temperature for 30 minutes, LCMS indicated the reaction was complete. The reaction mixture was filtered, and the filtrate was purified directly by reversed phase preparative HPLC to give compound **28** (52 mg, 6% yield for 3 steps) as a white solid. ESI-MS (m/z): 381.5 [M+H]⁺; ¹H NMR (500 MHz, DMSO-*d*₆) δ 12.65 (br s, 1H), 7.91 (s, 1H), 7.63 (s, 1H), 7.31 (s, 1H), 7.28(s, 1H), 6.66 (s, 1H), 4.60 (q, *J* = 7.1 Hz, 2H), 4.25 (s, 2H), 2.17 (s, 3H), 1.35 (t, *J* = 7.1 Hz, 3H), 1.15-1.09 (m, 2H), 1.06-0.99 (m, 2H).

### Example 29

### 2-(1-Ethyl-3-methyl-1H-pyrazole-5-carboxamido)-3-methyl-3-((methyl(phenethyl)amino)m ethyl)-3,4-dihydro-5-oxa-1,2a-diazaacenaphthylene-7-carboxamide

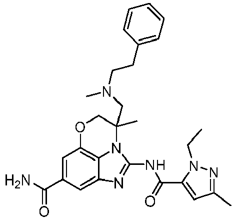

### Synthetic scheme:

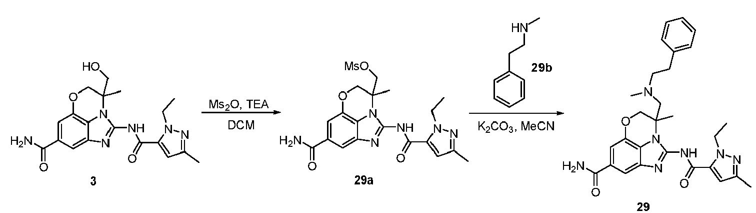

Step 1: To a solution of compound **3** (50 mg, 0.13 mmol) in DCM (3 mL) was added triethylamine (73 mg, 0.72 mmol) and methanesulfonic anhydride (67 mg, 0.38 mmol). The mixture was stirred at room temperature for 30 minutes, LCMS indicated the reaction was complete. The reaction was diluted with water (10 mL) and extracted with EtOAc (20 mL × 3). The combined organic layers were washed with brine, dried over Na₂SO₄, filtered and concentrated to yield the product **29a** (30 mg) as yellow solid, which was used directly without further purification. ESI-MS (m/z): 477 [M+H]⁺.

Step 2: To a solution of compound **29a** (30 mg, from step 1) in acetonitrile (3 mL) was added K₂CO₃ (17 mg, 0.13 mmol) and compound **29b** (43 mg, 0.31 mmol). The mixture was heated at 40 °C for 5 hours, LCMS indicated the reaction was complete. The reaction mixture was diluted with water (10 mL) and extracted with EtOAc (20 mL × 3). The combined organic layers were washed with brine, dried over Na₂SO₄, filtered and concentrated. The residue was purified by reversed phase preparative HPLC to give the compound **29** (5 mg, 7% yield for 2 steps) as white solid. ESI-MS (m/z): 516.5 [M+H]⁺; ¹H NMR (500 MHz, DMSO-*d*₆) δ 8.45 (t, *J* = 6.5 Hz, 1H), 7.84 (s, 1H), 7.69 (s, 1H), 7.31-7.24 (m, 2H), 7.23-7.15 (m, 5H), 6.45 (s, 1H), 4.41 (d, *J* = 11.7 Hz, 1H), 4.35-4.22 (m, 2H), 3.98 (d, *J* = 11.6 Hz, 1H), 3.84-3.74 (m, 1H), 3.50-3.40 (m, 4H), 2.93 (s, 3H), 2.86 (t, *J* = 7.9 Hz, 2H), 2.12 (s, 3H), 1.56 (s, 3H), 1.21 (t, *J* = 7.2 Hz, 3H).

### Example 30

### (S)-3-((Benzyloxy)methyl)-2-(1-ethyl-3-methyl-1H-pyrazole-5-carboxamido)-3,4-dihydro-5-oxa-1,2a-diazaacenaphthylene-7-carboxamide

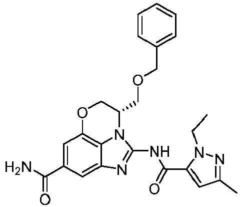

### Synthetic scheme:

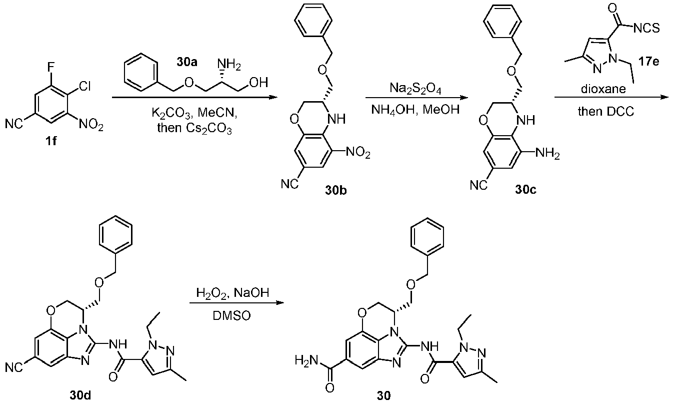

Step 1: To a stirring solution of compound **1f** (500 mg, 2.49 mmol) and compound **30a** (903 mg, 4.99 mmol) in acetonitrile (10 mL) was added K₂CO₃ (1.03 g, 7.48 mmol). The reaction mixture was heated at 70 °C and monitored TLC. After the starting material was consumed, indicated the starting material was consumed. Cs₂CO₃ (2.43 g, 7.48 mmol) was added to the reaction. Stirring was continued at 70 °C for 2 hours, and TLC indicated the product was formed. The reaction mixture was allowed to cool to room temperature, filtered through a pad of celite, and rinsed with EtOAc. The filtrated was concentrated, and the residue was purified by silica gel chromatography to give compound **30b** (670 mg, 83% yield) as yellow solid.

Step 2: Compound **30b** (670 mg, 1.94 mmol) was dissolved in MeOH (30 mL) and concentrated ammonium hydroxide (3 mL). Sodium dithionite (1.69 g, 9.70 mmol) was dissolved in water (3 mL) and added to the reaction mixture at room temperature. Stirring was continued at room temperature for half an hour, and TLC indicated the starting material was consumed. The reaction mixture was concentrated to remove the solvent, the residue was mixed with water (20 mL), extracted with EtOAc (15 mL × 3). The combined organic layers were washed with brine, dried over Na₂SO₄, filtered and concentrated to give crude compound **30c** (540 mg) as red solid, which was used directly without further purification. ESI-MS (m/z): 296.7 [M+H]⁺.

Step 3: Compound **30c** (100 mg, from step 2) was dissolved in dioxane (5 mL), and then compound **17e** (0.4M in dioxane, 0.85 mL, 0.338 mmol) was added. The reaction mixture was stirred at room temperature for 1 hour, LCMS indicated the starting material was consumed. DCC (70 mg, 0.34 mmol) was added, and the mixture was heated at 80 °C for 2 hours, LCMS indicated the product was formed. The reaction mixture was concentrated to give crude compound **30d** (220 mg) as brown oil, which was used directly without further purification. ESI-MS (m/z): 457.6 [M+H]⁺.

Step 4: To a stirring solution of compound **30d** (220 mg, from step 3) and NaOH (60 mg, 1.49 mmol) in DMSO (3 mL) at 50 °C was added hydrogen peroxide (30 wt. %, 0.6 mL) dropwise. The reaction was heated at 50 °C for 30 minutes, LCMS indicated the reaction was complete. The reaction mixture was filtered, and the filtrate was purified directly by reversed phase preparative HPLC to give compound **30** (30 mg, 16% yield for 3 steps) as a white solid. ESI-MS (m/z): 475.5 [M+H]⁺; ¹H NMR (500 MHz, DMSO-*d6*) δ 12.58 (s, 1H), 7.94 (s, 1H), 7.60 (s, 1H), 7.44-7.18 (m, 7H), 6.58 (s, 1H), 4.80 (br s, 1H), 4.75 (d, *J* = 12.0 Hz, 1H), 4.66-4.45 (m, 4H), 4.37 (d, *J* = 11.5 Hz, 1H), 3.88-3.71 (m, 2H), 2.18 (s, 3H), 1.33 (t, *J* = 6.8 Hz, 3H).

### Example 31

### (S)-3-(3-Cinnamamidopropyl)-2-(1-ethyl-3-methyl-1H-pyrazole-5-carboxamido)-3,4-d ihydro-5-oxa-1,2a-diazaacenaphthylene-7-carboxamide

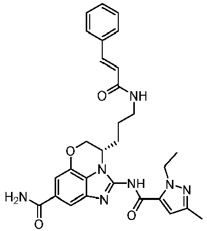

### Synthetic scheme:

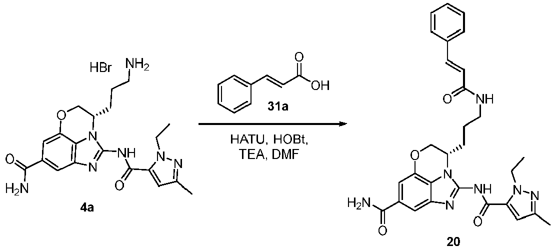

Step 1: To a solution of cinnamic acid (45 mg, 0.30 mmol) in DMF (2 mL) at room temperature was added HATU (154 mg, 0.41 mmol), HOBt (28 mg, 0.20 mmol) under nitrogen atmosphere. The mixture was stirred at room temperature for 30 minutes, triethylamine (123 mg, 1.22 mmol) was added. Then after 10 minutes, compound **4a** (100 mg, 0.20 mmol) was added to the reaction. The reaction mixture was stirred at room temperature overnight. LCMS indicated the reaction was complete. The mixture was purified directly by reversed phase preparative HPLC to give compound **31** (22 mg, 13% yield) as white solid. ESI-MS (m/z): 542.5 [M+H]⁺; ¹H NMR (500 MHz, DMSO-*d6*) δ 12.71 (br s, 1H), 8.12 (s, 1H), 7.93 (s, 1H), 7.60 (s, 1H), 7.52 (d, *J* = 7.0 Hz, 2H), 7.45-7.33 (m, 5H), 7.31 (s, 1H), 6.64 (s, 1H), 6.55 (d, *J* = 16.0 Hz, 1H), 4.73-4.52 (m, 4H), 4.28 (d, *J* = 11.0 Hz, 1H), 3.26-3.15 (m, 2H), 2.14 (s, 3H), 1.90-1.79 (m, 2H), 1.68-1.57 (m, 2H), 1.35 (t, *J* = 7.1 Hz, 3H).

### Example 32

### Pyridin-3-ylmethyl (S)-(3-(7-carbamoyl-2-(1-ethyl-3-methyl-1H-pyrazole-5-carboxamido)-3,4-dihydro-5-oxa-1, 2a-diazaacenaphthylen-3-yl)propyl)carbamate

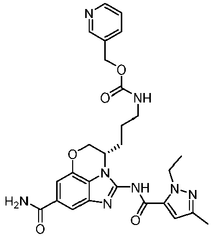

### Synthetic scheme:

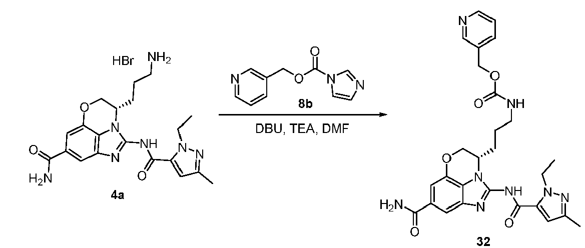

Step 1: To a solution of compound **4a** (100 mg) in DMF (2 mL) was added compound **8b** (103 mg, 0.51 mmol), DBU (77 mg, 0.51 mmol) and triethylamine (77 mg, 0.76 mmol), the reaction mixture was heated at 50 °C overnight. LCMS indicated the reaction was complete. The reaction mixture was diluted with water (15 mL), extracted with EtOAc (15 mL × 3). The combined organic layers were dried over Na₂SO₄, filtered and concentrated. The residue was purified by reversed phase preparative HPLC to give compound **32** (18 mg) as a white solid. ESI-MS (m/z): 547.5 [M+H]⁺; ¹H NMR (500 MHz, DMSO) δ 12.61 (br s, 1H), 8.53 (s, 1H), 8.51 (s, 1H), 8.34 (s, 1H), 7.93 (s, 1H), 7.70 (d, *J* = 7.2 Hz, 1H), 7.60 (s, 1H), 7.39-7.26 (m, 4H), 6.65 (s, 1H), 5.10-4.95 (m, 2H), 4.71-4.50 (m, 4H), 4.26 (d, *J* = 11.5 Hz, 1H), 3.13-2.97 (m, 2H), 2.17 (s, 3H), 1.84-1.77 (m, 2H), 1.62-1.53 (m, 2H), 1.35 (t, *J* = 6.2 Hz, 3H).

### Example 33

### (S)-2-(1-ethyl-3-methyl-1H-pyrazole-5-carboxamido)-3-(3-(2-phenoxyacetamido)propyl)-3,4 -dihydro-5-oxa-1,2a-diazaacenaphthylene-7-carboxamide

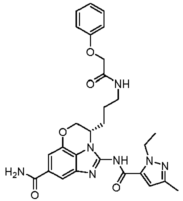

### Synthetic scheme:

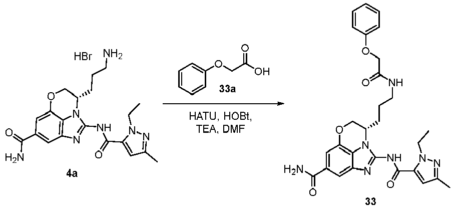

Step 1: To a solution of compound **33a** (58 mg, 0.38 mmol) in DMF (3 mL) was added HATU (144 mg, 0.38 mmol), HOBt (26 mg, 0.19 mmol). The mixture was stirred at room temperature for 30 minutes, triethylamine (57 mg, 0.57 mmol) was added. Then after 10 minutes, compound **4a** (100 mg, 0.20 mmol) was added to the reaction. The reaction mixture was stirred at room temperature overnight. LCMS indicated the reaction was complete. The mixture was purified directly by reversed phase preparative HPLC to give compound **33** (15 mg) as white solid. ESI-MS (m/z): 546.5 [M+H]⁺; ¹H NMR (500 MHz, DMSO) δ 12.71 (s, 1H), 8.11-8.04 (m, 1H), 7.93 (s, 1H), 7.61 (s, 1H), 7.35 (s, 1H), 7.30 (s, 1H), 7.27-7.20 (m, 2H), 6.97-6.85 (m, 3H), 6.66 (s, 1H), 4.72-4.55 (m, 4H), 4.45-4.35 (m, 2H), 4.26 (d, *J* = 11.5 Hz, 1H), 3.26-3.19 (m, 1H), 3.18-3.11 (m, 1H), 2.17 (s, 3H), 1.84-1.70 (m, 2H), 1.68-1.54 (m, 2H), 1.36 (t, *J* = 7.0 Hz, 3H).

### Example 34

### 2-(1-Ethyl-3-methyl-1H-pyrazole-5-carboxamido)-3-(((4-methoxybenzyl)(methyl)amino)met hyl)-3-methyl-3,4-dihydro-5-oxa-1,2a-diazaacenaphthylene-7-carboxamide

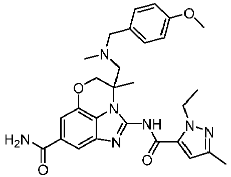

### Synthetic scheme:

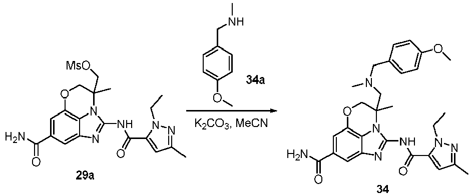

Step 1: To a solution of compound **29a** (50 mg, 0.11 mmol) in acetonitrile (3 mL) was added K₂CO₃ (29mg, 0.21 mmol) and compound **34a** (397mg, 2.62 mmol). The mixture was heated at 40 °C for 3 hours, LCMS indicated the reaction was complete. The reaction mixture was diluted with water (10 mL) and extracted with EtOAc (20 mL × 3). The combined organic layers were washed with brine, dried over Na₂SO₄, filtered and concentrated. The residue was purified by reversed phase preparative HPLC to give the compound **34** (14 mg, 25% yield) as white solid. ESI-MS (m/z): 532.5 [M+H]⁺; ¹H NMR (500 MHz, DMSO-*d*₆) δ 8.52 (t, *J* = 6.5 Hz, 1H), 7.84 (s, 1H), 7.70 (s, 1H), 7.31 (d, *J* = 8.5 Hz, 2H), 7.22 (s, 1H), 7.18 (s, 1H), 6.88 (d, *J* = 8.6 Hz, 2H), 6.46 (s, 1H), 4.46 (d, *J* = 11.6 Hz, 1H), 4.36-4.19 (m, 4H), 4.03 (d, *J* = 11.7 Hz, 1H), 3.92-3.81 (m, 1H), 3.74 (s, 3H), 3.58-3.48 (m, 1H), 2.71 (s, 3H), 2.11 (s, 3H), 1.68 (s, 3H), 1.21 (t, *J* = 7.1 Hz, 3H).

### Example 35

### (S, E)-2-(1-ethyl-3-methyl-1H-pyrazole-5-carboxamido)-3-(4-(3-(pyridin-3-yl)acrylamido)butyl )-3,4-dihydro-5-oxa-1,2a-diazaacenaphthylene-7-carboxamide

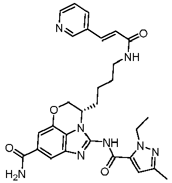

### Synthetic scheme:

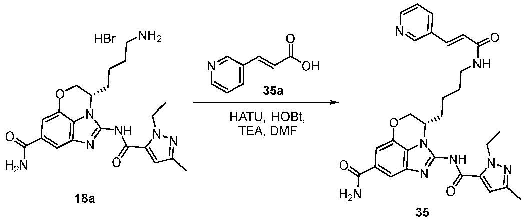

Step 1: To a solution of compound **18a** (30 mg) and compound **35a** (11 mg, 0.07 mmol) in DMF (2 mL) was added HATU (27 mg, 0.07 mmol), HOBt (9 mg, 0.07 mmol) and triethylamine (21 mg, 0.21 mmol). The reaction mixture was stirred at room temperature overnight. TLC indicated the conversion was complete. The reaction mixture was purified directly by reversed phase preparative HPLC to give compound **35** (8 mg) as white solid. ESI-MS (m/z): 557.4[M+H]⁺; ¹H NMR (500 MHz, DMSO-*d*₆) δ 12.72 (s, 1H), 8.74 (s, 1H), 8.55 (d, *J* = 4.7 Hz, 1H), 8.16 (t, *J* = 5.6 Hz, 1H), 8.03-7.91 (m, 2H), 7.60 (s, 1H), 7.49-7.40 (m, 2H), 7.36-7.30 (m, 2H), 6.69 (d, *J* = 16.0 Hz, 1H), 6.63 (s, 1H), 4.70-4.56 (m, 4H), 4.28 (d, *J* = 11.3 Hz, 1H), 3.23-3.14 (m, 2H), 2.16 (s, 3H), 1.88-1.78 (m, 2H), 1.59-1.46 (m, 4H), 1.35 (t, *J* = 7.1 Hz, 3H).

### Example 36

### (S, E)-2-(1-ethyl-3-methyl-1H-pyrazole-5-carboxamido)-3-(4-(3-(pyridin-4-yl)acrylamido)butyl )-3,4-dihydro-5-oxa-1,2a-diazaacenaphthylene-7-carboxamide

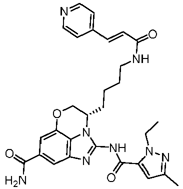

### Synthetic scheme:

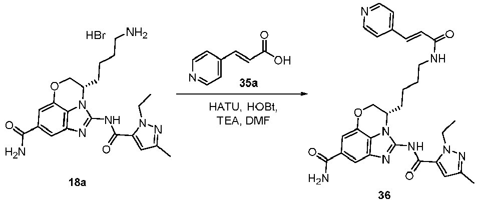

Step 1: To a solution of compound **18a** (30 mg) and compound **36a** (11 mg, 0.07 mmol) in DMF (2 mL) was added HATU (27 mg, 0.07 mmol), HOBt (9 mg, 0.07 mmol) and triethylamine (21 mg, 0.21 mmol). The reaction mixture was stirred at room temperature overnight. TLC indicated the conversion was complete. The reaction mixture was purified directly by reversed phase preparative HPLC to give compound **36** (8 mg). ESI-MS (m/z): 557.4 [M+H]⁺; ¹H NMR (500 MHz, DMSO-*d*₆) δ 12.74 (s, 1H), 8.67 (s, 2H), 8.28 (s, 1H), 7.94 (s, 1H), 7.65-7.57 (m, 3H), 7.38 (d, *J* = 15.5 Hz, 1H), 7.35-7.30 (m, 2H), 6.85 (d, *J* = 16.2 Hz, 1H), 6.64 (s, 1H), 4.70-4.57 (m, 4H), 4.28 (d, *J* = 11.6 Hz, 1H), 3.25-3.15 (m, 2H), 2.16 (s, 3H), 1.86-1.80 (m, 2H), 1.60-1.45 (m, 4H), 1.35 (t, *J* = 7.1 Hz, 3H).

### Example 37

### (S,E)-3-(2-(3-(4-cyanophenyl)acrylamido)ethyl)-2-(1-ethyl-3-methyl-1H-pyrazole-5-carboxa mido)-3,4-dihydro-5-oxa-1,2a-diazaacenaphthylene-7-carboxamide

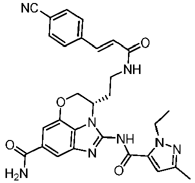

### Synthetic scheme:

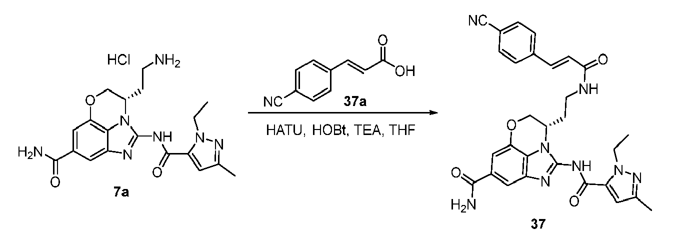

Step 1: To a solution of compound **7a** (75 mg) and compound **37a** (30 mg, 0.17 mmol) in DMF (3 mL) was added HATU (72mg, 0.19 mmol), HOBt (26 mg, 0.19 mmol) and triethylamine (53 mg, 0.52 mmol). The reaction mixture was stirred at room temperature overnight. LCMS indicated the conversion was complete. The reaction mixture was purified directly by reversed phase preparative HPLC to give compound **37** (25 mg) as white solid. ESI-MS (m/z): 553.4 [M+H]⁺; ¹H NMR (500 MHz, DMSO-*d*₆) δ 12.65 (br s, 1H), 8.42 (t, *J* = 5.9 Hz, 1H), 7.94 (s, 1H), 7.89 (d, *J* = 7.9 Hz, 2H), 7.77 (d, *J* = 8.0 Hz, 2H), 7.60 (s, 1H), 7.50 (d, *J* = 15.8 Hz, 1H), 7.36 (s, 1H), 7.33 (s, 1H), 6.77 (d, *J* = 15.8 Hz, 1H), 6.65 (s, 1H), 4.80-4.69 (m, 2H), 4.59 (q, *J* = 7.5 Hz, 2H), 4.32 (d, *J* = 11.5 Hz, 1H), 3.53-3.47 (m, 1H), 2.05 (s, 3H), 2.03-1.91 (m, 2H), 1.33 (t, *J* = 7.1 Hz, 3H).

### Example 38

### (S,E)-2-(1-ethyl-3-methyl-1H-pyrazole-5-carboxamido)-3-(2-(3-(3-fluorophenyl)acryla mido)ethyl)-3,4-dihydro-5-oxa-1,2a-diazaacenaphthylene-7-carboxamide

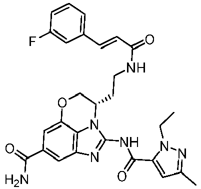

### Synthetic scheme:

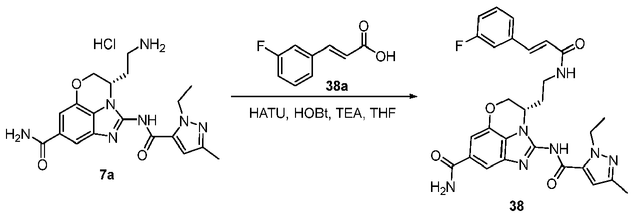

Step 1: To a solution of compound **7a** (75 mg) and compound **38a** (30 mg, 0.18 mmol) in DMF (3 mL) was added HATU (75 mg, 0.20 mmol), HOBt (27 mg, 0.20 mmol) and triethylamine (55 mg, 0.54 mmol). The reaction mixture was stirred at room temperature overnight. LCMS indicated the conversion was complete. The reaction mixture was purified directly by reversed phase preparative HPLC to give compound **38** (20 mg) as white solid. ESI-MS (m/z): 546.4 [M+H]⁺; ¹H NMR (500 MHz, DMSO-*d*₆) δ 12.74 (br s, 1H), 8.34 (t, *J* = 5.7 Hz, 1H), 7.95 (s, 1H), 7.60 (s, 1H), 7.50-7.40 (m, 4H), 7.37 (s, 1H), 7.33 (s, 1H), 7.23 (t, *J* = 8.8 Hz, 1H), 6.73-6.62 (m, 2H), 4.76 (d, *J* = 11.5 Hz, 1H), 4.71 (br s, 1H), 4.58 (q, *J* = 7.5 Hz, 2H), 4.32 (d, *J* = 11.0 Hz, 1H), 3.52-3.43 (m, 1H), 2.06 (s, 3H), 2.03-1.93 (m, 2H), 1.33 (t, *J* = 7.1 Hz, 3H).

### Example 39

### (S,E)-2-(1-ethyl-3-methyl-1H-pyrazole-5-carboxamido)-3-(2-(3-(2-methoxyphenyl)acr ylamido)ethyl)-3,4-dihydro-5-oxa-1,2a-diazaacenaphthylene-7-carboxamide

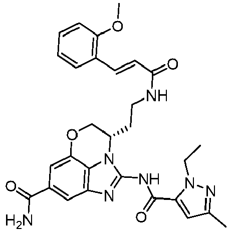

### Synthetic scheme:

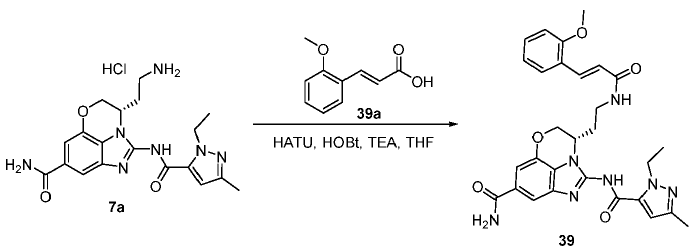

Step 1: To a solution of compound **7a** (73 mg) and compound **39a** (30 mg, 0.17 mmol) in DMF (3 mL) was added HATU (70 mg, 0.18 mmol), HOBt (25 mg, 0.18 mmol) and triethylamine (51 mg,0.51 mmol). The reaction mixture was stirred at room temperature overnight. LCMS indicated the conversion was complete. The reaction mixture was purified directly by reversed phase preparative HPLC to give compound **39** (21 mg) as white solid. ESI-MS (m/z): 558.4 [M+H]⁺; ¹H NMR (500 MHz, DMSO-*d*₆) δ 12.74 (br s, 1H), 8.30 (t, *J* = 5.8 Hz, 1H), 7.95 (s, 1H), 7.69 (d, *J* = 15.9 Hz, 1H), 7.60 (s, 1H), 7.53 (d, *J* = 7.6 Hz, 1H), 7.42-7.31 (m, 3H), 7.08 (d, *J* = 8.3 Hz, 1H), 6.99 (t, *J* = 7.5 Hz, 1H), 6.70-6.62 (m, 2H), 4.79-4.67 (m, 2H), 4.64-4.55 (m, 2H), 4.36-4.29 (m, 1H), 3.86 (s, 3H), 3.51-3.42 (m, 1H), 3.32 (s, 1H), 2.06 (s, 3H), 2.04-1.98 (m, 1H), 1.97-1.90 (m, 1H), 1.33 (t, *J* = 7.1 Hz, 3H).

### Example 40

### (S,E)-2-(1-ethyl-3-methyl-1H-pyrazole-5-carboxamido)-3-(2-(3-(pyridin-3-yl)acrylami do)ethyl)-3,4-dihydro-5-oxa-1,2a-diazaacenaphthylene-7-carboxamide

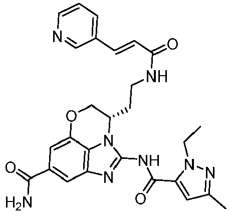

### Synthetic scheme:

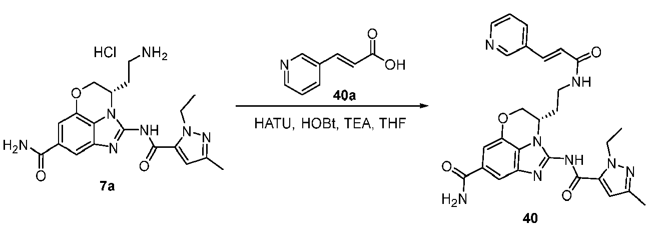

Step 1: To a solution of compound **7a** (133 mg) and compound **40a** (50mg, 0.33 mmol) in DMF (3 mL) was added HATU (127mg, 0.33 mmol), HOBt (45 mg, 0.33 mmol) and triethylamine (102 mg, 1mmol). The reaction mixture was stirred at room temperature overnight. LCMS indicated the conversion was complete. The reaction mixture was purified directly by reversed phase preparative HPLC to give compound **40** (17 mg) as white solid. ESI-MS (m/z): 529.4 [M+H]⁺; ¹H NMR (500 MHz, DMSO-*d*₆) δ 12.73 (br s, 1H), 8.77 (s, 1H), 8.56 (d, *J* = 4.7 Hz, 1H), 8.38 (t, *J* = 5.8 Hz, 1H), 8.00 (d, *J* = 8.0 Hz, 1H), 7.95 (s, 1H), 7.61 (s, 1H), 7.52-7.44 (m, 2H), 7.37 (s, 1H), 7.33 (s, 1H), 6.74 (d, *J* = 16.0 Hz, 1H), 6.66 (s, 1H), 4.76 (d, *J* = 11.5 Hz, 1H), 4.71 (br s, 1H), 4.58 (q, *J* = 7.5 Hz, 2H), 4.32 (d, *J* = 10.5 Hz, 1H), 3.53-3.43 (m, 1H), 2.05 (s, 3H), 2.03-1.92 (m, 2H), 1.33 (t, *J* = 7.1 Hz, 3H).

### Example 41

### Pyridin-2-ylmethyl(S)-(3-(7-carbamoyl-2-(1-ethyl-3-methyl-1H-pyrazole-5-carboxamido)-3, 4-dihydro-5-oxa-1,2a-diazaacenaphthylen-3-yl)propyl)carbamate

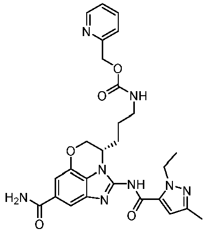

### Synthetic scheme:

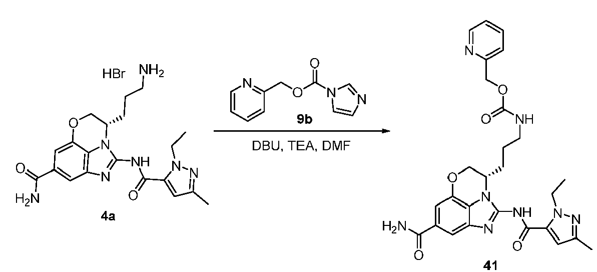

Step 1: To a solution of compound **4a** (100 mg) and compound **9b** (103mg, 0.51 mmol) in DMF (3 mL) was added triethylamine (64 mg, 0.64 mmol), and DBU (77 mg, 0.51 mmol). The reaction mixture was heated at 50 °C overnight, LCMS indicated the reaction was complete. The reaction mixture was diluted with water (15 mL), extracted with EtOAc (15 mL × 3). The combined organic layers were dried over Na₂SO₄, filtered and concentrated. The residue was purified by silica gel chromatography to give compound **41** (15 mg) as white solid. ESI-MS (m/z): 547.3 [M+H]⁺; ¹H NMR (500 MHz, DMSO) δ 8.49 (d, *J* = 3.9 Hz, 1H), 7.92 (s, 1H), 7.73 (t, *J* = 7.0 Hz, 1H), 7.59 (s, 1H), 7.40-7.22 (m, 5H), 6.64 (s, 1H), 5.11-4.95 (m, 2H), 4.69-4.54 (m, 4H), 4.26 (d, *J* = 12.0 Hz, 1H), 3.12-3.01 (mbr, 2H), 2.16 (s, 3H), 1.86-1.78 (m, 2H), 1.67-1.56 (m, 2H), 1.35 (t, *J* = 7.5 Hz, 3H).

### Example 42

### Phenethyl (S)-(2-(7-carbamoyl-2-(1-ethyl-3-methyl-1H-pyrazole-5-carboxamido)-3,4-dihydro-5-oxa-1, 2a-diazaacenaphthylen-3-yl)ethyl)carbamate

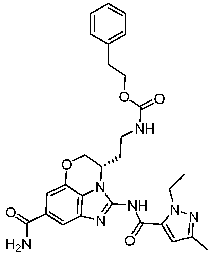

### Synthetic scheme:

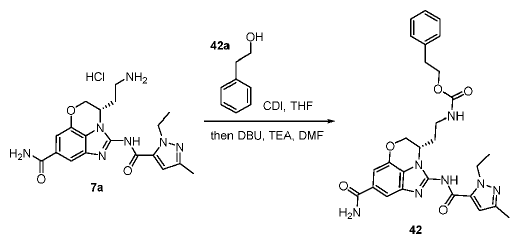

Step 1: To the solution of compound **42a** (28 mg, 0.23 mmol) in THF (5 mL) was added CDI (38 mg, 0.24 mmol). The mixture was stirred at room temperature for 1 hour, TLC indicated the starting material was consumed. Compound **7a** (50 mg) was dissolved in DMF (3 mL), and added to the reaction mixture, followed by the addition of DBU (35 mg, 0.23 mmol) and triethylamine (29 mg, 0.29 mmol). The resulting mixture was heated at 50 °C overnight. LCMS indicated the product was formed. The reaction mixture was purified directly by reversed phase preparative HPLC to give compound **42** (18 mg) as white solid.ESI-MS (m/z): 546.4 [M+H]⁺; ¹H NMR (500 MHz, DMSO-*d*₆) δ 12.72 (br s, 1H), 7.92 (s, 1H), 7.60 (s, 1H), 7.34 (s, 1H), 7.32-7.15 (m, 6H), 6.68 (s, 1H), 4.71-4.55 (m, 4H), 4.26 (d, *J* = 11.5 Hz, 1H), 4.20-4.08 (m, 2H), 3.23-3.16 (m, 1H), 3.15-3.07 (m, 1H), 2.85 (t, *J* = 7.0 Hz, 2H), 2.16 (s, 3H), 1.98-1.82 (m, 2H), 1.35 (t, *J* = 7.1 Hz, 3H).

### Example 43

### Benzyl (S)-(3-(8-carbamoyl-2-(1-ethyl-3-methyl-1H-pyrazole-5-carboxamido)-6-methyl-5,6-dihydro -4H-imidazo[1,5,4-de]quinoxalin-4-yl)propyl)carbamate

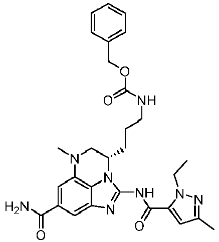

### Synthetic scheme:

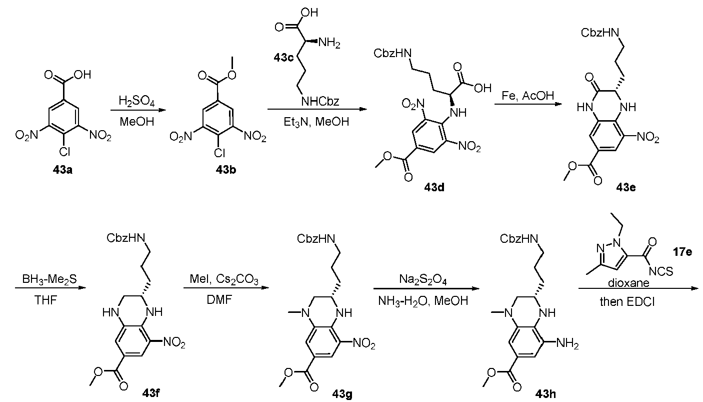

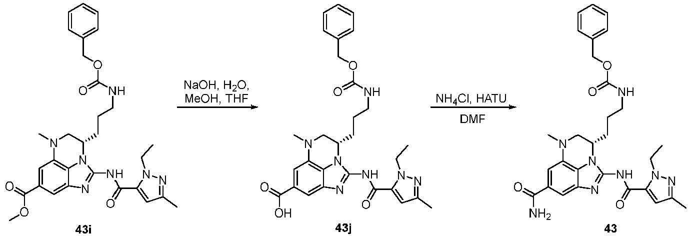

Step 1: Compound **43a** (10.0 g, 40.6 mmol) was dissolved in MeOH (200 mL), followed by addition of concentrated H₂SO₄ (2 mL) at room temperature. The mixture was refluxed overnight, TLC indicated the starting material was consumed. The reaction mixture was concentrated, the residue was mixed with water (20 mL), extracted with EtOAc (15 mL × 3). The combined organic layers were washed with saturated NaHCO₃ solution (10 mL) and brine (10 mL), dried over Na₂SO₄, filtered and concentrated. The residue was purified by silica chromatography to give the compound **43b** (8.2 g, 78% yield) as yellow solid.

Step 2: To a stirring solution of compound **43b** (4.0 g, 15.4 mmol) and compound **43c** (4.9 g, 18.5 mmol) in MeOH (100 mL) was added trimethylamine (3.1 g, 30.8 mmol). The reaction mixture was heated at 80 °C for 3 hours, and LCMS indicated the reaction was complete. The reaction mixture was concentrated to give compound **43d** (4.3 g) as red oil, which was used directly without further purification. ESI-MS (m/z): 490.9 [M+H]⁺.

Step 3: Compound **43d** (4.3 g, from step 2) was dissolved in acetic acid (30 mL), and Fe powder (0.94 g, 16.7 mmol)) was added by portions at room temperature. The reaction mixture was heated at 80 °C for 3 hours, LCMS indicated the product was formed. The mixture was diluted with water (20 mL), and extracted with EtOAc (15 mL × 3). The combined organic layers were washed with saturated NaHCO₃ solution (10 mL) and brine (10 mL), dried over Na₂SO₄, filtered and concentrated. The residue was purified by silica chromatography to give the compound **43e** (3.9 g, 57% yield for 2 steps) as red oil. ESI-MS (m/z): 443.0 [M+H]⁺.

Step 4: A solution of **43e** (3.9 g, 8.85 mmol) in THF (50 mL) was treated with borane-methyl sufide complex in THF (2 M, 8.8 mL, 17.6 mmol), and the solution was heated at 70 °C for 2 hours. LCMS indicated the reaction was complete. The mixture was concentrated, the residue was purified by silica gel chromatography (petroleum ether/EtOAc = 1/1) to give compound **43f** (1.3 g, 34% yield) as red oil. ESI-MS (m/z): 429.0 [M+H]⁺.

Step 5: To a solution of compound **43f** (1.6 g, 3.73 mmol) in DMF (30 mL) at room temperature was added Cs₂CO₃ (2.4 g, 7.48 mmol), followed by the addition of MeI (0.8 g, 5.6 mmol). The reaction mixture was heated at 90°C for 8 hours, LCMS indicated the reaction was complete. The reaction was cooled to room temperature, diluted with water (15 mL) and extracted with EtOAc (15 mL × 4). The combined organic layers were washed with brine, dried over Na₂SO₄, filtered and concentrated. The residue was purified by silica gel chromatography (petroleum ether/EtOAc = 1/1) to give compound **43g** (1.3 g, 76% yield) as red oil. ESI-MS (m/z): 443.0 [M+H]⁺.

Step 6: To a solution of compound **43g** (2.6 g, 5.88 mmol) in a mixture of MeOH (50 mL) and concentrated ammonium hydroxide (18 mL) at 0 °C was added dropwise the solution of sodium dithionite (10.2 g, 58.8 mmol) in water (20 mL). Stirring was continued at room temperature for 1 hour, and LCMS indicated the product was formed. The reaction mixture was diluted with water (200 mL), and extracted with EtOAc (80 mL × 4). The combined organic layers were washed with brine (40 mL), dried over Na₂SO₄, filtered, and concentrated. The residue was purified by silica gel chromatography to give compound **43h** (1.7 g, 70% yield) as red oil. ESI-MS (m/z): 413.0 [M+H]⁺.

Step 7: Compound **43h** (250 mg, 0.61 mmol) was dissolved in 1, 4-dioxane (10 mL), and then compound **17e** (0.4 M in dioxane, 1.7 mL, 0.67 mmol) was added. The reaction mixture was stirred at room temperature for 1 hour, LCMS indicated the starting material was consumed. EDCI (140 mg, 0.15 mmol) was added, and the mixture was heated at 80 °C for 4 hours, LCMS indicated the product was formed. The reaction mixture was cooled to room temperature, diluted with water (50 mL), and extracted with EtOAc (30 mL × 4). The combined organic layers were washed with brine, dried over Na₂SO₄, filtered and concentrated. The residue was purified by silica gel chromatography (pure EtOAc) to give compound **43i** (190 mg, 55% yield) as brown solid. ESI-MS (m/z): 574.0 [M+H]⁺.

Step 8: To a solution of compound **43i** (260 mg, 0.45 mmol) in MeOH (10 mL) and THF (10 mL) was added 1M NaOH in water (1.82 mL, 1.82 mmol). The mixture was stirred at room temperature for 48 hours, LCMS indicated the reaction was complete. The mixture was concentrated, the residue was suspended in water and carefully adjusted to pH 3-4 with 2M HCl aqueous solution. The formed solid was collected by filtration and dried in vacuo to give the compound **43j** (165 mg, 65% yield) as white solid.

Step 9: To a solution of compound **43j** (150 mg, 0.27 mmol) and NH₄Cl (143 mg, 2.68 mmol) in DMF (10 mL) was added EDCI (77 mg, 0.40 mmol), HOBt (54 mg, 0.40 mmol) and DIPEA (104 mg, 0.81 mmol). The mixture was stirred at room temperature for 16 hours, LCMS indicated the reaction was complete. The reaction mixture was poured into water (30 mL) and extracted with EtOAc (20 mL × 3). The combined organic layers were washed with brine, dried over Na₂SO₄, filtered and concentrated. The residue was purified by Preparative TLC to give compound **43** (22 mg, 15% yield) as white solid. ESI-MS (m/z): 559.4 [M+H]⁺; ¹H NMR (400 MHz, DMSO-*d*₆) δ 12.61 (s, 1H), 7.87 (s, 1H), 7.38 (s, 1H), 7.33-7.21 (m, 6H), 7.07 (s, 1H), 6.61 (s, 1H), 5.04-4.92 (m, 2H), 4.69-4.54 (m, 3H), 3.43 (d, *J* = 12.0 Hz, 1H), 3.25 (d, *J* = 11.6 Hz, 1H), 3.10-3.00 (m, 2H), 2.98 (s, 3H), 2.16 (s, 3H), 1.84-1.69 (m, 2H), 1.66-1.50 (m, 2H), 1.35 (t, *J* = 7.0 Hz, 3H).

### Example 44

### 1-(1-Ethyl-4-fluoro-3-methyl-1H-pyrazole-5-carboxamido)-8,8-dimethyl-8,9-dihydro-7H-6-o xa-2,9a-diazabenzo[cd]azulene-4-carboxamide

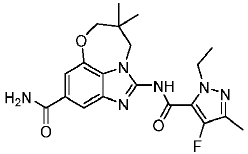

### Synthetic scheme:

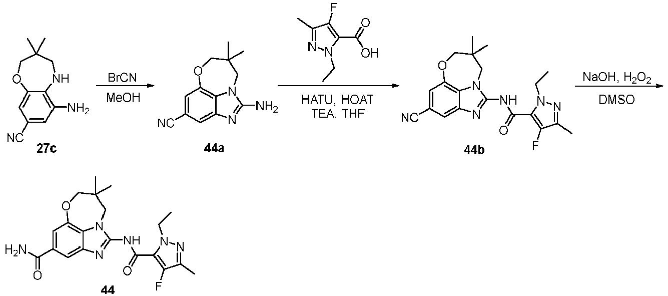

Step 1: Compound **27c** (600 mg, 2.76 mmol) was dissolved in MeOH (10 mL), and cyanogen bromide (1.46 g, 13.81 mmol) was added. The resulting mixture was stirred at room temperature overnight, LCMS indicated the product was formed. The mixture was concentrated in vacuo to remove the solvent. The residue was purified by silica gel chromatography to give compound **44a** (500 mg, 74% yield) as white solid. ESI-MS (m/z): 243.2[M+H]⁺.

Step 2: To a stirring solution of compound **44a** (50 mg, 0.21 mmol) and 1-ethyl-4-fluoro-3-methyl-1H-pyrazole-5-carboxylic acid (39 mg, 0.22 mmol) in THF (10 mL) was added HATU (94 mg, 0.24 mmol), HOAT (33 mg, 0.24 mmol) and triethylamine (0.09 mL, 0.62 mmol). The mixture was stirred at room temperature overnight, LCMS indicated the reaction was complete. The mixture was concentrated to give crude compound **44b** (80 mg), which was used directly without further purification. ESI-MS (m/z): 397.2[M+H]⁺.

Step 3: To a stirring solution of crude compound **44b** (80 mg, from step 2) and NaOH (24 mg, 0.60 mmol) in DMSO (3 mL) at 60 °C was added dropwise hydrogen peroxide (30wt. %, 1 mL). Stirring was continued at 60 °C for 5 minutes, and LCMS indicated the starting material was consumed. The mixture was purified directly by reversed phase preparative HPLC to give compound **44** (17 mg, 20% yield for 2 steps) as white solid. ESI-MS (m/z): 415.4 [M+H]⁺; ¹H NMR (500 MHz, DMSO-d6) δ 12.96 (br s, 1H),7.94 (s, 1H), 7.66 (d, *J* = 1.5 Hz, 1H), 7.34 (d, *J* = 1.5 Hz, 1H), 4.55 (q, *J* = 7.0 Hz, 2H), 4.15 (s, 2H), 3.96 (s, 2H), 2.16 (s, 3H), 1.34(t, *J* = 7.0 Hz, 3H), 1.08 (s, 6H).

### Example 45

### 1-(4-Chloro-1-ethyl-3-methyl-1H-pyrazole-5-carboxamido)-8,8-dimethyl-8,9-dihydro-7H-6-oxa-2,9a-diazabenzo[cd]azulene-4-carboxamide

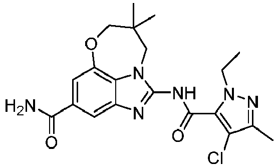

### Synthetic scheme:

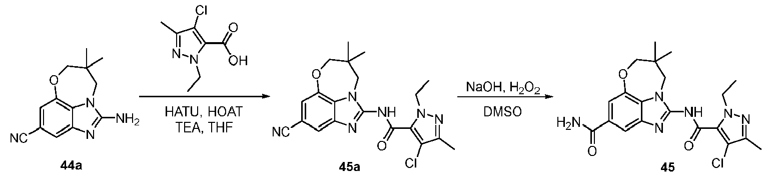

Step 1: To a stirring solution of compound **44a** (50 mg, 0.21 mmol) and 4-chloro-1-ethyl-3-methyl-1H-pyrazole-5-carboxylic acid (42 mg, 0.22 mmol) in THF (10 mL) was added HATU (94 mg, 0.24 mmol), HOAT (33 mg, 0.24 mmol) and triethylamine (0.09 mL, 0.62 mmol). The mixture was stirred at room temperature overnight, LCMS indicated the reaction was complete. The mixture was concentrated to give crude compound **45a** (80 mg), which was used directly without further purification. ESI-MS (m/z): 413.3 [M+H]⁺.

Step 2: To a stirring solution of crude compound **45a** (80 mg, from step 1) and NaOH (23 mg, 0.58 mmol) in DMSO (3 mL) at 60 °C was added dropwise hydrogen peroxide (30wt. %, 1 mL). Stirring was continued at 60 °C for 5 minutes, and LCMS indicated the starting material was consumed. The mixture was purified directly by reversed phase preparative HPLC to give compound **45** (13 mg, 15% yield for 2 steps) as white solid. ESI-MS (m/z): 431.3 [M+H]⁺; ¹H NMR (500 MHz, DMSO-*d6*) δ 13.02 (s, 1H), 7.95 (s, 1H), 7.67 (d, *J* = 1.5 Hz, 1H), 7.40-7.30 (m, 2H), 4.59 (q, *J* = 7.0 Hz, 2H), 4.15 (s, 2H), 4.01 (s, 2H), 2.17 (s, 3H), 1.36 (t, *J* = 7.0 Hz, 3H), 1.08 (s, 6H).

### Example 46

### 1-(4-Bromo-1-ethyl-3-methyl-1H-pyrazole-5-carboxamido)-8,8-dimethyl-8,9-dihydro-7H-6-oxa-2,9a-diazabenzo[cd]azulene-4-carboxamide

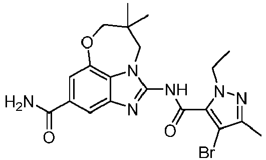

### Synthetic scheme:

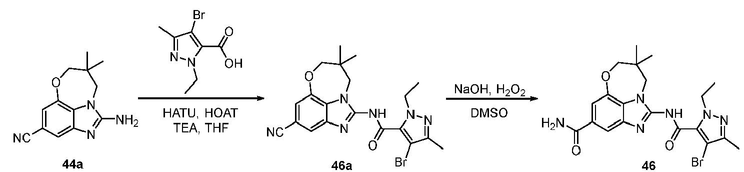

Step 1: To a stirring solution of compound **44a** (50 mg, 0.21 mmol) and 4-bromo-1-ethyl-3-methyl-1H-pyrazole-5-carboxylic acid (52 mg, 0.22 mmol) in THF (10 mL) was added HATU (94 mg, 0.24 mmol), HOAT (33 mg, 0.24 mmol) and triethylamine (0.09 mL, 0.62 mmol). The mixture was stirred at room temperature overnight, LCMS indicated the reaction was complete. The mixture was concentrated to give crude compound **46a** (90 mg), which was used directly without further purification. ESI-MS (m/z): 457.4 [M+H]⁺.

Step 2: To a stirring solution of crude compound **46a** (80 mg, from step 1) and NaOH (23 mg, 0.58 mmol) in DMSO (3 mL) at 60 °C was added dropwise hydrogen peroxide (30wt. %, 1 mL). Stirring was continued at 60 °C for 5 minutes, and LCMS indicated the starting material was consumed. The mixture was purified directly by reversed phase preparative HPLC to give compound **46** (7 mg, 7% yield for 2 steps) as white solid. ESI-MS (m/z): 475.3 [M+H]⁺; ¹H NMR (500 MHz, DMSO-*d6*) δ 12.96 (br s, 1H), 7.94 (s, 1H), 7.68 (s, 1H), 7.40-7.30 (m, 2H), 4.59 (q, *J* = 7.0 Hz, 2H), 4.15 (s, 2H), 4.04 (s, 2H), 2.17 (s, 3H), 1.36 (t, *J* = 7.0 Hz, 3H), 1.08 (s, 6H).

### Example 47

### (S)-3-Ethyl-2-(1-ethyl-4-fluoro-3-methyl-1H-pyrazole-5-carboxamido)-3,4-dihydro-5-oxa-1, 2a-diazaacenaphthylene-7-carboxamide

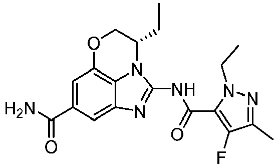

### Synthetic scheme:

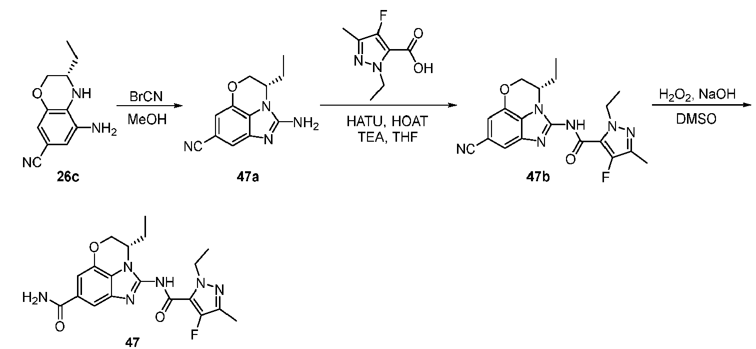

Step 1: Compound **26c** (1.0 g, 4.92 mmol) was dissolved in MeOH (20 mL), and cyanogen bromide (2.61 g, 24.6 mmol) was added. The resulting mixture was stirred at room temperature overnight, LCMS indicated the product was formed. The mixture was concentrated in vacuo to remove the solvent. The residue was purified by silica gel chromatography to give compound **47a** (1.0 g, 89% yield) as off-white solid. ESI-MS (m/z): 229.2 [M+H]⁺.

Step 2: To a stirring solution of compound **47a** (50 mg, 0.21 mmol) and 1-ethyl-4-fluoro-3-methyl-1H-pyrazole-5-carboxylic acid (41 mg, 0.24 mmol ) in THF (10 mL) was added HATU (99 mg, 0.26 mmol), HOAT (35 mg, 0.26 mmol) and triethylamine (0.09 mL, 0.65 mmol). The mixture was stirred at room temperature overnight, LCMS indicated the reaction was complete. The mixture was concentrated to give crude compound **47b** (80 mg), which was used directly without further purification. ESI-MS (m/z): 383.3 [M+H]⁺.

Step 3: To a stirring solution of crude compound **47b** (80 mg, from step 2) and NaOH (25 mg, 0.62 mmol) in DMSO (3 mL) at 60 °C was added dropwise hydrogen peroxide (30wt. %, 1 mL). Stirring was continued at 60 °C for 5 minutes, and LCMS indicated the starting material was consumed. The mixture was purified directly by reversed phase preparative HPLC to give compound **47** (18 mg, 20% yield for 2 steps) as white solid. ESI-MS (m/z): 401.1 [M+H]⁺; ¹H NMR (500 MHz, DMSO-*d₆*) δ 12.60 (br s, 1H), 7.94 (s, 1H), 7.59 (s, 1H), 7.38-7.30 (m, 2H), 4.66 (dd, *J* = 12.0, 1.5 Hz, 1H), 4.63-4.56 (m, 1H), 4.56-4.47 (m, 2H), 4.27 (dd, *J=* 12.0, 3.0 Hz, 1H), 2.16 (s, 3H), 1.92-1.79 (m, 2H), 1.34 (t, *J* = 7.0 Hz, 3H), 0.98 (t, *J* = 7.5 Hz, 3H).

### Example 48

### (S)-2-(4-Chloro-1-ethyl-3-methyl-1H-pyrazole-5-carboxamido)-3-ethyl-3,4-dihydro-5-oxa-1, 2a-diazaacenaphthylene-7-carboxamide

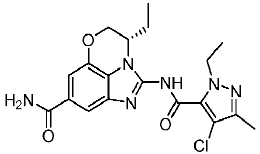

### Synthetic scheme:

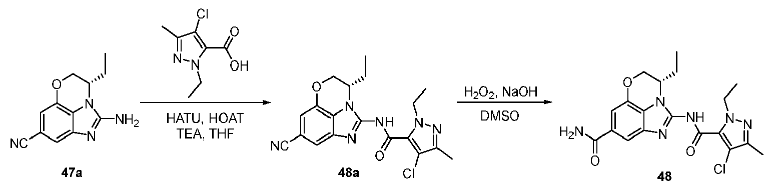

Step 1: To a stirring solution of compound 47a (50 mg, 0.22 mmol) and 4-chloro-1-ethyl-3-methyl-1H-pyrazole-5-carboxylic acid (45 mg, 0.24 mmol) in THF (10 mL) was added HATU (99 mg, 0.26 mmol), HOAT (35 mg, 0.26 mmol) and triethylamine (0.09 mL, 0.65 mmol). The mixture was stirred at room temperature overnight, LCMS indicated the reaction was complete. The mixture was concentrated to give crude compound **48a** (80 mg), which was used directly without further purification. MS (ESI): m/z 399.3 [M+H]⁺.

Step 2: To a stirring solution of crude compound **48a** (80 mg, from step 1) and NaOH (24 mg, 0.60 mmol) in DMSO (3 mL) at 60 °C was added dropwise hydrogen peroxide (30wt. %, 1 mL). Stirring was continued at 60 °C for 5 minutes, and LCMS indicated the starting material was consumed. The mixture was purified directly by reversed phase preparative HPLC to give compound **48** (24 mg, 29% yield for 2 steps) as white solid. ESI-MS (m/z): 417.4 [M+H]⁺; ¹H-NMR (500 MHz, DMSO-*d₆*) δ 12.85 (br s, 1H), 7.95 (s, 1H), 7.60 (s, 1H), 7.40-7.31 (m, 2H), 4.72-4.67 (m, 1H), 4.67-4.59 (m, 1H), 4.59-4.51 (m, 2H), 4.27 (dd, *J=* 12.0, 3.0 Hz, 1H), 2.16 (s, 3H), 1.94-1.86 (m, 1H), 1.85-1.75 (m, 1H), 1.37 (t, *J=* 7.0 Hz, 3H), 0.98 (t, *J=* 7.5 Hz, 3H).

### Example 49

### (S)-2-(4-Bromo-1-ethyl-3-methyl-1H-pyrazole-5-carboxamido)-3-ethyl-3,4-dihydro-5-oxa-1, 2a-diazaacenaphthylene-7-carboxamide

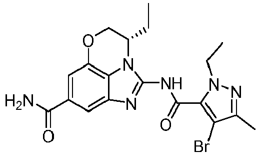

### Synthetic scheme:

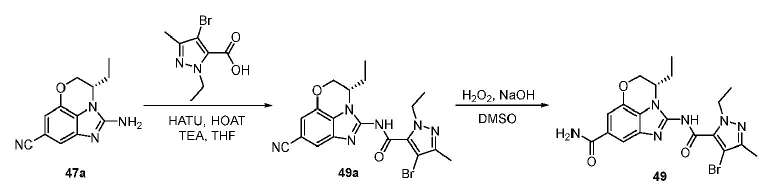

Step 1: To a stirring solution of compound **47a** (50 mg, 0.22 mmol) and 4-bromo-1-ethyl-3-methyl-1H-pyrazole-5-carboxylic acid (56 mg, 0.24 mmol) in THF (10 mL) was added HATU (99 mg, 0.26 mmol), HOAT (35 mg, 0.26 mmol) and triethylamine (0.09 mL, 0.65 mmol). The mixture was stirred at room temperature overnight, LCMS indicated the reaction was complete. The mixture was concentrated to give crude compound **49a** (90 mg), which was used directly without further purification. ESI-MS (m/z): 443.2 [M+H]⁺.

Step 2: To a stirring solution of crude compound **49a** (90 mg, from step 1) and NaOH (24 mg, 0.61 mmol) in DMSO (3 mL) at 60 °C was added dropwise hydrogen peroxide (30wt. %, 1 mL). Stirring was continued at 60 °C for 5 minutes, and LCMS indicated the starting material was consumed. The mixture was purified directly by reversed phase preparative HPLC to give compound **49** (46 mg, 49% yield for 2 steps) as white solid. ESI-MS (m/z): 463.4 [M+H]⁺; ¹H-NMR (500 MHz, DMSO-*d₆*) δ 12.87 (s, 1H), 7.94 (s, 1H), 7.61 (s, 1H), 7.40-7.30 (m, 2H), 4.75-4.51 (m, 4H), 4.27 (dd, *J=* 12.0, 3.0 Hz, 1H), 2.17 (s, 3H), 1.94-1.87 (m, 1H), 1.84-1.75 (m, 1H), 1.37 (t, *J* = 7.0 Hz, 3H), 0.99 (t, *J* = 7.5 Hz, 3H).

### Example 50

### (S)-3-(3-Aminopropyl)-2-(3-methyl-1-(pent-4-en-1-yl)-1H-pyrazole-5-carboxamido)-3,4-dih ydro-5-oxa-1,2a-diazaacenaphthylene-7-carboxamide

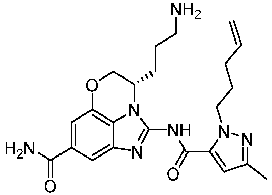

### Synthetic scheme:

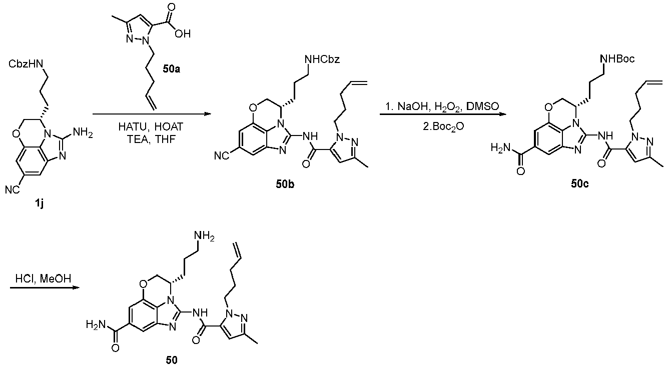

Step 1: To a solution of compound **1j** (3.0 g, 7.66 mmol) and compound **50a** (1.56 g, 8.05 mmol) in THF (10 mL) was added HATU (3.21 g, 8.43 mmol), HOAT (1.15 g, 8.43 mmol) and triethylamine (2.12 mL, 15.33 mmol). The mixture was stirred at room temperature overnight. LCMS indicated the starting material was consumed. The reaction mixture was concentrated, the residue suspended in EtOAc (100 mL) and washed with saturated NH₄Cl solution and brine. The organic layer was dried over Na₂SO₄, filtered and concentrated to afford crude compound **50b** (4.0 g), which was used directly without further purification. ESI-MS (m/z): 568.5 [M+H]⁺.

Step 2: To a stirring solution of **50b** (4.0 g, from step 1) and NaOH (845 mg, 21 mmol) in DMSO (40 mL) at 60 °C was added dropwise hydrogen peroxide (30 wt. %, 3 mL). Stirring was continued at 60 °C for 10 minutes, and LCMS indicated the starting material was consumed. The reaction was cooled to room temperature, Boc₂O (2.31 g, 10.57 mmol) was added, and the reaction was stirred at room temperature for half an hour. LCMS indicated the product was formed. The reaction mixture was poured into water (150 mL) and extracted with EtOAc. The combined organic layers were dried over Na₂SO₄, filtered and concentrated. The residue was purified by silica gel chromatography to give compound **50c** (1.8 g, 40% yield for 2 steps) as white solid. ESI-MS (m/z): 552.4 [M+H]⁺.

Step 3: To a stirring solution of **50c** (1.8 g, 3.26 mmol) in MeOH (20 mL) was added 4M HCl in dioxane (5 mL, 20 mmol). The mixture was stirred at room temperature for 4 hours, LCMS indicated the product was formed. The reaction mixture was concentrated to give compound **50** (1.5 g, 94% yield) as white solid. ESI-MS (m/z): 452.4 [M+H]⁺; ¹H-NMR (500 MHz, DMSO-*d₆*) δ 8.44 (s, 1H), 7.92 (s, 1H), 7.60 (s, 1H), 7.40-7.20 (m, 2H), 6.67 (s, 1H), 5.90-5.75 (m, 1H), 5.07-5.00 (m, 1H), 4.97 (d, *J* = 10.5 Hz, 1H), 4.70-4.55 (m, 4H), 4.37-4.20 (m, 1H), 2.80-2.70 (m, 2H), 2.19(s, 3H), 2.04 (q, *J* = 7.0 Hz, 2H), 1.95-1.80 (m, 4H), 1.70-1.60 (m, 2H).

### Example 51

### (S)-3-(3-Aminopropyl)-2-(1-ethyl-3-methyl-4-vinyl-1H-pyrazole-5-carboxamido)-3,4-dihydr o-5-oxa-1,2a-diazaacenaphthylene-7-carboxamide

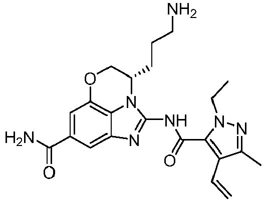

### Synthetic scheme:

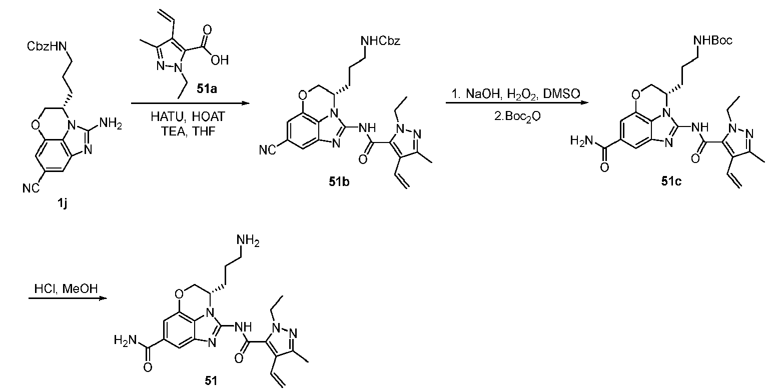

Step 1: To a solution of compound **1j** (3.0 g, 7.66 mmol) and compound **51a** (1.45 g, 8.05 mmol) in THF (10 mL) was added HATU (3.21 g, 8.43 mmol), HOAT (1.15 g, 8.43 mmol) and triethylamine (2.12 mL, 15.33 mmol). The mixture was stirred at room temperature overnight. LCMS indicated the starting material was consumed. The reaction mixture was concentrated, the residue suspended in EtOAc (100 mL) and washed with saturated NH₄Cl solution and brine. The organic layer was dried over Na₂SO₄, filtered and concentrated to afford crude compound **51b** (4.0 g), which was used directly without further purification. ESI-MS (m/z): 554.8 [M+H]⁺.

Step 2: To a stirring solution of **51b** (4.0 g, from step 1) and NaOH (845 mg, 21.14 mmol) in DMSO (40 mL) at 60 °C was added dropwise hydrogen peroxide (30 wt. %, 10 mL). Stirring was continued at 60 °C for 10 minutes, and LCMS indicated the starting material was consumed. The reaction was cooled to room temperature, Boc₂O (2.37 g, 10.84 mmol) was added, and the reaction was stirred at room temperature for half an hour. LCMS indicated the product was formed. The reaction mixture was poured into water (150 mL) and extracted with EtOAc. The combined organic layers were dried over Na₂SO₄, filtered and concentrated. The residue was purified by silica gel chromatography to give compound **51c** (1.4 g, 32% yield for 2 steps) as white solid. ESI-MS (m/z): 538.5 [M+H]⁺.

Step 3: To a stirring solution of **51c** (1.4 g, 2.60 mmol) in MeOH (20 mL) was added 4M HCl in dioxane (7 mL). The mixture was stirred at room temperature for 6 hours, LCMS indicated the product was formed. The reaction mixture was concentrated to give compound **51** (1.1 g, 89% yield) as white solid. ESI-MS (m/z): 438.4 [M+H]⁺; ¹H NMR (500 MHz, DMSO-*d₆*) δ 8.39 (s, 1H), 7.93 (s, 1H), 7.61 (s, 1H), 7.55-7.45 (m, 1H), 7.40-7.30 (m, 2H), 5.36 (dd, *J =* 18.5, 2.0 Hz,1H), 5.23 (dd, *J* = 11.5, 2.0 Hz, 1H), 4.70-4.61 (m, 2H), 4.59-4.52 (m, 2H), 4.37-4.22 (m, 1H), 2.75-2.58 (m, 2H), 2.28 (s, 3H), 1.85 (q, *J* = 8.0 Hz, 2H), 1.75-1.55 (m, 2H), 1.36 (t, *J* = 7.0 Hz, 3H).

### Example 52

### (S)-2-(1-ethyl-3-methyl-1H-pyrazole-5-carboxamido)-4-(hydroxymethyl)-3,4-dihydro-5-oxa-1,2a-diazaacenaphthylene-7-carboxamide

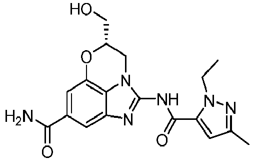

### Synthetic scheme:

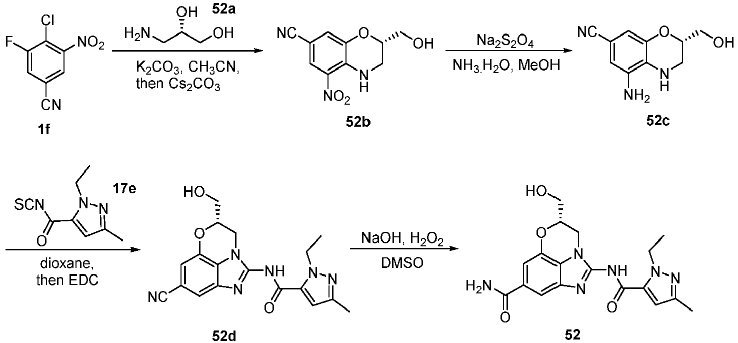

**Step 1:** To a stirring solution of compound **1f** (2.0 g, 9.97 mmol) and compound **52a** (1.36 g,14.96 mmol) in acetonitrile (50 mL) was added K₂CO₃ (3.45 g, 24.93 mmol). The reaction mixture was heated at 80 °C for 4 hours, then Cs₂CO₃ (1.62 g,4.99 mmol) was added to the reaction. Stirring was continued at 80 °C overnight. The reaction mixture was allowed to cool to room temperature, filtered through a pad of celite, and rinsed with DCM. The filtrated was concentrated, and the residue was purified by silica gel chromatography to give compound **52b** (800 mg, 34% yield) as yellow solid.

**Step 2:** Compound **52b** (800 mg, 3.40 mmol) was dissolved in MeOH (20 mL) and concentrated ammonium hydroxide (4 mL). Sodium dithionite (1.78 g, 10.2 mmol) was dissolved in water (2 mL) and added to the reaction mixture at room temperature. Stirring was continued at room temperature for half an hour, and TLC indicated the starting material was consumed. The reaction mixture was concentrated to remove the solvent, the residue was mixed with water (20 mL), extracted with EtOAc (15 mL × 3). The combined organic layers were washed with brine, dried over Na₂SO₄, filtered and concentrated to give crude compound **52c** (500 mg, 71% yield) as red solid. ESI-MS (m/z): 206.2 [M+H]⁺.

**Step 3:** Compound **52c** (500 mg, 2.44 mmol) was dissolved in dioxane (10 mL), and then compound **17e** (1M in dioxane, 2.68 mL,2.68 mmol) was added. The reaction mixture was stirred at room temperature for 10 minutes, LCMS indicated the starting material was consumed. EDCI (513 mg, 2.68 mmol) was added, and the mixture was heated at 80 °C for 2 hours, LCMS indicated the product was formed. The reaction mixture was concentrated, and the residue was purified by silica gel chromatography to give compound **52d** (600 mg, 67% yield) as a red solid. ESI-MS (m/z): 367.4 [M+H]⁺.

**Step 4:** To a stirring solution of compound **52d** (50 mg,0.13 mmol) and NaOH (16 mg, 0.40 mmol) in DMSO (3 mL) at room temperature was added hydrogen peroxide (30 wt. %, 1 mL) dropwise. The reaction was heated at room temperature for 30 minutes, LCMS indicated the reaction was complete. The reaction mixture was purified directly by reversed phase preparative HPLC to give compound **52** (6 mg, 11% yield) as a white solid. ESI-MS (m/z): 385.5 [M+H]⁺; ¹H NMR (500 MHz, DMSO-*d6*) δ 12.64 (br s, 1H), 8.43 (s, 1H), 7.92 (s, 1H), 7.58 (s, 1H), 7.31(s, 2H), 6.66 (s, 1H), 5.28 (br s, 1H), 4.61 (q, *J* = 7.0 Hz, 2H), 4.45-4.38 (m, 2H), 3.95-3.88 (m, 1H), 3.82-3.75 (m, 2H), 2.17 (s, 3H), 1.35 (t, *J* = 7.0 Hz, 3H).

### Example 53

### 1-(1-Ethyl-3-methyl-1H-pyrazole-5-carboxamido)-8,8-difluoro-8,9-dihydro-7H-6-oxa-2,9a-d iazabenzo[cd]azulene-4-carboxamide

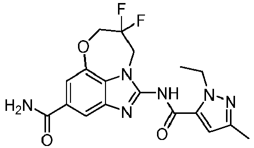

### Synthetic scheme:

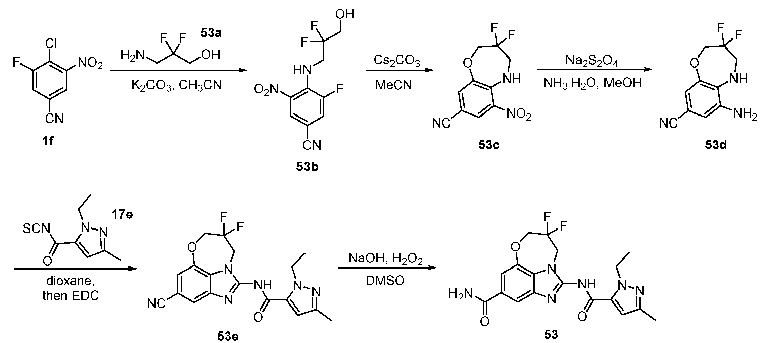

**Step 1:** To a stirring solution of compound **1f** (800 mg, 3.99 mmol) and compound **53a** (509 mg, 4.59 mmol) in acetonitrile (10 mL) was added K₂CO₃ (1.10 g, 7.98 mmol). The reaction mixture was heated at 70 °C for 4 hours, and TLC indicated the starting material was consumed. The reaction mixture was allowed to cool to room temperature, filtered through a pad of celite. The filtrated was concentrated, and the residue was purified by silica gel chromatography to give compound **53b** (350 mg, 31% yield) as yellow oil.

**Step 2:** To a stirring solution of compound **53b** (350 mg, 1.27 mmol) in acetonitrile (10 mL) was added Cs₂CO₃ (621 mg, 1.91 mmol). The reaction mixture was heated at 70 °C for 2 hours, and TLC indicated the reaction was complete. The reaction mixture was allowed to cool to room temperature, filtered through a pad of celite. The filtrated was concentrated, and the residue was purified by silica gel chromatography to give compound **53c** (200 mg, 61% yield) as yellow oil.

**Step 3:** Compound **53c** (200 mg, 0.78 mmol) was dissolved in MeOH (10 mL) and concentrated ammonium hydroxide (2 mL). Sodium dithionite (409 mg, 2.35 mmol) was dissolved in water (2 mL) and added to the reaction mixture at room temperature. Stirring was continued at room temperature for 10 minutes, and TLC indicated the starting material was consumed. The reaction mixture was concentrated, and the residue was purified by silica gel chromatography to give compound **53d** (60 mg, 34% yield) as a white solid. ESI-MS (m/z): 226.1[M+H]⁺.

**Step 4:** Compound **53d** (20 mg, 0.08 mmol) was dissolved in dioxane (5 mL), and then compound **17e** (1M in dioxane, 0.1 mL, 0.1 mmol) was added. The reaction mixture was stirred at room temperature for 10 minutes, LCMS indicated the starting material was consumed. EDCI (20 mg, 0.10 mmol) was added, and the mixture was heated at 80 °C for 2 hours, LCMS indicated the product was formed. The reaction mixture was concentrated to give crude compound **53e** (30 mg) which was used directly without further purification. ESI-MS (m/z): 387.3 [M+H]⁺.

**Step 5:** To a stirring solution of compound **53e** (30 mg, from step 4) and NaOH (9 mg, 0.23 mmol) in DMSO (3 mL) at room temperature was added hydrogen peroxide (30 wt. %, 0.5 mL) dropwise. The reaction was stirred at room temperature for 30 minutes, LCMS indicated the reaction was complete. The reaction mixture was purified directly by reversed phase preparative HPLC to give compound **53** (18 mg, 51% yield for 2 steps) as a white solid. ESI-MS (m/z): 405.1 [M+H]⁺; ¹H NMR (500 MHz, DMSO-*d6*) δ 12.97 (br s, 1H), 7.98 (s, 1H), 7.74 (s, 1H), 7.50-7.35 (m, 2H), 6.80 (s, 1H), 4.80-4.68 (m, 4H), 4.60 (q, *J* = 7.0 Hz, 2H), 2.18 (s, 3H), 1.35 (t, *J* = 7.0 Hz, 3H).

### Example 54

### (S)-2-(1-Ethyl-4-fluoro-3-methyl-1H-pyrazole-5-carboxamido)-3-isopropyl-3,4-dihydro-5-ox a-1,2a-diazaacenaphthylene-7-carboxamide

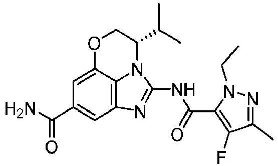

### Synthetic scheme:

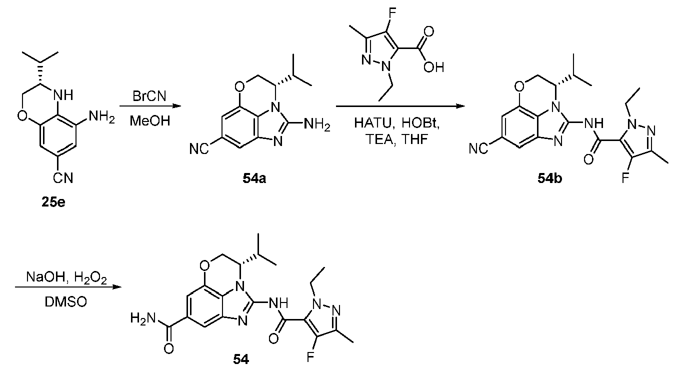

**Step 1:** Compound **25e** (1.94 g, 8.94 mmol) was dissolved in MeOH (30 mL), and cyanogen bromide (2.84 g, 26.79 mmol) was added. The resulting mixture was stirred at room temperature overnight, LCMS indicated the product was formed. The mixture was concentrated in vacuo to remove the solvent. The residue was mixed with EtOAc (80 mL), and washed with saturated Na₂CO₃ soultion (40 mL). The aqueous layer was extracted with EtOAc (50 mL × 2). The combined organic layers were washed with brine, dried over Na₂SO₄, filtered and concentrated. The residue was purified by silica gel chromatography to give compound **54a** (1.68 g, 77 % yield) as yellow solid. ESI-MS (m/z): 243.6 [M+H]⁺.

**Step 2:** To a stirring solution of compound **54a** (300 mg, 1.24 mmol) and 1-ethyl-4-fluoro-3-methyl-1H-pyrazole-5-carboxylic acid (214 mg, 1.24 mmol) in THF (8 mL) was added HATU (471 mg, 1.24 mmol), HOBt (84 mg, 0.62 mmol) and triethylamine (0.52 mL, 3.72 mmol). The mixture was stirred at room temperature overnight, LCMS indicated the reaction was complete. The mixture was poured into water (20 mL) and extracted with EtOAc (25 mL × 3). The combined organic layers were washed brine, dried over Na₂SO₄, filtered and concentrated. The residue was purified by silica gel chromatography to give compound **54b** (295 mg, 60% yield) as white solid. ESI-MS (m/z): 397.6 [M+H]⁺.

**Step 3:** To a stirring solution of compound **54b** (150 mg, 0.37 mmol) and NaOH (50 mg, 1.25 mmol) in DMSO (3 mL) at 60 °C was added hydrogen peroxide (30 wt. %, 1.5 mL) dropwise. The reaction was heated at 60 °C for 30 minutes, LCMS indicated the reaction was complete. The reaction mixture was purified directly by reversed phase preparative HPLC to give compound **54** (40 mg, 26% yield) as a white solid. ESI-MS (m/z): 415.4 [M+H]⁺; ¹H NMR (500 MHz, DMSO-*d*₆) δ 12.81 (br s, 1H), 7.91 (s, 1H), 7.58 (s, 1H), 7.42-7.19 (m, 2H), 4.82-4.72 (m, 1H), 4.62-4.45 (m, 2H), 4.40-4.32 (m, 1H), 4.24-4.14 (m, 1H), 2.38-2.28 (m, 1H), 2.13 (s, 3H), 1.32 (t, *J* = 7.1 Hz, 3H), 0.99 (d, *J =* 6.9 Hz, 3H), 0.89 (d, *J =* 6.9 Hz, 3H).

### Example 55

### (S)-2-(4-chloro-1-ethyl-3-methyl-1H-pyrazole-5-carboxamido)-3-isopropyl-3,4-dihydro-5-ox a-1,2a-diazaacenaphthylene-7-carboxamide

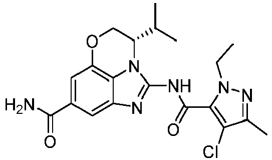

### Synthetic scheme:

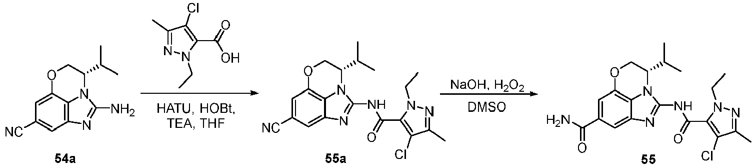

**Step 1:** To a stirring solution of compound **54a** (200 mg, 0.83 mmol) and 4-chloro-1-ethyl-3-methyl-1H-pyrazole-5-carboxylic acid (155 mg, 0.83 mmol) in THF (8 mL) was added HATU (315 mg, 0.83 mmol), HOBt (55 mg, 0.42 mmol) and triethylamine (0.33 mL, 2.55 mmol). The mixture was stirred at room temperature overnight, LCMS indicated the reaction was complete. The mixture was poured into water (20 mL) and extracted with EtOAc (25 mL × 3). The combined organic layers were washed brine, dried over Na₂SO₄, filtered and concentrated. The residue was purified by silica gel chromatography to give compound 55a (224 mg, 66% yield) as white solid. ESI-MS (m/z): 413.6 [M+H]⁺.

**Step 2:** To a stirring solution of compound **55a** (224 mg, 0.54 mmol) and NaOH (65 mg, 1.62 mmol) in DMSO (4 mL) at 60 °C was added hydrogen peroxide (30 wt. %, 2 mL) dropwise. Stirring was continued at 60 °C for 30 minutes, LCMS indicated the reaction was complete. The reaction mixture was purified directly by reversed phase preparative HPLC to give compound **55** (45 mg, 20% yield) as a white solid. ESI-MS (m/z): 431.5 [M+H]⁺; ¹H NMR (500 MHz, DMSO-*d*₆) δ 12.87 (br s, 1H), 7.91 (s, 1H), 7.59 (s, 1H), 7.39-7.20 (m, 2H), 4.82-4.77 (m, 1H), 4.66-4.49 (m, 2H), 4.45-4.37 (m, 1H), 4.27-4.15 (m, 1H), 2.45-2.34 (m, 1H), 2.14 (s, 3H), 1.34 (t, *J* = 7.1 Hz, 3H), 1.01 (d, *J* = 7.0 Hz, 3H), 0.85 (d, *J* = 6.8 Hz, 3H).

### Example 56

### (S)-2-(4-Bromo-1-ethyl-3-methyl-1H-pyrazole-5-carboxamido)-3-isopropyl-3,4-dihydro-5-o xa-1,2a-diazaacenaphthylene-7-carboxamide

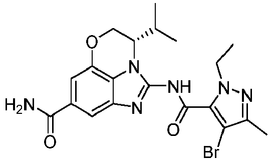

### Synthetic scheme:

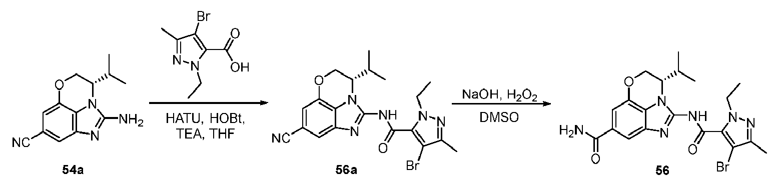

**Step 1:** To a stirring solution of compound **54a** (200 mg, 0.83 mmol) and 4-bromo-1-ethyl-3-methyl-1H-pyrazole-5-carboxylic acid (191 mg, 0.83 mmol) in THF (8 mL) was added HATU (315 mg, 0.83 mmol), HOBt (55 mg, 0.42 mmol) and triethylamine (0.33 mL, 2.55 mmol). The mixture was stirred at room temperature overnight, LCMS indicated the reaction was complete. The mixture was poured into water (30 mL) and extracted with EtOAc (40 mL × 3). The combined organic layers were washed brine, dried over Na₂SO₄, filtered and concentrated. The residue was purified by silica gel chromatography to give compound **56a** (185 mg, 49% yield) as white solid. ESI-MS (m/z): 457.1[M+H]⁺.

**Step 2:** To a stirring solution of compound **56a** (180 mg, 0.39 mmol) and NaOH (50 mg, 1.25 mmol) in DMSO (3 mL) at 60 °C was added hydrogen peroxide (30 wt. %, 1.5 mL) dropwise. Stirring was continued at 60 °C for 30 minutes, LCMS indicated the reaction was complete. The reaction mixture was purified directly by reversed phase preparative HPLC to give compound **56** (53 mg, 29% yield) as a white solid. ESI-MS (m/z): 475.3 [M+H]⁺; ¹H NMR (500 MHz, DMSO-*d*₆) δ 12.87 (br s, 1H), 7.91 (s, 1H), 7.59 (s, 1H), 7.46-7.19 (m, 2H), 4.84-4.74 (m, 1H), 4.68-4.50 (m, 2H), 4.48-4.41 (m, 1H), 4.24-4.14 (m, 1H), 2.44-2.37 (m, 1H), 2.15 (s, 3H), 1.34 (t, *J* = 7.1 Hz, 3H), 1.02 (d, *J* = 6.9 Hz, 3H), 0.84 (d, *J* = 6.9 Hz, 3H).

### Example 57

### 1-(1-Ethyl-3-methyl-1H-pyrazole-5-carboxamido)-8,9-dihydro-7H-6-oxa-2,9a-diazabenzo[c d]azulene-4-carboxamide

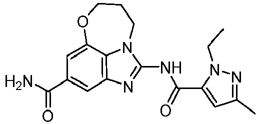

### Synthetic scheme:

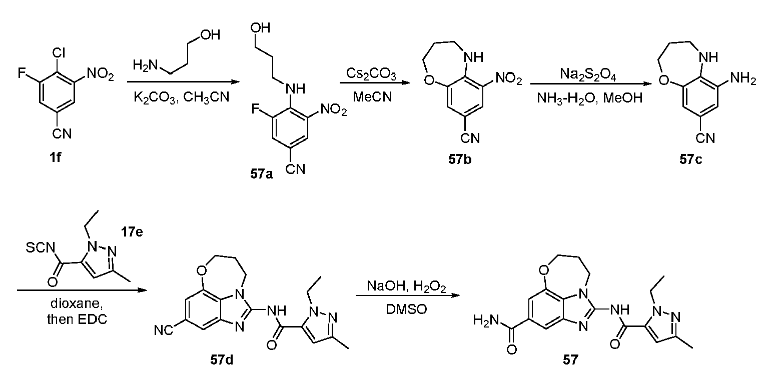

Step **1**: To a stirring solution of compound **1f** (1.00 g, 5.00 mmol) and 3-aminopropan-1-ol (562 mg, 7.50 mmol) in acetonitrile (15 mL) was added K₂CO₃ (1.4 g, 10.00 mmol). The reaction mixture was heated at 70 °C for 16 hours, and TLC indicated the starting material was consumed. The reaction mixture was allowed to cool to room temperature, filtered through a pad of celite, and rinsed with DCM (100 mL). The filtrated was concentrated, and the residue was purified by silica gel chromatography to give compound **57a** (1.05 g, 87% yield) as yellow oil. ESI-MS (m/z): 240.5 [M+H]⁺.

Step 2: To a stirring solution of compound **57a** (1.05 g, 4.39 mmol) in acetonitrile (20 mL) was added Cs₂CO₃ (2.85 g, 8.78 mmol). The reaction mixture was heated at 70 °C for 3 hours, and TLC indicated the reaction was complete. The reaction mixture was allowed to cool to room temperature, filtered through a pad of celite, and rinsed with DCM. The filtrated was concentrated to give crude compound **57b** (880 mg) as brown oil, which was used directly without further purification. ESI-MS (m/z): 220.4 [M+H]⁺.

Step 3: Compound **57b** (880 mg, from step 2) was dissolved in MeOH (60 mL) and concentrated ammonium hydroxide (5 mL). Sodium dithionite (3.5 g, 20.11 mmol) was dissolved in water (5 mL) and added dropwise to the reaction mixture at room temperature. Stirring was continued at room temperature for 10 minutes, and LCMS indicated the starting material was consumed. The reaction mixture was poured into water (50 mL), extracted with EtOAc (50 mL × 3). The combined organic layers were washed with brine, dried over Na₂SO₄, filtered and concentrated to give crude compound **57c** (455 mg) as brown oil, which was used directly without further purification. ESI-MS (m/z): 190.6[M+H]⁺.

Step 4: Compound **57c** (455 mg, from step 3) was dissolved in dioxane (20 mL), and then compound **17e** (1M in dioxane, 2.4 mL, 2.40 mmol) was added. The reaction mixture was stirred at room temperature for 30 minutes, DCC (920 mg, 4.81 mmol) was added, and the mixture was heated at 80 °C overnight, LCMS indicated the product was formed. The reaction mixture was concentrated, and the residue was purified by silica gel chromatography to give compound **57d** (280 mg, 20% yield for 3 steps) as white solid. ESI-LC-MS (m/z): 351.4 [M+H]⁺.

Step 5: To a stirring solution of compound **57d** (280 mg, 0.80 mmol) and NaOH (100 mg, 2.50 mmol) in DMSO (3 mL) at 60 °C was added hydrogen peroxide (30 wt. %, 0.5 mL) dropwise. Stirring was continued at 60 °C for 30 minutes, LCMS indicated the reaction was complete. The reaction mixture was purified directly by reversed phase preparative HPLC to give compound **57** (50 mg, 17% yield) as white solid. ESI-MS (m/z): 369.5 [M+H]⁺; ¹H NMR (500 MHz, DMSO-*d*₆) δ 12.79 (br s, 1H), 7.89 (s, 1H), 7.64 (d, *J* = 1.6 Hz, 1H), 7.31 (d, *J* = 1.7 Hz, 1H), 7.28 (s, 1H), 6.66 (s, 1H), 4.60 (q, *J* = 7.1 Hz, 2H), 4.41-4.35 (m, 2H), 4.15 (t, *J* = 5.7 Hz, 2H), 2.37-2.29 (m, 2H), 2.16 (s, 3H), 1.33 (t, *J* = 7.1 Hz, 3H).

### Example 58

### (S)-2-(1-ethyl-3-methyl-1H-pyrazole-5-carboxamido)-3-(hydroxymethyl)-3,4-dihydro-5-oxa-1,2a-diazaacenaphthylene-7-carboxamide

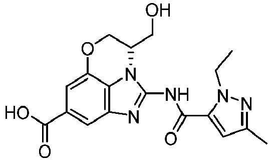

### Synthetic scheme:

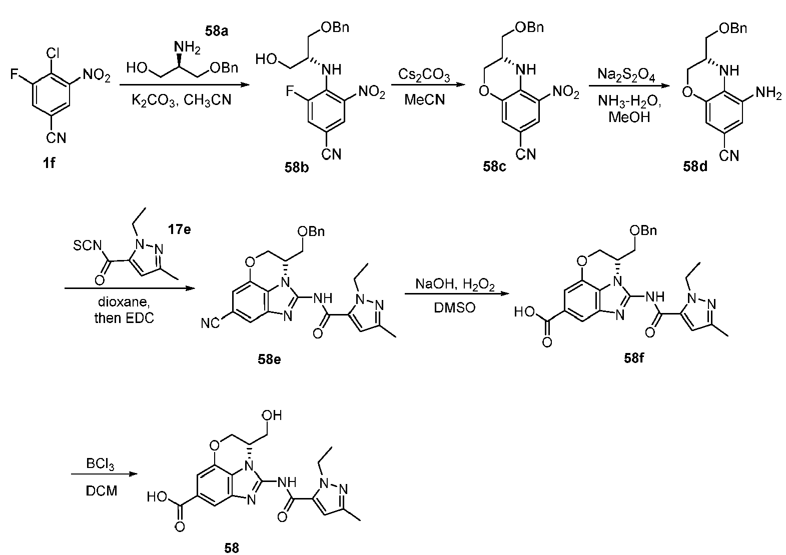

Step **1**: To a stirring solution of compound **1f** (1.00 g, 5.00 mmol) and **58a** (1.36 g, 7.50 mmol) in acetonitrile (30 mL) was added K₂CO₃ (1.4 g, 10.00 mmol). The reaction mixture was heated at 70 °C for 16 hours, and TLC indicated the starting material was consumed. The reaction mixture was allowed to cool to room temperature, filtered through a pad of celite, and rinsed with DCM (100 mL). The filtrated was concentrated, and the residue was purified by silica gel chromatography to give compound **58b** (1.55 g, 89% yield) as yellow oil. ESI-MS (m/z): 346.7 [M+H]⁺.

Step 2: To a stirring solution of compound **58b** (1.55 g, 4.49 mmol) in acetonitrile (30 mL) was added Cs₂CO₃ (2.92 g, 8.98 mmol). The reaction mixture was heated at 70 °C for 3 hours, and TLC indicated the reaction was complete. The reaction mixture was allowed to cool to room temperature, filtered through a pad of celite, and rinsed with DCM. The filtrated was concentrated to give crude compound **58c** (1.31 g) as brown oil, which was used directly without further purification. ESI-MS (m/z): 326.5 [M+H]⁺.

Step 3: Compound **58c** (1.31 g, from step 2) was dissolved in MeOH (80 mL) and concentrated ammonium hydroxide (5 mL). Sodium dithionite (3.5 g, 20.11 mmol) was dissolved in water (5 mL) and added dropwise to the reaction mixture at room temperature. Stirring was continued at room temperature for 30 minutes, and LCMS indicated the starting material was consumed. The reaction mixture was poured into water (50 mL), extracted with EtOAc (50 mL × 3). The combined organic layers were washed with brine, dried over Na₂SO₄, filtered and concentrated to give crude compound **58d** (594 mg) as yellow oil, which was used directly without further purification. ESI-MS (m/z): 296.6 [M+H]⁺.

Step 4: Compound **58d** (590 mg, from step 3) was dissolved in dioxane (15 mL), and then compound **17e** (1M in dioxane, 2.0 mL, 2.0 mmol) was added. The reaction mixture was stirred at room temperature for 30 minutes, DCC (765 mg, 4.0 mmol) was added, and the mixture was heated at 80 °C overnight, LCMS indicated the product was formed. The reaction mixture was concentrated, and the residue was purified by silica gel chromatography to give compound **58e** (605 mg, 29% yield for 3 steps) as white solid. ESI-MS (m/z): 457.3 [M+H]⁺.

Step 5: To a stirring solution of compound **58e** (605 mg, 1.33 mmol) and NaOH (160 mg, 4.0 mmol) in DMSO (5 mL) at 60 °C was added hydrogen peroxide (30 wt. %, 0.5 mL) dropwise. Stirring was continued at 60 °C for 30 minutes, LCMS indicated the reaction was complete. The reaction mixture was cooled to room temperature, poured into water (50 mL), and extracted with EtOAc 50 mL × 3). The combined organic layers were washed with brine, dried over Na₂SO₄, filtered and concentrated. The residue was purified by silica gel chromatography to give compound **58f** (510 mg, 81% yield) as white solid. ESI-MS (m/z): 475.7 [M+H]⁺.

Step 6: To a solution of compound **58f** (510 mg, 1.07 mmol) in DCM (15 mL) at room temperature was added boron trichloride (1M in DCM, 11 mL, 11 mmol) dropwise. The mixture was stirred at room temperature for 30 minutes, LCMS indicated the starting material was consumed. The reaction mixture was carefully quenched by dropwise addition of MeOH, then concentrated. The residue was purified by reversed phase preparative HPLC to give compound **58** (270 mg, 65% yield) as white solid. ESI-MS (m/z): 385.2 [M+H]⁺; ¹H NMR (500 MHz, DMSO-*d*₆) δ 12.70 (br s, 1H), 7.90 (s, 1H), 7.58 (s, 1H), 7.32 (s, 1H), 7.28 (s, 1H), 6.64 (s, 1H), 5.45 (br s, 1H), 4.71 (dd, *J* = 11.8, 2.1 Hz, 1H), 4.66-4.56 (m, 2H), 4.53 (s, 1H), 4.32 (dd, *J* = 11.9 and 3.0 Hz, 1H), 3.84-3.76 (m, 1H), 3.69-3.61 (m, 1H), 2.18 (s, 3H), 1.36 (t, *J* = 7.1 Hz, 3H).

### Example 59

### 1-(1-Ethyl-3-methyl-1H-pyrazole-5-carboxamido)-7H,9H-6-oxa-2,9a-diazaspiro[benzo[cd]a zulene-8,3'-oxetan]-1,2a,2a¹(5a),4-tetraene-4-carboxamide

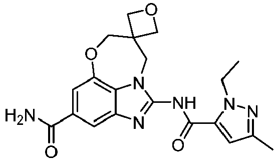

### Synthetic scheme:

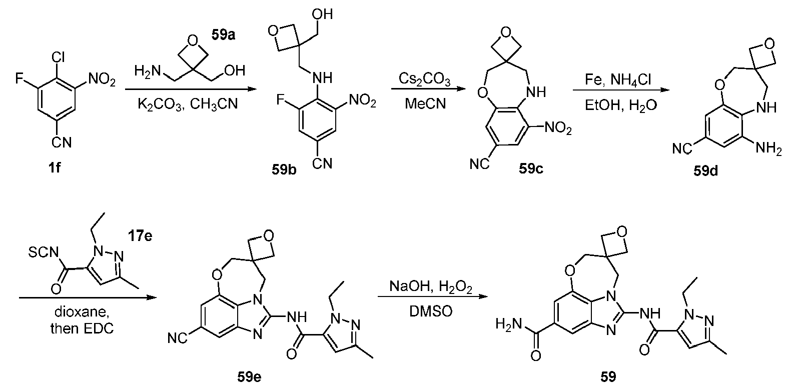

Step **1**: To a stirring solution of compound **1f** (200 mg, 0.99 mmol) and **59a** (152 mg, 1.30 mmol) in acetonitrile (10 mL) was added K₂CO₃ (275 mg, 1.99 mmol). The reaction mixture was heated at 70 °C for 4 hours, and TLC indicated the starting material was consumed. The reaction mixture was concentrated, and the residue was purified by silica gel chromatography to give compound **59b** (200 mg, 71% yield) as yellow solid. ¹H NMR (500 MHz, DMSO-*d₆*) δ 8.59 (q, *J* = 5.0 Hz, 1H), 8.41 (t, *J* = 1.5 Hz, 1H), 7.99 (dd, J = 14.5, 2.0 Hz, 1H), 5.28 (t, *J* = 5.0 Hz, 1H), 4.39-4.33 (m, 4H), 3.92 (dd, *J* = 5.5, 4.0 Hz, 2H), 3.74 (d, *J* = 5.0 Hz, 2H).

Step 2: To a stirring solution of compound **59b** (200 mg, 0.71 mmol) in acetonitrile (10 mL) was added Cs₂CO₃ (463 mg, 1.42 mmol). The reaction mixture was heated at 70 °C for 1 hour, and TLC indicated the reaction was complete. The reaction mixture was concentrated, and the residue was purified by silica gel chromatography to give compound **59c** (50 mg, 26% yield) as yellow solid.

Step 3: To a solution of compound **59c** (50 mg, 0.19 mmol) in EtOH (10 mL) and H₂O (2 mL) was added NH₄Cl (33 mg, 0.63 mmol) and iron powder (35 mg, 0.63 mmol). The mixture was heated at 70 °C for 1 hour, and LCMS indicated the starting material was consumed. The reaction mixture was filtrated through a pad of celite, and the filtrate was concentrated to give compound **59d** (40 mg, 90% yield) as yellow oil. ESI- MS (m/z): 232.0 [M+H]⁺.

Step 4: Compound **59d** (40 mg, 0.17 mmol) was dissolved in dioxane (5 mL), and then compound **17e** (1M in dioxane, 0.19 mL, 0.19 mmol) was added. The reaction mixture was stirred at room temperature for 10 minutes, LCMS indicated the starting material was consumed. EDCI (43 mg, 0.22 mmol) was added, and the mixture was heated at 80 °C overnight, LCMS indicated the product was formed. The reaction mixture was concentrated to give crude compound **59e** (60 mg), which was used directly without further purification. ESI-MS (m/z): 393.4 [M+H]⁺.

Step 5: To a stirring solution of compound **59e** (60 mg, from step 4) and NaOH (18 mg, 0.45 mmol) in DMSO (3 mL) at 60 °C was added hydrogen peroxide (30 wt. %, 0.5 mL) dropwise. Stirring was continued at 60 °C for 5 minutes, LCMS indicated the reaction was complete. The reaction mixture was purified directly by reversed phase preparative HPLC to give compound **59** (17 mg, 24% yield for 2 steps) as white solid. ESI-MS (m/z): 411.4 [M+H]⁺; ¹H NMR (500 MHz, DMSO-*d₆*) δ 12.74 (br s, 1H), 7.90 (s, 1H), 7.66 (d, *J* = 1.5 Hz, 1H), 7.34 (d, *J* = 1.5 Hz, 1H), 7.24 (s, 1H), 6.79 (s, 1H), 4.70-4.54 (m, 6H), 4.50 (s, 2H), 4.42(d, *J* = 6.5 Hz, 2H), 2.20 (s, 3H), 1.37 (t, *J* = 7.0 Hz, 3H).

### Example 60

### 1-(1-ethyl-3-methyl-1H-pyrazole-5-carboxamido)-6-methyl-6,7,8,9-tetrahydro-2,6,9a-triaza benzo[cd]azulene-4-carboxamide

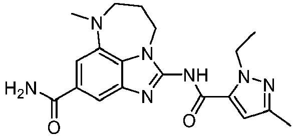

### Synthetic scheme:

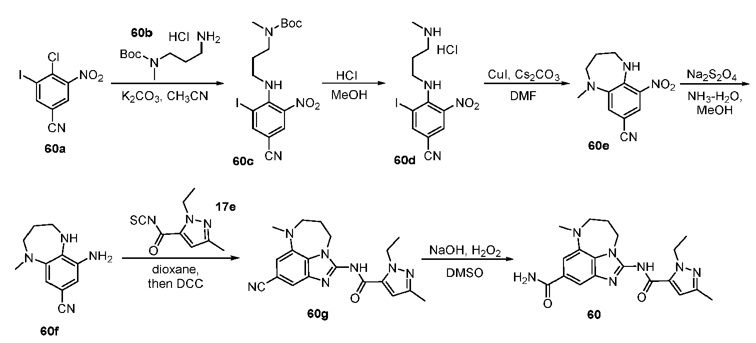

Step 1: To a stirring solution of compound **60a** (1.00 g, 3.25 mmol) and **60b** (1.10 g, 4.90 mmol) in acetonitrile (30 mL) was added K₂CO₃ (900 mg, 6.50 mmol). The reaction mixture was heated at 70 °C for 16 hours, and TLC indicated the starting material was consumed. The reaction mixture was cooled to room temperature, filtered through a pad of silica gel, and rinsed with DCM (100 mL). The filtrate was concentrated, and the residue was purified by silica gel chromatography to give compound **60c** (1.27 g, 85% yield) as yellow solid. ESI-MS (m/z): 461.4 [M+H]⁺.

Step 2: To a stirring solution of **60c** (1.27 g, 2.76 mmol) in MeOH (15 mL) was added 4M HCl in dioxane (3.5 mL, 14 mmol). The mixture was stirred at room temperature for 1 hour, LCMS indicated the product was formed. The reaction mixture was concentrated, and the residue was purified by silica gel chromatography to give compound **60d** (980 mg, 89% yield) as yellow solid. ESI- MS (m/z): 361.7 [M+H]⁺.

Step **3**: To a solution of compound **60d** (200 mg, 0.50 mmol) in DMF (4 mL) was added copper(l) iodide (57 mg, 0.3 mmol) and Cs₂CO₃ (325 mg, 1.0 mmol) at room temperature under nitrogen atmosphere. The reaction mixture was heated at 130 °C by microwave for 1 hour. TLC indicated the starting material was consumed. The reaction mixture was poured into water (30 mL), and extracted with EtOAc (50 mL × 3). The combined organic layers were washed with brine, dried over Na₂SO₄, filtered and concentrated. The residue was purified by silica gel chromatography to give compound **60e** (70 mg, 60% yield) as yellow solid. ESI-MS (m/z): 233.6 [M+H]⁺.

Step 4: Compound **60e** (70 mg, 0.30 mmol) was dissolved in a mixture of MeOH (5 mL) and concentrated ammonium hydroxide (0.2 mL). And sodium dithionite (260 mg, 1.49 mmol) was dissolved in water (1 mL), the resultant solution was added to the reaction mixture at room temperature. Stirring was continued at room temperature for half an hour. LCMS indicated the reaction was complete. The reaction mixture was poured into water (20 mL) and extracted with ethyl acetate (30 mL × 3). The combined organic layers were washed with brine, dried over Na₂SO₄, filtered and concentrated under reduced pressure to give compound **60f** (45 mg) as yellow oil, which was used directly without further purification. ESI-LC-MS (m/z): 203.7 [M+H]⁺.

Step 5: Compound **60f** (45 mg, from step 4) was dissolved in dioxane (5 mL), and then compound **17e** (1M in dioxane, 0.2 mL, 0.2 mmol) was added. The reaction mixture was stirred at room temperature for 30 minutes, DCC (76 mg, 0.40 mmol) was added, and the mixture was heated at 80 °C for 1 hour, LCMS indicated the product was formed. The reaction mixture was purified directly by silica gel chromatography to give compound **60g** (50 mg, 47% yield for 2 steps) as white solid. ESI- MS (m/z): 364.2 [M+H]⁺.

Step 6: To a stirring solution of compound **60g** (50 mg, 0.14 mmol) and NaOH (20 mg, 0.5 mmol) in DMSO (1 mL) at 60 °C was added hydrogen peroxide (30 wt. %, 0.5 mL) dropwise. The reaction mixture was stirred at 60 °C for 30 minutes, LCMS indicated the reaction was complete. The reaction mixture was purified directly by reversed phase preparative HPLC to give compound **60** (33 mg, 63% yield) as white solid. ESI-MS (m/z): 382.4; ¹H NMR (500 MHz, DMSO-*d*₆) δ 7.88 (s, 1H), 7.44 (s, 1H), 7.20 (s, 1H), 6.96 (s, 1H), 6.63 (s, 1H), 4.59 (q, *J* = 7.1 Hz, 2H), 4.17-4.05 (m, 2H), 3.47-3.41 (m, 2H), 3.05 (s, 3H), 2.20-2.15 (m, 5H), 1.33 (t, *J* = 7.1 Hz, 3H).

### Example 61

### 1-(1-ethyl-3-methyl-1H-pyrazole-5-carboxamido)-7H,9H-6-oxa-2,9a-diazaspiro[benzo[cd]az ulene-8,1'-cyclopropan]-1,2a,3,5-tetraene-4-carboxamide

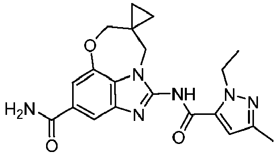

### Synthetic scheme:

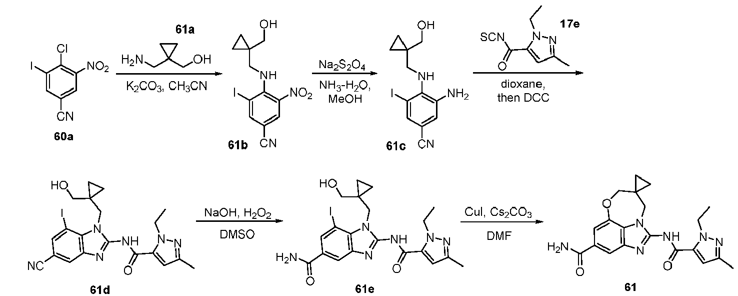

Step **1**: To a stirring solution of compound **60a** (1.00 g, 3.25 mmol) and **61a** (670mg, 4.90 mmol) in acetonitrile (30 mL) was added K₂CO₃ (900 mg, 6.50 mmol). The reaction mixture was heated at 70 °C for 16 hours, and TLC indicated the starting material was consumed. The reaction mixture was cooled to room temperature, filtered through a pad of silica gel, and rinsed with DCM (100 mL). The filtrate was concentrated, and the residue was purified by silica gel chromatography to give compound **61b** (1.07 g, 89% yield) as yellow solid. ESI-MS (m/z): 374.3 [M+H]⁺.

Step 2: Compound **61b** (1.07 g, 2.87 mmol) was dissolved in a mixture of MeOH (30 mL) and concentrated ammonium hydroxide (4 mL). And sodium dithionite (2.5 g, 14.3 mmol) was dissolved in water (4 mL), the resultant solution was added to the reaction mixture at room temperature. Stirring was continued at room temperature for half an hour. LCMS indicated the reaction was complete. The reaction mixture was poured into water (100 mL) and extracted with ethyl acetate (100 mL × 3). The combined organic layers were washed with brine, dried over Na₂SO₄, filtered and concentrated under reduced pressure to give compound **61c** (690 mg) as yellow oil, which was used directly without further purification. ESI-LC-MS (m/z): 344.7 [M+H]⁺.

Step 3: Compound **61c** (690 mg, from step 2) was dissolved in dioxane (30 mL), and then compound **17e** (1M in dioxane, 2.1 mL, 2.1 mmol) was added. The reaction mixture was stirred at room temperature for 30 minutes, DCC (765 mg, 4.02 mmol) was added, and the mixture was heated at 80 °C for 2 hours, LCMS indicated the product was formed. The reaction mixture was purified directly by silica gel chromatography to give compound **61d** (700 mg, 48% yield for 2 steps) as white solid. ESI- MS (m/z): 505.2 [M+H]⁺.

Step 4: To a stirring solution of compound **61d** (700 mg, 1.38 mmol) and NaOH (170 mg, 4.25 mmol) in DMSO (8 mL) at 60 °C was added hydrogen peroxide (30 wt. %, 5 mL) dropwise. The reaction mixture was stirred at 60 °C for 30 minutes, LCMS indicated the reaction was complete. The reaction mixture was purified directly by reversed phase preparative HPLC to give compound **61e** (510 mg, 70% yield) as white solid. ESI-MS (m/z): 523.3 [M+H]⁺.

Step **5**: To a solution of compound **61e** (50 mg, 0.09 mmol) in DMF (2 mL) was added copper(l) iodide (13 mg, 0.07 mmol) and Cs₂CO₃ (60 mg, 0.18 mmol) at room temperature under nitrogen atmosphere. The reaction mixture was heated at 130 °C by microwave for 1 hour. TLC indicated the starting material was consumed. The reaction mixture was purified directly by reversed phase preparative HPLC to give compound **61** (15 mg, 41% yield) as white solid. ESI-MS (m/z): 395.4 [M+H]⁺; ¹H NMR (500 MHz, DMSO-*d*₆) δ 7.89 (s, 1H), 7.64 (s, 1H), 7.34-7.22 (m, 2H), 6.65 (s, 1H), 4.57 (q, *J* = 7.1 Hz, 2H), 4.27 (s, 2H), 4.01 (s, 2H), 2.15 (s, 3H), 1.31 (t, *J* = 7.0 Hz, 3H), 0.91-0.76 (m, 4H).

### Example 62

### (S)-1-(1-ethyl-3-methyl-1H-pyrazole-5-carboxamido)-9-methyl-8,9-dihydro-7H-6-oxa-2,9a-d iazabenzo[cd]azulene-4-carboxamide

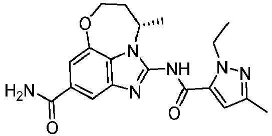

### Synthetic scheme:

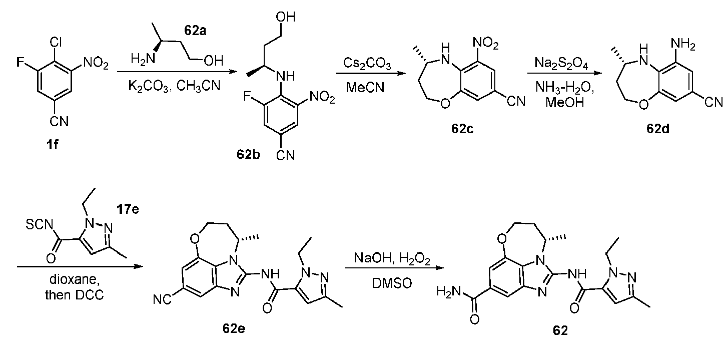

Step 1: To a stirring solution of compound **1f** (1.00 g, 5.00 mmol) and 62a (940 mg, 7.50 mmol) in acetonitrile (20 mL) was added K₂CO₃ (1.38 g, 10.05 mmol). The reaction mixture was heated at 70 °C for 16 hours, and TLC indicated the starting material was consumed. The reaction mixture was cooled to room temperature, filtered through a pad of silica gel, and rinsed with DCM (100 mL). The filtrate was concentrated, and the residue was purified by silica gel chromatography to give compound **62b** (1.05 g, 83% yield) as yellow solid. ESI-MS (m/z): 254.5 [M+H]⁺.

Step 2: To a stirring solution of compound **62b** (1.05 g, 4.15 mmol) in acetonitrile (40 mL) was added Cs₂CO₃ (2.70 g, 8.30 mmol). The reaction mixture was heated at 70 °C for 3 hours, and TLC indicated the reaction was complete. The reaction mixture was cooled to room temperature, filtered through a pad of silica gel, and rinsed with DCM. The filtrate was concentrated to give compound **62c** (760 mg) as brown oil, which was used directly without further purification. ESI-MS (m/z): 234.4 [M+H]⁺.

Step 3: Compound **62c** (760 mg, from step 2) was dissolved in a mixture of MeOH (50 mL) and concentrated ammonium hydroxide (5 mL). And sodium dithionite (2.8 g, 16.10 mmol) was dissolved in water (5 mL), the resultant solution was added to the reaction mixture at room temperature. Stirring was continued at room temperature for half an hour. LCMS indicated the reaction was complete. The reaction mixture was poured into water (100 mL) and extracted with ethyl acetate (100 mL × 3). The combined organic layers were washed with brine, dried over Na₂SO₄, filtered and concentrated under reduced pressure to give compound **62d** (390 mg) as brown oil, which was used directly without further purification. ESI-MS (m/z): 204.6 [M+H]⁺.

Step 4: Compound **62d** (390 mg from step 3) was dissolved in dioxane (30 mL), and then compound **17e** (1M in dioxane, 2.0 mL, 2.0 mmol) was added. The reaction mixture was stirred at room temperature for 30 minutes, DCC (730 mg, 3.84 mmol) was added, and the mixture was heated at 80 °C for 2 hours, LCMS indicated the product was formed. The reaction mixture was concentrated, and the residue was purified by silica gel chromatography to give compound 62e (300 mg, 20% yield for 3 steps) as white solid. ESI- MS (m/z): 365.3[M+H]⁺.

Step 5: To a stirring solution of compound **62e** (300 mg, 0.82 mmol) and NaOH (100 mg, 2.50 mmol) in DMSO (8 mL) at 60 °C was added hydrogen peroxide (30 wt. %, 5 mL) dropwise. The reaction mixture was stirred at 60 °C for 30 minutes, LCMS indicated the reaction was complete. The reaction mixture was purified directly by reversed phase preparative HPLC to give compound **62** (110 mg, 35% yield) as white solid. ESI-MS (m/z): 383.3; ¹H NMR (500 MHz, DMSO-*d*₆) δ 12.86 (s, 1H), 7.88 (s, 1H), 7.67-7.57 (m, 1H), 7.34-7.21 (m, 2H), 6.64 (s, 1H), 5.01-4.91 (m, 1H), 4.68-4.51 (m, 3H), 4.47-4.38 (m, 1H), 2.58-2.50 (m, 1H), 2.37-2.25 (m, 1H), 2.16 (s, 3H), 1.44 (d, *J=* 6.6 Hz, 3H), 1.34 (t, *J=* 7.1 Hz, 3H).

### Example 63

### (S)-1-(1-ethyl-4-fluoro-3-methyl-1H-pyrazole-5-carboxamido)-9-methyl-8,9-dihydro-7H-6-o xa-2,9a-diazabenzo[cd]azulene-4-carboxamide

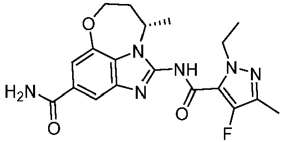

### Synthetic scheme:

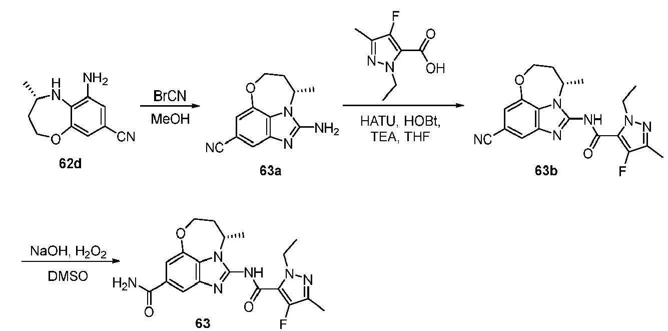

Step 1: Compound 62d (300mg, 1.48 mmol) was dissolved in MeOH (10 mL), and cyanogen bromide (780 mg, 7.43 mmol) was added. The resulting mixture was stirred at room temperature overnight, LCMS indicated the product was formed. The mixture was concentrated in vacuo to remove the solvent. The residue was mixed with EtOAc (100 mL), and washed with saturated Na₂CO₃ soultion (60 mL). The aqueous layer was extracted with EtOAc (100 mL × 2). The combined organic layers were washed with brine, dried over Na₂SO₄, filtered and concentrated. The residue was purified by silica gel chromatography to give compound **63a** (255 mg, 75% yield) as yellow solid. ESI-MS (m/z): 229.3 [M+H]⁺.

Step 2: To a stirring solution of compound **63a** (250 mg, 1.09 mmol) and 1-ethyl-4-fluoro-3-methyl-1H-pyrazole-5-carboxylic acid (188 mg, 1.09 mmol) in THF (10 mL) was added HATU (416 mg, 1.09 mmol), HOBt (74 mg, 0.55 mmol) and triethylamine (0.42 mL, 3.27 mmol). The mixture was stirred at room temperature overnight, LCMS indicated the reaction was complete. The mixture was poured into water (60 mL) and extracted with EtOAc (80 mL × 3). The combined organic layers were washed brine, dried over Na₂SO₄, filtered and concentrated. The residue was purified by silica gel chromatography to give compound **63b** (379 mg, 90% yield) as white solid. ESI-MS (m/z): 383.5 [M+H]⁺.

Step 3: To a stirring solution of compound **63b** (379 mg, 0.99 mmol) and NaOH (120 mg, 3.00 mmol) in DMSO (4 mL) at 60 °C was added hydrogen peroxide (30 wt. %, 3 mL) dropwise. The reaction mixture was stirred at 60 °C for 30 minutes, LCMS indicated the reaction was complete. The reaction mixture was purified directly by reversed phase preparative HPLC to give compound **63** (160 mg, 40% yield) as white solid. ESI-MS (m/z): 401.0 [M+H]⁺; ¹H NMR (500 MHz, DMSO-*d*₆) δ 12.92 (br s, 1H), 7.89 (s, 1H), 7.63 (d, *J =* 1.6 Hz, 1H), 7.34-7.23 (m, 2H), 5.00-4.89 (m, 1H), 4.66-4.36 (m, 4H), 2.59-2.50 (m, 1H), 2.38-2.28 (m, 1H), 2.14 (s, 3H), 1.45 (d, *J* = 6.7 Hz, 3H), 1.32 (t, *J* = 7.1 Hz, 3H).

### Example 64

### 1-(1-ethyl-4-fluoro-3-methyl-1H-pyrazole-5-carboxamido)-7,9-dihydrospiro[6-oxa-2,9a-diaz abenzo[cd]azulene-8,1'-cyclopropane]-4-carboxamide

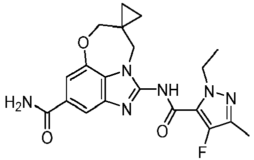

### Synthetic scheme:

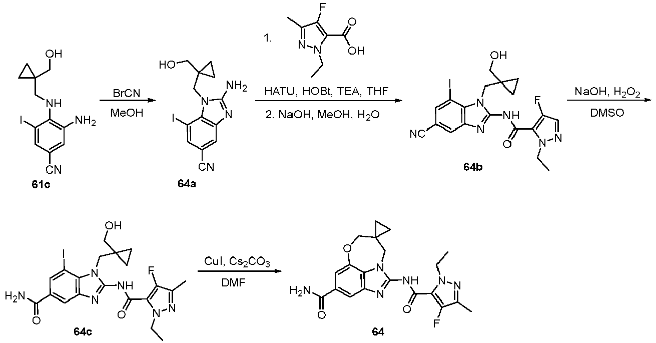

Step 1: Compound **61c** (50 mg, 0.14 mmol) was dissolved in MeOH (5 mL), and cyanogen bromide (75 mg, 0.71 mmol) was added. The resulting mixture was stirred at room temperature overnight, LCMS indicated the product was formed. The mixture was concentrated in vacuo to remove the solvent. The residue was mixed with EtOAc (30 mL), and washed with saturated Na₂CO₃ soultion (20 mL). The aqueous layer was extracted with EtOAc (30 mL × 2). The combined organic layers were washed with brine, dried over Na₂SO₄, filtered and concentrated. The residue was purified by silica gel chromatography to give compound **64a** (42 mg, 80% yield) as yellow solid. ESI-MS (m/z): 369.1 [M+H]⁺.

Step 2: To a stirring solution of compound **64a** (42 mg, 0.11 mmol) and 1-ethyl-4-fluoro-3-methyl-1H-pyrazole-5-carboxylic acid (20 mg, 0.12 mmol) in THF (5 mL) was added HATU (443 mg, 0.11 mmol), HOBt (8 mg, 0.06 mmol) and triethylamine (35 mg, 0.34 mmol). The mixture was stirred at room temperature overnight. Then MeOH (2.5 mL), water (2.5 mL) and NaOH (15 mg, 0.37 mmol) were added, and the mixture was stirred at room temperatue for 1 hour. LCMS indicated the product was formed. The mixture was poured into water (40 mL) and extracted with EtOAc (30 mL × 3). The combined organic layers were washed brine, dried over Na₂SO₄, filtered and concentrated. The residue was purified by silica gel chromatography to give compound **64b** (32 mg, 56% yield) as white solid. ESI-MS (m/z): 509.5 [M+H]⁺.

Step 3: To a stirring solution of compound **64b** (32 mg, 0.06 mmol) and NaOH (10 mg, 0.25 mmol) in DMSO (1 mL) at 60 °C was added hydrogen peroxide (30 wt. %, 0.3 mL) dropwise. The reaction mixture was stirred at 60 °C for 30 minutes, LCMS indicated the reaction was complete. The reaction mixture was purified directly by reversed phase preparative HPLC to give compound **64c** (15 mg, 45% yield) as white solid. ESI-MS (m/z): 541.5 [M+H]⁺.

Step **4**: To a solution of compound **64c** (15 mg, 0.03 mmol) in DMF (1 mL) was added copper(l) iodide (4 mg, 0.02 mmol) and Cs₂CO₃ (20 mg, 0.06 mmol) at room temperature under nitrogen atmosphere. The reaction mixture was heated at 130 °C by microwave for 1 hour. TLC indicated the starting material was consumed. The reaction mixture was purified directly by reversed phase preparative HPLC to give compound **64** (4.3 mg, 37% yield) as white solid. ESI-MS (m/z): 413.4; ¹H NMR (500 MHz, DMSO-*d*₆) δ 8.39 (br s, 1H), 7.90 (s, 1H), 7.64 (s, 1H), 7.30 (s, 1H), 4.56-4.47 (m, 2H), 4.27 (s, 2H), 4.00 (s, 2H), 2.12 (s, 3H), 1.30 (t, *J =* 7.0 Hz, 3H), 0.93-0.70 (m, 4H).

### Example 65

### 1-(1-ethyl-4-fluoro-3-methyl-1H-pyrazole-5-carboxamido)-8,9-dihydro-7H-6-oxa-2,9a-diaza benzo[cd]azulene-4-carboxamide

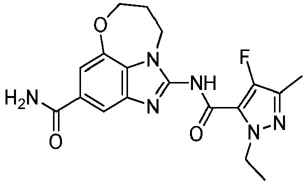

### Synthetic scheme:

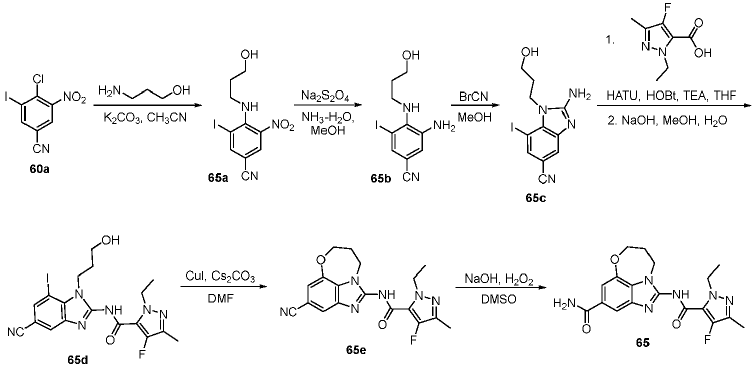

Step **1**: To a stirring solution of compound **60a** (200 mg, 0.65 mmol) and 3-aminopropan-1-ol (110 mg, 0.99 mmol) in acetonitrile (30 mL) was added K₂CO₃ (180 mg, 1.30 mmol). The reaction mixture was heated at 70 °C for 16 hours, and TLC indicated the starting material was consumed. The reaction mixture was cooled to room temperature, filtered through a pad of silica gel, and rinsed with DCM (50 mL). The filtrate was concentrated, and the residue was purified by silica gel chromatography to give compound **65a** (190 mg, 85% yield) as yellow solid. ESI-MS (m/z): 348.2 [M+H]⁺.

Step 2: Compound **65a** (190 mg, 0.54 mmol) was dissolved in MeOH (20 mL) and concentrated ammonium hydroxide (2 mL). Sodium dithionite (475 mg, 2.73 mmol) was dissolved in water (2 mL) and added dropwise to the reaction mixture at room temperature. Stirring was continued at room temperature for 30 minutes, and LCMS indicated the starting material was consumed. The reaction mixture was poured into water (40 mL), extracted with EtOAc (50 mL × 3). The combined organic layers were washed with brine, dried over Na₂SO₄, filtered and concentrated to give crude compound **65b** (102 mg) as yellow oil, which was used directly without further purification. ESI -MS (m/z): 318.4[M+H]⁺.

Step 3: Compound **65b** (102 mg, from step 2) was dissolved in MeOH (5 mL), and cyanogen bromide (170 mg, 1.62 mmol) was added. The resulting mixture was stirred at room temperature overnight, LCMS indicated the product was formed. The mixture was concentrated in vacuo to remove the solvent. The residue was mixed with EtOAc (40 mL), and washed with saturated Na₂CO₃ soultion (30 mL). The aqueous layer was extracted with EtOAc (40 mL × 2). The combined organic layers were washed with brine, dried over Na₂SO₄, filtered and concentrated. The residue was purified by silica gel chromatography to give compound **65c** (55 mg, 30% yield for 2 steps) as yellow solid. ESI-MS (m/z): 343.1 [M+H]⁺.

Step 4: To a stirring solution of compound **65c** (55 mg, 0.16 mmol) and 1-ethyl-4-fluoro-3-methyl-1H-pyrazole-5-carboxylic acid (28 mg, 0.16 mmol) in THF (5 mL) was added HATU (62 mg, 0.16 mmol), HOBt (11 mg, 0.08 mmol) and triethylamine (50 mg, 0.49 mmol). The mixture was stirred at room temperature overnight. Then MeOH (2.5 mL), water (2.5 mL) and NaOH (20 mg, 0.50 mmol) were added, and the mixture was stirred at room temperatue for 1 hour. LCMS indicated the product was formed. The mixture was poured into water (40 mL) and extracted with EtOAc (40 mL × 3). The combined organic layers were washed brine, dried over Na₂SO₄, filtered and concentrated. The residue was purified by silica gel chromatography to give compound **65d** (33 mg, 42% yield) as white solid. ESI-MS (m/z): 497.3 [M+H]⁺.

Step **5**: To a solution of compound **65d** (33 mg, 0.07 mmol) in DMF (1 mL) was added copper(l) iodide (9 mg, 0.05 mmol) and Cs₂CO₃ (46 mg, 0.14 mmol) at room temperature under nitrogen atmosphere. The reaction mixture was heated at 130 °C by microwave for 1 hour. TLC indicated the starting material was consumed. The reaction mixture was purified directly by reversed phase preparative HPLC to give compound **65e** (18 mg, 78% yield) as yellow solid. ESI-MS (m/z): 369.5 [M+H]⁺.

Step 6: To a stirring solution of compound **65e** (18 mg, 0.05mmol) and NaOH (10 mg, 0.25 mmol) in DMSO (1 mL) at 60 °C was added hydrogen peroxide (30 wt. %, 0.3 mL) dropwise. The reaction mixture was stirred at 60 °C for 30 minutes, LCMS indicated the reaction was complete. The reaction mixture was purified directly by reversed phase preparative HPLC to give compound **65** (9.4 mg, 48% yield) as white solid. ESI-MS (m/z): 387.4 [M+H]⁺; ¹H NMR (500 MHz, DMSO-*d*₆) δ 7.90 (s, 1H), 7.64 (s, 1H), 7.31 (s, 1H), 7.28 (s, 1H), 4.63-4.49 (m, 2H), 4.42-4.33 (m, 2H), 4.23-4.10 (m, 2H), 2.40-2.25 (m, 2H), 2.13 (s, 3H), 1.32 (t, *J=* 7.1 Hz, 3H).

### Example 66

### 1-(1-Ethyl-4-fluoro-3-methyl-1H-pyrazole-5-carboxamido)-6-methyl-6,7,8,9-tetrahydro-2,6, 9a-triazabenzo[cd]azulene-4-carboxamide

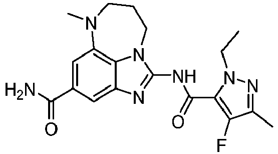

### Synthetic scheme:

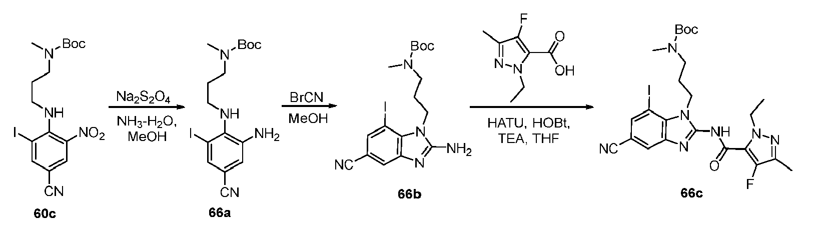

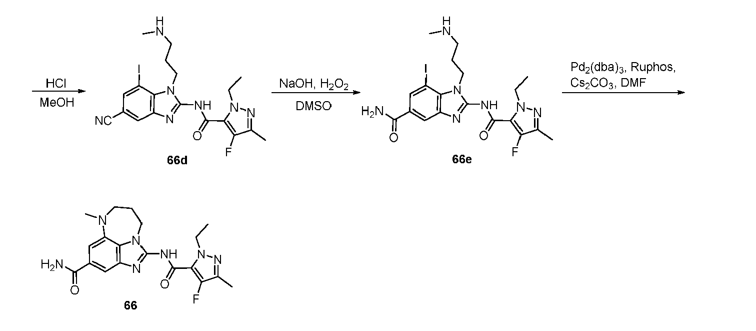

Step 1: Compound **60c** (200 mg, 0.43 mmol) was dissolved in MeOH (20 mL) and concentrated ammonium hydroxide (2 mL). Sodium dithionite (380 mg, 2.18 mmol) was dissolved in water (3 mL) and added dropwise to the reaction mixture at room temperature. Stirring was continued at room temperature for 30 minutes, and LCMS indicated the starting material was consumed. The reaction mixture was poured into water (70 mL), extracted with EtOAc (80 mL × 3). The combined organic layers were washed with brine, dried over Na₂SO₄, filtered and concentrated to give crude compound **66a** (140 mg) as yellow solid, which was used directly without further purification. ESI-MS (m/z): 431.6 [M+H]⁺.

Step 2: Compound **66a** (140 mg, from step 1) was dissolved in MeOH (15 mL), and cyanogen bromide (170 mg, 1.62 mmol) was added. The resulting mixture was stirred at room temperature overnight, LCMS indicated the product was formed. The mixture was concentrated in vacuo to remove the solvent. The residue was mixed with EtOAc (70 mL), and washed with saturated Na₂CO₃ soultion (50 mL). The aqueous layer was extracted with EtOAc (60 mL × 2). The combined organic layers were washed with brine, dried over Na₂SO₄, filtered and concentrated. The residue was purified by silica gel chromatography to give compound **66b** (115 mg, 59% yield for 2 steps) as yellow solid. ESI-MS (m/z): 456.1 [M+H]⁺.

Step 3: To a stirring solution of compound **66b** (115 mg, 0.25 mmol) and 1-ethyl-4-fluoro-3-methyl-1H-pyrazole-5-carboxylic acid (45 mg, 0.26 mmol) in THF (12 mL) was added HATU (95 mg, 0.25 mmol), HOBt (18 mg, 0.13 mmol) and triethylamine (75 mg, 0.75 mmol). The mixture was stirred at room temperature overnight. Then MeOH (2.5 mL), water (2.5 mL) and NaOH (20 mg, 0.50 mmol) were added, and the mixture was stirred at room temperatue for 1 hour. LCMS indicated the product was formed. The mixture was poured into water (50 mL) and extracted with EtOAc (50 mL × 3). The combined organic layers were washed brine, dried over Na₂SO₄, filtered and concentrated. The residue was purified by silica gel chromatography to give compound **66c** (110 mg, 71% yield) as white solid. ESI-MS (m/z): 610.5 [M+H]⁺.

Step 4: To a stirring solution of **66c** (110 mg, 0.18 mmol) in MeOH (15 mL) was added 4M HCl in dioxane (0.25 mL, 1 mmol). The mixture was stirred at room temperature for 1 hour, LCMS indicated the product was formed. The reaction mixture was concentrated, and the residue was purified by silica gel chromatography to give compound **66d** (90 mg) as white solid. ESI-MS (m/z): 510.6 [M+H]⁺.

Step 5: To a stirring solution of compound **66d** (90 mg, 0.16 mmol) and NaOH (20 mg, 0.50 mmol) in DMSO (3 mL) at 60 °C was added hydrogen peroxide (30 wt. %, 2 mL) dropwise. The reaction mixture was stirred at 60 °C for 30 minutes, LCMS indicated the reaction was complete. The reaction mixture was poured into water (20 mL), extracted with EtOAc (30 mL × 3). The combined organic layers were washed with brine, dried over Na₂SO₄, filtered and concentrated. The residue was purified by silica gel chromatography to give compound **66e** (44 mg, 51% yield) as white solid. ESI-MS (m/z): 528.1 [M+H]⁺.

Step 6: To a solution of compound **66e** (44 mg, 0.08 mmol) in DMF (2 mL) was added Pd₂(dba)₃ (8 mg, 0.01 mmol), Ruphos (8 mg, 0.02 mmol) and Cs₂CO₃ (55 mg, 0.17 mmol) at room temperature under nitrogen atmosphere. The reaction mixture was heated at 90 °C overnight. LCMS indicated the starting material was consumed. The reaction mixture was purified directly by reversed phase preparative HPLC to give compound **66** (7.8 mg, 24% yield) as white solid. ESI-MS (m/z): 400.3 [M+H]⁺; ¹H NMR (500 MHz, DMSO-*d*₆) δ 12.78 (br s, 1H), 7.90 (s, 1H), 7.44 (d, *J* = 1.5 Hz, 1H), 7.22 (s, 1H), 6.96 (d, *J* = 1.5 Hz, 1H), 4.54 (q, *J* = 7.1 Hz, 2H), 4.17-4.02 (m, 2H), 3.48-3.40 (m, 2H), 3.05 (s, 3H), 2.21-2.15 (m, 2H), 2.13 (s, 3H), 1.31 (t, *J* = 7.1 Hz, 3H).

### Example 67

### 1-(1-Ethyl-3-methyl-1H-pyrazole-5-carboxamido)-8,8-dimethyl-6,7,8,9-tetrahydro-2,6,9a-tri azabenzo[cd]azulene-4-carboxamide

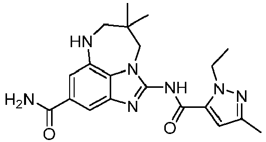

### Synthetic scheme:

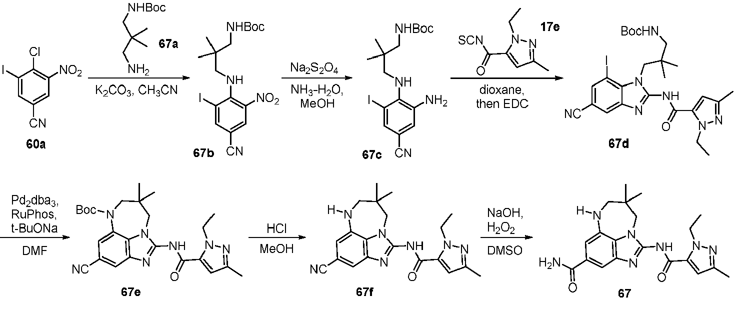

Step **1**: To a stirring solution of compound **60a** (4.0 g, 12.97 mmol) and **67a** (3.15 g, 15.56 mmol) in acetonitrile (50 mL) was added K₂CO₃ (3.58 g, 25.94 mmol). The reaction mixture was heated at 70 °C for 6 hours, and TLC indicated the starting material was consumed. The reaction mixture was cooled to room temperature, filtered through a pad of silica gel. The filtrate was concentrated, and the residue was purified by silica gel chromatography to give compound **67b** (4.0 g, 65% yield) as yellow solid. ESI-MS (m/z): 475.2 [M+H]⁺.

Step 2: Compound **67b** (4.0 g, 8.43 mmol) was dissolved in MeOH (40 mL) and concentrated ammonium hydroxide (8 mL). Sodium dithionite (7.34 g, 42.17 mmol) was dissolved in water (8 mL) and added dropwise to the reaction mixture at room temperature. Stirring was continued at room temperature for 5 minutes, and LCMS indicated the starting material was consumed. The reaction mixture was filtered and the filtrate was concentrated. The residue was purified by silica gel chromatography to give compound **67c** (3.4 g, 90% yield) as pink solid. ESI -MS (m/z): 445.3 [M+H]⁺.

Step 3: Compound **67c** (1.8 g, 4.05 mmol) was dissolved in dioxane (20 mL), and then compound **17e** (1M in dioxane, 4.25 mL, 4.25 mmol) was added. The reaction mixture was stirred at room temperature for 10 minutes, LCMS indicated the starting material was consumed. EDCI (931 mg, 4.86 mmol) was added, and the mixture was heated at 80 °C for 2 hours, LCMS indicated the product was formed. The reaction mixture was concentrated, and the residue was purified by silica gel chromatography to give compound **67d** (1.5 g, 61% yield) as pink solid. ESI- MS (m/z): 606.5 [M+H]⁺.

Step **4**: To a solution of compound **67d** (500 mg, 0.82 mmol) in DMF (10 mL) was added Pd₂(dba)₃ (75 mg, 0.08 mmol), Ruphos (77 mg, 0.16 mmol) and Sodium tert-butoxide (237 mg, 2.47 mmol) at room temperature under nitrogen atmosphere. The reaction mixture was heated at 110 °C by microwave for 2.5 hours. LCMS indicated the starting material was consumed. The reaction mixture was poured into water (30 mL), and extracted with EtOAc (15 mL × 3). The combined organic layers were washed with brine, dried over Na₂SO₄, filtered and concentrated to give crude compound **67e** (350 mg) as brown oil, which was used directly without further purification. ESI-MS (m/z): 478.6 [M+H]⁺.

Step **5**: To a stirring solution of **67e** (350 mg, from step 4) in MeOH (10 mL) was added 4M HCl in dioxane (5 mL, 20 mmol). The mixture was stirred at room temperature for 5 hours, LCMS indicated the product was formed. The reaction mixture was concentrated to give crude compound **67f** (280 mg) as yellow solid, which was used directly without further purification. ESI-MS (m/z): 378.6 [M+H]⁺.

Step 6: To a stirring solution of compound **67f** (280 mg, from step 5) and NaOH (81 mg, 2.02 mmol) in DMSO (4 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound 67 (51 mg, 15% yield for 3 steps) as white solid. ESI-MS (m/z): 396.6 [M+H]⁺; ¹H NMR (500 MHz, DMSO-*d6*) δ 12.61 (br s, 1H), 7.71 (s, 1H), 7.21 (d, *J* = 1.5 Hz, 1H), 7.13 (s, 1H), 6.98 (d, *J=* 1.5 Hz, 1H), 6.65 (s, 1H), 6.42 (t, *J =* 3.0 Hz, 1H), 4.61 (q, J=7.0Hz, 2H), 3.89 (s, 2H), 3.09 (d, *J* = 3.0 Hz, 2H), 2.17 (s, 3H), 1.35 (t, *J=* 7.0 Hz, 3H), 1.03 (s, 6H).

### Example 68

### 1-(1-Ethyl-3-methyl-1H-pyrazole-5-carboxamido)-6,8,8-trimethyl-6,7,8,9-tetrahydro-2,6,9a-triazabenzo[cd]azulene-4-carboxamide

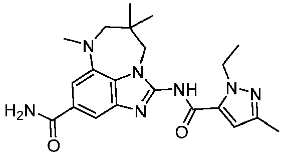

### Synthetic scheme:

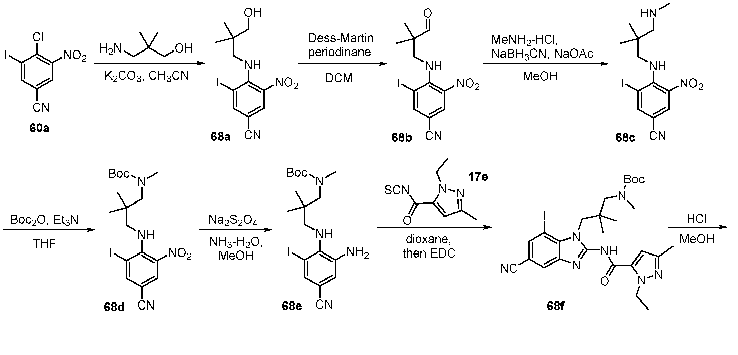

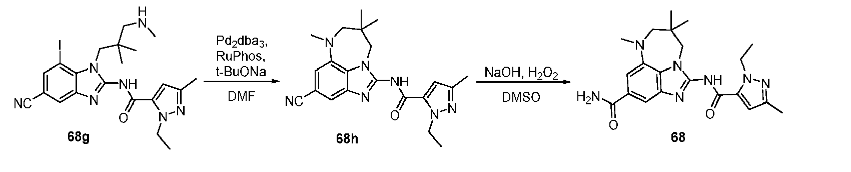

Step **1**: To a stirring solution of compound **60a** (2.0 g, 6.48 mmol) and 3-amino-2,2-dimethylpropan-1-ol (1.0 g, 9.69 mmol) in acetonitrile (30 mL) was added K₂CO₃ (1.79 g, 12.97 mmol). The reaction mixture was heated at 70 °C for 4 hours, and TLC indicated the starting material was consumed. The reaction mixture was cooled to room temperature, filtered through a pad of silica gel. The filtrate was concentrated, and the residue was purified by silica gel chromatography to give compound **68a** (2.3 g, 94% yield) as yellow solid. ESI-MS (m/z): 376.3 [M+H]⁺; ¹H NMR (500 MHz, DMSO-d(5) δ 8.37 (s, 1H), 8.30 (s, 1H), 7.03 (t, *J* = 4.5 Hz, 1H), 5.40 (s, 1H), 3.35 (s, 2H), 2.76 (d, *J* = 4.0 Hz, 2H), 0.87(s, 6H).

Step 2: To a solution of compound **68a** (2.3 g, 6.13 mmol) in DCM (30 mL) at 0 °C was added Dess-Martin periodinane (3.90 g, 9.2 mmol) by portions. The mixture was stirred at 0 °C for 2 hours, TLC indicated the starting material was consumed. The reaction mixture was washed with saturated NaHCO₃ solution, the organic layer was dried over Na₂SO₄, filtered and concentrated. The residue was purified by silica gel chromatography to give compound **68b** (2 g, 87% yield) as yellow solid. ¹H NMR (500 MHz, DMSO-*d6*) δ 9.49 (s, 1H), 8.44 (d, *J* = 2.0 Hz, 1H), 8.37 (d, *J* = 2.0 Hz, 1H), 5.98 (t, *J* = 5.5 Hz, 1H), 3.11 (d, *J* = 5.5 Hz, 2H), 1.08 (s, 6H).

Step 3: To a solution of compound **68b** (550 mg, 1.47 mmol) in MeOH (10 mL) was added methylamine hydrochloride (298 mg, 4.42 mmol) and sodium acetate (423 mg, 5.16 mmol). The mixture was stirred at room temperature for 3 hours, then sodium cyanoborohydride (277 mg, 4.42 mmol) was added. The resultant mixture was stirred at room temperature overnight. LCMS indicated the product was formed. The reaction mixture was concentrated, and the residue was purified by silica gel colomn chromatography to give compound **68c** (370 mg, 64% yield) as yellow oil. ¹H NMR (500 MHz, DMSO-*d6*) δ 8.32 (d, *J* = 2.0 Hz, 1H), 8.26 (d, *J* = 2.0 Hz, 1H), 2.71 (s, 2H), 2.55 (s, 2H), 2.35 (s, 3H), 0.87(s, 6H).

Step 4: To a solution of compound **68c** (370 mg, 0.95 mmol) in THF (10 mL) was added Boc₂O (228 mg, 1.05 mmol) and triethylamine (0.29 mL, 2.1 mmol). The mixture was stirred at room temperature overnight. LCMS indicated the reaction was complete. The reaction mixture was concentrated to give crude compound **68d** (450 mg) as yellow oil, which was used directly without further purification. ESI-MS (m/z): 489.2 [M+H]⁺.

Step 5: Compound **68d** (450 mg, from step 4) was dissolved in a mixture of MeOH (5 mL) and concentrated ammonium hydroxide (1 mL). And sodium dithionite (802 mg, 4.61 mmol) was dissolved in water (3 mL), the resultant solution was added to the reaction mixture at room temperature. Stirring was continued at room temperature for 5 minutes, LCMS indicated the reaction was complete. The reaction mixture was filtered, and the filtrate was concentrated. The residue was purified by silica gel chromatography to give compound **68e** (240 mg, 55% yield) as red oil. ESI -MS (m/z): 459.2 [M+H]⁺.

Step 6: Compound **68e** (240 mg, 0.52 mmol) was dissolved in dioxane (10 mL), and then compound **17e** (1M in dioxane, 0.57 mL, 0.57 mmol) was added. The reaction mixture was stirred at room temperature for 10 minutes, LCMS indicated the starting material was consumed. EDCI (130 mg, 0.68 mmol) was added, and the mixture was heated at 80 °C for 2 hours, LCMS indicated the product was formed. The reaction mixture was concentrated, and the residue was purified by silica gel chromatography to give compound **68f** (160 mg, 49% yield) as pink solid. ESI- MS (m/z): 620.1 [M+H]⁺.

Step 7: To a stirring solution of **68f** (160 mg, 0.25 mmol) in MeOH (5 mL) was added 4M HCl in dioxane (3 mL, 12 mmol). The mixture was stirred at room temperature for 3 hours, LCMS indicated the product was formed. The reaction mixture was concentrated to give crude compound **68g** (140 mg) as yellow solid, which was used directly without further purification. ESI-MS (m/z): 520.3 [M+H]⁺.

Step 8: To a solution of compound **68g** (140 mg, from step 7) in DMF (5 mL) was added Pd₂(dba)₃ (23 mg, 0.025 mmol), Ruphos (23 mg, 0.050 mmol) and sodium tert-butoxide (72 mg, 0.75 mmol) at room temperature under nitrogen atmosphere. The reaction mixture was heated at 110 °C by microwave for 2.5 hours. LCMS indicated the starting material was consumed. The reaction mixture was poured into water (20 mL), and extracted with EtOAc (10 mL × 3). The combined organic layers were washed with brine, dried over Na₂SO₄, filtered and concentrated to give crude compound **68h** (60 mg) as brown oil, which was used directly without further purification. ESI-MS (m/z): 392.6 [M+H]⁺.

Step 9: To a stirring solution of compound **68h** (60 mg, from step 8) and NaOH (18 mg, 0.45 mmol) in DMSO (3 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound **68** (6.5 mg, 6% yield for 3 steps) as white solid. ESI-MS (m/z): 410.4 [M+H]⁺; ¹H NMR (500 MHz, DMSO-*d6*) δ 12.73 (s, 1H), 7.90 (s, 1H), 7.45 (s, 1H), 7.24 (s, 1H), 6.96 (s, 1H), 6.65 (s, 1H), 4.67-4.55 (m, 2H), 3.94 (s, 2H), 3.19 (s, 2H), 3.05 (s, 3H), 2.17(s, 3H), 1.35 (t, *J =* 7.0 Hz, 3H), 1.07 (s, 6H).

### Example 69

### 6-Ethyl-1-(1-ethyl-3-methyl-1H-pyrazole-5-carboxamido)-8,8-dimethyl-6,7,8,9-tetrahy dro-2,6,9a-triazabenzo[cd]azulene-4-carboxamide

### Synthetic scheme:

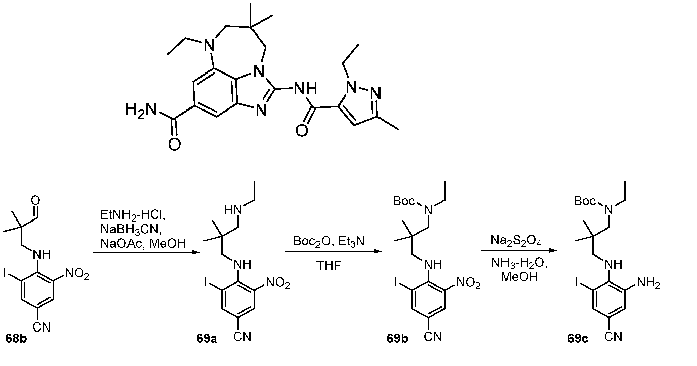

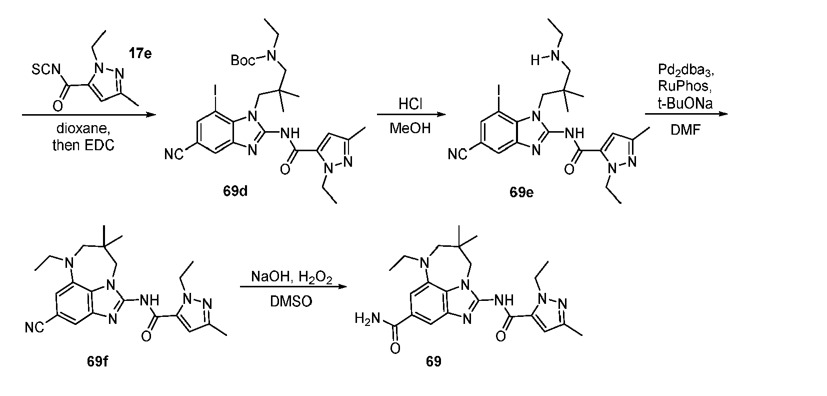

Step 1: To a solution of compound **68b** (500 mg, 1.34 mmol) in MeOH (10 mL) was added ethylamine hydrochloride (327 mg, 4.02 mmol) and sodium acetate (384 mg, 4.69 mmol). The mixture was stirred at room temperature for 3 hours, then sodium cyanoborohydride (252 mg, 4.02 mmol) was added. The resultant mixture was stirred at room temperature overnight. LCMS indicated the product was formed. The reaction mixture was concentrated, and the residue was purified by silica gel colomn chromatography to give compound **69a** (350 mg, 64% yield) as yellow oil. ESI-MS (m/z): 403.7 [M+H]⁺.

Step 2: To a solution of compound **69a** (350 mg, 0.87 mmol) in THF (10 mL) was added Boc₂O (208 mg, 0.95 mmol) and triethylamine (0.27 mL, 1.91 mmol). The mixture was stirred at room temperature overnight. LCMS indicated the reaction was complete. The reaction mixture was concentrated to give crude compound **69b** (420 mg) as yellow oil, which was used directly without further purification. ESI-MS (m/z): 503.2 [M+H]⁺_{∘}

Step 3: Compound **69b** (420 mg, from step 2) was dissolved in a mixture of MeOH (5 mL) and concentrated ammonium hydroxide (1 mL). And sodium dithionite (727 mg, 4.18 mmol) was dissolved in water (3 mL), the resultant solution was added to the reaction mixture at room temperature. Stirring was continued at room temperature for 5 minutes, LCMS indicated the reaction was complete. The reaction mixture was filtered, and the filtrate was concentrated. The residue was purified by silica gel chromatography to give compound **69c** (300 mg, 73% yield) as red oil. ESI -MS (m/z): 473.3 [M+H]⁺.

Step 4: Compound **69c** (300 mg, 0.63 mmol) was dissolved in dioxane (10 mL), and then compound **17e** (1M in dioxane, 0.7 mL, 0.7 mmol) was added. The reaction mixture was stirred at room temperature for 10 minutes, LCMS indicated the starting material was consumed. EDCI (158 mg, 0.82 mmol) was added, and the mixture was heated at 80 °C for 2 hours, LCMS indicated the product was formed. The reaction mixture was concentrated, and the residue was purified by silica gel chromatography to give compound **69d** (250 mg, 62% yield) as red oil. ESI-MS (m/z): 634.5 [M+H]⁺.

Step 5: To a stirring solution of **69d** (250 mg, 0.31 mmol) in MeOH (10 mL) was added 4M HCl in dioxane (5 mL, 20 mmol). The mixture was stirred at room temperature for 3 hours, LCMS indicated the product was formed. The reaction mixture was concentrated to give crude compound **69e** (170 mg) as pink solid. ESI-MS (m/z): 534.5 [M+H]⁺.

Step 6: To a solution of compound **69e** (170 mg, from step 7) in DMF (5 mL) was added Pd₂(dba)₃ (27 mg, 0.029 mmol), Ruphos (27 mg, 0.059 mmol) and sodium tert-butoxide (86 mg, 0.89 mmol) at room temperature under nitrogen atmosphere. The reaction mixture was heated at 110 °C by microwave for 2.5 hours. LCMS indicated the starting material was consumed. The reaction mixture was poured into water (20 mL), and extracted with EtOAc (10 mL × 3). The combined organic layers were washed with brine, dried over Na₂SO₄, filtered and concentrated to give crude compound **69f** (70 mg) as brown oil, which was used directly without further purification. ESI-MS (m/z): 406.4 [M+H]⁺.

Step 7: To a stirring solution of compound **69f** (70 mg, from step 6) and NaOH (20 mg, 0.51 mmol) in DMSO (3 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound **69** (33 mg, 19% yield) as white solid. ESI-MS (m/z): 424.5 [M+H]⁺; ¹H NMR (500 MHz, DMSO-*d6*) δ 12.67 (br s, 1H), 7.90 (s, 1H), 7.40 (s, 1H), 7.22 (d, *J* = 1.5 Hz, 1H), 7.00 (d, *J=* 1.5 Hz, 1H), 6.64 (s, 1H), 4.61 (q, *J* = 7.0 Hz, 2H), 3.93 (s, 2H), 3.48 (q, *J* = 7.0 Hz, 2H), 3.19 (s, 2H), 2.17 (s, 3H), 1.35 (t, *J=* 7.0 Hz, 3H), 1.19 (t, *J=* 7.0 Hz, 3H), 1.06 (s, 6H).

### Example 70

### (R)-1-(1-Ethyl-3-methyl-1H-pyrazole-5-carboxamido)-8-methyl-8,9-dihydro-7H-6-oxa-2,9a-diazabenzo[cd]azulene-4-carboxamide

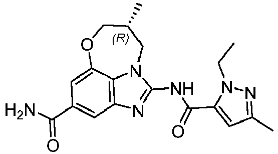

### Synthetic scheme:

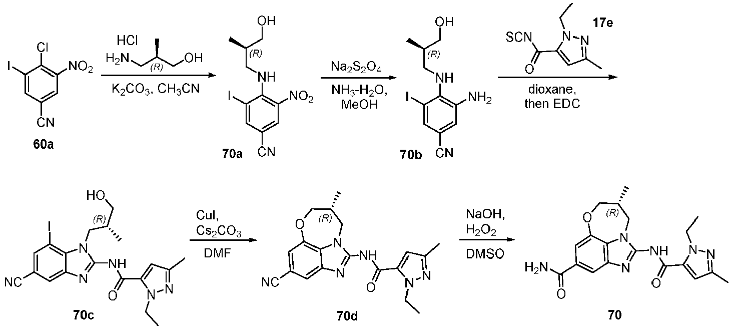

Step **1**: To a stirring solution of compound **60a** (4.0 g, 12.97 mmol) and (R)-3-amino-2-methylpropan-1-ol hydrochloride (3.0 g, 23.88 mmol) in acetonitrile (50 mL) was added K₂CO₃ (7.17 g, 51.87 mmol). The reaction mixture was heated at 60 °C for 16 hours, and TLC indicated the starting material was consumed. The reaction mixture was cooled to room temperature, filtered through a pad of silica gel. The filtrate was concentrated, and the residue was purified by silica gel chromatography to give compound **70a** (3.5 g, 74% yield) as yellow solid. ESI-MS (m/z): 362.1 [M+H]⁺.

Step 2: Compound **70a** (2.0 g, 5.54 mmol) was dissolved in a mixture of MeOH (20 mL) and concentrated ammonium hydroxide (4 mL). And sodium dithionite (5.79 g, 33.23 mmol) was dissolved in water (10 mL), the resultant solution was added to the reaction mixture at room temperature. Stirring was continued at room temperature for 5 minutes, LCMS indicated the reaction was complete. The reaction mixture was filtered, and the filtrate was concentrated. The residue was purified by silica gel chromatography to give compound **70b** (0.8 g, 43% yield) as yellow oil. ESI -MS (m/z): 332.3 [M+H]⁺.

Step 3: Compound **70b** (350 mg, 1.06 mmol) was dissolved in dioxane (10 mL), and then compound **17e** (1M in dioxane, 1.11 mL, 1.11 mmol) was added. The reaction mixture was stirred at room temperature for 10 minutes, LCMS indicated the starting material was consumed. EDCI (1.01 g, 5.28 mmol) was added, and the mixture was heated at 80 °C for 4 hours, LCMS indicated the product was formed. The reaction mixture was concentrated, and the residue was purified by silica gel chromatography to give compound **70c** (450 mg, 88% yield) as yellow solid. ESI- MS (m/z): 493.4 [M+H]⁺.

Step 4: To a solution of compound **70c** (250 mg, 0.51 mmol) in DMF (4 mL) was added copper(I) iodide (96 mg, 0.51 mmol) and Cs₂CO₃ (496 mg, 1.52 mmol) at room temperature under nitrogen atmosphere. The reaction mixture was heated at 130 °C by microwave for 4 hours. TLC indicated the starting material was consumed. The reaction mixture was purified directly by silica gel chromatography to give compound **70d** (65 mg, 35% yield) as yellow solid. ESI-MS (m/z): 365.1 [M+H]⁺.

Step 5: To a stirring solution of compound **70d** (65 mg, 0.17 mmol) and NaOH (35 mg, 0.89 mmol) in DMSO (2 mL) at 60 °C was added hydrogen peroxide (30 wt. %, 1 mL) dropwise. Stirring was continued at 60 °C for 1 hour, LCMS indicated the reaction was complete. The reaction mixture was purified directly by reversed phase preparative HPLC to give compound **70** (2 mg, 3% yield) as white solid. ESI-MS (m/z): 383.5 [M+H]⁺; ¹H NMR (500 MHz, DMSO-*d6*) δ 8.29 (s, 1H), 7.90 (s, 1H), 7.65 (d, *J=* 1.5 Hz, 1H), 7.32 (d, J=1.5 Hz, 1H), 7.28 (s, 1H), 6.69 (s, 1H), 4.61 (q, *J* = 7.0 Hz, 2H), 4.35-4.29 (m, 1H), 4.28-4.22 (m, 2H), 3.93-3.86 (m, 1H), 2.56-2.50 (m, 1H), 2.18 (s, 3H), 1.35 (t, *J* = 7.1 Hz, 3H), 1.09 (d, *J* = 7.1 Hz, 3H).

### Example 71

### (S)-1-(1-Ethyl-3-methyl-1H-pyrazole-5-carboxamido)-8-methyl-8,9-dihydro-7H-6-oxa-2,9a-diazabenzo[cd]azulene-4-carboxamide

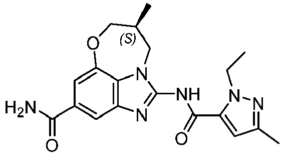

### Synthetic scheme:

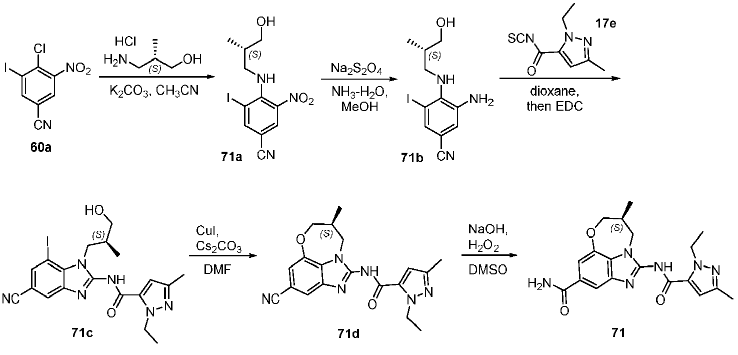

Step **1**: To a stirring solution of compound **60a** (4.0 g, 12.97 mmol) and (S)-3-amino-2-methylpropan-1-ol hydrochloride (3.0 g, 23.88 mmol) in acetonitrile (50 mL) was added K₂CO₃ (7.17 g, 51.87 mmol). The reaction mixture was heated at 60 °C for 16 hours, and TLC indicated the starting material was consumed. The reaction mixture was cooled to room temperature, filtered through a pad of silica gel. The filtrate was concentrated, and the residue was purified by silica gel chromatography to give compound **71a** (4.0 g, 85% yield) as yellow oil. ESI-MS (m/z): 362.1 [M+H]⁺.

Step 2: Compound **70a** (2.0 g, 5.54 mmol) was dissolved in a mixture of MeOH (20 mL) and concentrated ammonium hydroxide (4 mL). And sodium dithionite (5.79 g, 33.23 mmol) was dissolved in water (10 mL), the resultant solution was added to the reaction mixture at room temperature. Stirring was continued at room temperature for 5 minutes, LCMS indicated the reaction was complete. The reaction mixture was filtered, and the filtrate was concentrated. The residue was purified by silica gel chromatography to give compound **71b** (1.4 g, 76% yield) as yellow oil. ESI -MS (m/z): 332.3 [M+H]⁺.

Step 3: Compound **71b** (500 mg, 1.51 mmol) was dissolved in dioxane (2 mL), and then compound **17e** (1M in dioxane, 1.59 mL, 1.59 mmol) was added. The reaction mixture was stirred at room temperature for 10 minutes, LCMS indicated the starting material was consumed. EDCI (1.45 g, 7.55 mmol) was added, and the mixture was heated at 80 °C for 4 hours, LCMS indicated the product was formed. The reaction mixture was concentrated, and the residue was purified by silica gel chromatography to give compound **71c** (460 mg, 61% yield) as white solid. ESI- MS (m/z): 493.4 [M+H]⁺.

Step **4**: To a solution of compound **71c** (500 mg, 1.02 mmol) in DMF (4 mL) was added copper(I) iodide (290 mg, 1.52 mmol) and Cs₂CO₃ (661 mg, 2.03 mmol) at room temperature under nitrogen atmosphere. The reaction mixture was heated at 130 °C by microwave for 2 hours. TLC indicated the starting material was consumed. The reaction mixture was purified directly by silica gel chromatography to give compound **71d** (300 mg, 81% yield) as yellow solid. ESI-MS (m/z): 365.1 [M+H]⁺.

Step 5: To a stirring solution of compound **71d** (300 mg, 0.41 mmol) and NaOH (82 mg, 2.06 mmol) in DMSO (4 mL) at 60 °C was added hydrogen peroxide (30 wt. %, 2 mL) dropwise. Stirring was continued at 60 °C for 1 hour, LCMS indicated the reaction was complete. The reaction mixture was purified directly by reversed phase preparative HPLC to give compound **71** (57 mg, 36% yield) as white solid. ESI-MS (m/z): 383.4 [M+H]⁺; ¹H NMR (500 MHz, DMSO-*d*₆) δ 12.74 (br s, 1H), 8.32 (s, 1H), 7.91 (s, 1H), 7.66 (s, 1H), 7.33 (s, 1H), 7.29 (s, 1H), 6.69 (s, 1H), 4.61 (q, *J* = 7.1 Hz, 2H), 4.35-4.20 (m, 3H), 3.94-3.85 (m, 1H), 2.62-2.52 (m, 1H), 2.18 (s, 3H), 1.35 (t, *J* = 7.1 Hz, 3H), 1.09 (d, *J* = 7.1 Hz, 3H).

### Example 72

### 1-(1-Ethyl-4-fluoro-3-methyl-1H-pyrazole-5-carboxamido)-7H,9H-6-oxa-2,9a-diazaspiro[be nzo[cd]azulene-8,3'-oxetan]-1,2a,2a¹(5a),4-tetraene-4-carboxamide

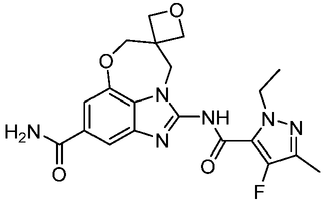

### Synthetic scheme:

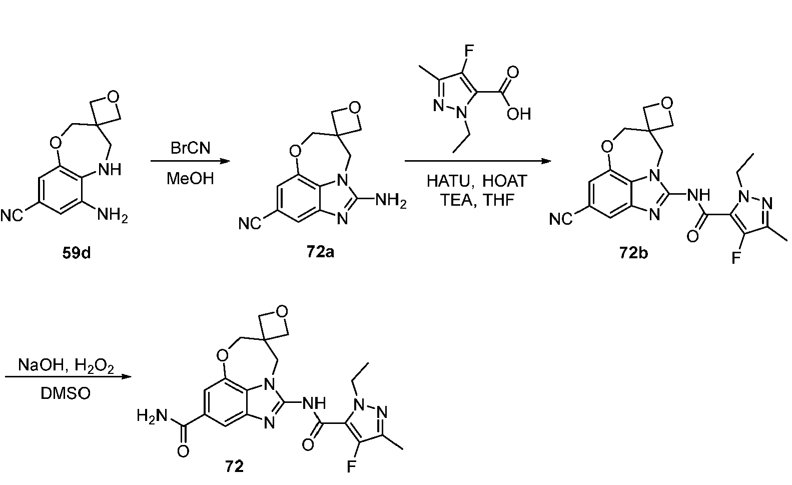

Step 1: Compound 59d (180 mg, 0.77 mmol) was dissolved in MeOH (10 mL), and cyanogen bromide (256 mg, 2.34 mmol) was added. The resulting mixture was stirred at room temperature overnight, LCMS indicated the product was formed. The mixture was concentrated in vacuo to remove the solvent. The residue was purified by silica gel chromatography to give compound **72a** (60 mg, 30% yield) as pink solid. ESI-MS (m/z): 257.2 [M+H]⁺.

Step 2: To a stirring solution of compound **72a** (30 mg, 0.11 mmol) and 1-ethyl-4-fluoro-3-methyl-1H-pyrazole-5-carboxylic acid (22 mg, 0.12 mmol) in THF (10 mL) was added HATU (53 mg, 0.14 mmol), HOAt (19 mg, 0.14 mmol) and triethylamine (0.05 mL, 0.35 mmol). The mixture was stirred at room temperature overnight. LCMS indicated the product was formed. The mixture was concentrated to give crude compound **72b** (40 mg), which was used directly without further purification. ESI-MS (m/z): 411.5 [M+H]⁺.

Step 3: To a stirring solution of compound **72b** (40 mg, from step 2) and NaOH (11 mg, 0.29 mmol) in DMSO (3 mL) at 60 °C was added hydrogen peroxide (30 wt. %, 0.5 mL) dropwise. Stirring was continued at 60 °C for 5 mimutes, LCMS indicated the reaction was complete. The reaction mixture was purified directly by reversed phase preparative HPLC to give compound **72** (4 mg, 8% yield for 2 steps) as white solid. ESI-MS (m/z): 429.4 [M+H]⁺; ¹H NMR (500 MHz, DMSO-*d6*) δ 12.95 (br s, 1H), 7.92 (s, 1H), 7.66 (s, 1H), 7.40-7.25 (m, 2H), 4.70-4.52 (m, 6H), 4.50 (s,2H), 4.40-4.30 (m, 2H), 2.17 (s, 3H), 1.37 (t, *J* = 7.0 Hz, 3H).

### Example 73

### 1-(1-ethyl-3-methyl-1H-pyrazole-5-carboxamido)-6-(2-methoxyethyl)-8,8-dimethyl-6,7,8,9-t etrahydro-2,6,9a-triazabenzo[cd]azulene-4-carboxamide

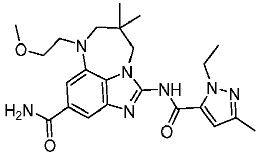

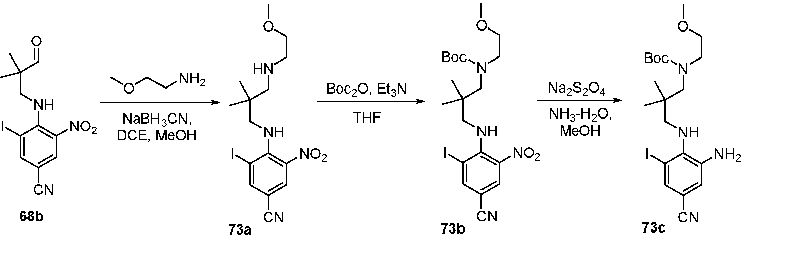

### Synthetic scheme:

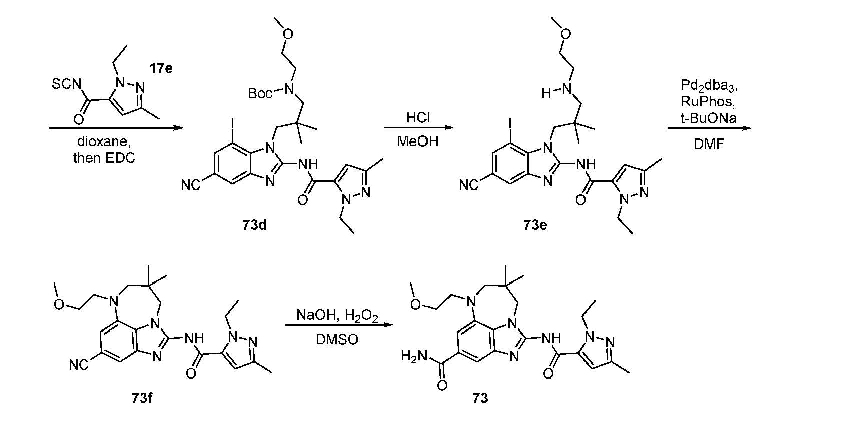

Step 1: To a solution of compound **68b** (500 mg, 1.34 mmol) in MeOH (3 mL) and DCE (15 mL) was added 2-methoxyethanamine (503 mg, 6.70 mmol). The mixture was stirred at room temperature overnight, then sodium cyanoborohydride (252 mg, 4.02 mmol) was added. The resultant mixture was stirred at room temperature for 2 days. LCMS indicated the product was formed. The reaction mixture was concentrated, and the residue was purified by silica gel colomn chromatography to give compound **73a** (400 mg, 69% yield) as yellow oil. ESI-MS (m/z): 433.2 [M+H]⁺.

Step 2: To a solution of compound **73a** (400 mg, 0.92 mmol) in THF (10 mL) was added Boc₂O (262 mg, 1.20 mmol) and triethylamine (0.2 mL, 1.39 mmol). The mixture was stirred at room temperature overnight. LCMS indicated the reaction was complete. The reaction mixture was concentrated to give crude compound **73b** (450 mg) as yellow oil, which was used directly without further purification. ESI-MS (m/z): 533.3 [M+H]⁺.

Step 3: Compound **73b** (450 mg, from step 2) was dissolved in a mixture of MeOH (5 mL) and concentrated ammonium hydroxide (1 mL). And sodium dithionite (727 mg, 4.18 mmol) was dissolved in water (3 mL), the resultant solution was added to the reaction mixture at room temperature. Stirring was continued at room temperature for 5 minutes, LCMS indicated the reaction was complete. The reaction mixture was filtered, and the filtrate was concentrated. The residue was purified by silica gel chromatography to give compound **73c** (390 mg, 84% yield) as red oil. ESI -MS (m/z): 503.4 [M+H]⁺.

Step 4: Compound **73c** (390 mg, 0.77 mmol) was dissolved in dioxane (10 mL), and then compound **17e** (1M in dioxane, 0.85 mL, 0.85 mmol) was added. The reaction mixture was stirred at room temperature for 10 minutes, LCMS indicated the starting material was consumed. EDCI (193 mg, 1.01 mmol) was added, and the mixture was heated at 80 °C for 2 hours, LCMS indicated the product was formed. The reaction mixture was concentrated, and the residue was purified by silica gel chromatography to give compound **73d** (300 mg, 58% yield) as red oil. ESI-MS (m/z): 664.6 [M+H]⁺.

Step 5: To a stirring solution of **73d** (300 mg, 0.45 mmol) in MeOH (10 mL) was added 4M HCl in dioxane (5 mL). The mixture was stirred at room temperature for 3 hours, LCMS indicated the product was formed. The reaction mixture was concentrated to give crude compound **73e** (260 mg) as pink solid. ESI-MS (m/z): 564.6 [M+H]⁺.

Step 6: To a solution of compound **73e** (260 mg, from step 5) in DMF (10 mL) was added Pd₂(dba)₃ (42 mg, 0.046 mmol), Ruphos (43 mg, 0.092 mmol) and sodium tert-butoxide (177 mg, 1.85 mmol) at room temperature under nitrogen atmosphere. The reaction mixture was heated at 110 °C by microwave for 2.5 hours. LCMS indicated the starting material was consumed. The reaction mixture was poured into water (40 mL), and extracted with EtOAc (15 mL × 3). The combined organic layers were washed with brine, dried over Na₂SO₄, filtered and concentrated to give crude compound **73f** (160 mg) as brown oil, which was used directly without further purification. ESI-MS (m/z): 435.7 [M+H]⁺.

Step 7: To a stirring solution of compound **73f** (160 mg, from step 6) and NaOH (44 mg, 1.10 mmol) in DMSO (5 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound **73** (32 mg, 15% yield) as white solid. ESI-MS (m/z): 454.6 [M+H]⁺; ¹H NMR (500 MHz, DMSO-*d₆*) δ 12.66 (br s, 1H), 8.36 (s, 1H), 7.90 (s, 1H), 7.41 (s, 1H), 7.23 (s, 1H), 7.01 (s, 1H), 6.64 (s, 1H), 4.61 (q, *J* = 7.0 Hz, 2H), 3.92 (s, 2H), 3.66-3.60 (m, 4H), 3.30-3.25 (m, 2H), 2.17 (s, 3H), 1.35 (t, *J* = 7.0 Hz,, 3H), 1.05 (s, 6H).

### Example 74

### 1-(1-Ethyl-3-methyl-1H-pyrazole-5-carboxamido)-6-(3-methoxypropyl)-8,8-dimethyl-6,7,8,9 -tetrahydro-2,6,9a-triazabenzo[cd]azulene-4-carboxamide

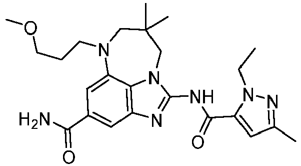

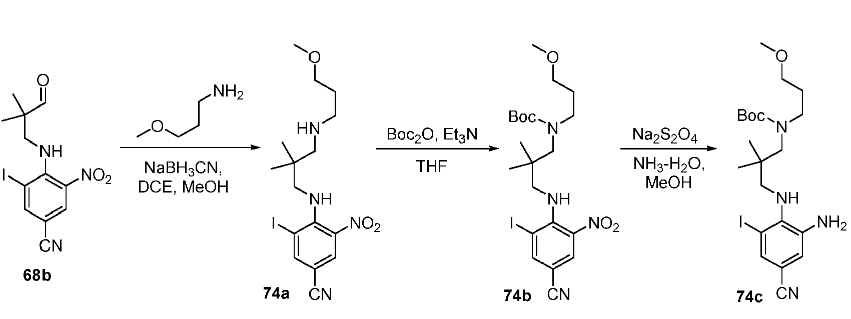

### Synthetic scheme:

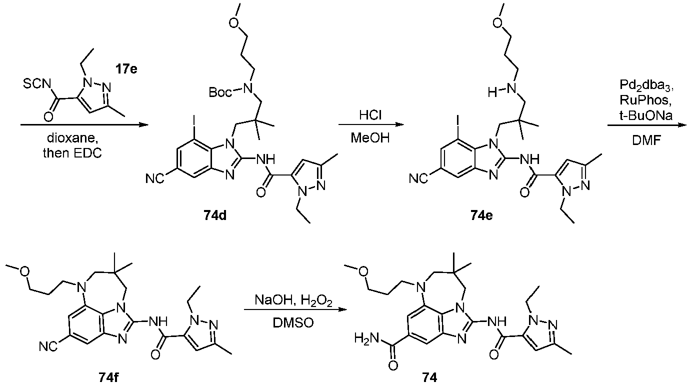

Step 1: To a solution of compound **68b** (500 mg, 1.34 mmol) in MeOH (3 mL) and DCE (15 mL) was added 3-methoxypropan-1-amine (597 mg, 6.70 mmol). The mixture was stirred at room temperature overnight, then sodium cyanoborohydride (421 m, 6.7 mmol) was added. The resultant mixture was stirred at room temperature for 2 days. LCMS indicated the product was formed. The reaction mixture was concentrated, and the residue was purified by silica gel colomn chromatography to give compound **74a** (280 mg, 46% yield) as yellow oil. ESI-MS (m/z): 447.3 [M+H]⁺.

Step 2: To a solution of compound **74a** (280 mg, 0.62 mmol) in THF (10 mL) was added Boc₂O (178 mg, 0.81 mmol) and triethylamine (0.13 mL, 0.94 mmol). The mixture was stirred at room temperature overnight. LCMS indicated the reaction was complete. The reaction mixture was concentrated to give crude compound **74b** (330 mg) as yellow oil, which was used directly without further purification. ESI-MS (m/z): 547.3 [M+H]⁺.

Step 3: Compound **74b** (330 mg, from step 2) was dissolved in a mixture of MeOH (5 mL) and concentrated ammonium hydroxide (1 mL). And sodium dithionite (525 mg, 3.02 mmo) was dissolved in water (3 mL), the resultant solution was added to the reaction mixture at room temperature. Stirring was continued at room temperature for 5 minutes, LCMS indicated the reaction was complete. The reaction mixture was filtered, and the filtrate was concentrated. The residue was purified by silica gel chromatography to give compound **74c** (290 mg, 89% yield for 2 steps) as red oil. ESI -MS (m/z): 517.3 [M+H]⁺.

Step 4: Compound **74c** (290 mg, 0.53 mmol) was dissolved in dioxane (10 mL), and then compound **17e** (1M in dioxane, 0.58 mL, 0.58 mmol) was added. The reaction mixture was stirred at room temperature for 10 minutes, LCMS indicated the starting material was consumed. EDCI (132 mg, 0.68 mmol) was added, and the mixture was heated at 80 °C for 2 hours, LCMS indicated the product was formed. The reaction mixture was concentrated, and the residue was purified by silica gel chromatography to give compound **74d** (300 mg, 83% yield) as red solid. ESI- MS (m/z): 678.3 [M+H]⁺.

Step 5: To a stirring solution of **74d** (300 mg, 0.44 mmol) in MeOH (10 mL) was added 4M HCl in dioxane (5 mL). The mixture was stirred at room temperature for 3 hours, LCMS indicated the product was formed. The reaction mixture was concentrated to give crude compound **74e** (250 mg) as pink solid. ESI-MS (m/z): 578.4 [M+H]⁺.

Step 6: To a solution of compound **74e** (250 mg, from step 5) in DMF (8 mL) was added Pd₂(dba)₃ (39 mg, 0.043 mmol), Ruphos (40 mg, 0.086 mmol) and sodium tert-butoxide (166 mg, 1.73 mmol) at room temperature under nitrogen atmosphere. The reaction mixture was heated at 110 °C by microwave for 2.5 hours. LCMS indicated the starting material was consumed. The reaction mixture was poured into water (30 mL), and extracted with EtOAc (15 mL × 3). The combined organic layers were washed with brine, dried over Na₂SO₄, filtered and concentrated to give crude compound **74f** (130 mg) as brown oil, which was used directly without further purification. ESI-MS (m/z): 449.4 [M+H]⁺.

Step 7: To a stirring solution of compound **74f** (130 mg, from step 6) and NaOH (34 mg, 0.86 mmol) in DMSO (5 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound **74** (4.9 mg, 2.3% yield for 3 steps). ESI-MS (m/z): 468.2 [M+H] ⁺; ¹H NMR (500 MHz, DMSO-*d*₆) δ 12.68 (s, 1H), 8.36 (s, 1H), 7.85 (s, 1H), 7.39 (s, 1H), 7.23 (s, 1H), 7.00 (s, 1H), 6.64 (s, 1H), 4.61 (q, *J* = 7.0 Hz, 2H), 3.94 (s, 2H), 3.50-3.45 (m, 4H), 3.27 (s, 3H), 3.21 (s, 2H), 2.17 (s, 3H), 1.90-1.82 (m, 2H), 1.35 (t, *J* = 7.0 Hz, 3H), 1.06 (s, 6H).

### Example 75

### 6-(2-(benzyloxy)ethyl)-1-(1-ethyl-3-methyl-1H-pyrazole-5-carboxamido)-8,8-dimethyl-6,7,8, 9-tetrahydro-2,6,9a-triazabenzo[cd]azulene-4-carboxamide

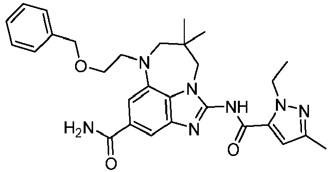

### Synthetic scheme:

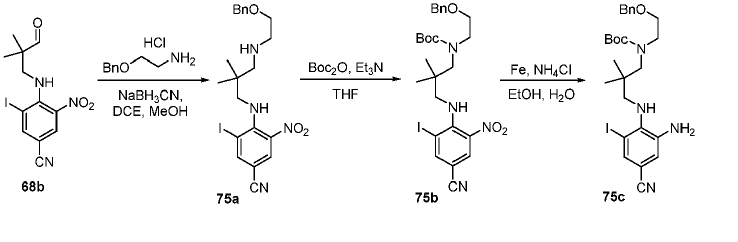

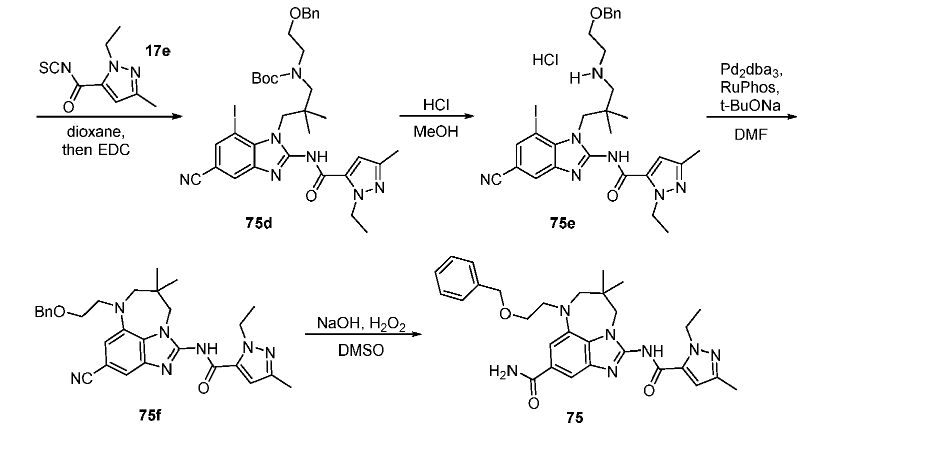

Step 1: To a solution of compound **68b** (500 mg, 1.34 mmol) in MeOH (3 mL) and DCE (15 mL) was added 2-(benzyloxy)ethanamine hydrochloride (754 mg, 4.02 mmol). The mixture was stirred at room temperature overnight, then sodium cyanoborohydride (294 mg, 4.69 mmol) was added. The resultant mixture was stirred at room temperature overnight. LCMS indicated the product was formed. The reaction mixture was concentrated, and the residue was purified by silica gel colomn chromatography to give compound **75a** (340 mg, 49% yield) as yellow oil. ESI-MS (m/z): 509.4 [M+H]⁺ .

Step 2: To a solution of compound **75a** (340 mg, 0.66 mmol) in THF (10 mL) was added Boc₂O (175 mg, 0.80 mmol) and triethylamine (0.13 mL, 1.0 mmol). The mixture was stirred at room temperature overnight. LCMS indicated the reaction was complete. The reaction mixture was concentrated to give crude compound **75b** (400 mg) as yellow oil, which was used directly without further purification. ESI-MS (m/z): 609.3 [M+H]⁺.

Step 3: To a solution of compound **75b** (400 mg, from step 2) in ethanol (10 mL) and water (2 mL) was added NH₄Cl (116 mg, 2.17 mmol). The mixture was heated at 50 °C, then Fe powder (121 mg, 2.17 mmol) by portions. The resultant mixture was stirred at 70 °C for 2 hours, LCMS indicated the reaction was complete. The reaction mixture was cooled to room temperature, filtered through a pad of celite. The filtrate was concentrated to give crude compound **75c** (300 mg) as red oil, which was used directly without further purification. ESI-MS (m/z): 579.5 [M+H]⁺.

Step 4: Compound **75c** (300 mg, from step 3) was dissolved in dioxane (10 mL), and then compound **17e** (1M in dioxane, 0.57 mL, 0.57 mmol) was added. The reaction mixture was stirred at room temperature for 10 minutes, LCMS indicated the starting material was consumed. EDCI (129 mg, 0.67 mmol) was added, and the mixture was heated at 80 °C for 2 hours, LCMS indicated the product was formed. The reaction mixture was concentrated, and the residue was purified by silica gel chromatography to give compound **75d** (250 mg, 50% yield for 3 steps) as pink solid. ESI- MS (m/z): 740.2 [M+H]⁺.

Step 5: To a stirring solution of **75d** (250 mg, 0.33 mmol) in MeOH (10 mL) was added 4M HCl in dioxane (5 mL). The mixture was stirred at room temperature for 3 hours, LCMS indicated the product was formed. The reaction mixture was concentrated to give crude compound **75e** (220 mg) as pink solid. ESI-MS (m/z): 640.3 [M+H]⁺.

Step 6: To a solution of compound **75e** (200 mg, from step 5) in DMF (5 mL) was added Pd₂(dba)₃ (27 mg, 0.029 mmol), Ruphos (27 mg, 0.059 mmol) and sodium tert-butoxide (85 mg, 0.88 mmol) at room temperature under nitrogen atmosphere. The reaction mixture was heated at 110 °C by microwave for 2.5 hours. LCMS indicated the starting material was consumed. The reaction mixture was poured into water (30 mL), and extracted with EtOAc (15 mL × 3). The combined organic layers were washed with brine, dried over Na₂SO₄, filtered and concentrated to give crude compound **75f** (100 mg) as brown oil, which was used directly without further purification. ESI-MS (m/z): 512.6 [M+H]⁺.

Step 7: To a stirring solution of compound **75f** (100 mg, from step 6) and NaOH (23 mg, 0.58 mmol) in DMSO (5 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound **75** (18 mg, 11% yield for 3 steps) as white solid. ESI-MS (m/z): 529.9 [M+H] ⁺; ¹H NMR (500 MHz, DMSO-*d*₆) δ 12.68 (br s, 1H), 7.89 (s, 1H), 7.40 (d, *J* = 1.5 Hz, 1H), 7.36-7.31 (m, 4H), 7.29-7.22 (m, 2H), 7.05 (s, 1H), 6.64 (s, 1H), 4.60 (q, *J* = 7.0 Hz, 2H), 4.54 (s, 2H), 3.92 (s, 2H), 3.79-3.64 (m, 4H), 2.17 (s, 3H), 1.35 (t, *J* = 7.0 Hz, 3H), 1.04 (s, 6H).

### Example 76

### 1-(1-ethyl-3-methyl-1H-pyrazole-5-carboxamido)-6-(2-hydroxyethyl)-8,8-dimethyl-6,7,8,9-te trahydro-2,6,9a-triazabenzo[cd]azulene-4-carboxamide

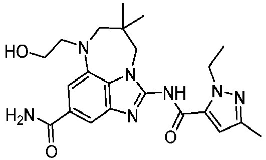

### Synthetic scheme:

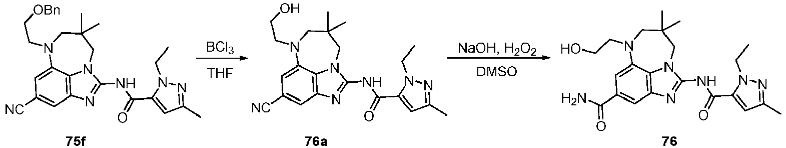

Step 1: To a solution of compound **75f** (200 mg, 0.39 mmol) in THF (10 mL) at 0 °C was added dropwise boron trichloride (1M in DCM, 3.9 mL, 3.9 mmol). The resultant mixture was stirred at room temperature for 2 hours, LCMS indicated the starting material was consumed. The reaction mixture was quenched with MeOH, then concentrated. The residue was purified by silica gel chromatography to give compound **76a** (150 mg, 91% yield) as yellow solid. ESI-MS (m/z): 422.4 [M+H]⁺.

Step 2: To a stirring solution of compound **76a** (150 mg, 0.35 mmol) and NaOH (42 mg, 1.06 mmol) in DMSO (5 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound **76** (67 mg, 43% yield) as white solid. ESI-MS (m/z): 440.4 [M+H] ⁺; ¹H NMR (500 MHz, DMSO-*d*₆) δ 12.67 (br s, 1H), 7.84 (s, 1H), 7.37 (d, *J* = 1.5 Hz, 1H), 7.23 (s, 1H), 7.00 (d, *J* = 1.5 Hz, 1H), 6.64 (s, 1H), 4.72 (br s, 1H), 4.61 (q, *J* = 7.0 Hz, 2H), 3.94 (s, 2H), 3.67 (t, *J* = 6.5 Hz, 2H), 3.50 (t, *J* = 6.5 Hz, 2H), 3.30 (s, 2H), 2.17 (s, 3H), 1.35 (t, *J* = 7.0Hz,, 3H), 1.06 (s, 6H).

### Example 77

### 1-(1-ethyl-3-methyl-1H-pyrazole-5-carboxamido)-7H,9H-6-oxa-2,9a-diazaspiro[benzo[cd]az ulene-8,1'-cyclobutan]-1,2a,2al(5a),4-tetraene-4-carboxamide

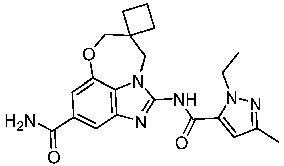

### Synthetic scheme:

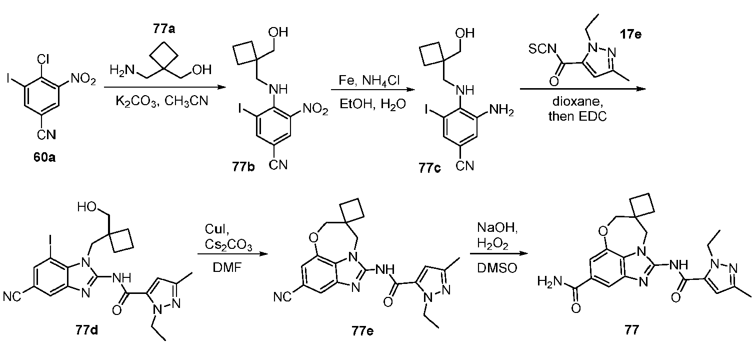

Step **1**: To a stirring solution of compound **60a** (1.3 g, 4.21 mmol) and (1-(aminomethyl)cyclobutyl)methanol (582 mg, 5.06 mmol) in acetonitrile (20 mL) was added K₂CO₃ (1.16 g, 8.43 mmol). The reaction mixture was heated at 70 °C for 4 hours, and TLC indicated the starting material was consumed. The reaction mixture was cooled to room temperature, filtered through a pad of silica gel. The filtrate was concentrated, and the residue was purified by silica gel chromatography to give compound **77b** (1.3 g, 79% yield) as yellow solid. ESI-MS (m/z): 388.2 [M+H] ⁺; ¹H NMR (500 MHz, DMSO-*d*₆) δ 8.37 (d, *J* = 2.0 Hz, 1H), 8.32 (d, *J* = 2.0 Hz, 1H), 6.73 (t, *J* = 4.5Hz, 1H), 5.40-5.23 (m, 1H), 3.56 (s, 2H), 2.99 (d, *J=* 4.5 Hz, 2H), 1.90-1.72 (m, 4H), 1.68-1.55 (m, 2H).

Step 2: To a solution of compound **77b** (700 mg, 1.8 mmol) in ethanol (20 mL) and water (4 mL) was added NH₄Cl (483 mg, 9.04 mmol). The mixture was heated at 50 °C, then Fe powder (504 mg, 9.04 mmol) by portions. The resultant mixture was stirred at 70 °C for 2 hours, LCMS indicated the reaction was complete. The reaction mixture was cooled to room temperature, filtered through a pad of celite. The filtrate was concentrated to give crude compound **77c** (480 mg, 74% yield) as pink solid. ESI-MS (m/z): 358.3 [M+H]⁺.

Step 3: Compound **77c** (350 mg, 0.97 mmol) was dissolved in dioxane (15 mL), and then compound **17e** (1M in dioxane, 1.1 mL, 1.1 mmol) was added. The reaction mixture was stirred at room temperature for 10 minutes, LCMS indicated the starting material was consumed. EDCI (244 mg, 1.27 mmol) was added, and the mixture was heated at 80 °C for 2 hours, LCMS indicated the product was formed. The reaction mixture was concentrated, and the residue was purified by silica gel chromatography to give compound **77d** (250 mg, 49% yield) as pink solid. ESI-MS (m/z): 519.3 [M+H]⁺.

Step 4: To a solution of compound **77d** (200 mg, 0.38 mmol) in DMF (4 mL) was added copper(I) iodide (44 mg, 0.23 mmol) and Cs₂CO₃ (251 mg, 0.77 mmol) at room temperature under nitrogen atmosphere. The reaction mixture was heated at 130 °C by microwave for 1.5 hours. LCMS indicated the starting material was consumed. The reaction mixture was poured into water (30 mL), and sodium sulfide (60 mg, 0.77 mmol) was added. The resultant mixture was stirred at room temperature for 30 minutes, then extracted with EtOAc (15 mL × 3). The combined organic layers were washed with brine, dried over Na₂SO₄, filtered and concentrated to give crude compound **77e** (100 mg) as brown oil, which was used directly without further purification. ESI-MS (m/z): 391.3 [M+H]⁺.

Step 5: To a stirring solution of compound **77e** (100mg, from step 4) and NaOH (30 mg, 0.76 mmol) in DMSO (3 mL) at 60 °C was added hydrogen peroxide (30 wt. %, 1 mL) dropwise. Stirring was continued at 60 °C for 5 minutes, LCMS indicated the reaction was complete. The reaction mixture was purified directly by reversed phase preparative HPLC to give compound **77** (15 mg, 9% yield for 2 steps) as white solid. ESI-MS (m/z): 409.4 [M+H] ⁺; ¹H NMR (500 MHz, DMSO-*d*₆) δ 12.78 (br s, 1H), 7.89 (s, 1H), 7.63 (d, *J* = 1.5 Hz, 1H), 7.31 (d, *J* = 1.5 Hz, 1H), 7.27 (s, 1H), 6.73 (s, 1H), 4.63 (q, J = 7.0 Hz, 2H), 4.38 (s, 2H), 4.19 (s, 2H), 2.19 (s, 3H), 2.11-1.98 (m, 4H), 1.88-1.80 (m, 2H), 1.37 (t, *J* = 7.0 Hz, 3H).

### Example 78

### 1-(1-Ethyl-4-fluoro-3-methyl-1H-pyrazole-5-carboxamido)-7H,9H-6-oxa-2,9a-diazaspiro[be nzo[cd]azulene-8,1'-cyclobutan]-1,2a,2a1(5a),4-tetraene-4-carboxamide

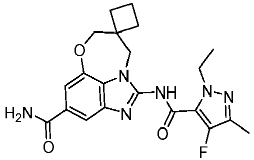

### Synthetic scheme:

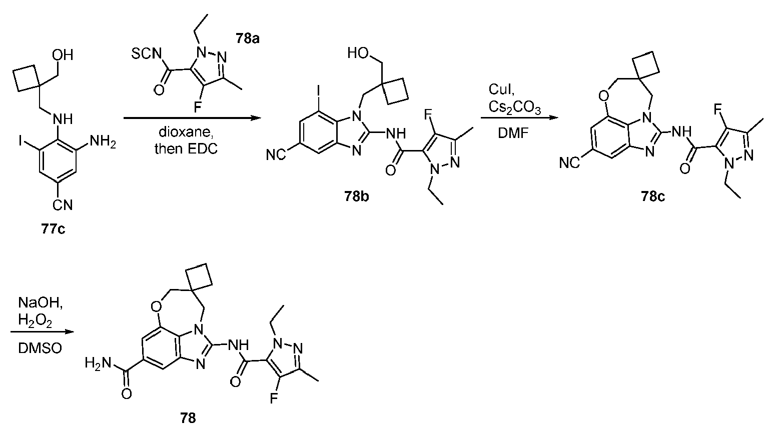

Step 1: Compound **77c** (130 mg, 0.36 mmol) was dissolved in dioxane (10 mL), and then compound **17e** (1M in dioxane, 0.4 mL, 0.4 mmol) was added. The reaction mixture was stirred at room temperature for 10 minutes, LCMS indicated the starting material was consumed. EDCI (90 mg, 0.47 mmol) was added, and the mixture was heated at 80 °C for 2 hours, LCMS indicated the product was formed. The reaction mixture was concentrated, and the residue was purified by silica gel chromatography to give compound **78b** (150 mg, 76% yield) as pink solid. ESI- MS (m/z): 537.3 [M+H]⁺.

Step 2: To a solution of compound **78b** (150 mg, 0.28 mmol) in DMF (4 mL) was added copper(I) iodide (31 mg, 0.16 mmol) and Cs₂CO₃ (182 mg, 0.55 mmol) at room temperature under nitrogen atmosphere. The reaction mixture was heated at 130 °C by microwave for 1.5 hours. LCMS indicated the starting material was consumed. The reaction mixture was poured into water (20 mL), and sodium sulfide (43 mg, 0.56 mmol) was added. The resultant mixture was stirred at room temperature for 30 minutes, then extracted with EtOAc (15 mL × 3). The combined organic layers were washed with brine, dried over Na₂SO₄, filtered and concentrated to give crude compound **78c** (82 mg) as brown oil, which was used directly without further purification. ESI-MS (m/z): 409.4 [M+H]⁺.

Step 3: To a stirring solution of compound **78c** (82 mg, from step 2) and NaOH (24 mg, 0.60 mmol) in DMSO (3 mL) at 60 °C was added hydrogen peroxide (30 wt. %, 0.5 mL) dropwise. Stirring was continued at 60 °C for 5 minutes, LCMS indicated the reaction was complete. The reaction mixture was purified directly by reversed phase preparative HPLC to give compound **78** (16 mg, 13% yield for 2 steps) as white solid. ESI-MS (m/z): 427.4 [M+H] ⁺; ¹H NMR (500 MHz, DMSO-*d*₆) δ 12.90 (br s, 1H), 7.90 (s, 1H), 7.63 (d, *J* = 1.5 Hz, 1H), 7.32 (d, *J* = 1.5 Hz, 1H), 7.29 (s, 1H), 4.58 (q, *J* = 7.0 Hz, 2H), 4.39 (s, 2H), 4.20 (s, 2H), 2.17 (s, 3H), 2.08-1.96 (m, 4H), 1.84-1.75 (m, 2H), 1.36 (t, *J* = 7.0 Hz, 3H).

### Biology screening result of STING agonist compound

### Example I: STING variants (WT & HAQ) activation by compounds in HEK-Blue^{™} ISG KO-STING cells (Method 1)

Activation of STING can be determined using a SEAP reporter assay in **HEK-Blue^{™}** ISG KO-STING cells (Invivogen, cat# hkb-kostg) transfected with plasmids expressing STING variants (WT & HAQ) (referred to WO2017/175147A1). STING variants (WT & HAQ) vector were constructed based on STING-232H vector (Origene, RC208418). GFP vector (VT2069) was bought from Youbio. The detailed protocol as follows: **HEK-Blue^{™}** ISG KO-STING cells were harvest and seeding into 96 well plate, the final cell number was 0.8×10⁵ cells/well. Transfections were prepared using Lipofectamine 2000 (Invitrogen, Cat#11668-027) following the manufacturer's instructions (Lipo2000: DNA =1:10), 20ul of transfection suspension containing 1ng STING vector was added to cell culture plate and incubated at 37°C, 5% CO2 for 24h. After 24 hours incubation, compounds were added at proper concentration, and the final DMSO concentration was 0.5%. After 24h incubation, the supernatant was collected to detect SEAP signal using Great EscAPe SEAP chemiluminescence KIT (Clontech, cat# 631738) and cells were collected to detect cell viability using CellTiter-Glo Luminescent Cell Viability Assay (Promega, cat# G7573) following the manufacturer's instructions. The data were described by the ratio of the stimulating signal of compounds to 0.5% DMSO.

| Compounds | HEK-Blue^{™} ISG-KO-Sting/variants reporter assay | | |
|---|---|---|---|
| | Concentration (µM) | Fold change related to DMSO control | |
| | | WT | HAQ |
| 1 | 31.25 | 3.08 | 21.49 |
| 2 | 77.9 | 2.25 | 6.46 |
| 3 | 125 | 1.79 | 3.54 |
| 5 | 15.65 | 1.52 | 1.20 |
| 6 | 169.6 | 0.76 | 4.41 |
| 7 | 115.2 | 2.70 | 13.64 |
| 8 | 73.3 | 3.47 | 13.17 |
| 9 | 206.6 | 2.86 | 21.28 |
| 11 | 27.9 | 2.26 | 4.20 |
| 12 | 52.6 | 3.31 | 27.37 |
| 15 | 75.95 | 3.68 | 20.00 |
| 16 | 42.8 | 3.00 | 15.06 |
| 14 | 25 | 1.59 | 27.44 |
| 17 | 25 | 1.76 | 37.64 |
| 18 | 153.2 | 1.58 | 17.64 |
| 19 | 125.3 | 1.65 | 20.80 |
| 20 | 127.7 | 1.43 | 16.22 |
| 21 | 25 | 2.18 | 8.87 |
| 22 | 25 | 3.02 | 95.64 |
| 23 | 25 | 2.62 | 81.27 |
| 24 | 25 | 2.33 | 65.85 |
| 25 | 25 | 2.24 | 56.18 |
| 26 | 25 | 2.00 | 63.91 |
| 27 | 25 | 2.37 | 56.43 |
| 28 | 25 | 2.34 | 38.16 |
| 29 | 25 | 1.06 | 1.14 |
| 30 | 25 | 1.63 | 35.09 |
| 31 | 25 | 2.71 | 22.11 |
| 32 | 25 | 1.45 | 2.88 |
| 33 | 25 | 2.49 | 20.81 |
| 37 | 3.11 | 1.07 | 1.85 |
| 38 | 25 | 2.96 | 22.76 |
| 39 | 25 | 2.78 | 25.85 |
| 42 | 21.4 | 1.10 | 10.52 |
| 43 | 2.5 | 2.02 | - |
| | 5 | - | 23.88 |

| | | | |
|---|---|---|---|
| - Not detected | | | |

### Example II: STING variants (WT & HAQ) activation by compounds in HEK-Blue^{™} ISG KO-STING cells (Method 2)

Activation of STING can be determined using a SEAP reporter assay in HEK-Blue^{™} ISG KO-STING cells (Invivogen, cat#hkb-kostg) transfected with plasmids expressing STING variants (WT & HAQ) (referred to WO2017/175147A1). STING variants (WT & HAQ) vector were constructed based on STING-232H vector (Origene, RC208418). GFP vector (VT2069) was bought from Youbio. The detailed protocol as follows: HEK-Blue^{™} ISG KO-STING cells were harvest and seeding into 96 well plate and the final cell number was 0.8x105 cells/well. Transfections were prepared using Lipofectamine 2000 (Invitrogen, Cat# 11668-027) following the manufacturer's instructions (Lipo2000: DNA =1:10), 20ul of transfection suspension containing 1ng (WT variant) or 0.0625ng (HAQ variant) STING vector was added to cell culture plate and incubated at 37°C, 5% CO2 for 24h. After 24 hours incubation, compounds were added at proper concentration, the final DMSO concentration was 0.5%. After 24h incubation, the supernatant was collected to detect SEAP signal using Great EscAPe SEAP chemiluminescence KIT (Clontech,cat# 631738) and cells were collected to detect cell viability using CellTiter-Glo Luminescent Cell Viability Assay(Promega,cat# G7573) following the manufacturer's instructions. The data were described by the ratio of the stimulating signal of compounds to 0.5% DMSO.

| - Compounds | HEK-Blue^{™} ISG-KO-Sting/variants reporter assay | | |
|---|---|---|---|
| | Concentration (mM) | Fold change related to DMSO control | |
| | | WT | HAQ |
| 44 | 2.5 | 2.48 | - |
| | 5 | - | 15.38 |
| 45 | 2.5 | 1.18 | - |
| | 5 | - | 1.59 |
| 46 | 2.5 | 1.41 | - |
| | 5 | - | 2.12 |
| 47 | 2.5 | 6.91 | - |
| | 5 | - | 27.64 |
| 48 | 2.5 | 1.85 | - |
| | 5 | - | 3.46 |
| 49 | 2.5 | 1.49 | - |
| | 5 | - | 1.94 |
| 50 | 2.5 | 0.83 | - |
| | 5 | - | 1.27 |
| 51 | 2.5 | 0.79 | - |
| | 5 | - | 0.63 |
| 52 | 2.5 | 0.82 | - |
| | 5 | - | 0.82 |
| 53 | 2.5 | 1.53 | - |
| | 5 | - | 2.42 |
| 54 | 2.5 | 2.29 | - |
| | 5 | - | 7.79 |
| 55 | 2.5 | 1.38 | - |
| | 5 | - | 2.96 |
| 56 | 2.5 | 1.16 | - |
| | 5 | - | 1.17 |
| 57 | 2.5 | 1.34 | - |
| | 5 | - | 3.59 |
| 58 | 2.5 | 1.06 | - |
| | 5 | - | 0.95 |
| 59 | 2.5 | 1.22 | - |
| | 5 | - | 1.13 |
| 60 | 2.5 | 6.55 | - |
| | 5 | - | 73.46 |
| 61 | 2.5 | 1.90 | - |
| | 5 | - | 8.67 |
| 62 | 2.5 | 1.84 | - |
| | 5 | - | 13.40 |
| 63 | 2.5 | 2.10 | - |
| | 5 | - | 15.19 |
| 64 | 2.5 | 1.53 | - |
| | 5 | - | 5.84 |
| 65 | 2.5 | 1.63 | - |
| | 5 | - | 8.89 |
| 66 | 2.5 | 7.15 | - |
| | 5 | - | 71.56 |
| 67 | 2.5 | 7.90 | - |
| | 5 | - | 49.72 |
| 68 | 2.5 | 9.81 | - |
| | 5 | - | 67.23 |
| 69 | 2.5 | 7.69 | - |
| | 5 | - | 74.83 |
| 70 | 2.5 | 2.37 | - |
| | 5 | - | 10.57 |
| 71 | 2.5 | 2.97 | - |
| | 5 | - | 18.10 |
| 72 | 2.5 | 1.24 | - |
| | 5 | - | 3.27 |

| | | | |
|---|---|---|---|
| - Not detected | | | |

### Example III: Compound stimulate THP1 to release IFNβ by STING activation

In this assay, activation of STING by compounds was evaluated by detecting their ability to stimulate the secretion of IFN-β (interferon-beta) from THP1. THP1 was purchased from National Collection of Authenticated Cell Cultures (Cat# TCHu 57). The top dose of compounds was setted according to their solubility. First, Compounds were 3 times diluted with medium, 8-dose points in total. The final DMSO concentration was 0.2%. THP1 during the logarithmic phase was resuspended to 2×10⁶ cells/ml in assay medium. The THP1-cell suspension was dispensed into a 96-well U bottom plate containing 50ul of compound diluted in medium. After 24h incubation at 37°C,5% CO₂, the supernatant was collected. The concentration of IFN-β in the supernatant was measured using a human IFNβ ELISA KIT (R&D, DY814-05). The data was fitted with GraphPad Prism or XLfit to calculate EC₅₀ values.

| Compound | hIFNβ ELISA (EC50, µM) |
|---|---|
| 1 | inactive |
| 2 | inactive |
| 3 | NT |
| 5 | NT |
| 6 | NT |
| 7 | inactive |
| 8 | inactive |
| 9 | inactive |
| 11 | inactive |
| 12 | >8.9 |
| 15 | inactive |
| 16 | inactive |
| 14 | NT |
| 17 | NT |
| 18 | inactive |
| 19 | inactive |
| 20 | inactive |
| 21 | NT |
| 22 | inactive |
| 23 | inactive |
| 24 | inactive |
| 25 | > 16.7 |
| 26 | 11.4 |
| 27 | 22.1 |
| 28 | inactive |
| 29 | NT |
| 30 | NT |
| 31 | inactive |
| 32 | inactive |
| 33 | inactive |
| 37 | NT |
| 38 | NT |
| 39 | NT |
| 42 | NT |
| 43 | inactive |
| 44 | 19.8 |
| 45 | inactive |
| 46 | >5.6 |
| 47 | >5.2 |
| 48 | inactive |
| 49 | >2.5 |
| 50 | inactive |
| 51 | inactive |
| 52 | inactive |
| 53 | >22.7 |
| 54 | >16.7 |
| 55 | inactive |
| 56 | inactive |
| 57 | inactive |
| 58 | >50 |
| 59 | >21.6 |
| 60 | 15.8 |
| 61 | >16.7 |
| 62 | >27.2 |
| 63 | >16.7 |
| 64 | >12.8 |
| 65 | >11.6 |
| 66 | 2.8 |
| 67 | 14.9 |
| 68 | 4.7 |
| 69 | 7.6 |
| 70 | >50 |
| 71 | >17.6 |
| 72 | >38.5 |
| 73 | 6.8 |
| 74 | 11.8 |
| 75 | 7.7 |
| 76 | 9.9 |
| 77 | 20.8 |
| 78 | 19.0 |

| | |
|---|---|
| inactive: indicated that IFNβ was not detected at the maximum concentration of compounds; NT: not tested. | |

## Claims

1. A compound having a structure of Formula (I) or (II), 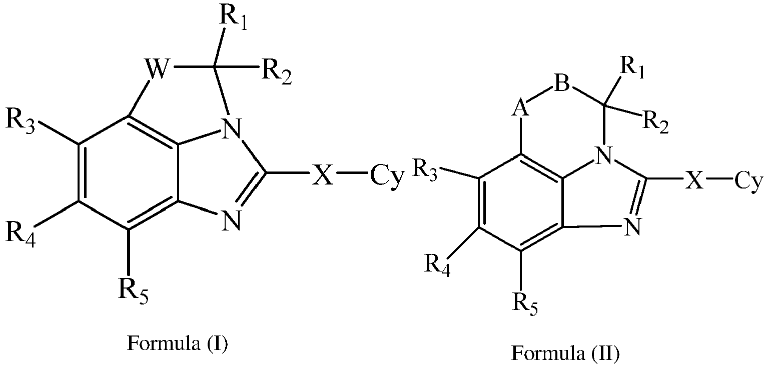
wherein W represents (CR^{a}R^{a'})ₘ, wherein any one CR^{a}R^{a'} is optionally substituted by 0, 1 or 2 O, S or NR^{b};
wherein A and B each independently represent CR^{a}R^{a'}, NR^{b}, O or S;
R₁ and R₂ are each independently selected from hydrogen, halogen, hydroxy, amino, mercapto, C₁-C₆ alkyl, C₁-C₆ alkoxy, C₁-C₆ alkylthio, C₁-C₆ alkylamino, di(C₁-C₆ alkyl) amino, C₂-C₆ alkenyl, C₂-C₆ alkynyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), and -(C₀-C₆ alkylene)-(5- to 12-membered heteroaryl), or R₁ and R₂ together with atoms adjacent thereto are cyclized to form a 3- to 6-membered ring optionally containing 0, 1 or 2 heteroatoms selected from O, N and S;
R₃, R₄ and R₅ are each independently selected from hydrogen, halogen, cyano, C₁-C₆ alkyl, C₂-C₆ alkenyl, C₂-C₆ alkynyl, -(C₀-C₆ alkylene)-(C₃-C₆ cycloalkyl), -(C₀-C₆ alkylene)-(4- to 7-membered heterocycloalkyl), -OR^{c}, -NR^{c}R^{c'}, -OC(O)R^{c'}, -C(O)R^{c}, -CO₂R^{c}, -CON(R^{c})(R^{c'}), -C(=NR^{c})N(R^{c'})(R^{c"}), -NHC(O)R^{c}, -NHS(O)₂R^{c}-, -NHS(O)R^{c}-, -SO₂R^{c}, -SO₂NR^{c}R^{c'}, -(C₀-C₆ alkylene)-(6- to 12-membered aryl), and -(C₀-C₆ alkylene)-(5- to 12-membered heteroaryl);
or R₃ and R₄ are cyclized together to form a 5- to 8-membered ring optionally containing 0, 1, 2, 3 or 4 heteroatoms selected from O, S and N;
or R₄ and R₅ are cyclized together to form a 5- to 8-membered ring optionally containing 0, 1, 2, 3 or 4 heteroatoms selected from O, S and N;
X represents -NR^{d}C(O)-, or -NR^{d}C(=NR^{d'})-;
Cy represents 6- to 12-membered aryl or 5- to 12-membered heteroaryl;
m represents an integer of 1, 2 or 3;
R^{a} and R^{a'} each independently represent hydrogen, halogen, hydroxy, C₁-C₆ alkyl, C₁-C₆ alkylthio, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), -(C₀-C₆ alkylene)-(5- to 12-membered heteroaryl), -NR^{e}R^{e'}, -NR^{e}COR^{e'}, -NR^{e}SO₂R^{e'}, -OR^{e} or -OCOR^{e}, or R^{a} and R^{a'} together with atoms adjacent thereto, are cyclized into a 3- to 6-membered ring optionally containing 0, 1, or 2 heteroatoms selected from O, N and S; or any one CR^{a}R^{a'} is taken together to form -C=O;
R^{b} each independently represents hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene) -(6- to 12-membered aryl), -(C₀-C₆ alkylene)-(5- to 12-membered heteroaryl), -C(O)R^{f}, -SO₂R^{f}, -SOR^{f}, -C(O)OR^{f} or -C(O)NR^{f}R^{f'};
R^{c}, R^{c'}, R^{c"}, R^{d}, R^{d'}, R^{e}, R^{e'}, R^{f}, and R^{f'} each independently represent hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl) or -(C₀-C₆ alkylene)-(5- to 12-membered heteroaryl), or when the above-mentioned substituents are collectively bound to a single N atom, they are optionally cyclized with the bound N atom to form a 3- to 8-membered ring;
the above-mentioned alkyl, alkylene, aryl, heteroaryl, ring, cycloalkyl, heterocycloalkyl, alkenyl, alkynyl, and alkoxy are each optionally independently substituted by 0, 1, 2, 3, or 4 substituents selected from the following groups cosisting of: halogen, hydroxyl, cyano, carboxyl, C₁-C₆ alkyl, C₁-C₆ haloalkyl, sulfo, -OR^{g}, -SR^{g}, -NR^{g}R^{g'}, -NR^{g}COR^{g'}, -NR^{g}COOR^{g'}, -COR^{g}, -CO₂R^{g}, -SOR^{g}, -SO₂R^{g}, -OCONR^{g}R^{g'}-, -OCOR^{g}, -CONR^{g}R^{g'}, -NR^{g}SO₂R^{g'}, -SO₂NR^{g}R^{g'}, and -OP(O)(OR^{g}R^{g'})₂;
or for the aryl and heteroaryl, or when the number of substituents is 2, the adjacent two substituents are also optionally cyclized to each other into a 5- to 6-membered saturated or unsaturated carbocycle or heterocycle, the heterocycle being a ring containing 0, 1, 2, 3 or 4 heteroatoms selected from O, S and N;
wherein R^{g} and R^{g'} each independently consist of hydrogen, or the following groups optionally substituted by 0, 1, 2, 3 or 4 groups selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino and di(C₁-C₆ alkyl) amino: C₁-C₆ alkyl, -(C₀-C₆ alkylene)-(C₃-C₆ cycloalkyl), -(C₀-C₆ alkylene)-O-(C₁-C₆ alkyl), -(C₀-C₆ alkylene)-O-CO (C₁-C₆ alkyl), -(C₀-C₆ alkylene)-C(O)O (C₁-C₆ alkyl), -(C₀-C₆ alkylene)-(6- to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆ alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl);
wherein the 6- to 12-membered aryl is preferably phenyl; the 5- to 12-membered heteroaryl is preferably pyridyl, imidazolyl, or pyrazolyl; or for the above-mentioned 6- to 12-membered aryl or 5- to 12-membered heteroaryl, when the number of substituents is 2, the adjacent two substituents may also be cyclized to each other into a 5- to 6-membered saturated or unsaturated carbocycle or heterocycle;
provided that the following compounds are excluded:

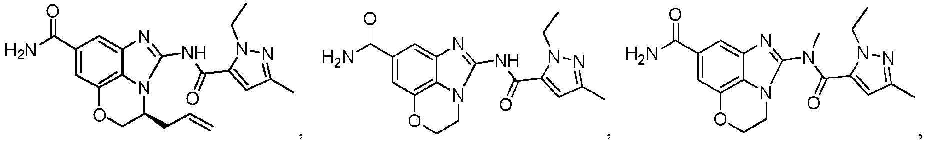

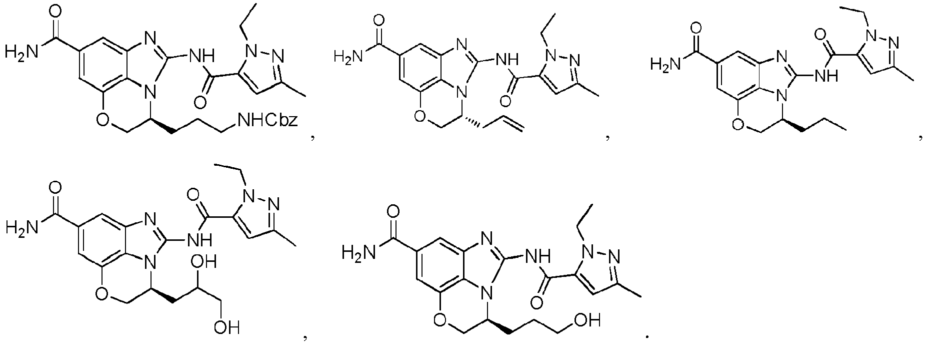

2. The compound according to claim 1, having a structure of Formula (III) or (IV), 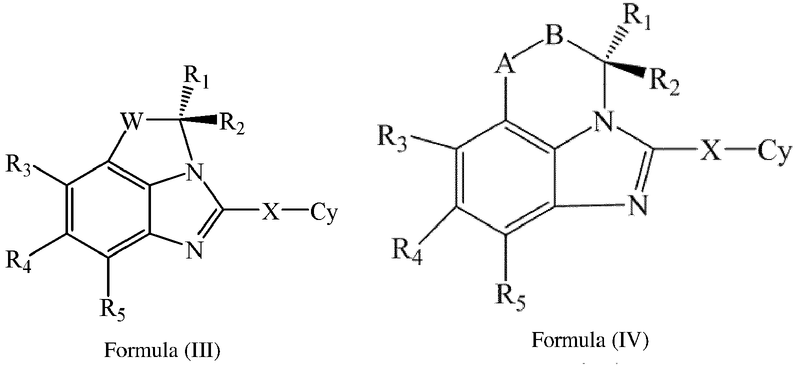 wherein R₁, R₃, R₄, R₅, W, X, Cy, A, and B have the meaning as defined in claim 1; R₂ represents hydrogen or C₁-C₆ alkyl, and R₁ and R₂ represent different substituents.

3. The compound according to claim 1 or 2, wherein A represents O, and B represents CR^{a}R^{a'}.

4. The compound according to any one of claims 1 to 3, wherein R₄ represents -CONR^{c}R^{c'}, and R^{c} and R^{c'} are independently represent hydrogen or C₁-C₆ alkyl;
wherein X represents -NR^{d}C(O)-, and R^{d} represents hydrogen or C₁-C₆ alkyl; or
wherein the Cy is each independently selected from phenyl, pyridyl, pyrazolyl, pyrimidinyl, pyrazinyl, furanyl, thiazolyl, oxazolyl, imidazolyl, thienyl, triazolyl, and tetrazolyl; preferably pyrazolyl, imidazolyl, oxazolyl, triazolyl and tetrazolyl; preferably imidazolyl; and optionally the Cy is each independently substituted by 0, 1, 2, 3 or 4 substituents selected from the following groups consisting of: halogen, hydroxyl, cyano, carboxyl, C₁-C₆ alkyl, C₁-C₆ haloalkyl, sulfo, C₁-C₆ alkoxy, -amino, nitro, (C₁-C₆ alkyl) amino, and di(C₁-C₆ alkyl) amino.

5. The compound according to any one of claims 1 to 4, wherein R₁ consists of C₁-C₆ alkyl, C₂-C₆ alkenyl, -(C₀-C₆ alkylene)-(C₃-C₆ cycloalkyl), -(C₀-C₆ alkylene)-(4- to 7-membered heterocycloalkyl), or -(C₀-C₆ alkylene)-(6- to 12-membered aryl); preferably C₁-C₆ alkyl, C₂-C₆ alkenyl, -(C₀-C₆ alkylene)-(C₃-C₆ cycloalkyl); more preferably C₁-C₆ alkyl, and C₂-C₆ alkenyl; and R₁ is optionally substituted by a substituent selected from the following substituents: -NR^{g}COR^{g'}; and R^{g} consists of hydrogen or C₁-C₆ alkyl; R^{g'} consists of the following groups substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino and di(C₁-C₆ alkyl) amino: C₁-C₆ alkyl, -(C₀-C₆ alkylene)-(C₃-C₆ cycloalkyl), -(C₀-C₆ alkylene)-O-(C₁-C₆ alkyl), -(C₀-C₆ alkylene)-O-CO (C₁-C₆ alkyl), -(C₀-C₆ alkylene)-C(O)O (C₁-C₆ alkyl), -(C₀-C₆ alkylene)-(6- to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl);
wherein the 6- to 12-membered aryl is preferably phenyl; the 5- to 12-membered heteroaryl is preferably pyridyl, imidazolyl, or pyrazolyl; or for the above-mentioned 6- to 12-membered aryl or 5- to 12-membered heteroaryl, when the number of substituents is 2, the adjacent two substituents are also optionally cyclized to each other into a 5- to 6-membered saturated or unsaturated carbocycle or heterocycle.

6. The compound according to any one of claims 1 to 5, wherein R₁ consists of -(C₁-C₆ alkylene)-NR^{g}COR^{g'}, and -(C₂-C₆ alkenylene)-NR^{g}COR^{g'}, wherein R^{g} consists of hydrogen or C₁-C₆ alkyl; R^{g'} consists of the following groups substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino and di(C₁-C₆ alkyl) amino: -(C₀-C₆ alkylene)-(6-to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆ alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl);
wherein the 6- to 12-membered aryl is preferably phenyl; the 5- to 12-membered heteroaryl is preferably pyridyl; or for the above-mentioned 6-to 12-membered aryl or 5-to 12-membered heteroaryl, when the number of substituents is 2, the adjacent two substituents are also optionally cyclized to each other into a 5-to 6-membered saturated or unsaturated carbocycle or heterocycle; more preferably -O-(C₁-C₆ alkylene)-phenyl, -O-(C₁-C₆ alkylene)-pyridyl, -(C₁-C₆ alkylene)-O-phenyl, -(C₁-C₆ alkylene)-O-pyridyl, -(C₁-C₆ alkylene)-phenyl, -(C₁-C₆ alkylene)-pyridyl, -(C₂-C₆ alkenylene)-phenyl, or -(C₂-C₆ alkenylene)-pyridyl; and the phenyl, and pyridyl are optionally independently substituted by 0, 1, 2, 3 or 4 substituents selected from hydroxy, halogen, amino, sulfonyl, cyano, nitro, C₁-C₆ alkoxy, and C₁-C₆ haloalkyl.

7. The compound according to any one of claims 1 to 6, wherein R₂ represents hydrogen or C₁-C₆ alkyl; or
wherein R₃ and R₅ each independently represent hydrogen, halogen, or C₁-C₆ alkyl.

8. The compound according to claim 1, having a structure of Formula (V), 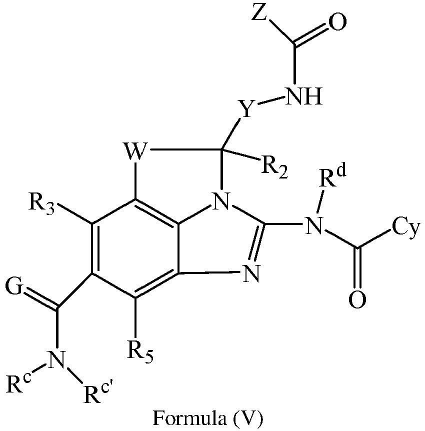
wherein W represents (CR^{a}R^{a'})ₘ, wherein any one CR^{a}R^{a'} is optionally substituted by 0, 1 or 2 O, S or NR^{b};
R₂ independently represents hydrogen, halogen, hydroxy, amino, mercapto, C₁-C₆ alkyl, C₁-C₆ alkoxy, C₁-C₆ alkylthio, C₁-C₆ alkylamino, di(C₁-C₆ alkyl) amino, C₂-C₆ alkenyl, C₂-C₆ alkynyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), or -(C₀-C₆ alkylene)-(5- to 12-membered heteroaryl);
R₃ and R₅ are each independently selected from hydrogen, halogen, cyano, C₁-C₆ alkyl, C₂-C₆ alkenyl, C₂-C₆ alkynyl, -(C₀-C₆ alkylene)-(C₃-C₆ cycloalkyl), -OR^{c}, -NR^{c}R^{c'}, -OC(O)R^{c'}, -C(O)R^{c}, -CO₂R^{c}, -CON(R^{c})(R^{c'}), -C(=NR^{c})N(R^{c'})(R^{c"}), -NHC(O)R^{c}, -NHS(O)₂R^{c}-, -NHS(O)R^{c}-, -SO₂R^{c}, -SO₂NR^{c}R^{c'}, -(C₀-C₆ alkylene)-(4- to 7-membered heterocycloalkyl), -(C₀-C₆ alkylene)-(6- to 12-membered aryl), and -(C₀-C₆ alkylene)-(5- to 12-membered heteroaryl);
Cy represents 6- to 12-membered aryl, or 5- to 12-membered heteroaryl;
m represents an integer of 1, 2 or 3;
R^{a} and R^{a'} each independently represent hydrogen, halogen, hydroxy, C₁-C₆ alkyl, C₁-C₆ alkylthio, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), -(C₀-C₆ alkylene)-(5- to 12-membered heteroaryl), -NR^{e}R^{e'}, -NR^{e}COR^{e'}, -NR^{e}SO₂R^{e'}, -OR^{e} or -OCOR^{e}, or R^{a} and R^{a'} together with the atoms adjacent thereto, are cyclized into a 3- to 6-membered ring optionally containing 0, 1, or 2 heteroatoms selected from O, N and S; or any one CR^{a}R^{a'} is taken together to form -C=O;
R^{b} each independently represents hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene) -(6- to 12-membered aryl), -(C₀-C₆ alkylene)-(5- to 12-membered heteroaryl), -C(O)R^{f}, -SO₂R^{f}, -SOR^{f}, -C(O)OR^{f} or -C(O)NR^{f}R^{f'};
G represents O or NR^{c};
R^{c}, R^{c'}, R^{c"}, R^{d}, R^{e}, R^{e'}, R^{f}, and R^{f'} each independently represent hydrogen, C₁-C₆ alkyl, -(C₀-C₆ alkylene)-(C₃-C₆ cycloalkyl), -(C₀-C₆ alkylene)-(4- to 7-membered heterocycloalkyl), -(C₀-C₆ alkylene)-(6- to 12-membered aryl), or -(C₀-C₆ alkylene)-(5-to 12-membered heteroaryl), or when the above-mentioned substituents are collectively bound to a single N atom, they are optionally cyclized with the bound N atom to form a 3- to 8-membered ring;
the above-mentioned alkyl, alkylene, aryl, heteroaryl, ring, cycloalkyl, heterocycloalkyl, alkenyl, alkynyl, and alkoxy are each optionally independently substituted by 0, 1, 2, 3, or 4 substituents selected from the following groups consisting of: halogen, oxo, hydroxy, cyano, carboxy, C₁-C₆ alkyl, C₁-C₆ haloalkyl, sulfo, C₁-C₆ alkoxy, -OR^{g}, -SR^{g}, -N(R^{g})(R^{g'}), -NR^{g}COR^{g'}, -NR^{g}COOR^{g'}, -COR^{g}, -CO₂R^{g}, -SOR^{g}, -SO₂R^{g}, -OCONR^{g}R^{g'}-, -OCOR^{g}, -CONR^{g}R^{g'}, -NR^{g}SO₂R^{g'}, -SO₂NR^{g}R^{g'}, and -OP(O)(OR^{g}R^{g'})₂;
or for the aryl and heteroaryl, or when the number of substituents is 2, the adjacent two substituents are also optionally cyclized to each other into a 5- to 6-membered saturated or unsaturated carbocycle or heterocycle, the heterocycle being a ring containing 0, 1, 2, 3 or 4 heteroatoms selected from O, S and N;
wherein R^{g} and R^{g'} each independently represent hydrogen, or the following groups optionally substituted by 0, 1, 2, 3 or 4 groups selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, di(C₁-C₆ alkyl) amino: C₁-C₆ alkyl, -(C₀-C₆ alkylene)-(C₃-C₆ cycloalkyl), halo (C₁-C₆ alkyl), -(C₀-C₆ alkyl)-OH, -(C₀-C₆ alkylene)-O-(C₁-C₆ alkyl), -C₀-C₆ alkylene)-O-CO (C₁-C₆ alkyl), -(C₀-C₆ alkylene)-C(O)O (C₁-C₆ alkyl), -(C₀-C₆ alkylene)-(6- to 12-membered aryl), -C₂-C₆ alkenylene-( 6- to 12-membered aryl), -(C₀-C₆ alkylene)-O-(6- to 12-membered aryl), -(C₀-C₆ alkylene)-O-C₁-C₆ alkyl, -O-(C₁-C₆ alkylene)-(6- to 12-membered aryl), -(C₀-C₆ alkylene)-(5-to 12-membered heteroaryl), -C₂-C₆ alkenylene-(5- to 12-membered heteroaryl), -(C₀-C₆ alkylene)-O-(5- to 12-membered heteroaryl), or -O-(C₀-C₆ alkylene)-(5- to 12-membered heteroaryl); or for the above-mentioned 6-to 12-membered aryl or 5-to 12-membered heteroaryl, when the number of substituents is 2, the adjacent two substituents are also optionally cyclized to each other into a 5-to 6-membered saturated or unsaturated carbocycle or heterocycle;
Y represents the following groups optionally substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino: -C₁-C₆ alkylene-, -(C₀-C₆ alkylene)-(C₃-C₆ cycloalkyl)-(C₀-C₆ alkylene), -(C₀-C₆ alkylene)-(4- to 7-membered heterocycloalkyl)-(C₀-C₆ alkylene), (C₀-C₆ alkylene)-(6- to 12-membered aryl)-(C₀-C₆ alkylene), -(C₀-C₆ alkylene)-(5- to 12-membered heteroaryl)-(C₀-C₆ alkylene), or -C₂-C₆ alkenylene-;
Z represents the following groups optionally substituted by 0, 1, 2, 3 or 4 substituents selected from hydroxy, halogen, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino: C₁-C₆ alkyl, -(C₀-C₆ alkylene)-(C₃-C₆ cycloalkyl), -(C₀-C₆ alkylene)-O-(C₁-C₆ alkyl), -(C₀-C₆ alkylene)-O-CO(C₁-C₆ alkyl), -(C₀-C₆ alkylene)-C(O)O(C₁-C₆ alkyl), -(C₀-C₆ alkylene)-(6- to 12-membered aryl), -(C₀-C₆ alkylene)-(5- to 12-membered heteroaryl), -(C₂-C₆ alkenylene)-(6- to 12-membered aryl), -(C₂-C₆ alkenylene)-(5- to 12-membered heteroaryl), -O-(C₀-C₆ alkylene)-(6- to 12-membered aryl), -O-(C₀-C₆ alkylene)-(5- to 12-membered heteroaryl), -O-(C₂-C₆ alkenylene)-(6- to 12-membered aryl), -O-(C₂-C₆ alkenylene)-(5- to 12-membered heteroaryl), -(C₀-C₆ alkylene)-O-(6- to 12-membered aryl), -(C₀-C₆ alkylene)-O-(5- to 12-membered heteroaryl), -(C₂-C₆ alkenylene)-O-(6- to 12-membered aryl), or -(C₂-C₆ alkenylene)-O-(5- to 12-membered heteroaryl); or for the above-mentioned 6-to 12-membered aryl or 5-to 12-membered heteroaryl, when the number of substituents is 2, the adjacent two substituents are also optionally cyclized to each other into a 5-to 6-membered saturated or unsaturated carbocycle or heterocycle;
wherein the 6- to 12-membered aryl is preferably phenyl; the 5- to 12-membered heteroaryl is preferably pyridyl, imidazolyl, or pyrazolyl; or for the above-mentioned 6- to 12-membered aryl or 5- to 12-membered heteroaryl, when the number of substituents is 2, the adjacent two substituents are also optionally cyclized to each other into a 5- to 6-membered saturated or unsaturated carbocycle or heterocycle.

9. The compound according to claim 8, having a structure of Formula (VI), 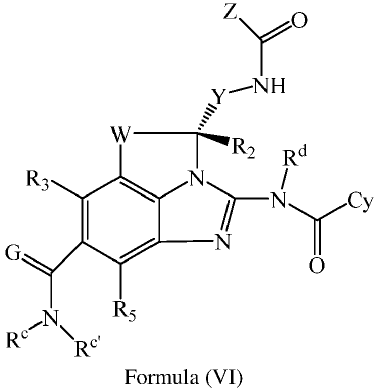 wherein R₂ is selected from hydrogen or C₁-C₆ alkyl, and W, R₃, R₅, R^{c}, R^{c'}, R^{d}, G, Z, Y, and Cy are all as defined in claim 8.

10. The compound according to any one of claims 8 to 9, wherein G consists of O or NH;
wherein Y consists of the following groups substituted by 0, 1, 2, 3, or 4 substituents selected from hydroxy, halo, and C₁-C₆ alkyl: -C₁-C₆ alkylene-, -C₂-C₆ alkenylene-, or -C₃-C₆ cycloalkyl-;
wherein W consists of -CR^{a}R^{a'}-O, -O-CR^{a}R^{a'}-, -C(O)-NR^{b}-, or -NR^{b}-C(O)-, wherein R^{a}, R^{a'},
and R^{b} each independently represent hydrogen, C₁-C₆ alkyl, or C₃-C₆ cycloalkyl;
wherein Z consists of -O-(C₀-C₆ alkylene)-(6- to 12-membered aryl), -(C₀-C₆ alkylene)-(6- to 12-membered aryl), -(C₂-C₆ alkenylene)-(6- to 12-membered aryl), -(C₀-C₆ alkylene)-(5- to 12-membered heteroaryl), -(C₂-C₆ alkenylene)-(5- to 12-membered heteroaryl), -O-(C₂-C₆ alkenylene)-(6- to 12-membered aryl), -(C₀-C₆ alkylene)-O-(6- to 12-membered aryl), -O-(C₀-C₆ alkylene)-(5- to 12-membered heteroaryl), -O-(C₂-C₆ alkenylene)-(5- to 12-membered heteroaryl), or -( C₀-C₆ alkylene)-O-(5- to 12-membered heteroaryl), and optionally the 6- to 12-membered aryl (preferably phenyl) or 5- to 12-membered heteroaryl (preferably pyridyl) is each independently substituted by 0, 1, 2, 3 or 4 substituents selected from the following groups consisting of: halo, hydroxy, nitro, C₁-C₆ alkyl, halo (C₁-C₆ alkyl), amino, sulfonyl, cyano, C₁-C₆ alkoxy, C₁-C₆ alkylthio, C₁-C₆ amino, and di(C₁-C₆ alkyl) amino;
wherein R² represents hydrogen or C₁-C₆ alkyl;
wherein R³ and R⁵ each independently represent halogen, hydrogen, or C₁-C₆ alkyl;
wherein R^{c} and R^{c'} represent hydrogen or C₁-C₆ alkyl;
wherein R^{d} represents hydrogen or C₁-C₆ alkyl; or
wherein Cy represents pyrazolyl, and is optionally substituted by 0, 1, 2, or 3 C₁-C₆ alkyl.

11. The compound according to claim 1, having a structure selected from:
| No. | Compound structure | No. | Compound structure |
|---|---|---|---|
| 1 | 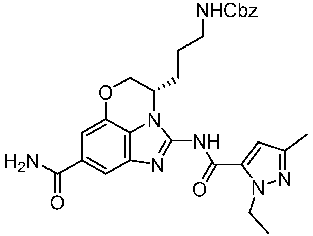 | 2 | |
| 3 | 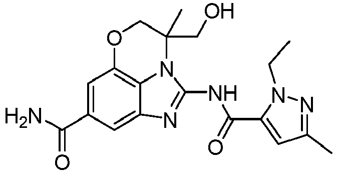 | 4 | 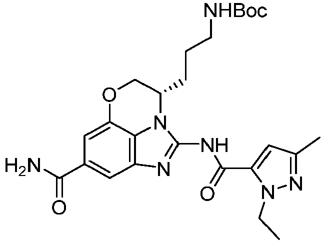 |
| 5 | 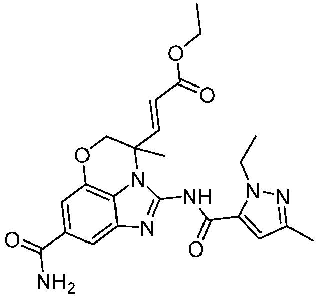 | 6 | 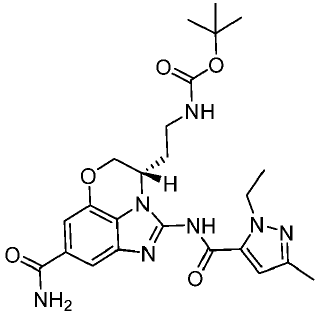 |
| 7 | 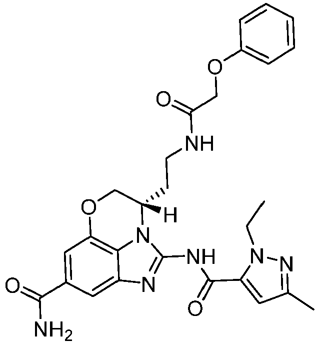 | 8 | 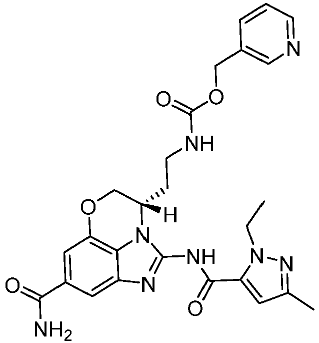 |
| 9 | 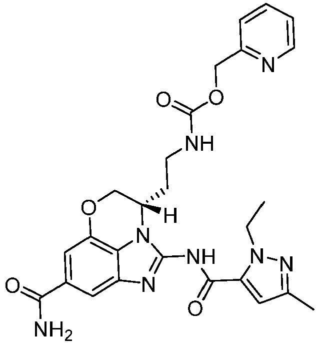 | 10 | 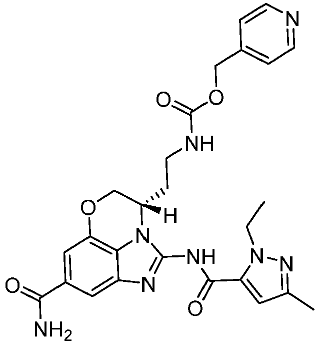 |
| 11 | 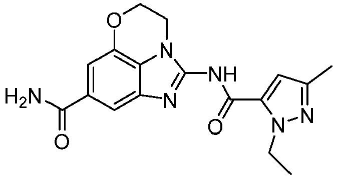 | 12 | 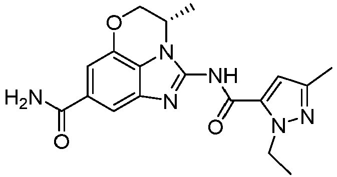 |
| 13 | 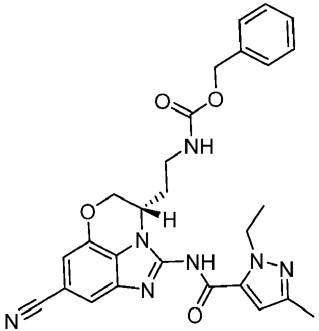 | 14 | 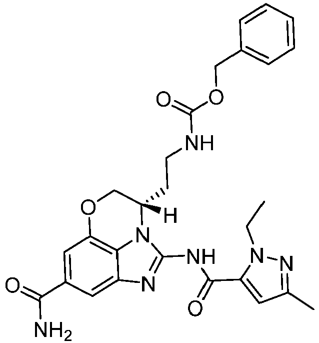 |
| 15 | 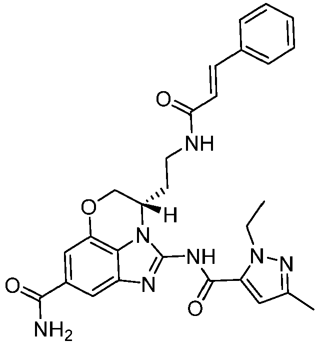 | 16 | 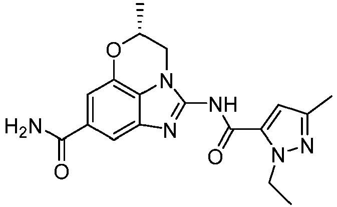 |
| 17 | 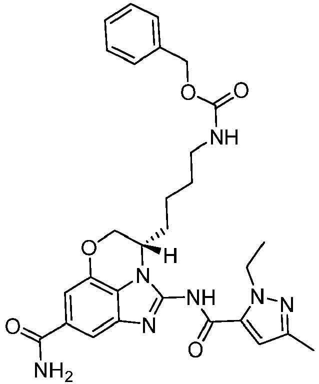 | 18 | 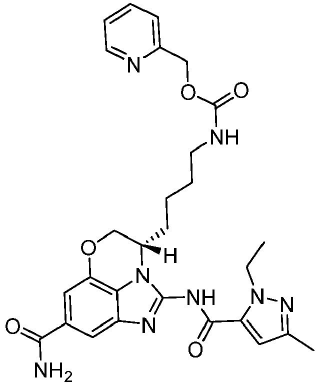 |
| 19 | 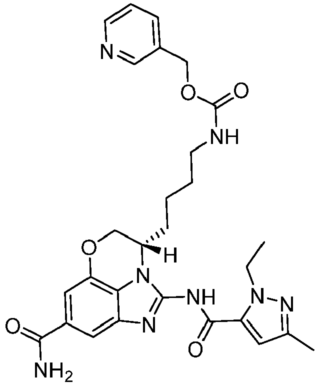 | 20 | 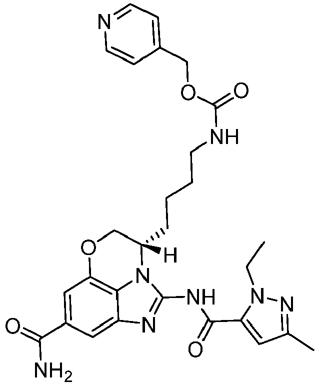 |
| 21 | 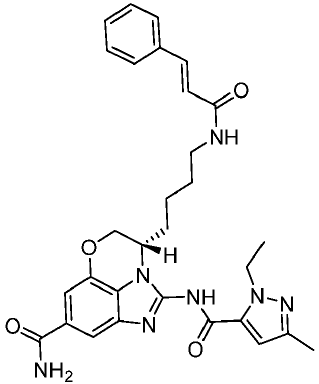 | 22 | 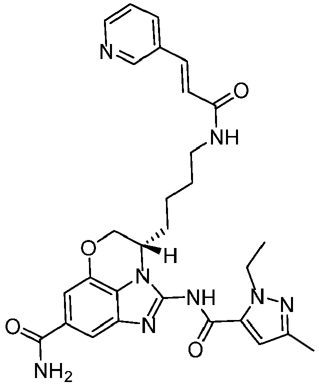 |
| 23 | 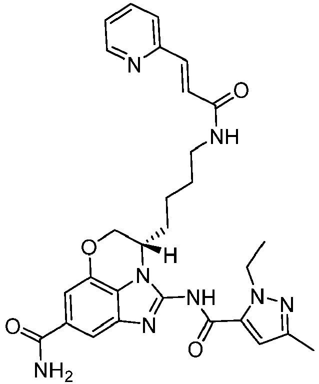 | 24 | 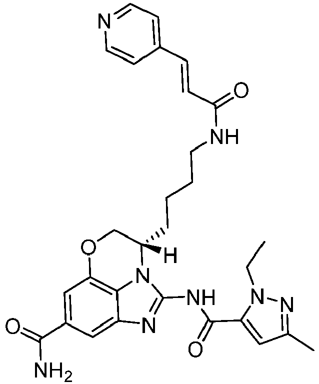 |
| 25 | 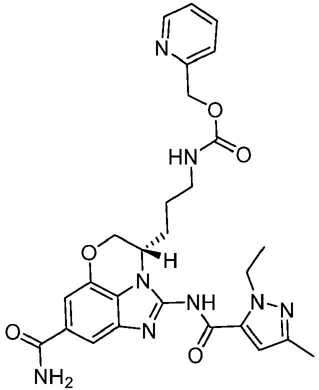 | 26 | 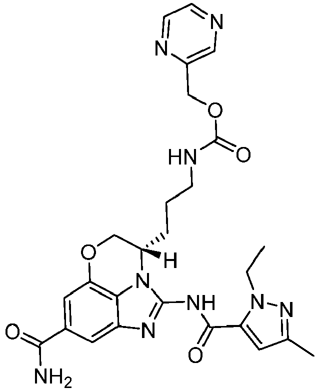 |
| 27 | 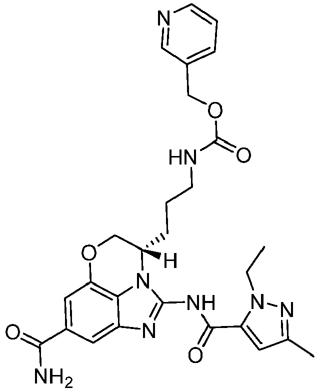 | 28 | 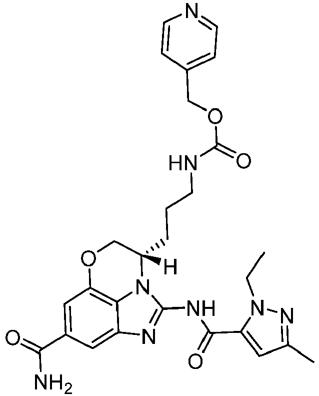 |
| 29 | 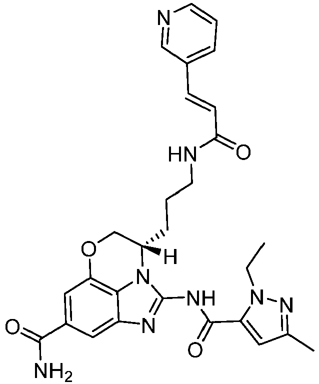 | 30 | 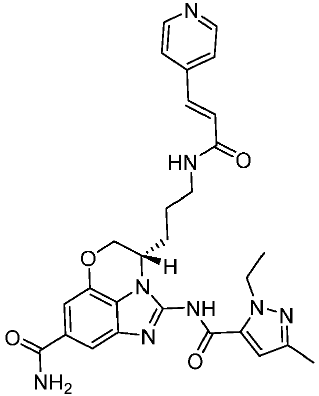 |
| 31 | 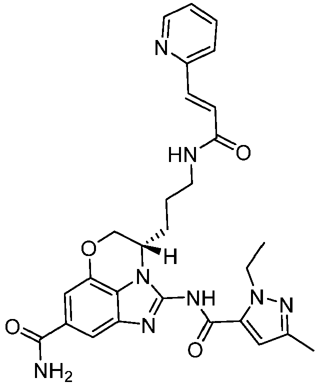 | 32 | 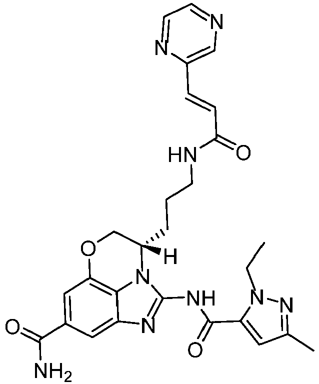 |
| 33 | 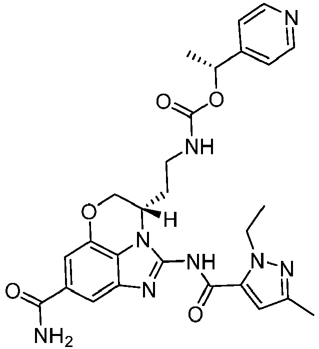 | 34 | 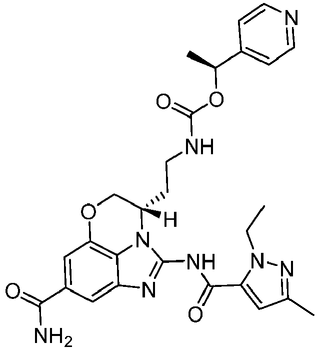 |
| 35 | 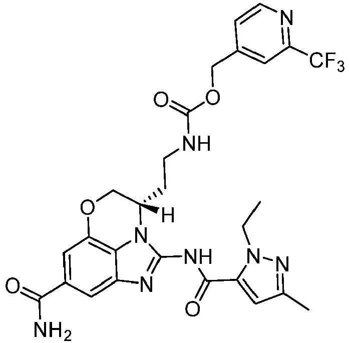 | 36 | 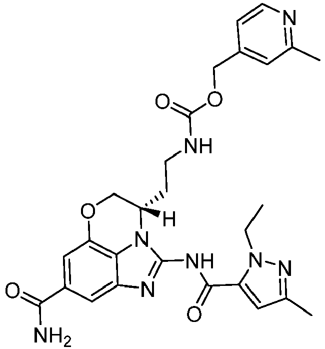 |
| 37 | 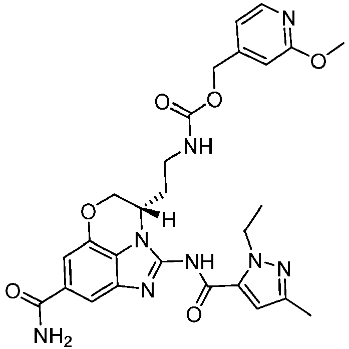 | 38 | 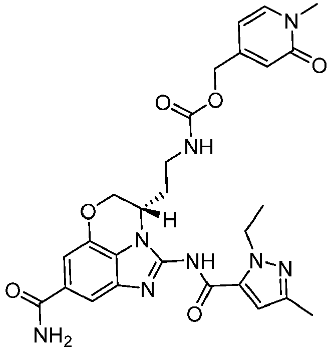 |
| 39 | 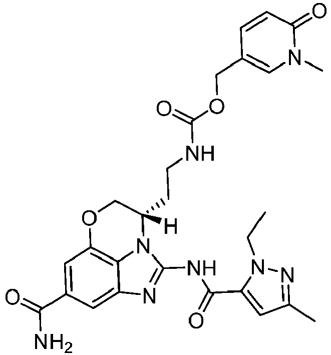 | 40 | 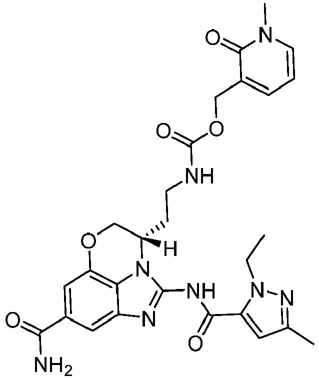 |
| 41 | 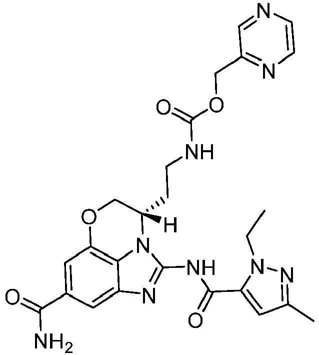 | 42 | 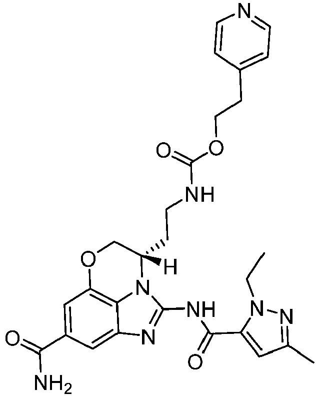 |
| 43 | 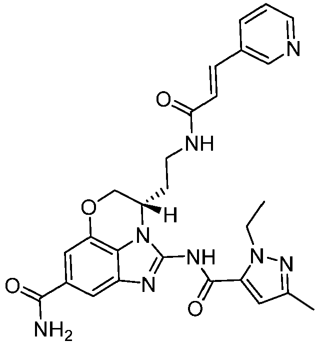 | 44 | 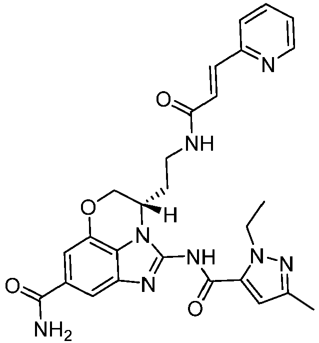 |
| 45 | 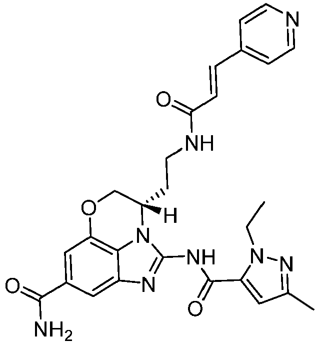 | 46 | 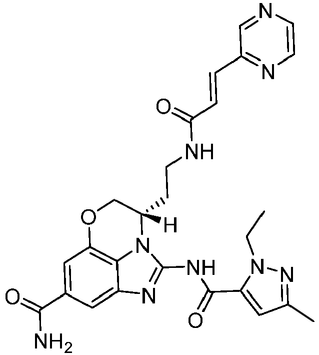 |
| 47 | 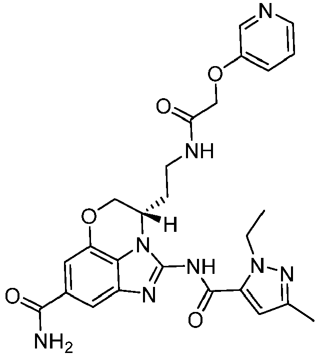 | 48 | 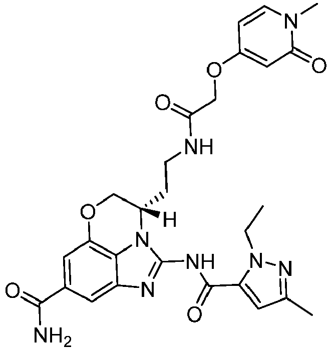 |
| 49 | 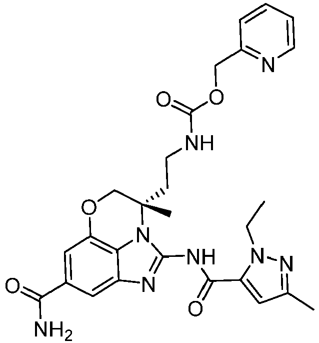 | 50 | 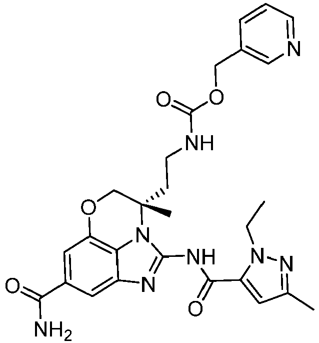 |
| 51 | 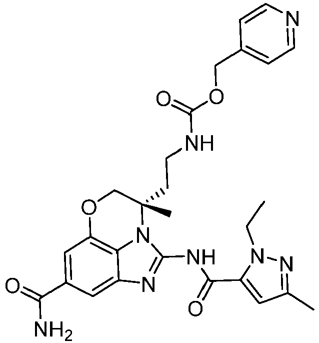 | 52 | 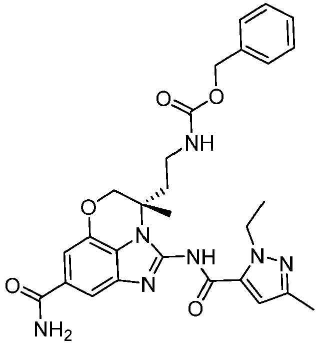 |
| 53 | 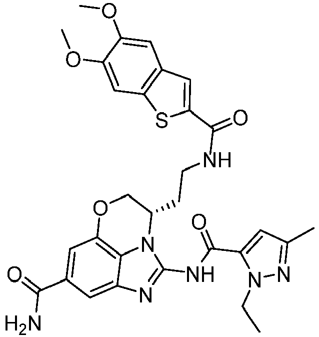 | 54 | 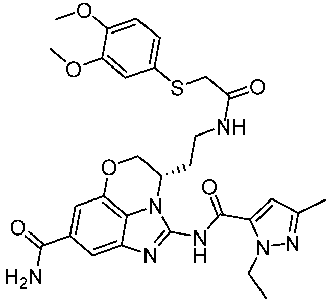 |
| 55 | 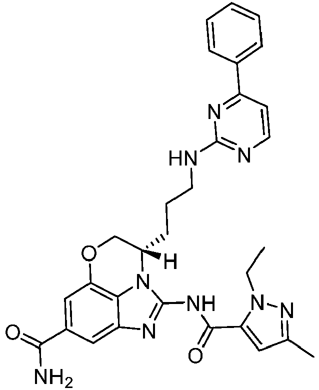 | 56 | 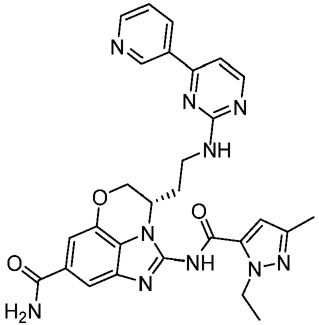 |
| 57 | 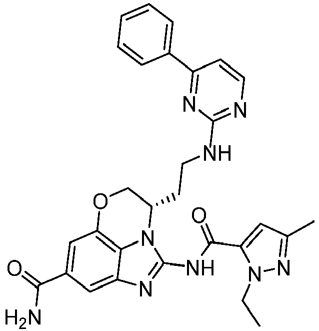 | 58 | 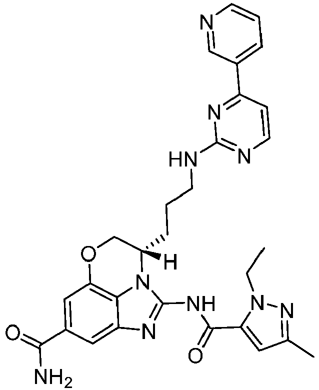 |
| 59 | 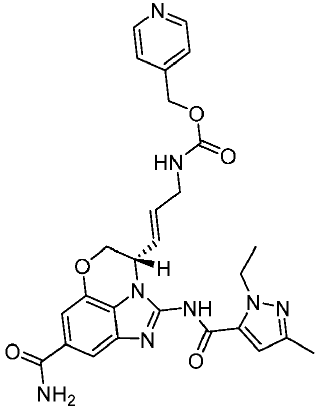 | 60 | 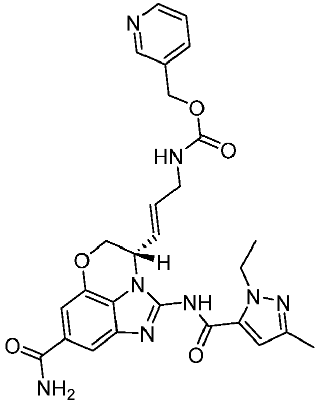 |
| 61 | 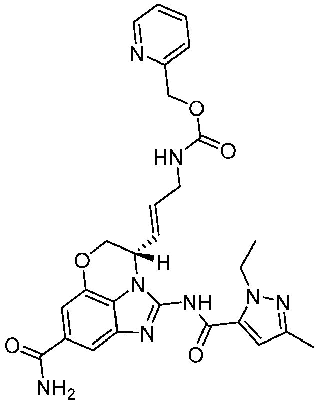 | 62 | 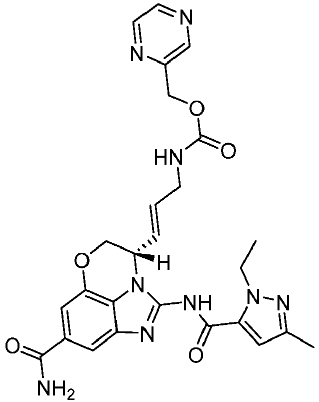 |
| 63 | 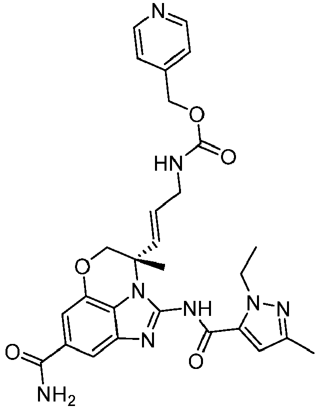 | 64 | 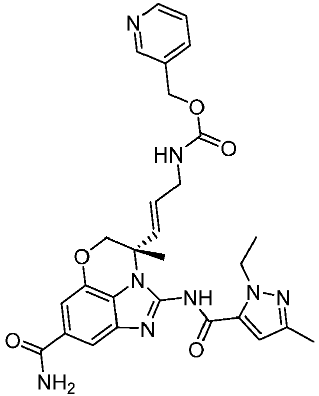 |
| 65 | 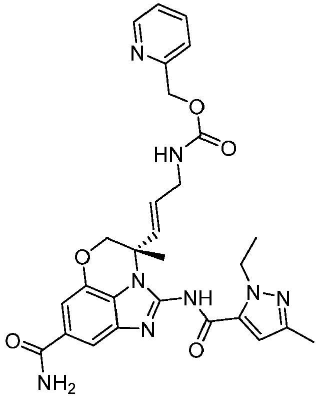 | 66 | 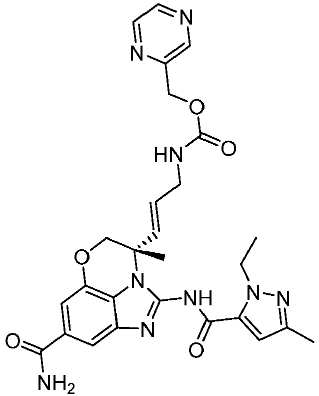 |
| 67 | 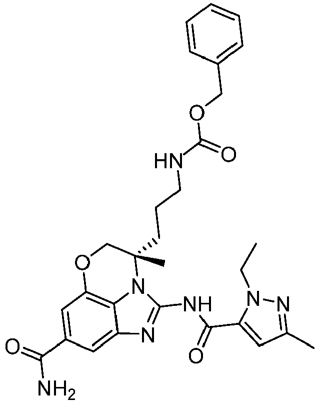 | 68 | 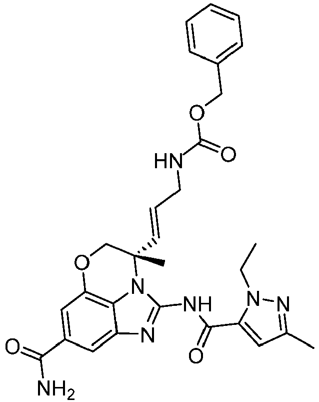 |
| 69 | 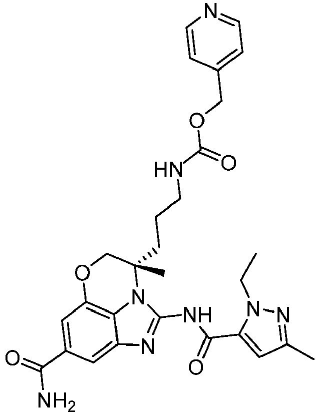 | 70 | 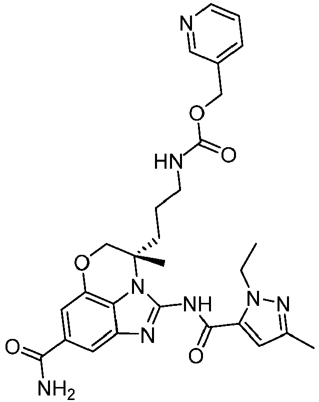 |
| 71 | 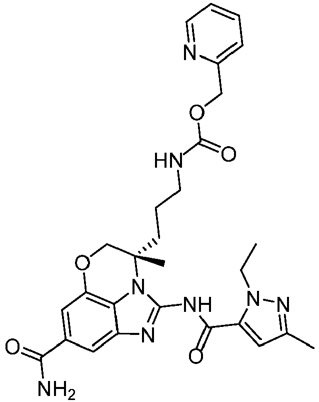 | 72 | 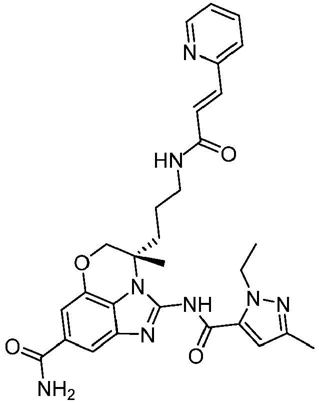 |
| 73 | 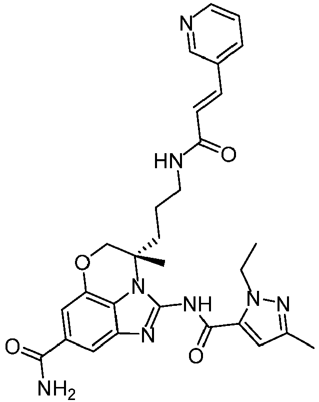 | 74 | 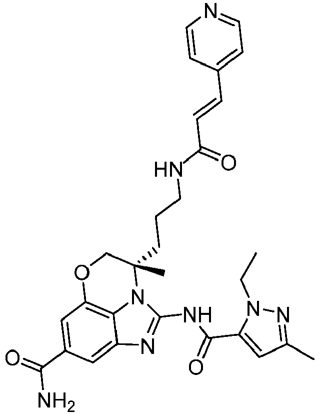 |
| 75 | 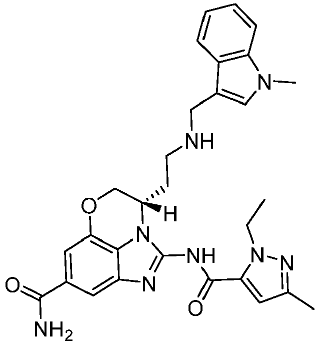 | 76 | 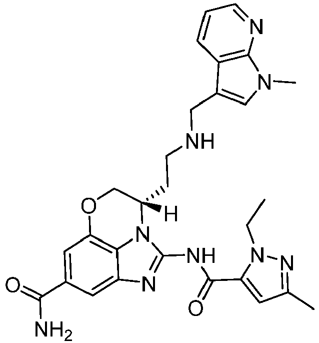 |
| 77 | 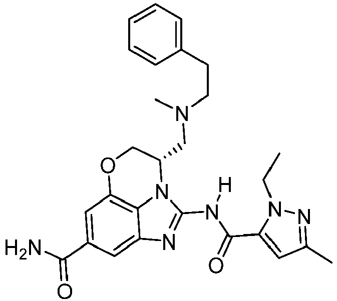 | 78 | 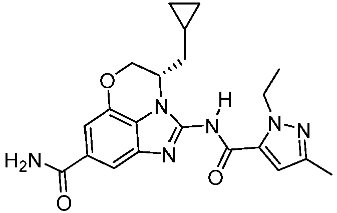 |
| 79 | 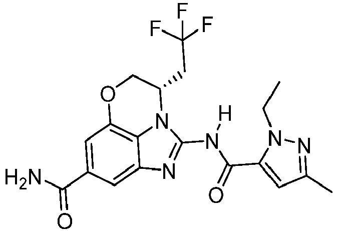 | 80 | 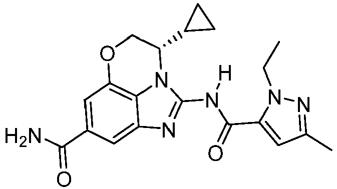 |
| 81 | 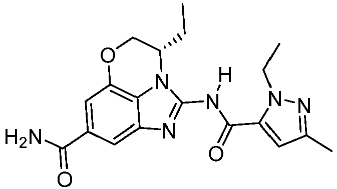 | 82 | 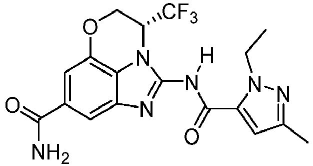 |
| 83 | 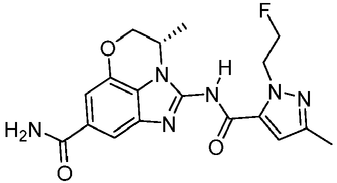 | 84 | 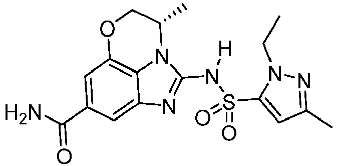 |
| 85 | 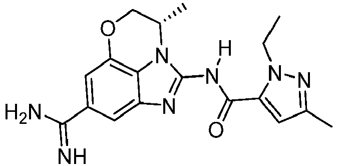 | 86 | 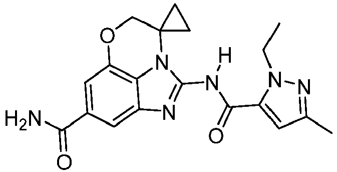 |
| 87 | 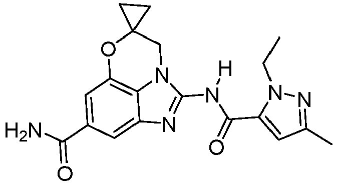 | 88 | 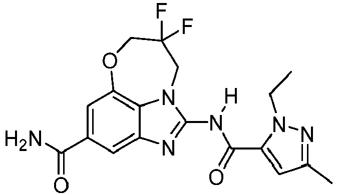 |
| 89 | 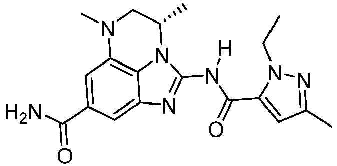 | 90 | 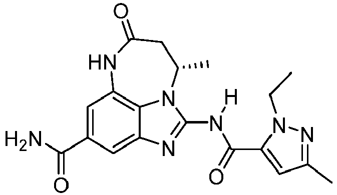 |
| 91 | 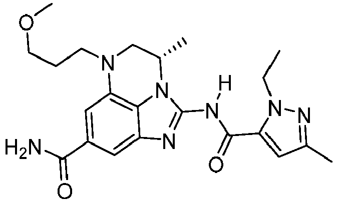 | 92 | 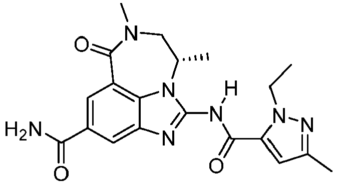 |
| 93 | 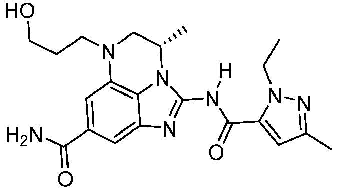 | 94 | 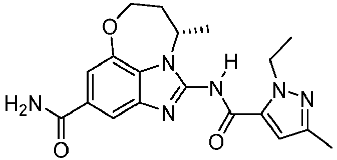 |
| 95 | 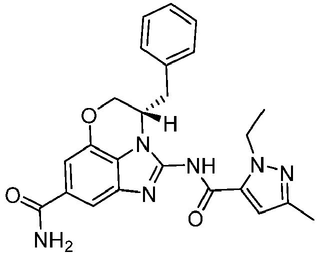 | 96 | 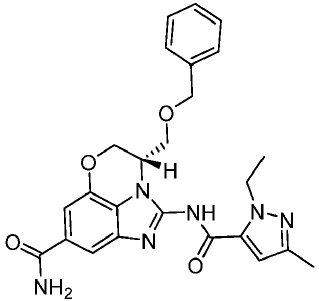 |
| 97 | 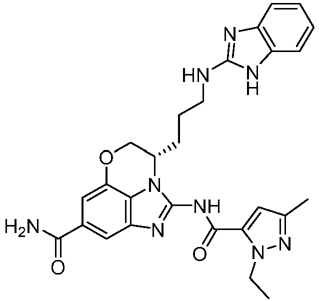 | 98 | 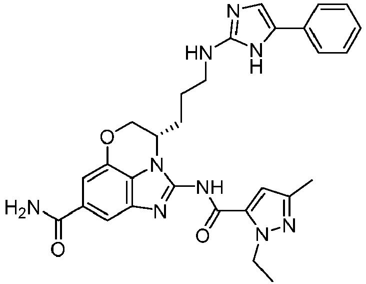 |
| 99 | 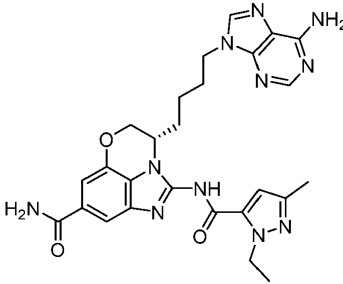 | 100 | 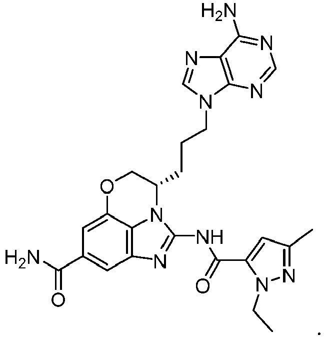 |
| 101 | 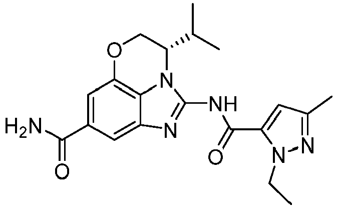 | 102 | 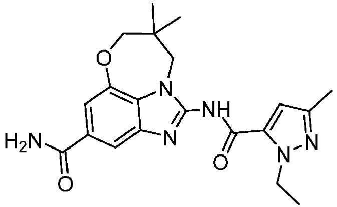 |
| 103 | 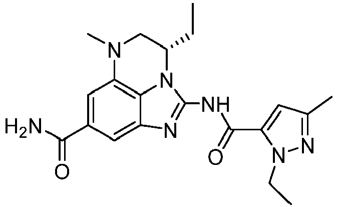 | 104 | 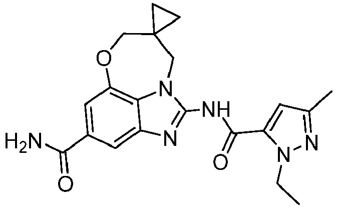 |
| 105 | 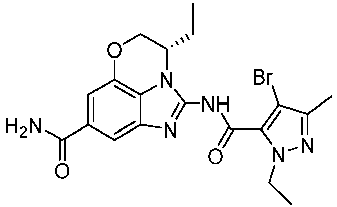 | 106 | 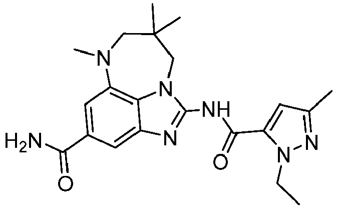 |
| 107 | 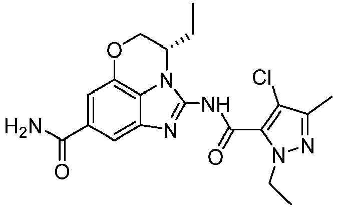 | 108 | 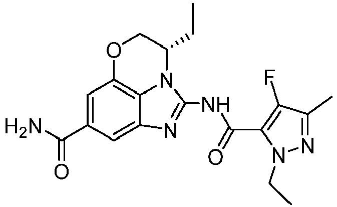 |
| 109 | 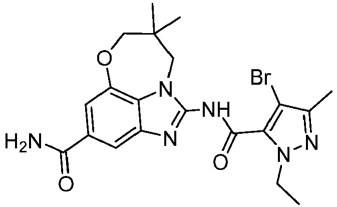 | 110 | 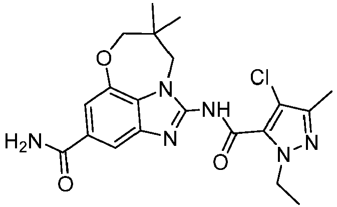 |
| 111 | 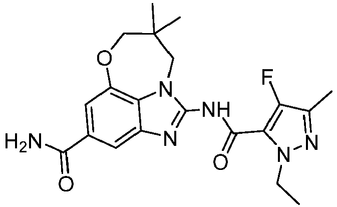 | 112 | 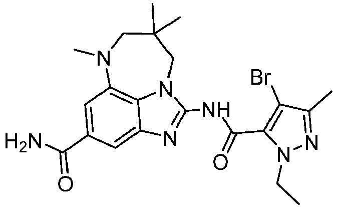 |
| 113 | 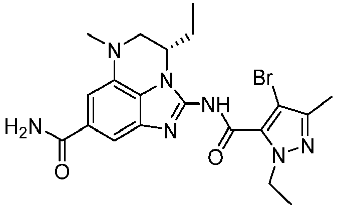 | 114 | 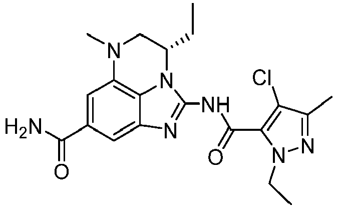 |
| 115 | 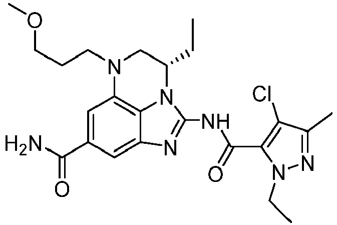 | 116 | 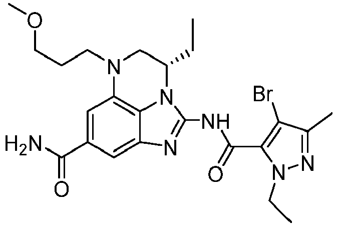 |
| 117 | 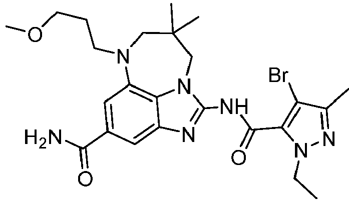 | 118 | 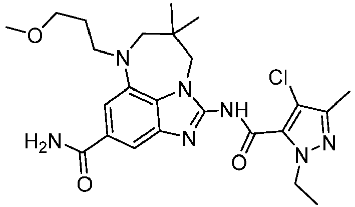 |
| 119 | 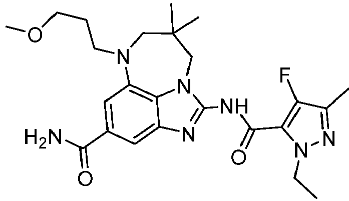 | 120 | 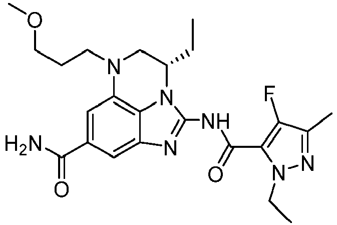 |
| 121 | 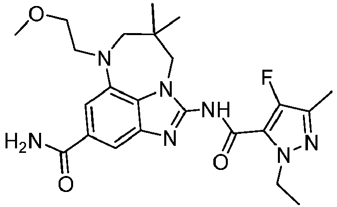 | 122 | 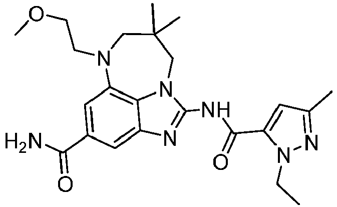 |
| 123 | 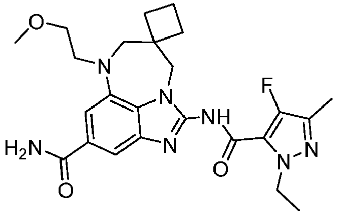 | 124 | 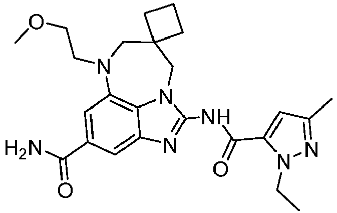 |
| 125 | 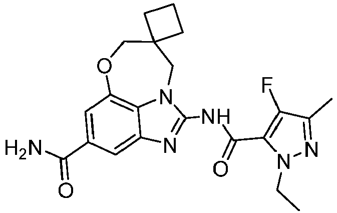 | 126 | 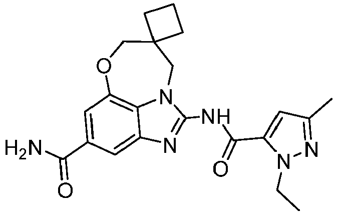 |
| 127 | 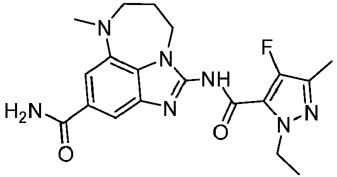 | 128 | 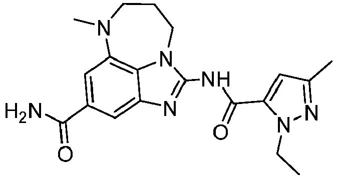 |
| 129 | 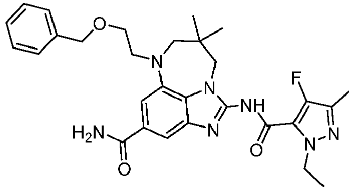 | 130 | 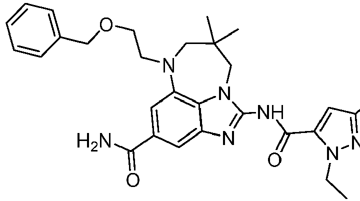 |
| 131 | 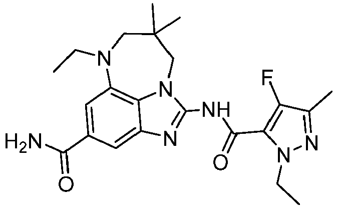 | 132 | 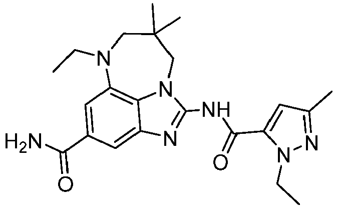 |
| 133 | 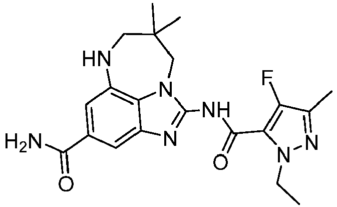 | 134 | 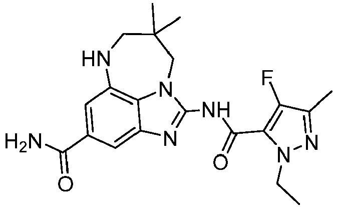 |
| 135 | 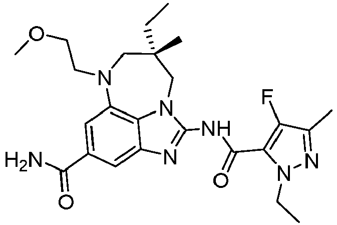 | 136 | 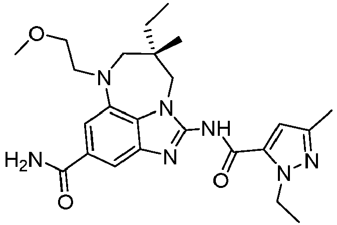 |
| 137 | 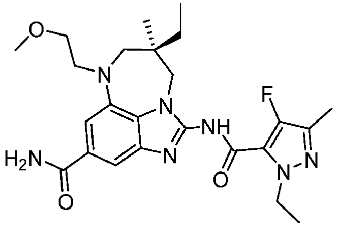 | 138 | 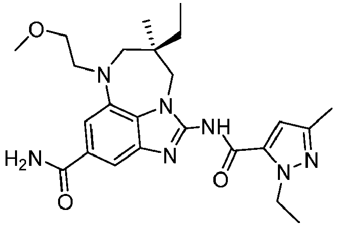 |
| 139 | 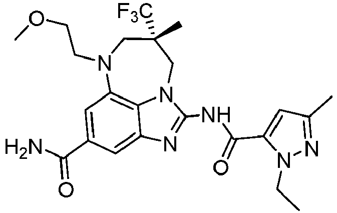 | 140 | 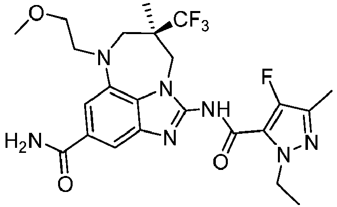 |
| 141 | 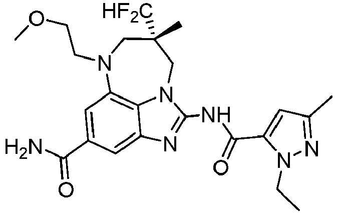 | 142 | 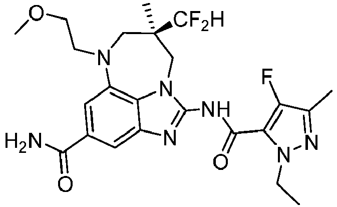 |
| 143 | 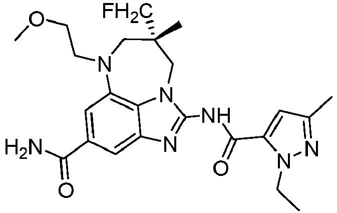 | 144 | 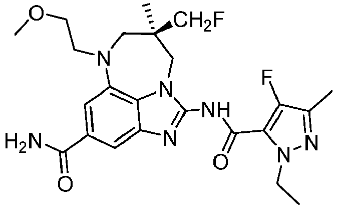 |
| 145 | 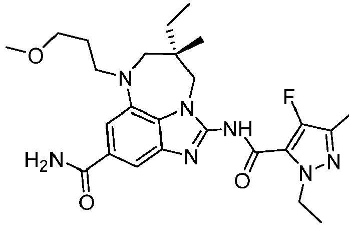 | 146 | 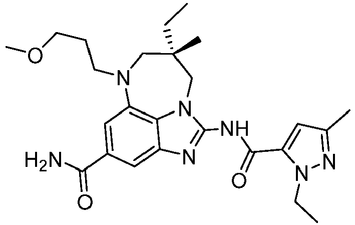 |
| 147 | 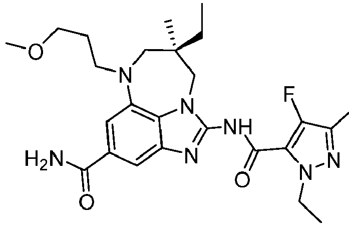 | 148 | 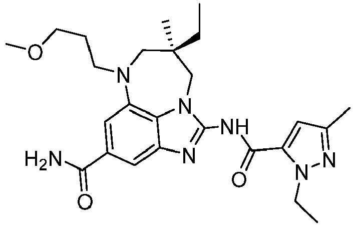 |
| 149 | 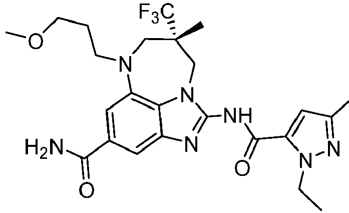 | 150 | 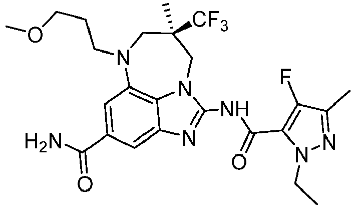 |
| 151 | 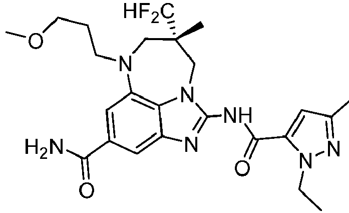 | 152 | 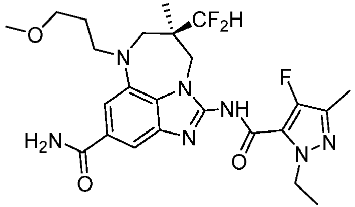 |
| 153 | 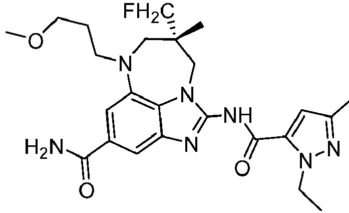 | 154 | 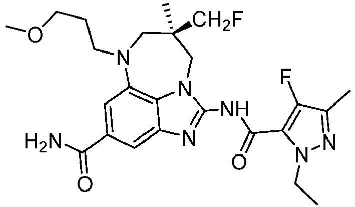 |
| 155 | 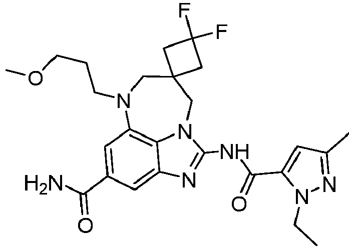 | 156 | 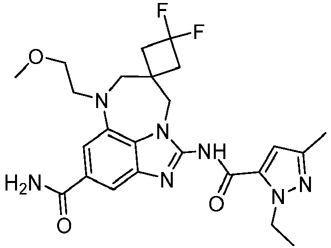 |
| 157 | 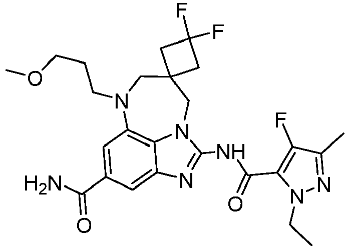 | 158 | 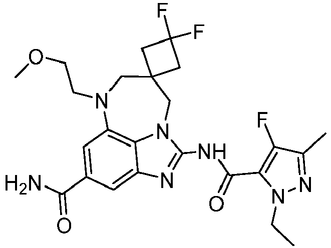 |

12. A pharmaceutical composition comprising a compound of any one of claims 1 to 11 and a pharmaceutically acceptable carrier.

13. The pharmaceutical composition according to claim 12, further comprising an additional therapeutic agent and/or an immune checkpoint inhibitor, wherein the additional therapeutic agent is preferably selected from Chlorambucil, Melphalan, Cyclophosphamide, Ifosfamide, Busulfan, Carmustine, Lomustine, Streptozotocin, Cisplatin, Carboplatin, Oxaliplatin, Dacarbazine, Temozolomide, Procarbazine, Methotrexate, Fluorouracil, Cytarabine, Gemcitabine, Mercaptopurine, Fludarabine, Vinblastine, Vincristine, Vinorelbine, Paclitaxel, Docetaxel, Topotecan, Irinotecan, Etoposide, Trabectedin, Dactinomycin, Doxorubicin, Epirubicin, Daunorubicin, Mitoxantrone, Bleomycin, Mitomycin C, Ixabepilone, Tamoxifen, Flutamide, Gonadorelin Analogs, Megestrol, Prednisone, Dexamethasone, Methylprednisolone, Thalidomide, Interferon A, Calcium Folinate, Sirolimus, Sirolimus Lipide, Everolimus, Afatinib, Alisertib, Amuvatinib, Apatinib, Axitinib, Bortezomib, Bosutinib, Brivanib, Cabozantinib, Cediranib, Crenolanib, Crizotinib, Dabrafenib, Dacomitinib, Danusertib, Dasatinib, Dovitinib, Erlotinib, Foretinib, Ganetespib, Gefitinib, Ibrutinib, Icotinib, Imatinib, Iniparib, Lapatinib, Lenvatinib, Linifanib, Linsitinib, Masitinib, Momelotinib, Motesanib, Neratinib Nilotinib, Niraparib, Oprozomib, Olaparib, Pazopanib, Pictiliisib, Ponatinib, Quizartinib, Regorafenib, Rigosertib, Rucaparib, Ruxolitinib, Saracatinib, Saridegib, Sorafenib, Sunitinib, Telatinib, Tivantinib, Tivozanib, Tofacitinib, Trametinib, Vandetanib, Veliparib, Vemurafenib, Erivedge, Volasertib, Alemtuzumab, Bevacizumab, Brentuximab Vedotin, Catumaxomab, Cetuximab, Denosumab, Gemtuzumab, Ipilimumab, Nimotuzumab, Ofatumumab, Panitumumab, Rituximab, Tositumomab, Trastuzumab, PI3K inhibitors, CSF1R inhibitors, A2A and/or A2B receptor antagonists, IDO inhibitors, anti-PD-1 antibodies, anti-PD-L1 antibodies, LAG3 antibodies, TIM-3 antibodies, and anti-CTLA-4 antibodies, or any combination thereof.

14. A compound according to any one of claims 1 to 11 or a pharmaceutical composition according to claim 12 or 13 for use in the prevention and/or treatment of tumors, cancer, viral infections, organ transplant rejection, neurodegenerative diseases, attention-related diseases, or autoimmune diseases.

15. The compound or pharmaceutical composition for use according to claim 14, wherein the tumor or cancer is selected from the group consisting of skin cancer, bladder cancer, ovarian cancer, breast cancer, gastric cancer, pancreatic cancer, prostate cancer, colon cancer, lung cancer, bone cancer, brain cancer, neurocytoma, rectal cancer, colon cancer, familial adenomatous polyposis cancer, hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, renal cancer, carcinoma of renal parenchyma, ovarian cancer, cervical cancer, corpus carcinoma, endometrial cancer, choriocarcinoma, pancreatic cancer, prostate cancer, testicular cancer, carcinoma of urinary system, melanoma, brain tumors such as glioblastoma, astrocytoma, meningioma, medulloblastoma and peripheral neuroectodermal tumors, Hodgkin's lymphoma, non-Hodgkin's lymphoma, Burkitt's lymphoma, acute lymphoblastic leukemia (ALL), chronic lymphocytic leukemia (CLL), acute myelogenous leukemia (AML), chronic myelogenous leukemia (CML), adult T-cell leukemia lymphoma, diffuse large B-cell lymphoma (DLBCL), hepatocellular carcinoma, gallbladder carcinoma, bronchial carcinoma, small cell lung carcinoma, non-small cell lung carcinoma, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma, or plasmacytoma.

## Patentansprüche

1. Verbindung mit einer Struktur der Formel (I) oder (II), 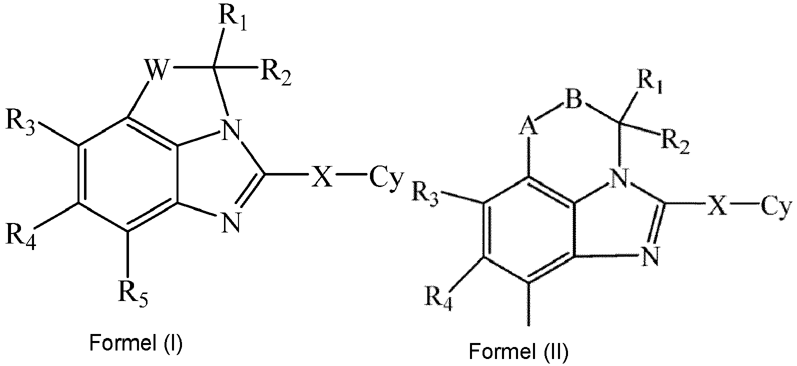
wobei W für (CR^{a}R^{a'})ₘ steht, wobei ein beliebiges CR^{a}R^{a'} optional durch 0, 1 oder 2 O, S oder NR^{b} substituiert ist;
wobei A und B jeweils unabhängig für CR^{a}R^{a'}, NR^{b}, O oder S stehen;
R₁ und R₂ jeweils unabhängig aus Wasserstoff, Halogen, Hydroxy, Amino, Mercapto, C₁-C₆ Alkyl, C₁-C₆ Alkoxy, C₁-C₆ Alkylthio, C₁-C₆ Alkylamino, Di(C₁-C₆ Alkyl)amino, C₂-C₆ Alkenyl, C₂-C₆ Alkinyl, -(C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6- bis 12-gliedriges Aryl) und -(C₀-C₆ Alkylen)-(5- bis 12-gliedriges Heteroaryl) ausgewählt sind, oder R1 und R2 zusammen mit dazu benachbarten Atomen cyclisiert sind, um einen 3- bis 6-gliedrigen Ring zu bilden, der wahlweise 0, 1 oder 2 Heteroatome, ausgewählt aus O, N und S, enthält;
R₃, R₄ und R₅ jeweils unabhängig aus Wasserstoff, Halogen, Cyano, C₁-C₆ Alkyl, C₂-C₆ Alkenyl, C₂-C₆ Alkinyl, -(C₀-C₆ Alkylen)-(C₃-C₆ Cycloalkyl), -(C₀-C₆ Alkylen)-(4- bis 7-gliedriges Heterocycloalkyl), -OR^{c}, -NR^{c}R^{c'}, -OC(O)R^{c'}, -C(O)R^{c}, -CO₂R^{c}, -CON(R^{c})(R^{c'}), -C(=NR^{c})N(R^{c'})(R^{c"}), -NHC(O)R^{c}, -NHS(O)₂R^{c}-, -NHS(O)R^{c}-, -SO₂R^{c}, -SO₂NR^{c}R^{c'}, -(C₀-C₆ Alkylen)-(6- bis 12-gliedriges Aryl), und -(C₀-C₆ Alkylen)-(5- bis 12-gliedriges Heteroaryl) ausgewählt sind;
oder R₃ und R₄ miteinander cyclisiert sind, um einen 5- bis 8-gliedrigen Ring zu bilden, der wahlweise 0, 1, 2, 3 oder 4 Heteroatome, ausgewählt aus O, S und N, enthält;
oder R₄ und R₅ zusammen cyclisiert sind, um einen 5- bis 8-gliedrigen Ring zu bilden, der wahlweise 0, 1, 2, 3 oder 4 Heteroatome, ausgewählt aus O, S und N, enthält;
X für -NR^{d}C(O)-, oder -NR^{d}C(=NR^{d'})- steht;
Cy für 6- bis 12-gliedriges Aryl oder 5- bis 12-gliedriges Heteroaryl steht;
m für eine ganze Zahl von 1, 2 oder 3 steht;
R^{a} und R^{a'} jeweils unabhängig für Wasserstoff, Halogen, Hydroxy, C₁-C₆ Alkyl, C₁-C₆ Alkylthio, -(C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6- bis 12-gliedriges Aryl), -(C₀-C₆ Alkylen)-(5- bis 12-gliedriges Heteroaryl), -NR^{e}R^{e'}, -NR^{e}COR^{e'}, -NR^{e}SO₂R^{e'}, -OR^{e} oder-OCOR^{e} stehen, oder R^{a} und R^{a'} zusammen mit dazu benachbarten Atomen zu einem 3- bis 6-gliedrigen Ring cyclisiert sind, der wahlweise 0, 1 oder 2 Heteroatome, ausgewählt aus O, N und S, enthält; oder ein beliebiges CR^{a}R^{a'} zusammengenommen wird, um -C=O zu bilden;
R^{b} jeweils unabhängig für Wasserstoff, C₁-C₆ Alkyl, -(C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6-bis 12-gliedriges Aryl), -(C₀-C₆ Alkylen)-(5- bis 12-gliedriges Heteroaryl), -C(O)R^{f}, -SO₂R^{f}, -SOR^{f}, -C(O)OR^{f} oder -C(O)NR^{f}R^{f'} steht;
R^{c}, R^{c'}, R^{c"}, R^{d}, R^{d'}, R^{e}, R^{e'}, R^{f}, und R^{f'} jeweils unabhängig für Wasserstoff, C₁-C₆ Alkyl, -(C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6- bis 12-gliedriges Aryl) oder -(C₀-C₆ Alkylen)-(5- bis 12-gliedriges Heteroaryl) stehen, oder wenn die oben erwähnten Substituenten gemeinsam an ein einziges N-Atom gebunden sind, die wahlweise mit dem gebundenen N-Atom zu einem 3- bis 8-gliedrigen Ring cyclisiert sind;
wobei die oben erwähnten Alkyl, Alkylen, Aryl, Heteroaryl, Ring, Cycloalkyl, Heterocycloalkyl, Alkenyl, Alkinyl und Alkoxy jeweils wahlweise unabhängig durch 0, 1, 2, 3 oder 4 Substituenten substituiert sind, die aus den folgenden Gruppen ausgewählt sind, die bestehen aus: Halogen, Hydroxyl, Cyano, Carboxyl, C₁-C₆ Alkyl, C₁-C₆ Halogenalkyl, Sulfo, -OR^{g}, -SR^{g}, -NR^{g}R^{g'}, -NR^{g}COR^{g'}, -NR^{g}COOR^{g'}, -COR^{g}, -CO₂R^{g}, - SOR^{g}, -SO₂R^{g}, -OCONR^{g}R^{g'}-, -OCOR^{g}, -CONR^{g}R^{g'}, -NR^{g}SO₂R^{g'}, -SO₂NR^{g}R^{g'}, und - OP(O)(OR^{g}R^{g'})₂;
oder für das Aryl und das Heteroaryl, oder wenn die Anzahl der Substituenten 2 beträgt, die benachbarten zwei Substituenten wahlweise auch miteinander zu einem 5-bis 6-gliedrigen gesättigten oder ungesättigten Carbocyclus oder Heterocyclus cyclisiert sind, wobei der Heterocyclus ein Ring ist, der 0, 1, 2, 3 oder 4 Heteroatome, ausgewählt aus O, S und N, enthält;
wobei R^{g} und R^{g'} jeweils unabhängig aus Wasserstoff oder den folgenden Gruppen bestehen, die wahlweise durch 0, 1, 2, 3 oder 4 Gruppen substituiert sind, die ausgewählt sind aus Hydroxy, Halogen, Nitro, C₁-C₆ Alkyl, Halogen(C₁-C₆ Alkyl), Amino, Sulfonyl, Cyano, C₁-C₆ Alkoxy, C₁-C₆ Alkylthio, C₁-C₆ Amino und Di(C₁-C₆ Alkyl)amino: C₁-C₆ Alkyl, -(C₀-C₆ Alkylen)-(C₃-C₆ Cycloalkyl), -(C₀-C₆ Alkylen)-O-(C₁-C₆ Alkyl), -(C₀-C₆ Alkylen)-O-CO (C₁-C₆ Alkyl), -(C₀-C₆ Alkylen)-C(O)O (C₁-C₆ Alkyl), -(C₀-C₆ Alkylen)-(6- bis 12-gliedriges Aryl), -(C₀-C₆ Alkylen)-(5- bis 12-gliedriges Heteroaryl), -(C₂-C₆ Alkenylen)-(6-bis 12-gliedriges Aryl), -(C₂-C₆ Alkenylen)-(5- bis 12-gliedriges Heteroaryl), -O-(C₀-C₆ Alkylen)-(6- bis 12-gliedriges Aryl), -O-(C₀-C₆ Alkylen)-(5- bis 12-gliedriges Heteroaryl), - O-(C₂-C₆ Alkenylen)-(6- bis 12-gliedriges Aryl), -O-(C₂-C₆ Alkenylen)-(5- bis 12-gliedriges Heteroaryl), -(C₀-C₆ Alkylen)-O-(6- bis 12-gliedriges Aryl), -(C₀-C₆ Alkylen)-O-(5- bis 12-gliedriges Heteroaryl), -(C₂-C₆ Alkenylen)-O-(6- bis 12-gliedriges Aryl), oder -(C₂-C₆ Alkenylen)-O-(5- bis 12-gliedriges Heteroaryl);
wobei das 6- bis 12-gliedrige Aryl vorzugsweise Phenyl ist; das 5- bis 12-gliedrige Heteroaryl vorzugsweise Pyridyl, Imidazolyl oder Pyrazolyl ist; oder für das oben erwähnte 6- bis 12-gliedrige Aryl oder 5- bis 12-gliedrige Heteroaryl, wenn die Anzahl der Substituenten 2 ist, die benachbarten zwei Substituenten auch miteinander zu einem 5-bis 6-gliedrigen gesättigten oder ungesättigten Carbocyclus oder Heterocyclus cyclisiert sein können;
mit der Maßgabe, dass die folgenden Verbindungen ausgeschlossen sind:

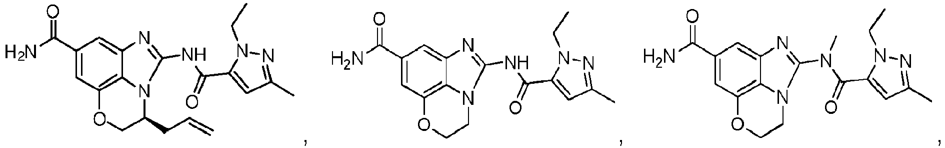

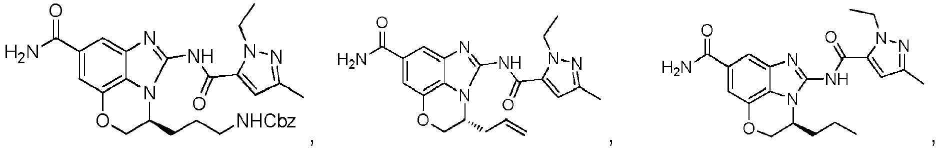

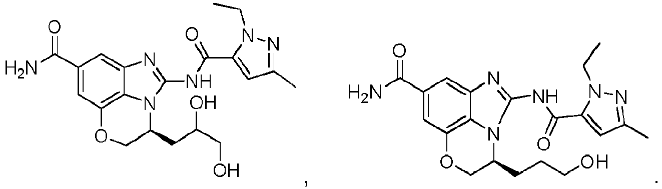

2. Verbindung nach Anspruch 1 mit einer Struktur der Formel (III) oder (IV), 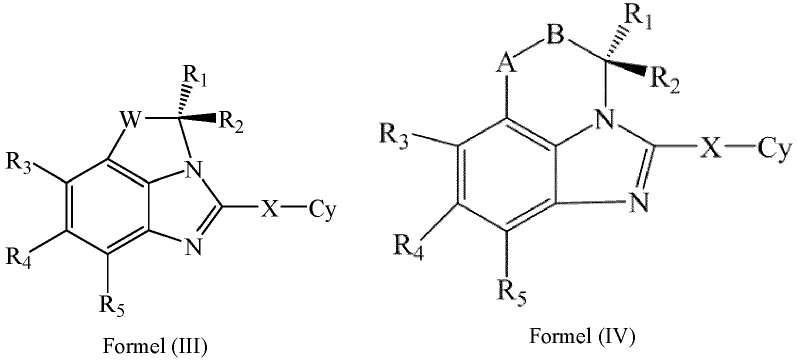 wobei R₁, R₃, R₄, R₅, W, X, Cy, A und B die in Anspruch 1 definierte Bedeutung haben; R₂ für Wasserstoff oder C₁-C₆ Alkyl steht, und R₁ und R₂ für verschiedene Substituenten stehen.

3. Verbindung nach Anspruch 1 oder 2, wobei A für O steht und B für CR^{a}R^{a'} steht.

4. Verbindung nach einem der Ansprüche 1 bis 3, wobei R₄ für -CONR^{c}R^{c'} steht, und R^{c} und R^{c'} unabhängig für Wasserstoff oder C₁-C₆ Alkyl stehen;
wobei X für -NR^{d}C(O)- steht, und R^{d} für Wasserstoff oder C₁-C₆ Alkyl steht; oder
wobei das Cy jeweils unabhängig ausgewählt ist aus Phenyl, Pyridyl, Pyrazolyl, Pyrimidinyl, Pyrazinyl, Furanyl, Thiazolyl, Oxazolyl, Imidazolyl, Thienyl, Triazolyl und Tetrazolyl; vorzugsweise Pyrazolyl, Imidazolyl, Oxazolyl, Triazolyl und Tetrazolyl; vorzugsweise Imidazolyl; und wobei wahlweise das Cy jeweils unabhängig substituiert durch 0, 1, 2, 3 oder 4 Substituenten ist, ausgewählt aus den folgenden Gruppen, bestehend aus: Halogen, Hydroxyl, Cyano, Carboxyl, C₁-C₆ Alkyl, C₁-C₆ Halogenalkyl, Sulfo, C₁-C₆ Alkoxy, -Amino, Nitro, (C₁-C₆ Alkyl)amino und Di(C₁-C₆ Alkyl)amino.

5. Verbindung nach einem der Ansprüche 1 bis 4, wobei R1 aus C₁-C₆ Alkyl, C₂-C₆ Alkenyl, -(C₀-C₆ Alkylen)-(C₃-C₆ Cycloalkyl), -(C₀-C₆ Alkylen)-(4- bis 7-gliedriges Heterocycloalkyl), oder -(C₀-C₆ Alkylen)-(6- bis 12-gliedriges Aryl) besteht; vorzugsweise C₁-C₆ Alkyl, C₂-C₆ Alkenyl, -(C₀-C₆ Alkylen)-(C₃-C₆ Cycloalkyl); weiter vorzugsweise C₁-C₆Alkyl und C₂-C₆ Alkenyl; und R₁ wahlweise durch einen Substituenten, ausgewählt aus den folgenden Substituenten, substituiert ist: -NR^{g}COR^{g'}; und R^{g} aus Wasserstoff oder C₁-C₆ Alkyl besteht; R^{g'} aus den folgenden Gruppen besteht, die durch 0, 1, 2, 3 oder 4 Substituenten substituiert sind, die aus Hydroxy, Halogen, Nitro, C₁-C₆ Alkyl, Halogen (C₁-C₆ Alkyl), Amino, Sulfonyl, Cyano, C₁-C₆ Alkoxy, C₁-C₆ Alkylthio, C₁-C₆ Amino und Di(C₁-C₆ Alkyl)amino ausgewählt sind: C₁-C₆ Alkyl, -(C₀-C₆ Alkylen)-(C₃-C₆ Cycloalkyl), - (C₀-C₆ Alkylen)-O-(C₁-C₆ Alkyl), -(C₀-C₆ Alkylen)-O-CO (C₁-C₆ Alkyl), -(C₀-C₆ Alkylen)-C(O)O (C₁-C₆ Alkyl), -(C₀-C₆ Alkylen)-(6- bis 12-gliedriges Aryl), -(C₀-C₆ Alkylen)-(5- bis 12-gliedriges Heteroaryl), -(C₂-C₆ Alkenylen)-(6- bis 12-gliedriges Aryl), -(C₂-C₆ Alkenylen)-(5- bis 12-gliedriges Heteroaryl), -O-(C₀-C₆ Alkylen)-(6- bis 12-gliedriges Aryl), -O-(C₀-C₆ Alkylen)-(5- bis 12-gliedriges Heteroaryl), -O-(C₂-C₆ Alkenylen)-(6- bis 12-gliedriges Aryl), -O-(C₂-C₆ Alkenylen)-(5- bis 12-gliedriges Heteroaryl), -(C₀-C₆ Alkylen)-O-(6- bis 12-gliedriges Aryl), -(C₀-C₆ Alkylen)-O-(5- bis 12-gliedriges Heteroaryl), -(C₂-C₆ Alkenylen)-O-(6- bis 12-gliedriges Aryl), oder -(C₂-C₆ Alkenylen)-O-(5- bis 12-gliedriges Heteroaryl);
wobei das 6- bis 12-gliedrige Aryl vorzugsweise Phenyl ist; das 5- bis 12-gliedrige Heteroaryl vorzugsweise Pyridyl, Imidazolyl oder Pyrazolyl ist; oder für das oben erwähnte 6- bis 12-gliedrige Aryl oder 5- bis 12-gliedrige Heteroaryl, wenn die Anzahl der Substituenten 2 ist, die benachbarten zwei Substituenten miteinander wahlweise auch zu einem 5- bis 6-gliedrigen gesättigten oder ungesättigten Carbocyclus oder Heterocyclus cyclisiert sind.

6. Verbindung nach einem der Ansprüche 1 bis 5, wobei R₁ aus -(C₁-C₆ Alkylen)-NR^{g}COR^{g'} und -(C₂-C₆ Alkenylen)-NR^{g}COR^{g'} besteht, wobei R^{g} aus Wasserstoff oder C₁-C₆ Alkyl besteht; R^{g'} aus den folgenden Gruppen besteht, die durch 0, 1, 2, 3 oder 4 Substituenten substituiert sind, die aus Hydroxy, Halogen, Nitro,C₁-C₆ Alkyl, Halogen(C₁-C₆ Alkyl), Amino, Sulfonyl, Cyano, C₁-C₆ Alkoxy, C₁-C₆ Alkylthio, C₁-C₆ Amino und Di(C₁-C₆ Alkyl)amino ausgewählt sind: -(C₀-C₆ Alkylen)-(6- bis 12-gliedriges Aryl), -(C₀-C₆ Alkylen)-(5- bis 12-gliedriges Heteroaryl), -(C₂-C₆ Alkenylen)-(6- bis 12-gliedriges Aryl), - (C₂-C₆ Alkenylen)-(5- bis 12-gliedriges Heteroaryl), -O-(C₀-C₆ Alkylen)-(6- bis 12-gliedriges Aryl), -O-(C₀-C₆ Alkylen)-(5- bis 12-gliedriges Heteroaryl), -O-(C₂-C₆ Alkenylen)-(6- bis 12-gliedriges Aryl), -O-(C₂-C₆ Alkenylen)-(5- bis 12-gliedriges Heteroaryl), -(C₀-C₆ Alkylen)-O-(6- bis 12-gliedriges Aryl), -(C₀-C₆ Alkylen)-O-(5- bis 12-gliedriges Heteroaryl), -(C₂-C₆ Alkenylen)-O-(6- bis 12-gliedriges Aryl), oder -(C₂-C₆ Alkenylen)-O-(5- bis 12-gliedriges Heteroaryl);
wobei das 6- bis 12-gliedrige Aryl vorzugsweise Phenyl ist; das 5- bis 12-gliedrige Heteroaryl vorzugsweise Pyridyl ist; oder für das oben erwähnte 6- bis 12-gliedrige Aryl oder 5- bis 12-gliedrige Heteroaryl, wenn die Anzahl der Substituenten 2 ist, die benachbarten zwei Substituenten wahlweise auch miteinander zu einem 5- bis 6-gliedrigen gesättigten oder ungesättigten Carbocyclus oder Heterocyclus cyclisiert sind; weiter vorzugsweise -O-(C₁-C₆ Alkylen)-phenyl, -O-(C₁-C₆ Alkylen)-pyridyl, -(C₁-C₆ Alkylen)-O-phenyl, -(C₁-C₆ Alkylen)-O-pyridyl, -(C₁-C₆ Alkylen)-phenyl, -(C₁-C₆ Alkylen)-pyridyl, -(C₂-C₆ Alkenylen)-phenyl, oder -(C₂-C₆ Alkenylen)-pyridyl; und das Phenyl und das Pyridyl wahlweise unabhängig durch 0, 1, 2, 3 oder 4 Substituenten substituiert sind, die aus Hydroxy, Halogen, Amino, Sulfonyl, Cyano, Nitro, C₁-C₆ Alkoxy und C₁-C₆ Halogenalkyl ausgewählt sind.

7. Verbindung nach einem der Ansprüche 1 bis 6, wobei R₂ für Wasserstoff oder C₁-C₆ Alkyl steht; oder
wobei R₃ und R₅ jeweils unabhängig für Wasserstoff, Halogen oder C₁-C₆ Alkyl stehen.

8. Verbindung nach Anspruch 1, die eine Struktur der Formel (V) aufweist, 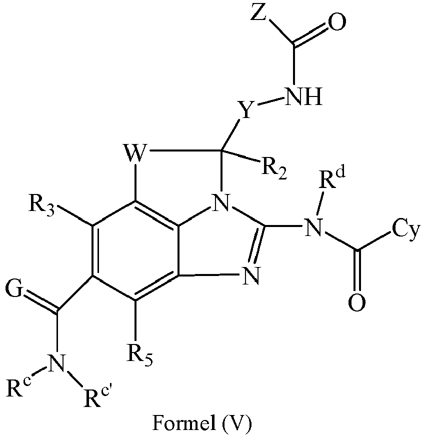
wobei Wfür (CR^{a}R^{a'})ₘ steht, wobei ein beliebiges CR^{a}R^{a'} wahlweise durch 0, 1 oder 2 O, S oder NR^{b} substituiert ist;
R₂ unabhängig für Wasserstoff, Halogen, Hydroxy, Amino, Mercapto, C₁-C₆ Alkyl, C₁-C₆ Alkoxy, C₁-C₆ Alkylthio, C₁-C₆ Alkylamino, Di(C₁-C₆ Alkyl)amino, C₂-C₆ Alkenyl, C₂-C₆ Alkinyl, -(C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6- bis 12-gliedriges Aryl), oder -(C₀-C₆ Alkylen)-(5-bis 12-gliedriges Heteroaryl) steht;
R₃ und R₅ jeweils unabhängig aus Wasserstoff, Halogen, Cyano, C₁-C₆ Alkyl, C₂-C₆ Alkenyl, C₂-C₆ Alkinyl, -(C₀-C₆ Alkylen)-(C₃-C₆ Cycloalkyl), -OR^{c}, -NR^{c}R^{c'}, -OC(O)R^{c'}, - C(O)R^{c}, -CO₂R^{c}, -CON(R^{c})(R^{c'}), -C(=NR^{c})N(R^{c'})(R^{c"}), -NHC(O)R^{c}, -NHS(O)₂R^{c}-, - NHS(O)R^{c}-, -SO₂R^{c}, -SO₂NR^{c}R^{c'}, -(C₀-C₆ Alkylen)-(4- bis 7-gliedriges Heterocycloalkyl), -(C₀-C₆ Alkylen)-(6- bis 12-gliedriges Aryl), und -(C₀-C₆ Alkylen)-(5- bis 12-gliedriges Heteroaryl) ausgewählt sind;
Cy für 6- bis 12-gliedriges Aryl oder einen 5- bis 12-gliedriges Heteroaryl steht;
m für eine ganze Zahl von 1, 2 oder 3 steht;
R^{a} und R^{a'} jeweils unabhängig für Wasserstoff, Halogen, Hydroxy, C₁-C₆ Alkyl, C₁-C₆ Alkylthio, -(C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6- bis 12-gliedriges Aryl), -(C₀-C₆ Alkylen)-(5- bis 12-gliedriges Heteroaryl), -NR^{e}R^{e'}, -NR^{e}COR^{e'}, -NR^{e}SO₂R^{e'}, -OR^{e} oder-OCOR^{e} stehen, oder R^{a} und R^{a'} zusammen mit den dazu benachbarten Atomen zu einem 3- bis 6-gliedrigen Ring cyclisiert sind, der wahlweise 0, 1 oder 2 Heteroatome, ausgewählt aus O, N und S, enthält; oder ein beliebiges CR^{a}R^{a'} zusammengenommen wird, um -C=O zu bilden;
R^{b} jeweils unabhängig für Wasserstoff, C₁-C₆ Alkyl, -(C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6-bis 12-gliedriges Aryl), -(C₀-C₆ Alkylen)-(5- bis 12-gliedriges Heteroaryl), -C(O)R^{f}, -SO₂R^{f}, -SOR^{f}, -C(O)OR^{f} oder -C(O)NR^{f}R^{f'} steht;
G für O oder NR^{c} steht;
R^{c}, R^{c'}, R^{c"}, R^{d}, R^{e}, R^{e'}, R^{f} und R^{f'} jeweils unabhängig für Wasserstoff, C₁-C₆ Alkyl, - (C₀-C₆ Alkylen)-(C₃-C₆ Cycloalkyl), -(C₀-C₆ Alkylen)-(4- bis 7-gliedriges Heterocycloalkyl), -(C₀-C₆ Alkylen)-(6- bis 12-gliedriges Aryl), oder -(C₀-C₆ Alkylen)-(5- bis 12-gliedriges Heteroaryl) stehen, oder wenn die oben erwähnten Substituenten gemeinsam an ein einzelnes N-Atom gebunden sind, die wahlweise mit dem gebundenen N-Atom zu einem 3- bis 8-gliedrigen Ring cyclisiert sind;
die oben erwähnten Alkyl, Alkylen, Aryl, Heteroaryl, Ring, Cycloalkyl, Heterocycloalkyl, Alkenyl, Alkinyl und Alkoxy jeweils wahlweise unabhängig durch 0, 1, 2, 3 oder 4 Substituenten substituiert sind, die aus den folgenden Gruppen ausgewählt sind, bestehend aus: Halogen, Oxo, Hydroxy, Cyano, Carboxy, C₁-C₆ Alkyl, C₁-C₆ Halogenalkyl, Sulfo, C₁-C₆ Alkoxy, -OR^{g}, -SR^{g}, -N(R^{g})(R^{g'}), -NR^{g}COR^{g'}, -NR^{g}COOR^{g'}, - COR^{g}, -CO₂R^{g}, -SOR^{g}, -SO₂R^{g}, -OCONR^{g}R^{g'}-, -OCOR^{g}, -CONR^{g}R^{g'}, -NR^{g}SO₂R^{g'}, - SO₂NR^{g}R^{g'} und -OP(O)(OR^{g}R^{g'})₂;
oder für das Aryl und das Heteroaryl, oder wenn die Anzahl der Substituenten 2 beträgt, die benachbarten zwei Substituenten wahlweise auch miteinander zu einem 5-bis 6-gliedrigen gesättigten oder ungesättigten Carbocyclus oder Heterocyclus cyclisiert sind, wobei der Heterocyclus ein Ring ist, der 0, 1, 2, 3 oder 4 Heteroatome, ausgewählt aus O, S und N, enthält;
wobei R^{g} und R^{g'} jeweils unabhängig für Wasserstoff oder die folgenden Gruppen stehen, die wahlweise durch 0, 1, 2, 3 oder 4 Gruppen, ausgewählt aus Hydroxy, Halogen, Nitro, C₁-C₆ Alkyl, Halogen (C₁-C₆ Alkyl), Amino, Sulfonyl, Cyano, C₁-C₆ Alkoxy, C₁-C₆ Alkylthio, C₁-C₆ Amino, Di(C₁-C₆ Alkyl)amino substituiert sind: C₁-C₆ Alkyl, -(C₀-C₆ Alkylen)-(C₃-C₆ Cycloalkyl), Halogen (C₁-C₆ Alkyl), -(C₀-C₆ Alkyl)-OH, -(C₀-C₆ Alkylen)-O-(C₁-C₆ Alkyl), -(C₀-C₆ Alkylen)-O-CO (C₁-C₆ Alkyl), -(C₀-C₆ Alkylen)-C(O)O (C₁-C₆ Alkyl), -(C₀-C₆ Alkylen)-(6- bis 12-gliedriges Aryl), -C₂-C₆ Alkenylen-( 6- bis 12-gliedriges Aryl), - (C₀-C₆ Alkylen)-O-(6- bis 12-gliedriges Aryl), -(C₀-C₆ Alkylen)-O-C₁-C₆ Alkyl, -O-(C₁-C₆ Alkylen)-(6- bis 12-gliedriges Aryl), -(C₀-C₆ Alkylen)-(5- bis 12-gliedriges Heteroaryl), -C₂-C₆ Alkenylen-(5- bis 12-gliedriges Heteroaryl), -(C₀-C₆ Alkylen)-O-(5- bis 12-gliedriges Heteroaryl), oder -O-(C₀-C₆ Alkylen)-(5- bis 12-gliedriges Heteroaryl); oder für das oben erwähnte 6- bis 12-gliedrige Aryl oder 5- bis 12-gliedrige Heteroaryl, wenn die Anzahl der Substituenten 2 beträgt, die benachbarten zwei Substituenten wahlweise auch miteinander zu einem 5- bis 6-gliedrigen gesättigten oder ungesättigten Carbocyclus oder Heterocyclus cyclisiert sind;
Y für die folgenden Gruppen steht, die wahlweise durch 0, 1, 2, 3 oder 4 Substituenten substituiert sind, die ausgewählt sind aus Hydroxy, Halogen, Nitro, C₁-C₆ Alkyl, Halogen(C₁-C₆ Alkyl), Amino, Sulfonyl, Cyano, C₁-C₆ Alkoxy, C₁-C₆ Alkylthio, C₁-C₆ Amino und Di(C₁-C₆ Alkyl)amino: -C₁-C₆ Alkylen-, -(C₀-C₆ Alkylen)-(C₃-C₆ Cycloalkyl)-(C₀-C₆ Alkylen), -(C₀-C₆ Alkylen)-(4- bis 7-gliedriges Heterocycloalkyl)-(C₀-C₆ Alkylen), (C₀-C₆ Alkylen)-(6- bis 12-gliedriges Aryl)-(C₀-C₆ Alkylen), -(C₀-C₆ Alkylen)-(5- bis 12-gliedriges Heteroaryl)-(C₀-C₆ Alkylen), oder -C₂-C₆ Alkenylen;
Z für die folgenden Gruppen steht, die wahlweise durch 0, 1, 2, 3 oder 4 Substituenten substituiert sind, die ausgewählt sind aus Hydroxy, Halogen, Nitro, C₁-C₆ Alkyl, Halogen(C₁-C₆ Alkyl), Amino, Sulfonyl, Cyano, C₁-C₆ Alkoxy, C₁-C₆ Alkylthio, C₁-C₆ Amino und Di(C₁-C₆ Alkyl)amino: C₁-C₆ Alkyl, -(C₀-C₆ Alkylen)-(C₃-C₆ Cycloalkyl), - (C₀-C₆ Alkylen)-O-(C₁-C₆ Alkyl), -(C₀-C₆ Alkylen)-O-CO(C₁-C₆ Alkyl), -(C₀-C₆ Alkylen)-C(O)O(C₁-C₆ Alkyl), -(C₀-C₆ Alkylen)-(6- bis 12-gliedriges Aryl), -(C₀-C₆ Alkylen)-(5- bis 12-gliedriges Heteroaryl), -(C₂-C₆ Alkenylen)-(6- bis 12-gliedriges Aryl), -(C₂-C₆ Alkenylen)-(5- bis 12-gliedriges Heteroaryl), -O-(C₀-C₆ Alkylen)-(6- bis 12-gliedriges Aryl), -O-(C₀-C₆ Alkylen)-(5- bis 12-gliedriges Heteroaryl), -O-(C₂-C₆ Alkenylen)-(6- bis 12-gliedriges Aryl), -O-(C₂-C₆ Alkenylen)-(5- bis 12-gliedriges Heteroaryl), -(C₀-C₆ Alkylen)-O-(6- bis 12-gliedriges Aryl), -(C₀-C₆ Alkylen)-O-(5- bis 12-gliedriges Heteroaryl), -(C₂-C₆ Alkenylen)-O-(6- bis 12-gliedriges Aryl), oder -(C₂-C₆ Alkenylen)-O-(5- bis 12-gliedriges Heteroaryl); oder für das oben erwähnte 6- bis 12-gliedrige Aryl oder 5- bis 12-gliedrige Heteroaryl, wenn die Anzahl der Substituenten 2 beträgt, die benachbarten zwei Substituenten wahlweise auch miteinander zu einem 5- bis 6-gliedrigen gesättigten oder ungesättigten Carbocyclus oder Heterocyclus cyclisiert sind;
wobei das 6- bis 12-gliedrige Aryl vorzugsweise Phenyl ist; das 5- bis 12-gliedrige Heteroaryl vorzugsweise Pyridyl, Imidazolyl oder Pyrazolyl ist; oder für das oben erwähnte 6- bis 12-gliedrige Aryl oder 5- bis 12-gliedrige Heteroaryl, wenn die Anzahl der Substituenten 2 ist, die benachbarten zwei Substituenten wahlweise auch zu einem 5-bis 6-gliedrigen gesättigten oder ungesättigten Carbocyclus oder Heterocyclus miteinander cyclisiert sind.

9. Verbindung nach Anspruch 8, die eine Struktur der Formel (VI) aufweist, 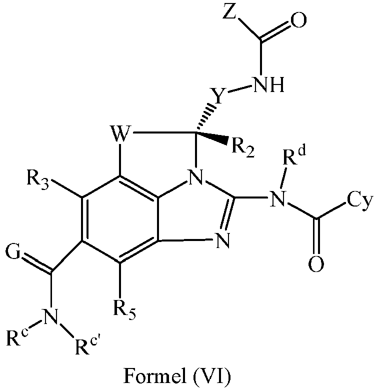 wobei R₂ ausgewählt ist aus Wasserstoff oder C₁-C₆ Alkyl, und W, R₃, R₅, R^{c}, R^{c'}, R^{d}, G, Z, Y und Cy alle wie in Anspruch 8 definiert sind.

10. Die Verbindung nach einem der Ansprüche 8 bis 9, wobei G aus O oder NH besteht;
wobei Y aus den folgenden Gruppen besteht, die durch 0, 1, 2, 3 oder 4 Substituenten, ausgewählt aus Hydroxy, Halogen und C₁-C₆ Alkyl, substituiert sind: -C₁-C₆ Alkylen, -C₂-C₆ Alkenylen oder -C₃-C₆ Cycloalkyl;
wobei W aus -CR^{a}R^{a'}-O, -O-CR^{a}R^{a'}-, -C(O)-NR^{b}-, oder -NR^{b}-C(O)- besteht, wobei R^{a}, R^{a'}, und R^{b} jeweils unabhängig für Wasserstoff, C₁-C₆ Alkyl oder C₃-C₆ Cycloalkyl stehen;
wobei Z aus -O-(C₀-C₆ Alkylen)-(6- bis 12-gliedriges Aryl), -(C₀-C₆ Alkylen)-(6- bis 12-gliedriges Aryl), -(C₂-C₆ Alkenylen)-(6- bis 12-gliedriges Aryl), -(C₀-C₆ Alkylen)-(5- bis 12-gliedriges Heteroaryl), -(C₂-C₆ Alkenylen)-(5- bis 12-gliedriges Heteroaryl), -O-(C₂-C₆ Alkenylen)-(6- bis 12-gliedriges Aryl), -(C₀-C₆ Alkylen)-O-(6- bis 12-gliedriges Aryl), -O-(C₀-C₆ Alkylen)-(5- bis 12-gliedriges Heteroaryl), -O-(C₂-C₆ Alkenylen)-(5- bis 12-gliedriges Heteroaryl), oder -(C₀-C₆ Alkylen)-O-(5- bis 12-gliedriges Heteroaryl) besteht, und wahlweise das 6- bis 12-gliedrige Aryl (vorzugsweise Phenyl) oder 5- bis 12-gliedrige Heteroaryl (vorzugsweise Pyridyl) jeweils unabhängig durch 0, 1, 2, 3 oder 4 Substituenten substituiert ist, die aus den folgenden Gruppen ausgewählt sind, bestehend aus: Halogen, Hydroxy, Nitro, C₁-C₆ Alkyl, Halogen (C₁-C₆ Alkyl), Amino, Sulfonyl, Cyano, C₁-C₆ Alkoxy, C₁-C₆ Alkylthio, C₁-C₆ Amino und Di(C₁-C₆ Alkyl)amino;
wobei R₂ für Wasserstoff oder C₁-C₆ Alkyl steht;
wobei R³ und R⁵ jeweils unabhängig für Halogen, Wasserstoff oder C₁-C₆ Alkyl stehen;
wobei R^{c} und R^{c'} für Wasserstoff oder C₁-C₆ Alkyl stehen;
wobei R^{d} für Wasserstoff oder C₁-C₆ Alkyl steht; oder
wobei Cy für Pyrazolyl steht und wahlweise durch 0, 1, 2 oder 3 C₁-C₆ Alkyl substituiert ist.

11. Verbindung nach Anspruch 1 mit einer Struktur, ausgewählt aus:
| No. | Struktur der Verbindung | No. | Struktur der Verbindung |
|---|---|---|---|
| 1 | 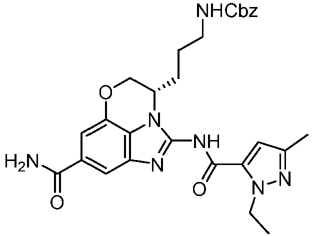 | 2 | |
| 3 | 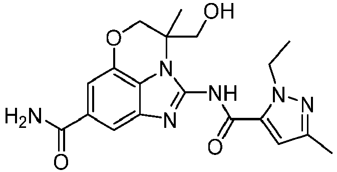 | 4 | 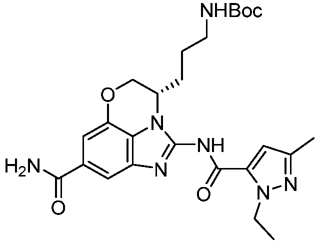 |
| 5 | 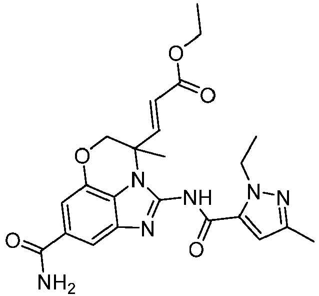 | 6 | 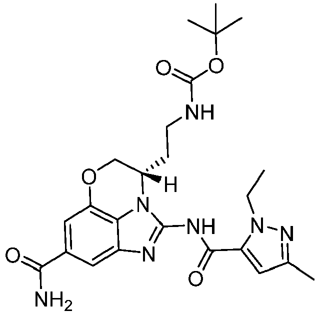 |
| 7 | 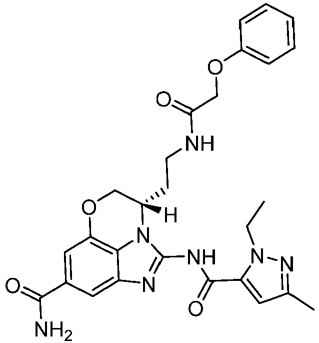 | 8 | 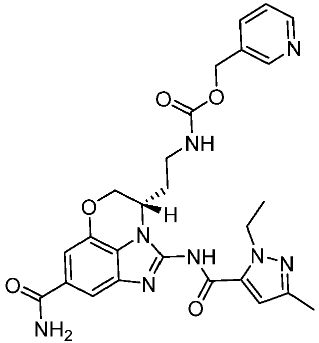 |
| 9 | 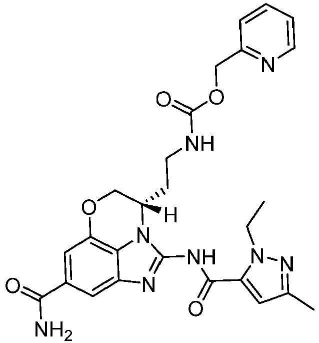 | 10 | 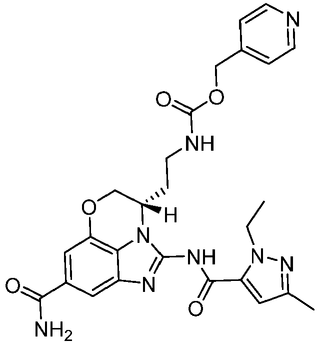 |
| 11 | 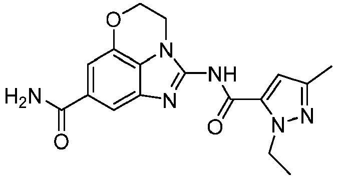 | 12 | 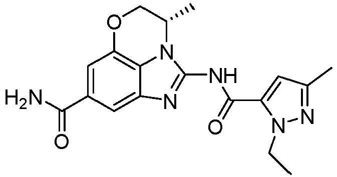 |
| 13 | 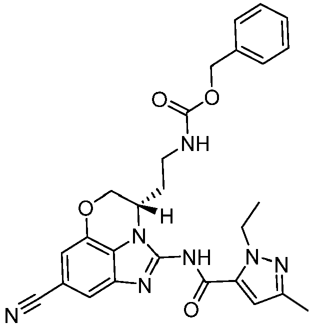 | 14 | 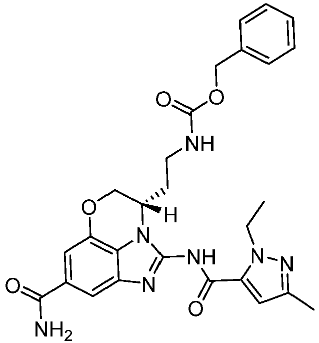 |
| 15 | 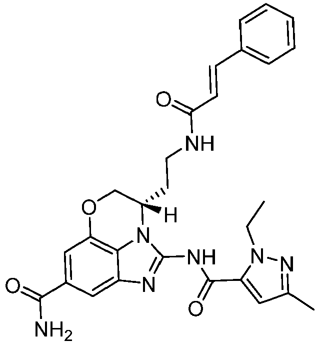 | 16 | 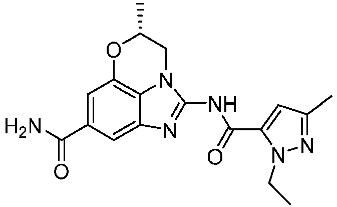 |
| 17 | 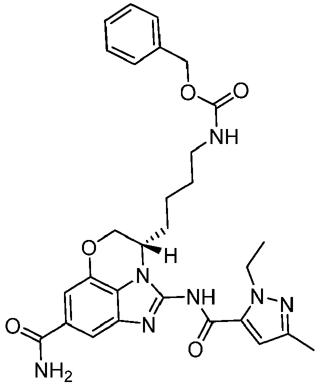 | 18 | 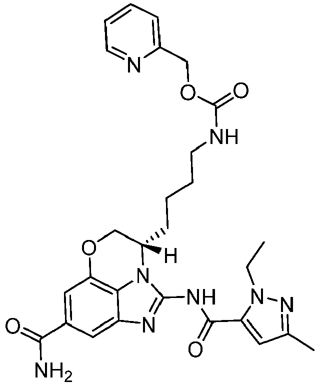 |
| 19 | 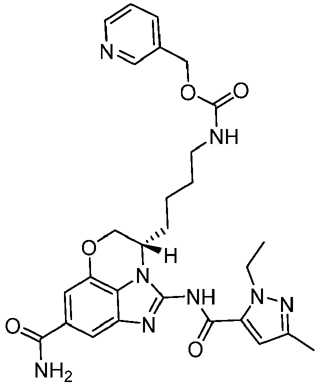 | 20 | 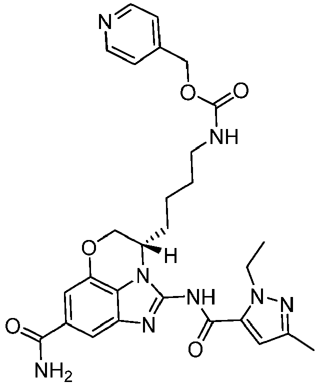 |
| 21 | 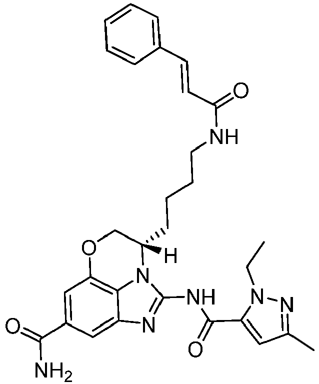 | 22 | 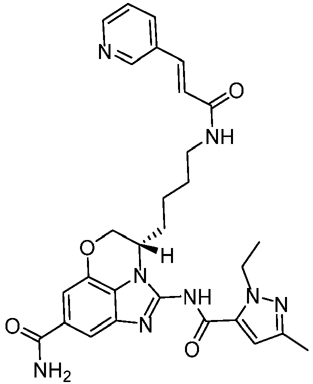 |
| 23 | 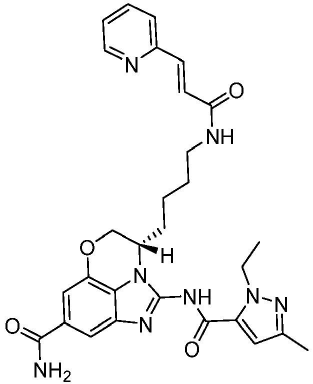 | 24 | 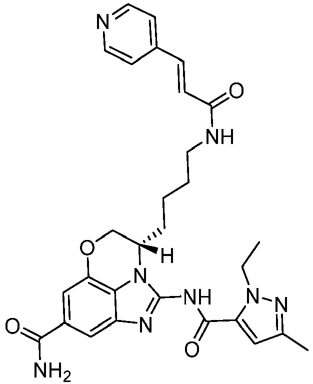 |
| 25 | 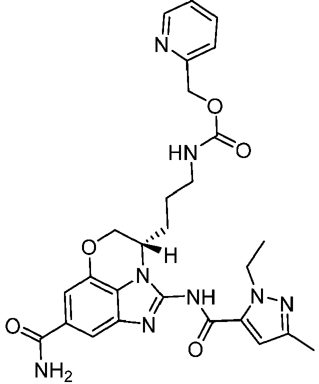 | 26 | 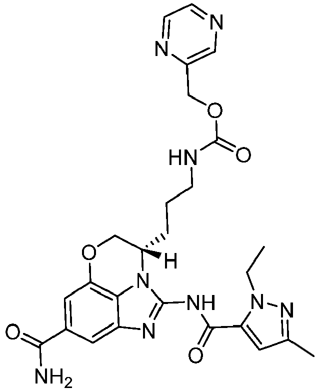 |
| 27 | 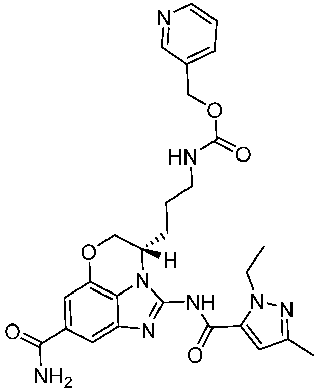 | 28 | 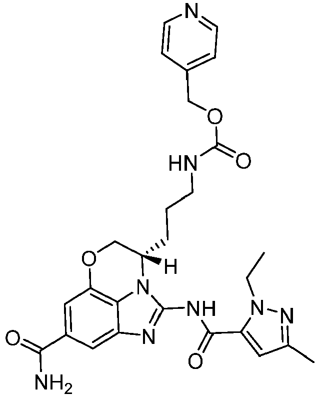 |
| 29 | 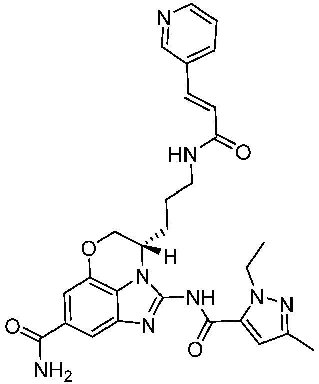 | 30 | 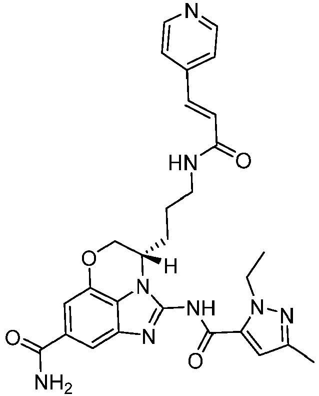 |
| 31 | 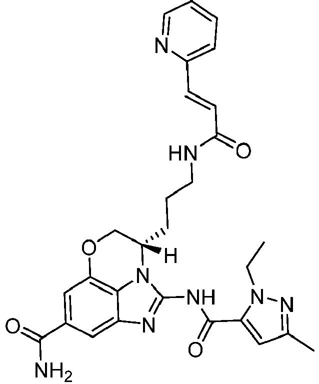 | 32 | 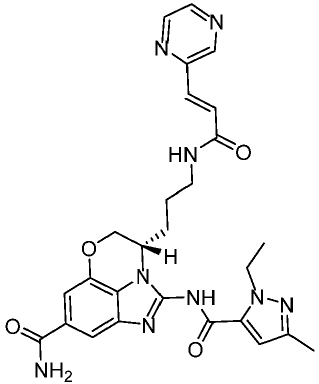 |
| 33 | 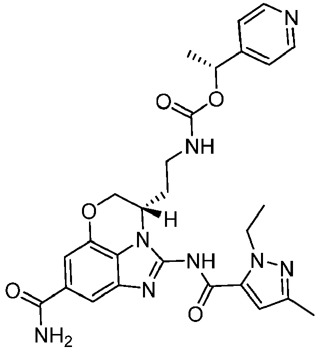 | 34 | 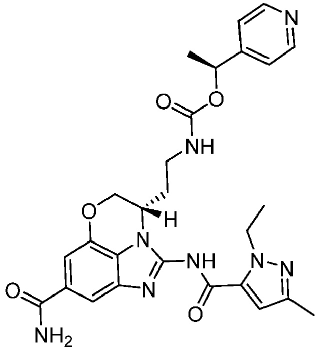 |
| 35 | 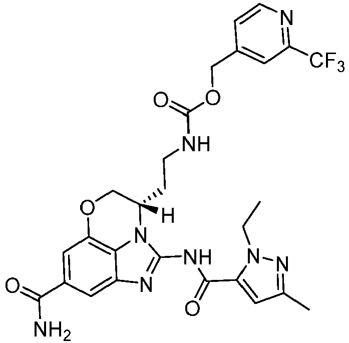 | 36 | 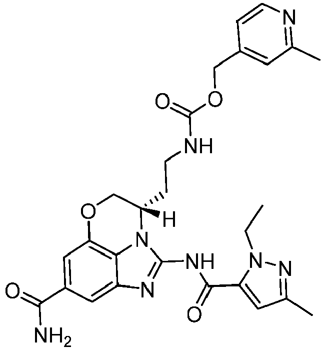 |
| 37 | 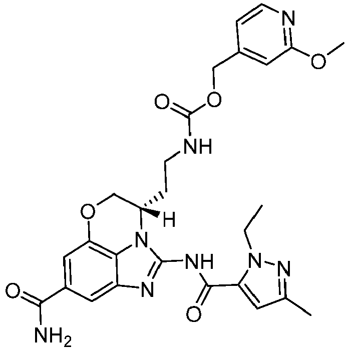 | 38 | 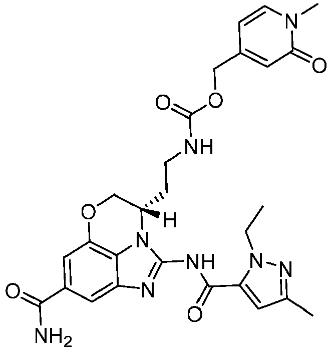 |
| 39 | 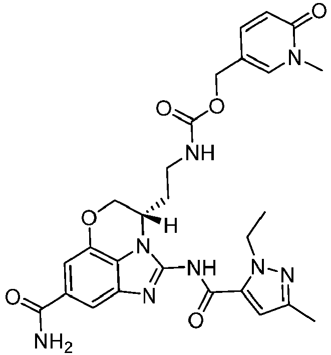 | 40 | 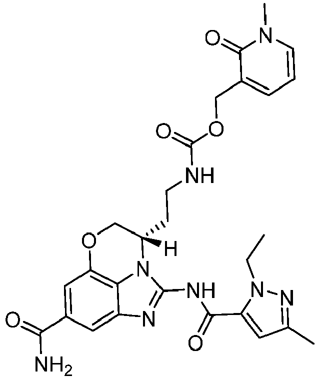 |
| 41 | 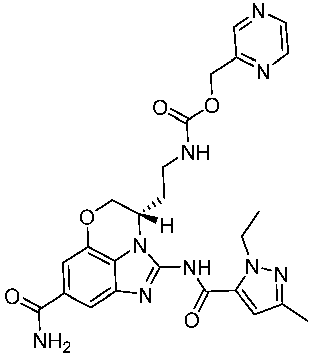 | 42 | 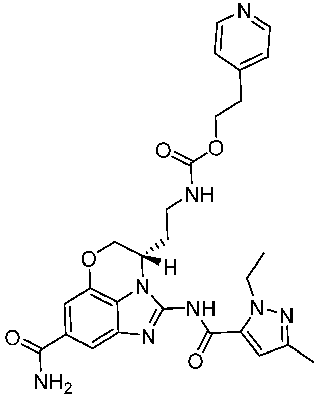 |
| 43 | 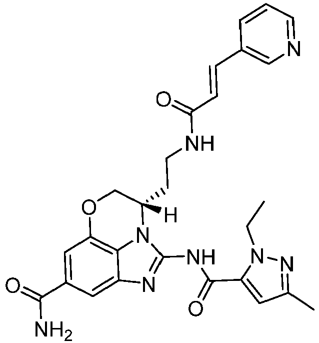 | 44 | 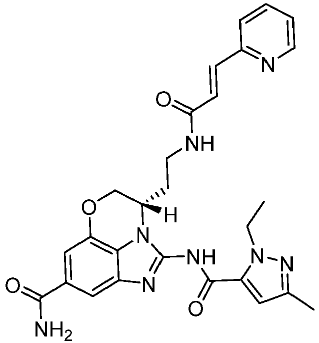 |
| 45 | 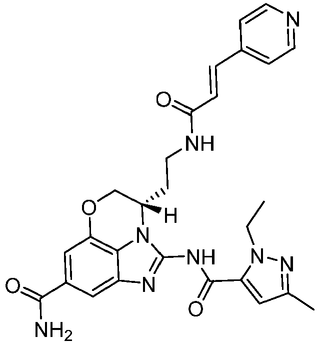 | 46 | 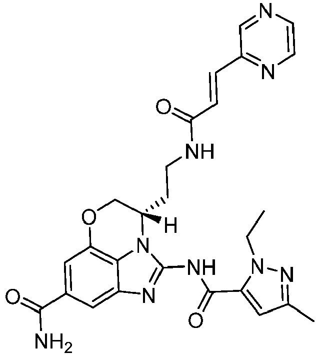 |
| 47 | 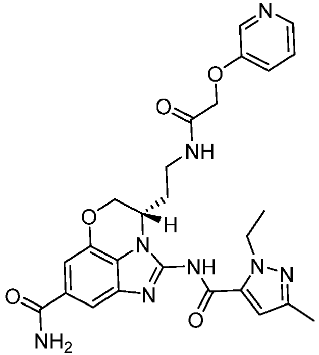 | 48 | 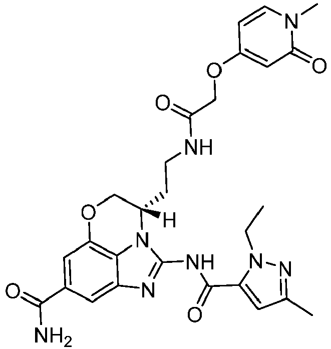 |
| 49 | 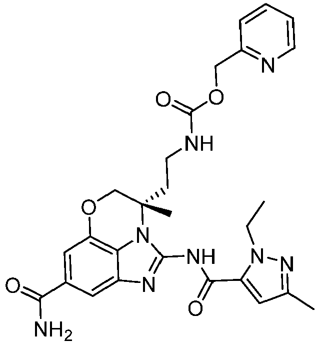 | 50 | 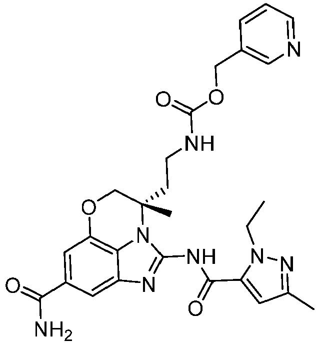 |
| 51 | 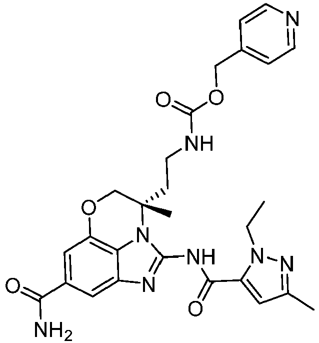 | 52 | 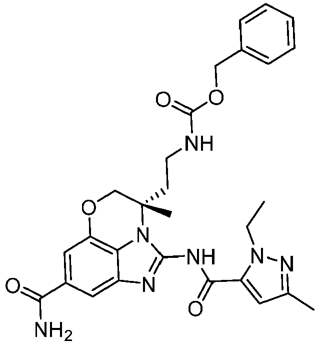 |
| 53 | 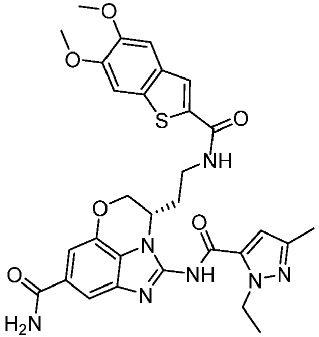 | 54 | 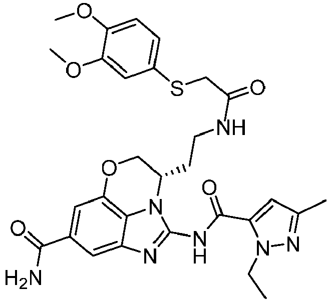 |
| 55 | 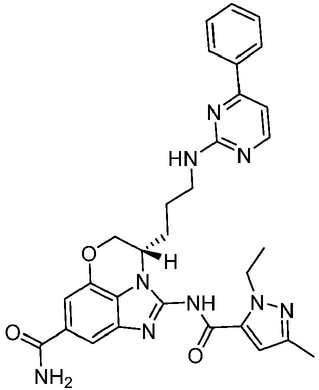 | 56 | 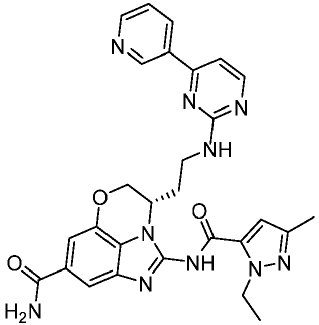 |
| 57 | 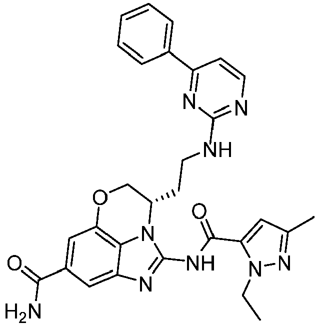 | 58 | 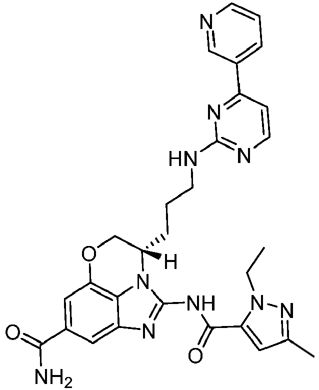 |
| 59 | 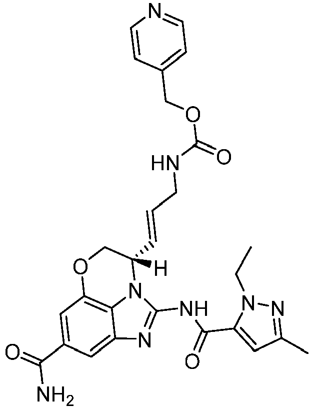 | 60 | 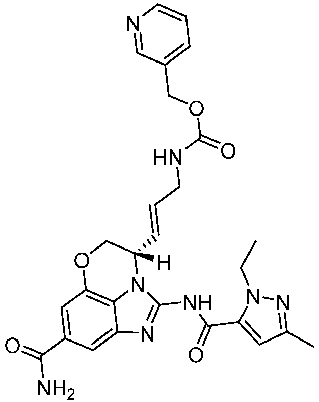 |
| 61 | 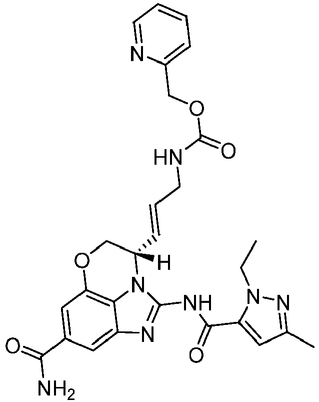 | 62 | 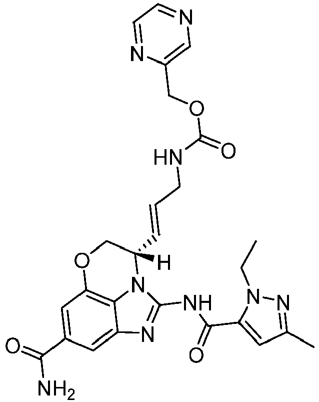 |
| 63 | 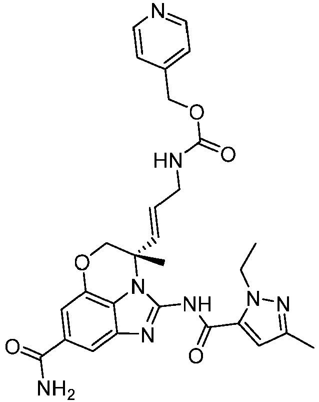 | 64 | 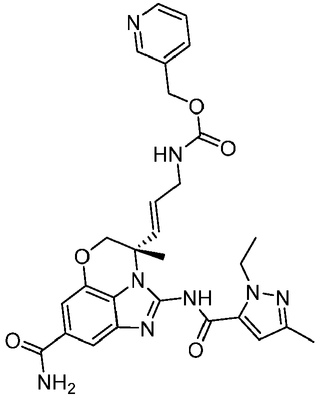 |
| 65 | 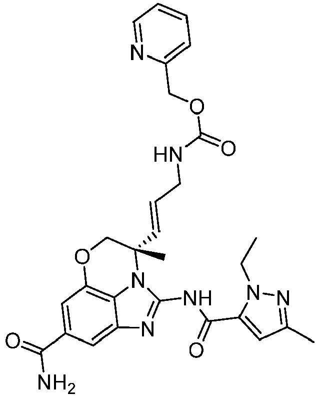 | 66 | 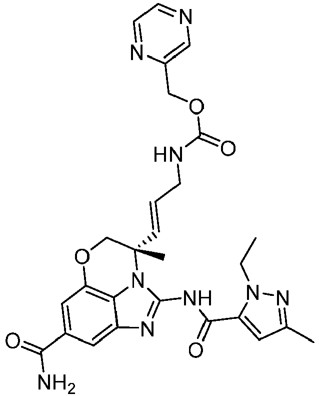 |
| 67 | 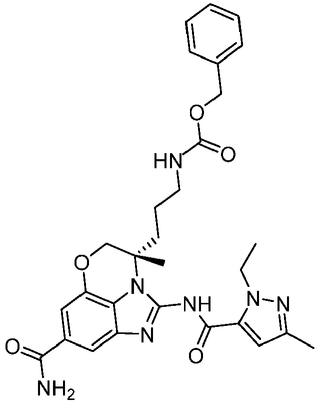 | 68 | 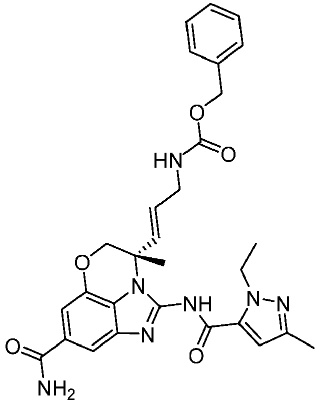 |
| 69 | 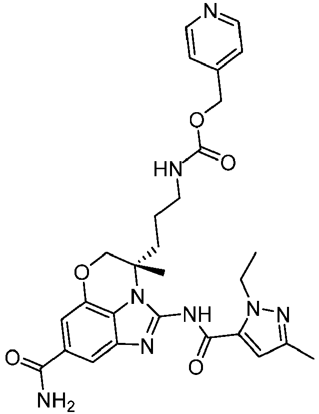 | 70 | 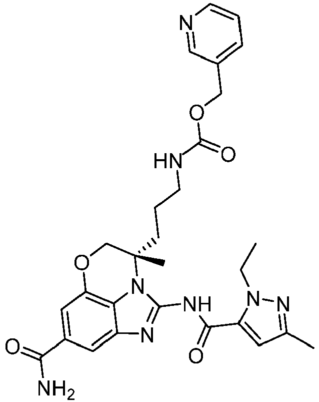 |
| 71 | 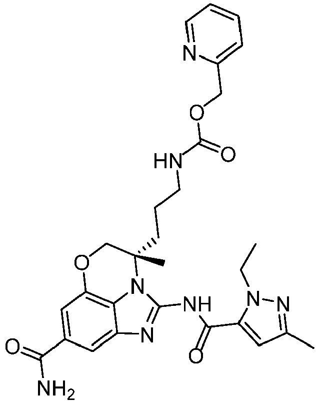 | 72 | 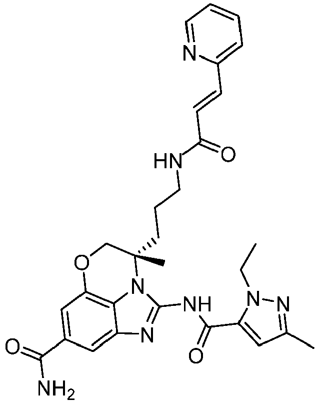 |
| 73 | 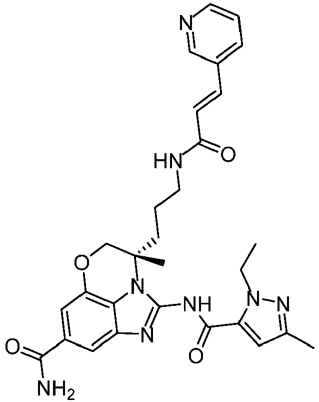 | 74 | 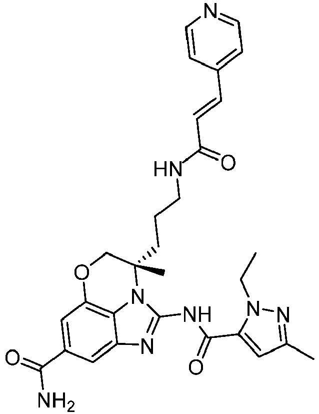 |
| 75 | 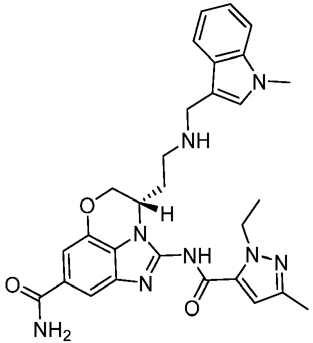 | 76 | 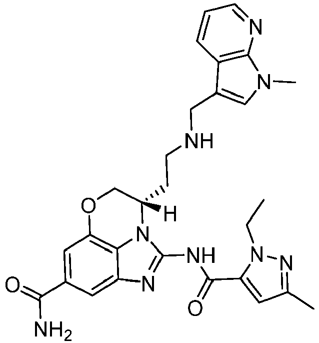 |
| 77 | 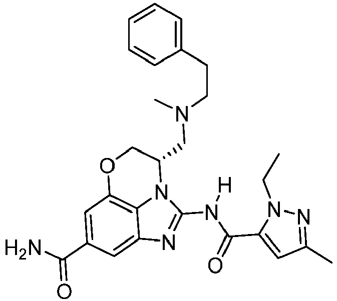 | 78 | 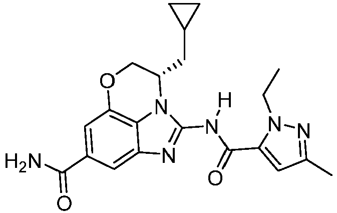 |
| 79 | 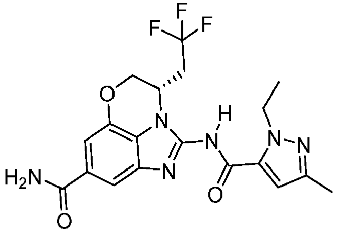 | 80 | 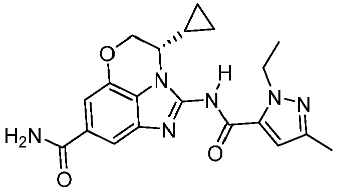 |
| 81 | 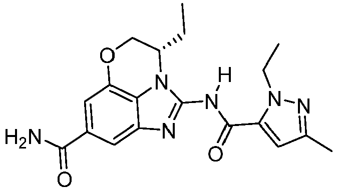 | 82 | 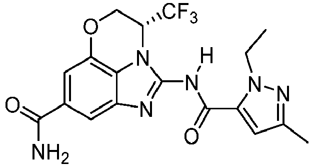 |
| 83 | 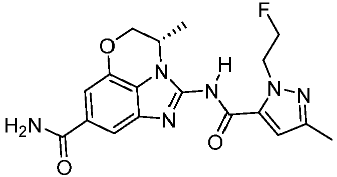 | 84 | 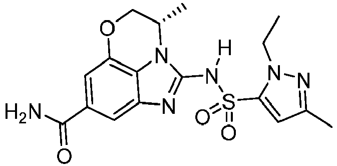 |
| 85 | 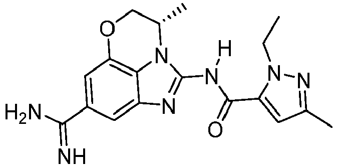 | 86 | 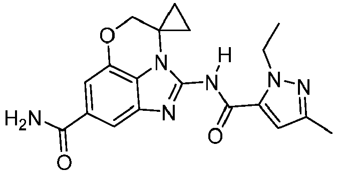 |
| 87 | 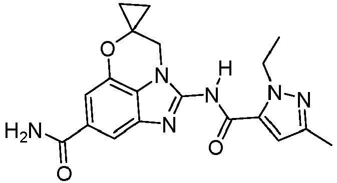 | 88 | 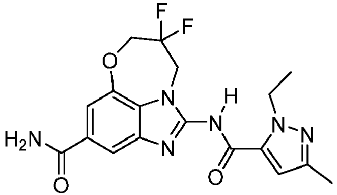 |
| 89 | 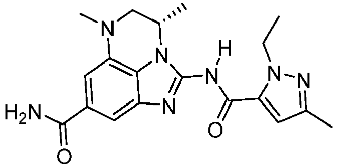 | 90 | 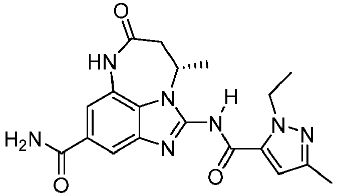 |
| 91 | 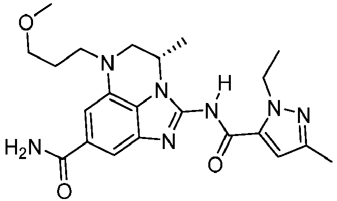 | 92 | 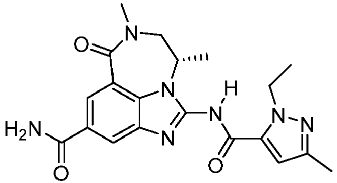 |
| 93 | 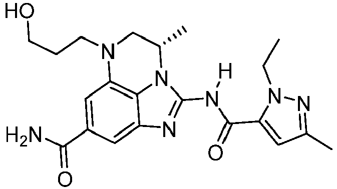 | 94 | 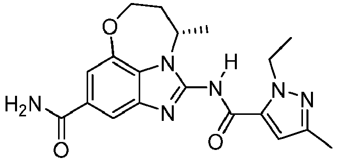 |
| 95 | 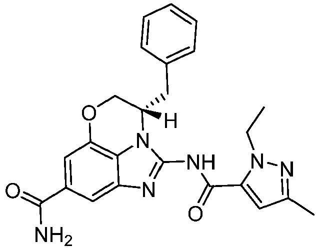 | 96 | 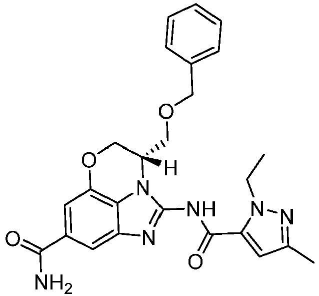 |
| 97 | 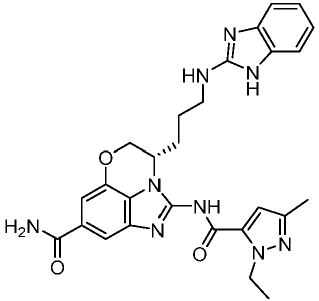 | 98 | 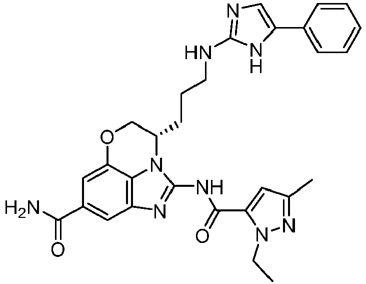 |
| 99 | 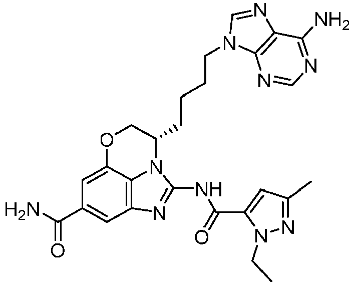 | 100 | 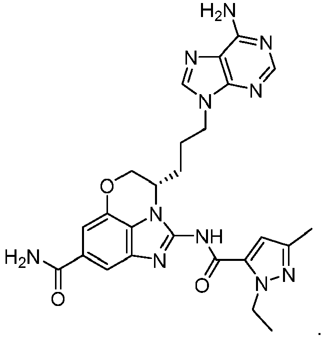 |
| 101 | 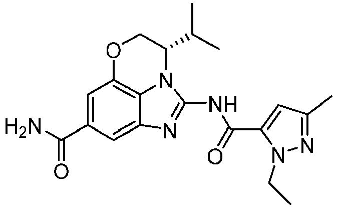 | 102 | 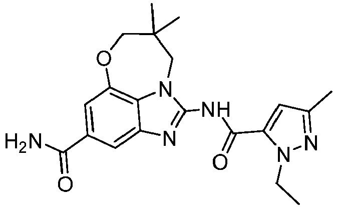 |
| 103 | 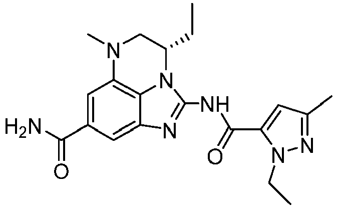 | 104 | 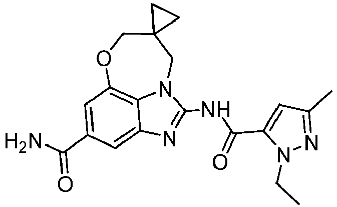 |
| 105 | 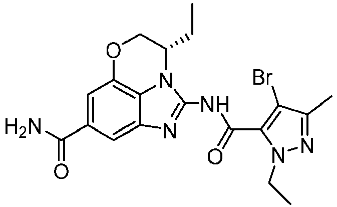 | 106 | 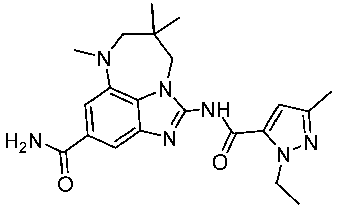 |
| 107 | 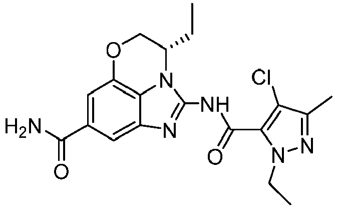 | 108 | 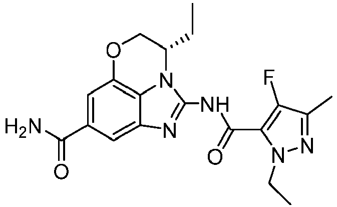 |
| 109 | 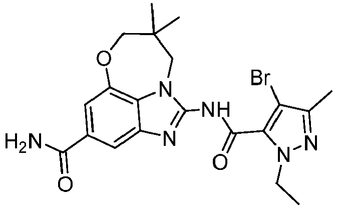 | 110 | 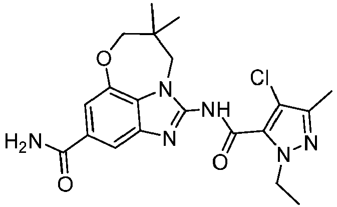 |
| 111 | 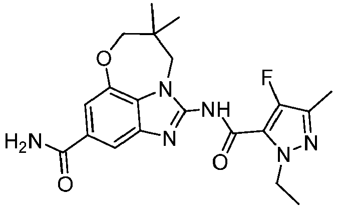 | 112 | 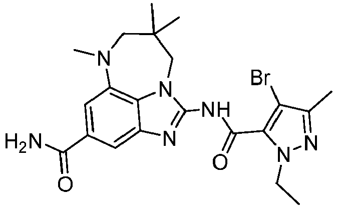 |
| 113 | 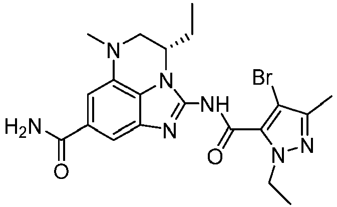 | 114 | 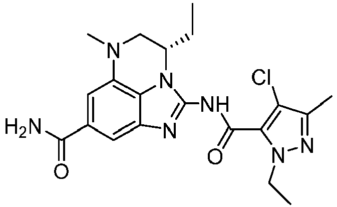 |
| 115 | 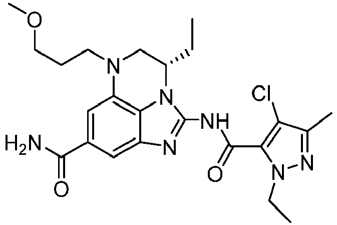 | 116 | 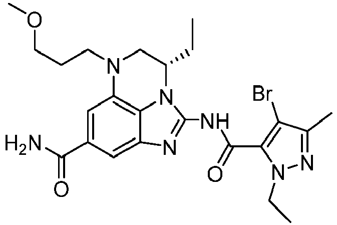 |
| 117 | 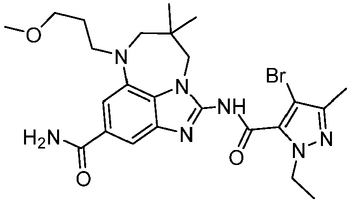 | 118 | 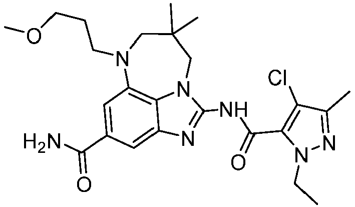 |
| 119 | 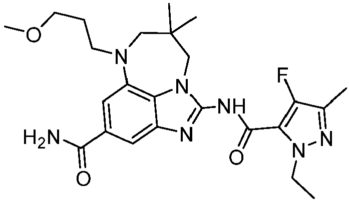 | 120 | 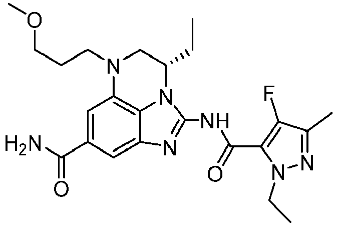 |
| 121 | 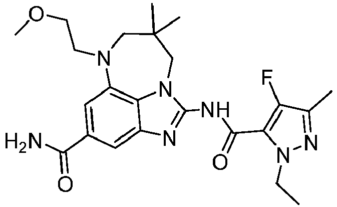 | 122 | 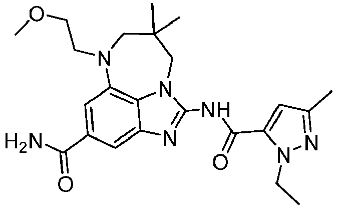 |
| 123 | 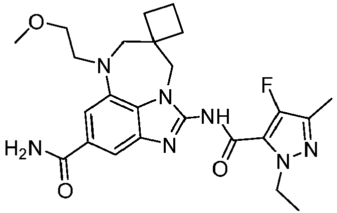 | 124 | 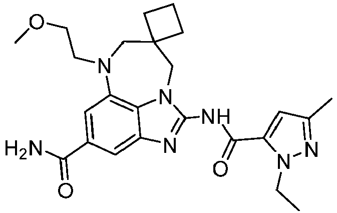 |
| 125 | 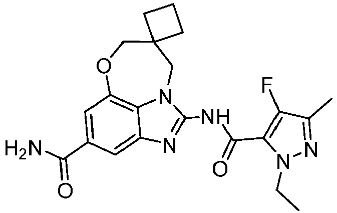 | 126 | 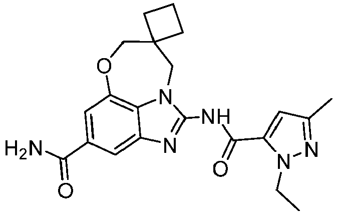 |
| 127 | 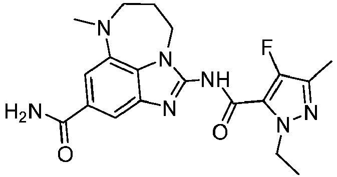 | 128 | 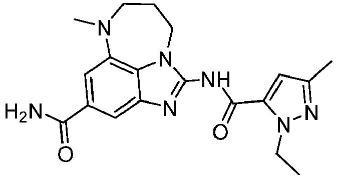 |
| 129 | 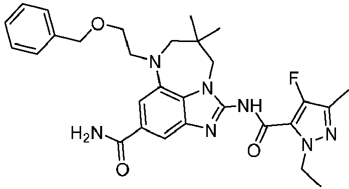 | 130 | 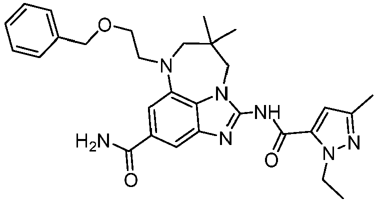 |
| 131 | 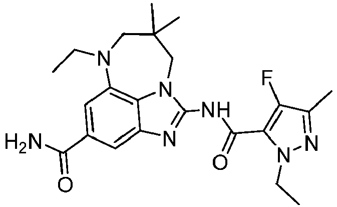 | 132 | 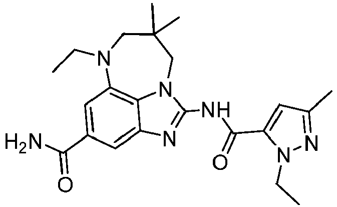 |
| 133 | 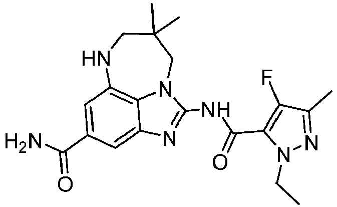 | 134 | 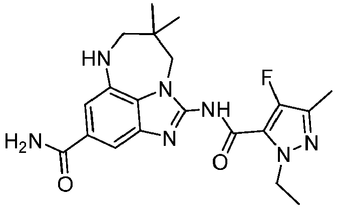 |
| 135 | 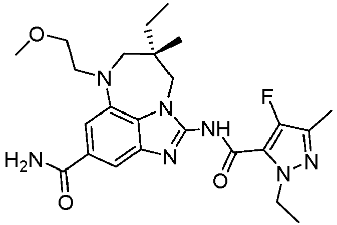 | 136 | 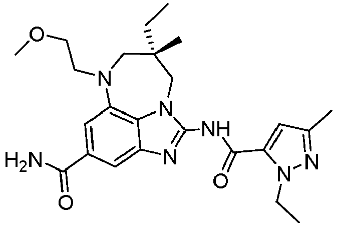 |
| 137 | 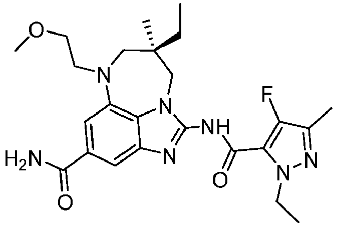 | 138 | 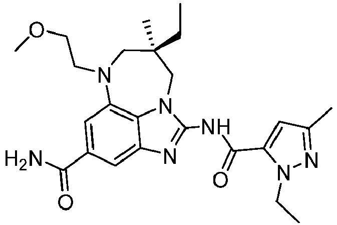 |
| 139 | 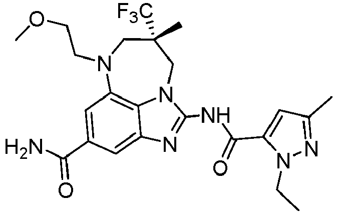 | 140 | 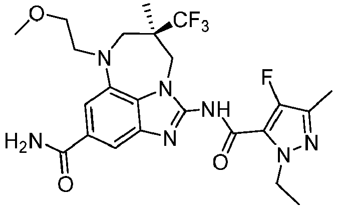 |
| 141 | 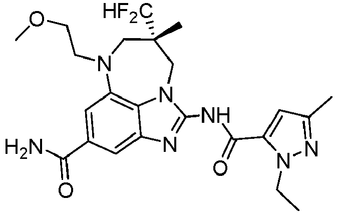 | 142 | 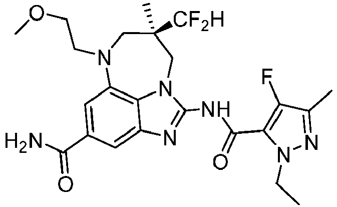 |
| 143 | 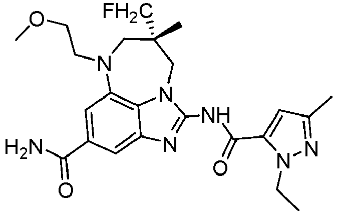 | 144 | 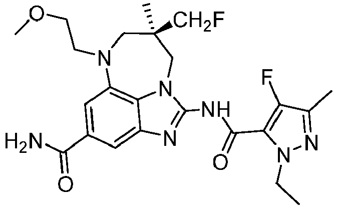 |
| 145 | 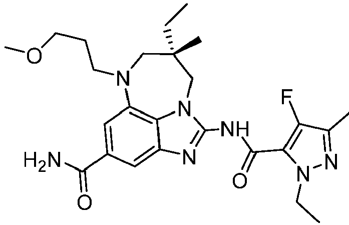 | 146 | 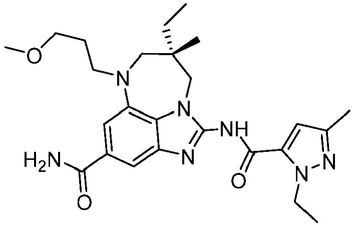 |
| 147 | 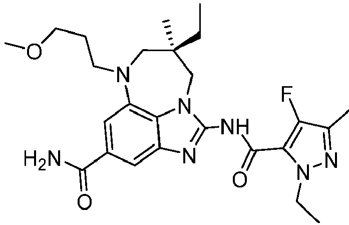 | 148 | 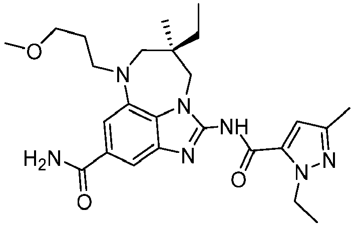 |
| 149 | 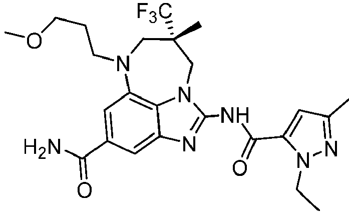 | 150 | 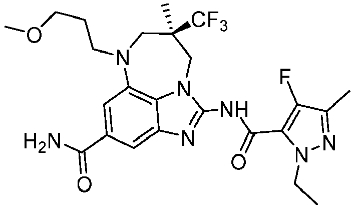 |
| 151 | 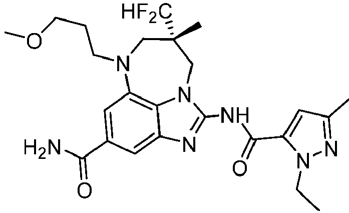 | 152 | 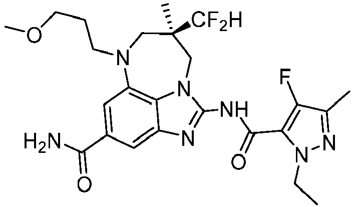 |
| 153 | 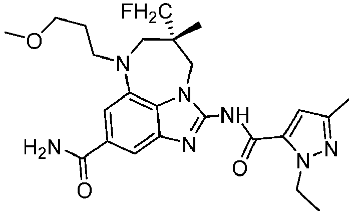 | 154 | 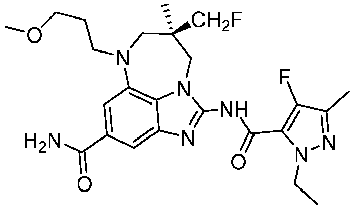 |
| 155 | 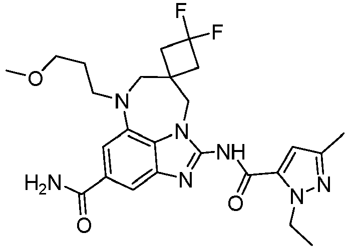 | 156 | 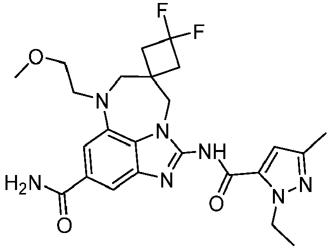 |
| 157 | 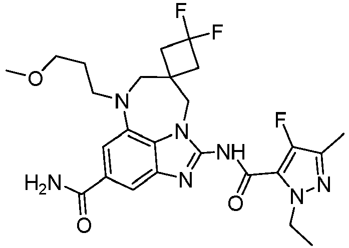 | 158 | 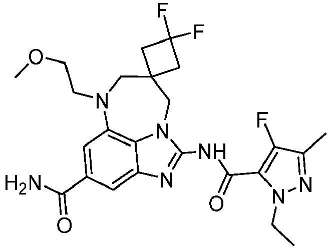 |

12. Pharmazeutische Zusammensetzung, umfassend eine Verbindung nach einem der Ansprüche 1 bis 11 und einen pharmazeutisch akzeptablen Träger.

13. Pharmazeutische Zusammensetzung nach Anspruch 12, ferner umfassend ein zusätzliches therapeutisches Mittel und/oder einen Immun-Checkpoint-Inhibitor, wobei das zusätzliche therapeutische Mittel vorzugsweise ausgewählt ist aus Chlorambucil, Melphalan, Cyclophosphamid, Ifosfamid, Busulfan, Carmustin, Lomustin, Streptozotocin, Cisplatin, Carboplatin, Oxaliplatin, Dacarbazin, Temozolomid, Procarbazin, Methotrexat, Fluorouracil, Cytarabin, Gemcitabin, Mercaptopurin, Fludarabin, Vinblastin, Vincristin, Vinorelbin, Paclitaxel, Docetaxel, Topotecan, Irinotecan, Etoposid, Trabectedin, Dactinomycin, Doxorubicin, Epirubicin, Daunorubicin, Mitoxantron, Bleomycin, Mitomycin C, Ixabepilon, Tamoxifen, Flutamid, Gonadorelin-Analoga, Megestrol, Prednison, Dexamethason, Methylprednisolon, Thalidomid, Interferon A, Calciumfolinat, Sirolimus, Sirolimuslipid, Everolimus, Afatinib, Alisertib, Amuvatinib, Apatinib, Axitinib, Bortezomib, Bosutinib, Brivanib, Cabozantinib, Cediranib, Crenolanib, Crizotinib, Dabrafenib, Dacomitinib, Danusertib, Dasatinib, Dovitinib, Erlotinib, Foretinib, Ganetespib, Gefitinib, Ibrutinib, Icotinib, Imatinib, Iniparib, Lapatinib, Lenvatinib, Linifanib, Linsitinib, Masitinib, Momelotinib, Motesanib, Neratinib Nilotinib, Niraparib, Oprozomib, Olaparib, Pazopanib, Pictiliisib, Ponatinib, Quizartinib, Regorafenib, Rigosertib, Rucaparib, Ruxolitinib, Saracatinib, Saridegib, Sorafenib, Sunitinib, Telatinib, Tivantinib, Tivozanib, Tofacitinib, Trametinib, Vandetanib, Veliparib, Vemurafenib, Erivedge, Volasertib, Alemtuzumab, Bevacizumab, Brentuximab Vedotin, Catumaxomab, Cetuximab, Denosumab, Gemtuzumab, Ipilimumab, Nimotuzumab, Ofatumumab, Panitumumab, Rituximab, Tositumomab, Trastuzumab, PI3K-Inhibitoren, CSF1R-Inhibitoren, A2A- und/oder A2B-Rezeptorantagonisten, IDO-Inhibitoren, Anti-PD-1-Antikörper, Anti-PD-L1-Antikörper, LAG3-Antikörper, TIM-3-Antikörper und Anti-CTLA-4-Antikörper oder eine beliebige Kombination davon.

14. Verbindung nach einem der Ansprüche 1 bis 11 oder pharmazeutische Zusammensetzung nach Anspruch 12 oder 13 zur Verwendung bei der Prävention und/oder Behandlung von Tumoren, Krebs, Virusinfektionen, Abstoßung von Organtransplantaten, neurodegenerativen Erkrankungen, aufmerksamkeitsbezogenen Erkrankungen oder Autoimmunerkrankungen.

15. Verbindung oder pharmazeutische Zusammensetzung zur Verwendung nach Anspruch 14, wobei der Tumor oder Krebs ausgewählt ist aus der Gruppe bestehend aus Hautkrebs, Blasenkrebs, Eierstockkrebs, Brustkrebs, Magenkrebs, Bauchspeicheldrüsenkrebs, Prostatakrebs, Dickdarmkrebs, Lungenkrebs, Knochenkrebs, Hirnkrebs, Neurozytom, Rektalkrebs, Dickdarmkrebs, familiärer adenomatöser Polyposis-Krebs, erblicher nichtpolypöser Darmkrebs, Speiseröhrenkrebs, Lippenkrebs, Kehlkopfkrebs, Hypopharynxkrebs, Zungenkrebs, Speicheldrüsenkrebs, Magenkrebs, Adenokarzinom, medullärer Schilddrüsenkrebs, papillärer Schilddrüsenkrebs, Nierenkrebs, Nierenparenchymkarzinom, Eierstockkrebs, Gebärmutterhalskrebs, Korpuskarzinom, Endometriumkarzinom, Choriokarzinom, Bauchspeicheldrüsenkrebs, Prostatakrebs, Hodenkrebs, Karzinom des Harnsystems, Melanom, Hirntumore wie Glioblastom, Astrozytom, Meningiom, Medulloblastom und periphere neuroektodermale Tumore, Hodgkin-Lymphom, Non-Hodgkin-Lymphom, Burkitt-Lymphom, akute lymphatische Leukämie (ALL), chronische lymphatische Leukämie (CLL), akute myeloische Leukämie (AML), chronische myeloische Leukämie (CML), Lymphom der adulten T-Zell-Leukämie, diffuses großzelliges B-Zell-Lymphom (DLBCL), hepatozelluläres Karzinom, Gallenblasenkarzinom, Bronchialkarzinom, kleinzelliges Lungenkarzinom, nichtkleinzelliges Lungenkarzinom, multiples Myelom, Basalzelltumor, Teratom, Retinoblastom, Aderhautmelanom, Seminom, Rhabdomyosarkom, Kraniopharyngiom, Osteosarkom, Chondrosarkom, Myosarkom, Liposarkom, Fibrosarkom, Ewing-Sarkom oder Plasmozytom.

## Revendications

1. Un composé ayant une structure de la formule (I) ou (II), 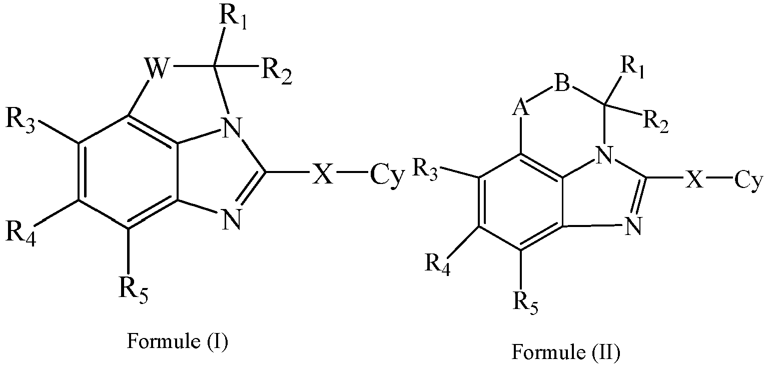
dans laquelle W représente (CR^{a}R^{a'})ₘ, dans lequel l'un quelconque des CR^{a}R^{a'} étant éventuellement substitué par 0, 1 ou 2 O, S ou Nb^{b};
dans lequel A et B représentent chacun indépendamment CR^{a}R^{a'}, NR^{b}, O or S;
R₁ et R₂ sont chacun indépendamment choisis parmi hydrogène, halogène, hydroxy, amino, mercapto, C₁-C₆ alkyle, C₁-C₆ alcoxy, C₁-C₆ alkylthio, C₁-C₆ alkylamino, di(C₁-C₆ alkyle) amino, C₂-C₆ alcényle, C₂-C₆ alcynyle, -(C₀-C₆ alkylène)-(C₃-C₆ cycloalkyle), -(C₀-C₆ alkylène)-(hétérocycloalkyle de 4 à 7 chaînons), -(C₀-C₆ alkylène)-(aryle ayant 6 à 12 chaînons) et -(C₀-C₆ alkylène)-(hétéroaryle ayant 5 à 12 chaînons), ou R₁ et R₂ ainsi que les atomes qui leur sont adjacents sont cyclisés pour former un cycle ayant 3 à 6 chaînons et contenant éventuellement 0, 1 ou 2 hétéroatomes choisis parmi O, N et S;
R₃, R₄ et R₅ sont chacun indépendamment choisis parmi hydrogène, halogène, cyano, C₁-C₆ alkyle, C₂-C₆ alcényle, C₂-C₆ alcynyle, -(C₀-C₆ alkylène)-(C3-C6 cycloalkyle), -(C₀-C₆ alkylène)-(hétérocycloalkyle ayant 4 à 7 chaînons), -OR^{c}, -NR^{c}R^{c'}, -OC(O)R^{c'}, -C(O)R^{c}, -CO₂R^{c}, - CON(R^{c})(R^{c'}), -C(=NR^{c})N(R^{c'})(R^{c"}), -NHC(O)R^{c}, -NHS(O)₂R^{c}-, -NHS(O)R^{c}-, -SO₂R^{c}, - SO₂NR^{c}R^{c'}, -(C₀-C₆ alkylène)-(aryle ayant 6 à 12 chaînons) et -(C₀-C₆ alkylène)-(hétéroaryle ayant 5 à 12 chaînons);
ou R₃ et R₄ sont cyclisés ensemble pour former un cycle ayant 5 à 8 chaînons et contenant éventuellement 0, 1, 2, 3 ou 4 hétéroatomes choisis parmi O, S et N;
ou R₄ et R₅ sont cyclisés ensemble pour former un cycle ayant 5 à 8 chaînons et contenant éventuellement 0, 1, 2, 3 ou 4 hétéroatomes choisis parmi O, S et N;
X représente -NR^{d}C(O)-, ou -NR^{d}C(=NR^{d'})-;
Cy représente un aryle ayant 6 à 12 chaînons ou un hétéroaryle ayant 5 à 12 chaînons;
m représente un nombre entier de 1, 2 ou 3;
R^{a} et R^{a'} représentent chacun indépendamment hydrogène, halogène, hydroxy, C₁-C₆ alkyle, C₁-C₆ alkylthio, -(C₀-C₆ alkylène)-(C₃-C₆ cycloalkyle), -(C₀-C₆ alkylène)-(hétérocycloalkyle ayant 4 à 7 chaînons), -(C₀-C₆ alkylène)-(aryle ayant 6 à 12 chaînons), -(C₀-C₆ alkylène)-(hétéroaryle ayant 5 à 12 chaînons), -NR^{e}R^{e'}, -NR^{e}COR^{e'}, -NR^{e}SO₂R^{e'}, -OR^{e} ou -OCOR^{e}, ou R^{a} et R^{a'}', ainsi que les atomes qui leur sont adjacents, sont cyclisés en un cycle de 3 à 6 chaînons contenant éventuellement 0, 1 ou 2 hétéroatomes sélectionnés parmi O, N et S; ou l'un quelconque des CR^{a}R^{a'} est pris ensemble pour former -C=O;
R^{b} représente chacun indépendamment hydrogène, C₁-C₆ alkyle, -(C₀-C₆ alkylène)-(C₃-C₆ cycloalkyle), -(C₀-C₆ alkylène)-(hétérocycloalkyle ayant 4 à 7 chaînons), -(C₀-C₆ alkylène) -(aryle ayant 6 à 12 chaînons), -(C₀-C₆ alkylène)-(hétéroaryle ayant 5 à 12 chaînons), -C(O)R^{f}, -SO₂R^{f}, - SOR^{f}, -C(O)OR^{f} or -C(O)NR^{f}R^{f'};
R^{c}, R^{c'}, R^{c"}, R^{d}, R^{d'}, R^{e}, R^{e'}, R^{f}, et R^{f'} représentent chacun indépendamment hydrogène, un C₁-C₆ alkyle, -(C₀-C₆ alkylène)-(C₃-C₆ cycloalkyle), -(C₀-C₆ alkylène)-(hétérocycloalkyle ayant 4 à 7 chaînons), -(C₀-C₆ alkylène)-(aryle ayant 6 à 12 chaînons) ou -(C₀-C₆ alkylène)-(alkylène ayant 5 à 12 chaînons), ou lorsque les substituants mentionnés ci-dessus sont collectivement liés à un seul atome N, ils sont éventuellement cyclisés avec l'atome N lié pour former un cycle de 3 à 8 chaînons;
les groupes alkyle, alkylène, aryle, hétéroaryle, cycle, cycloalkyle, hétérocycloalkyle, alcényle, alcynyle et alcoxy mentionnés ci-dessus sont chacun éventuellement indépendamment substitués par 0, 1, 2, 3 ou 4 substituants choisis parmi les groupes suivants constitués de : halogène, hydroxyle, cyano, carboxyle, C₁-C₆ alkyle, C₁-C₆ haloalkyle, sulfo, -OR^{g}, -SR^{g}, -NR^{g}R^{g'}, - NR^{g}COR^{g'}, -NR^{g}COOR^{g'}, -COR^{g}, -CO₂R^{g}, -SOR^{g}, -SO₂R^{g}, -OCONR^{g}R^{g'}-, -OCOR^{g}, -CONR^{g}R^{g'}, -NR^{g}SO₂R^{g'}, -SO₂NR^{g}R^{g'}, et -OP(O)(OR^{g}R^{g'})₂;
ou pour l'aryle et l'hétéroaryle, ou lorsque le nombre de substituants est de 2, les deux substituants adjacents sont également éventuellement cyclisés l'un par rapport à l'autre en un carbocycle ou hétérocycle saturé ou insaturé ayant 5 à 6 chaînons, l'hétérocycle étant un cycle contenant 0, 1, 2, 3 ou 4 hétéroatomes choisis parmi O, S et N,
dans laquelle R^{g} et R^{g'} sont chacun indépendamment constitués d'hydrogène, ou des groupes suivants éventuellement substitués par 0, 1, 2, 3 ou 4 groupes choisis parmi hydroxy, halogène, nitro, C₁-C₆ alkyle, halo(C₁-C₆ alkyle), amino, sulfonyle, cyano, C₁-C₆ alcoxy, C₁-C₆ alkylthio, C₁-C₆ amino et di(C₁-C₆ alkyle)amino; C₁-C₆ alkyle, -(C₀-C₆ alkylène)-(C₃-C₆ cycloalkyle), -(C₀-C₆ alkylène)-O-(C₁-C₆ alkyle), -(C₀-C₆ alkylène)-O-CO(C₁-C₆ alkyle), -(C₀-C₆ alkylène)-C(O)O(C₁-C₆ alkyle), -(C₀-C₆ alkylène)-(aryle ayant 6 à 12 chaînons), -(C₀-C₆ alkylène) -(hétéroaryle ayant 5 à 12 chaînons), -(C₂-C₆ alcénylène)-(aryle ayant 6 à 12 chaînons), -(C₂-C₆ alcénylène)-(hétéroaryle ayant 5 à 12 chaînons), -O-(C₀-C₆ alkylène)-(aryle ayant 6 à 12 chaînons), -O-(C₀-C₆ alkylène)-(hétéroaryle ayant 5 à 12 chaînons), -O-(C₂-C₆ alcénylène)-(aryle ayant 6 à 12 chaînons), -O-(C₂-C₆ alcénylène)-(hétéroaryle ayant 5 à 12 chaînons), -(C₀-C₆ alkylène)-O-(aryle ayant 6 à 12 chaînons), -(-C₀-C₆ alkylène)-O-(hétéroaryle ayant 5 à 12 chaînons), -(C₂-C₆ alcénylène)-O-(aryle ayant 6 à 12 chaînons) ou -(C₂-C₆ alcénylène)-O-(hétéroaryle ayant 5 à 12 chaînons);
dans lequel l'aryle ayant 6 à 12 chaînons est de préférence un phényle; l'hétéroaryle ayant 5 à 12 chaînons est de préférence un pyridyle, un imidazolyle ou un pyrazolyle; ou pour l'aryle ayant 6 à 12 chaînons ou l'hétéroaryle ayant 5 à 12 chaînons mentionnés ci-dessus, lorsque le nombre de substituants est de 2, les deux substituants adjacents peuvent également être cyclisés l'un à l'autre en un carbocycle ou un hétérocycle saturé ou insaturé ayant 5 à 6 chaînons;
à condition que les composés suivants soient exclus:

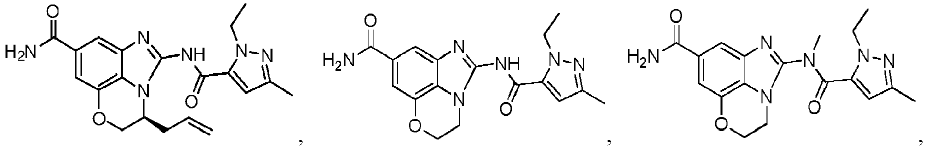

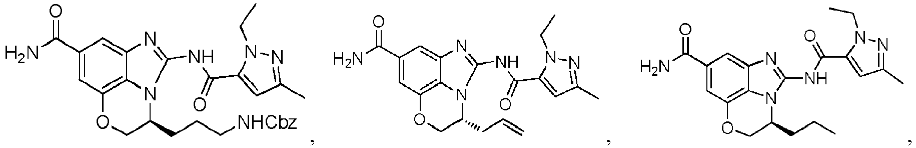

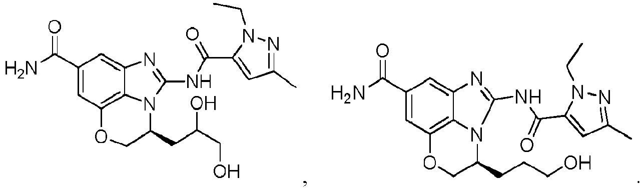

2. Le composé selon la revendition 1, ayant une structure de formule (III) ou (IV), 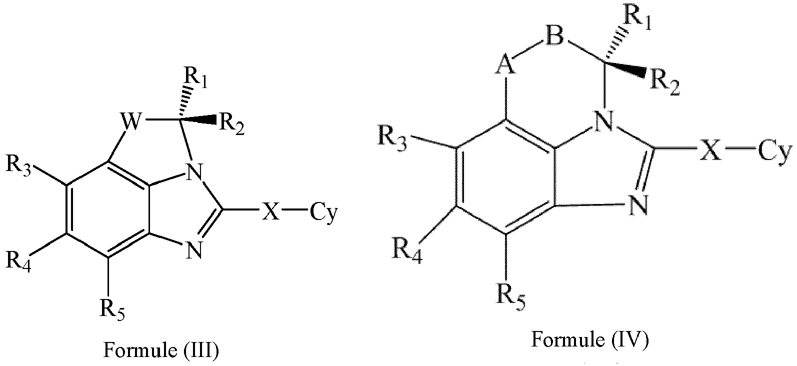 dans laquelle R₁, R₃, R₄, R₅, W, X, Cy, A et B ont la signification définie dans la revendication 1; R₂ représente un hydrogène ou un C₁-C₆ alkyle, et R₁ et R₂ représentent des substituants différents.

3. Le composé selon la revendication 1 ou 2, dans lequel A représente O et B représente CR^{a}R^{a'}.

4. Le composé selon l'une quelconque des revendications 1 à 3, dans lequel R₄ représente - CONR^{c}R^{c'}, et R^{c} et R^{c'} représentent indépendamment hydrogène ou C₁-C₆ alkyle;
dans laquelle X représente -NR^{d}C(O)-, et R^{d} représente hydrogène ou C₁-C₆ alkyle; ou
dans lequel Cy est chacun indépendamment choisi parmi phényle, pyridyle, pyrazolyle, pyrimidinyle, pyrazinyle, furanyle, thiazolyle, oxazolyle, imidazolyle, thiényle, triazolyle et tétrazolyle; de préférence pyrazolyl, imidazolyl, oxazolyl, triazolyl et tétrazolyl; de préférence imidazolyle; et éventuellement, Cy est chacun indépendamment substitué par 0, 1, 2, 3 ou 4 substituants choisis parmi les groupes suivants constitués de : halogène, hydroxyle, cyano, carboxyle, C₁-C₆ alkyle, C₁-C₆ haloalkyle, sulfo, C₁-C₆ alcoxy, -amino, nitro, (C₁-C₆ alkyle) amino et di(C₁-C₆ alkyle) amino.

5. Le composé selon l'une des revendications 1 à 4, dans lequel R₁ consiste en C₁-C₆ alkyle, C₂-C₆ alcényle, -(C₀-C₆ alkylène)-(C₃-C₆ cycloalkyle), -(C₀-C₆ alkylène)-(hétérocycloalkyle ayant 4 à 7 chaînons), ou -(C₀-C₆ alkylène)-(aryle ayant 6 à 12 chaînons); de préférence en C₁-C₆ alkyle, C₂-C₆ alcényle, -(C₀-C₆ alkylène)-(C₃-C₆ cycloalkyle), plus préférablement C₁-C₆ alkyle et C2-C6 alcényle, et R1 est éventuellement substitué par un substituant choisi parmi les substituants suivants : -NR^{g}COR^{g'}; et R^{g} consiste en hydrogène ou de C₁-C₆ alkyle; R^{g'} est consiste en les groupes suivants substitués par 0, 1, 2, 3 ou 4 substituants choisis parmi hydroxy, halogène, nitro, C₁-C₆ alkyle, halo (C₁-C₆ alkyle), amino, sulfonyle, cyano, C₁-C₆ alcoxy, C₁-C₆ alkylthio, C₁-C₆ amino et di(C₁-C₆ alkyle)amino; C₁-C₆ alkyle, -(C₀-C₆ alkylène)-(C₃-C₆ cycloalkyle), -(C₀-C₆ alkylène)-O-(C₁-C₆ alkyle), -(C₀-C₆ alkylène)-O-CO(C₁-C₆ alkyle), -(C₀-C₆ alkylène)-C(O)O(C₁-C₆ alkyle), -(C₀-C₆ alkylène)-(aryle ayant 6 à 12 chaînons), -(C₀-C₆ alkylène) -(hétéroaryle ayant 5 à 12 chaînons), -(C₂-C₆ alcénylène)-(aryle ayant de 6 à 12 chaînons), -(C₂-C₆ alcénylène)-(hétéroaryle ayant 5 à 12 chaînons), -O-(C₀-C₆ alkylène)-(aryle ayant 6 à 12 chaînons), -O-(C₀-C₆ alkylène)-(hétéroaryle ayant 5 à 12 chaînons), -O-(C₂-C₆ alcénylène)-(aryle ayant 6 à 12 chaînons), -O-(C₂-C₆ alcénylène)-(hétéroaryle ayant 5 à 12 chaînons), -(C₀-C₆ alkylène)-O-(aryle ayant 6 à 12 chaînons), -(C₀-C₆ alkylène)-O-(hétéroaryle ayant 5 à 12 chaînons), -(C₂-C₆ alcénylène)-O-(aryle ayant 6 à 12 chaînons) ou -(C₂-C₆ alcénylène)-O-(hétéroaryle ayant 5 à 12 chaînons);
dans lequel l'aryle ayant 6 à 12 chaînons est de préférence un phényle; l'hétéroaryle ayant 5 à 12 chaînons est de préférence un pyridyle, un imidazolyle ou un pyrazolyle; ou pour l'aryle ayant 6 à 12 chaînons ou l'hétéroaryle ayant 5 à 12 chaînons mentionnés ci-dessus, lorsque le nombre de substituants est de 2, les deux substituants adjacents sont éventuellement cyclisés l'un à l'autre en un carbocycle ou un hétérocycle saturé ou insaturé ayant 5 à 6 chaînons.

6. Le composé selon l'une quelconque des revendications 1 à 5, dans lequel R₁ consiste en -(C₁-C₆ alkylène)-NR^{g}COR^{g'} et -(C₂-C₆ alcénylène)-NR^{g}COR^{g'}, où R^{g} consiste en hydrogène ou de C₁-C₆ alkyle; R^{g'} consiste en les groupes suivants substitués par 0, 1, 2, 3 ou 4 substituants choisis parmi hydroxy, halogène, nitro, C₁-C₆ alkyle, halo(C₁-C₆ alkyle), amino, sulfonyle, cyano, C₁-C₆ alcoxy, C₁-C₆ alkylthio, C₁-C₆ amino et di(C₁-C₆ alkyle)amino : -(C₀-C₆ alkylène)-(aryle ayant 6 à 12 chaînons), -(C₀-C₆ alkylène)-(hétéroaryle ayant 5 à 12 chaînons), -(C₂-C₆ alcénylène)-(aryle ayant 6 à 12 chaînons), -(C₂-C₆ alcénylène)-(hétéroaryle ayant 5 à 12 chaînons), -O-(C₀-C₆ alkylène)-(aryle ayant 6 à 12 chaînons), -O-(C₀-C₆ alkylène)-(hétéroaryle ayant 5 à 12 chaînons), -O-(C₂-C₆ alcénylène)-(aryle ayant 6 à 12 chaînons), -O-(C₂-C₆ alcénylène)-(hétéroaryle ayant 5 à 12 chaînons), -(C₀-C₆ alkylène)-O-(aryle ayant 6 à 12 chaînons), -(C₀-C₆ alkylène)-O-(hétéroaryle ayant 5 à 12 chaînons), -(C₂-C₆ alcénylène)-O-(aryle ayant 6 à 12 chaînons), ou -(C₂-C₆ alcénylène)-O-(hétéroaryle ayant 5 à 12 chaînons);
dans lequel l'aryle ayant 6 à 12 chaînons est de préférence un phényle; l'hétéroaryle ayant 5 à 12 chaînons est de préférence pyridyle; ou pour l'aryle ayant 6 à 12 chaînons ou l'hétéroaryle ayant 5 à 12 chaînons mentionnés ci-dessus, lorsque le nombre de substituants est de 2, les deux substituants adjacents sont éventuellement cyclisés l'un à l'autre en un carbocycle ou un hétérocycle saturé ou insaturé ayant 5 à 6 chaînons; plus préférablement -O-(C₁-C₆ alkylène)-phényle, -O-(C₁-C₆ alkylène)-pyridyle, -(C₁-C₆ alkylène)-O-phényle, -(C₁-C₆ alkylène)-O-pyridyle, -(C₁-C₆ alkylène)-phényle, -(C₁-C₆ alkylène)-pyridyle, -(C₂-C₆ alcénylène)-phényle ou - (C₂-C₆ alcénylène)-pyridyle; et le phényle et le pyridyle sont éventuellement indépendamment substitués par 0, 1, 2, 3 ou 4 substituants choisis parmi hydroxy, halogène, amino, sulfonyle, cyano, nitro, C₁-C₆ alcoxy et C₁-C₆ haloalkyle .

7. Le composé selon l'une quelconque des revendications 1 à 6, dans lequel R₂ représente un hydrogène ou un C₁-C₆ alkyle; ou
dans laquel R₃ et R₅ représentent chacun indépendamment un hydrogène, un halogène ou un C₁-C₆ alkyle.

8. Le composé selon la revendition 1, ayant une structure de formule (V), 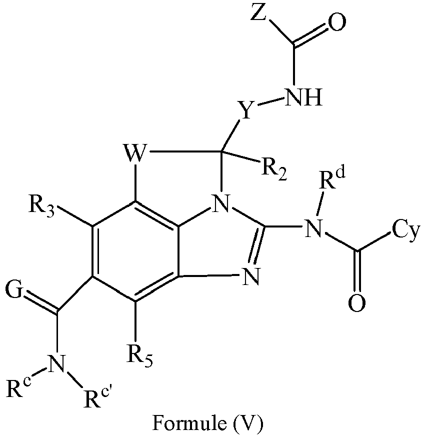
dans laquelle W représente (CR^{a}R^{a'})ₘ, dans lequel l'un quelconque des CR^{a}R^{a'} étant éventuellement substitué par 0, 1 ou 2 O, S ou Nb^{b};
R₂ représente indépendamment choisis parmi hydrogène, halogène, hydroxy, amino, mercapto, C₁-C₆ alkyle, C₁-C₆ alcoxy, C₁-C₆ alkylthio, C₁-C₆ alkylamino, di(C₁-C₆ alkyle) amino, C₂-C₆ alcényle, C₂-C₆ alcynyle, -(C₀-C₆ alkylène)-(C₃-C₆ cycloalkyle), -(C₀-C₆ alkylène)-(hétérocycloalkyle ayant 4 à 7 chaînons), -(C₀-C₆ alkylène)-(aryle ayant 4 à 12 chaînons) et -(C₀-C₆ alkylène)-(hétéroaryle ayant 5 à 12 chaînons),
R₃ et R₅ sont chacun indépendamment choisis parmi hydrogène, halogène, cyano, C₁-C₆ alkyle, C₂-C₆ alcényle, C₂-C₆ alcynyle, -(C₀-C₆ alkylène)-(C₃-C₆ cycloalkyle), -OR^{c}, -NR^{c}R^{c'}, - OC(O)R^{c'}, -C(O)R^{c}, -CO₂R^{c}, -CON(R^{c})(R^{c'}), -C(=NR^{c})N(R^{c'})(R^{c"}), -NHC(O)R^{c}, -NHS(O)₂R^{c}-, - NHS(O)R^{c}-, -SO₂R^{c}, -SO₂NR^{c}R^{c'}, -(alkylène C₀-C₆)-(hétérocycloalkyle ayant 4 à 7 chaînons), - (C₀-C₆ alkylène)-(aryle ayant 6 à 12 chaînons) et -(C₀-C₆ alkylène)-(hétéroaryle ayant 5 à 12 chaînons);
Cy représente un aryle ayant 6 à 12 chaînons ou un hétéroaryle ayant 5 à 12 chaînons;
m représente un nombre entier de 1, 2 ou 3;
R^{a} et R^{a'} représentent chacun indépendamment hydrogène, halogène, hydroxy, C₁-C₆ alkyle, C₁-C₆ alkylthio, -(C₀-C₆ alkylène)-(C₃-C₆ cycloalkyle), -(C₀-C₆ alkylène)-(hétérocycloalkyle ayant 4 à 7 chaînons), -(C₀-C₆ alkylène)-(aryle ayant 6 à 12 chaînons), -(C₀-C₆ alkylène)-(hétéroaryle ayant 5 à 12 chaînons), -NR^{e}R^{e'}, -NR^{e}COR^{e'}, -NR^{e}SO₂R^{e'}, -OR^{e} ou -OCOR^{e}, ou R^{a} et R^{a'}', ainsi que les atomes qui leur sont adjacents, sont cyclisés en un cycle de 3 à 6 chaînons contenant éventuellement 0, 1 ou 2 hétéroatomes sélectionnés parmi O, N et S; ou l'un quelconque des CR^{a}R^{a'} est pris ensemble pour former -C=O;
R^{b} représente chacun indépendamment hydrogène, C₁-C₆ alkyle, -(C₀-C₆ alkylène)-(C₃-C₆ cycloalkyle), -(C₀-C₆ alkylène)-(hétérocycloalkyle ayant 4 à 7 chaînons), -(C₀-C₆ alkylène) -(aryle ayant 6 à 12 chaînons), -(C₀-C₆ alkylène)-(hétéroaryle ayant 5 à 12 chaînons), -C(O)R^{f}, -SO₂R^{f}, - SOR^{f}, -C(O)OR^{f} or -C(O)NR^{f}R^{f'};
G représente O ou NR^{c};
R^{c}, R^{c'}, R^{c"}, R^{d}, R^{e}, R^{e'}, R^{f}, et R^{f'} représentent chacun indépendamment hydrogène, un C₁-C₆ alkyle, -(C₀-C₆ alkylène)-(C₃-C₆ cycloalkyle), -(C₀-C₆ alkylène)-(hétérocycloalkyle ayant 4 à 7 chaînons), -(C₀-C₆ alkylène)-(aryle ayant 6 à 12 chaînons) ou -(C₀-C₆ alkylène)-(alkylène ayant 5 à 12 chaînons), ou lorsque les substituants mentionnés ci-dessus sont collectivement liés à un seul atome N, ils sont éventuellement cyclisés avec l'atome N lié pour former un cycle de 3 à 8 chaînons;
les groupes alkyle, alkylène, aryle, hétéroaryle, cycle, cycloalkyle, hétérocycloalkyle, alcényle, alcynyle et alcoxy mentionnés ci-dessus sont chacun éventuellement indépendamment substitués par 0, 1, 2, 3 ou 4 substituants choisis parmi les groupes suivants constitués de : halogène, oxo, hydroxy, cyano, carboxy, C₁-C₆ alkyle, C₁-C₆ haloalkyle, sulfo, C₁-C₆ alkoxy, -OR^{g}, -SR^{g}, - N(R^{g})(R^{g'}), -NR^{g}COR^{g'}, -NR^{g}COOR^{g'}, -COR^{g}, -CO₂R^{g}, -SOR^{g}, -SO₂R^{g}, -OCONR^{g}R^{g'}-, -OCOR^{g}, -CONR^{g}R^{g'}, -NR^{g}SO₂R^{g'}, -SO₂NR^{g}R^{g'}, et -OP(O)(OR^{g}R^{g'})₂;
ou pour l'aryle et l'hétéroaryle, ou lorsque le nombre de substituants est de 2, les deux substituants adjacents sont également éventuellement cyclisés l'un par rapport à l'autre en un carbocycle ou hétérocycle saturé ou insaturé ayant 5 à 6 chaînons, l'hétérocycle étant un cycle contenant 0, 1, 2, 3 ou 4 hétéroatomes choisis parmi O, S et N;
dans laquelle R^{g} et R^{g'} chacun représentent indépendamment hydrogène, ou les groupes suivants éventuellement substitués par 0, 1, 2, 3 ou 4 groupes choisis parmi hydroxy, halogène, nitro, C₁-C₆ alkyle, halo(C₁-C₆ alkyle), amino, sulfonyle, cyano, C₁-C₆ alcoxy, C₁-C₆ alkylthio, C₁-C₆ amino, di(C₁-C₆ alkyle)amino; C₁-C₆ alkyle, -(C₀-C₆ alkylène)-(C₃-C₆ cycloalkyle), halo(C₁-C₆ alkyle), -(C₀-C₆ alkyle)-OH, -(C₀-C₆ alkylène)-O-(C₁-C₆ alkyle), (-C₀-C₆ alkylène)-O-CO(C₁-C₆ alkyle), -(C₀-C₆ alkylène)-C(O)O(C₁-C₆ alkyle), -(C₀-C₆ alkylène)-(aryle ayant 6 à 12 chaînons), - C₂-C₆ alcénylène -(aryle ayant 6 à 12 chaînons), -(C₀-C₆ alkylène)-O-(aryle ayant 6 à 12 chaînons), -(C₀-C₆ alkylène)-O-(C₁-C₆ alkyle), -O-(C₁-C₆ alkylène)-(aryle ayant 6 à 12 chaînons), -(C₀-C₆ alkylène)-(hétéroaryle ayant 5 à 12 chaînons), -(C₂-C₆ alcénylène)-(hétéroaryle ayant 5 à 12 chaînons), -(C₀-C₆ alkylène)-O-(hétéroaryle ayant 5 à 12 chaînons) ou -O-(C₀-C₆ alkylène)-(hétéroaryle ayant 5 à 12 chaînons); ou pour l'aryle ayant 6 à 12 chaînons ou l'hétéroaryle ayant 5 à 12 chaînons mentionnés ci-dessus, lorsque le nombre de substituants est de 2, les deux substituants adjacents sont également éventuellement cyclisés l'un à l'autre en un carbocycle ou un hétérocycle saturé ou insaturé ayant 5 à 6 chaînons;
Y représente les groupes suivants éventuellement substitués par 0, 1, 2, 3 ou 4 substituants choisis parmi hydroxy halogène, nitro, C₁-C₆ alkyle, halo(C₁-C₆ alkyle), amino, sulfonyle, cyano, C₁-C₆ alcoxy, C₁-C₆ alkylthio, C₁-C₆ amino et di(C₁-C₆ alkyle)amino; -C₁-C₆ alkylène, -(C₀-C₆ alkylène)-(C₃-C₆ cycloalkylène)-(C₀-C₆ alkylène), -(C₀-C₆ alkylène)-(hétérocycloalkyle ayant 4 à 7 chaînons)-(C₀-C₆ alkylène), (C₀-C₆ alkylène)-(aryle ayant 6 à 12 chaînons)-(C₀-C₆ alkylène), - (C₀-C₆ alkylène)-(hétéroaryle ayant 5 à 12 chaînons)-(C₀-C₆ alkylène), ou -C₂-C₆ alcénylène-;
Z représente les groupes suivants éventuellement substitués par 0, 1, 2, 3 ou 4 substituants choisis parmi hydroxy, halogène, nitro, C₁-C₆ alkyle, halo(C₁-C₆ alkyle), amino, sulfonyle, cyano, C₁-C₆ alcoxy, C₁-C₆ alkylthio, C₁-C₆ amino et di(C₁-C₆ alkyle)amino; C₁-C₆ alkyle, -(C₀-C₆ alkylène)-(C₃-C₆ cycloalkyle), -(C₀-C₆ alkylène)-O-(C₁-C₆ alkyle), -(C₀-C₆ alkylène)-O-CO(C₁-C₆ alkyle), -(C₀-C₆ alkylène)-C(O)O(C₁-C₆ alkyle), -(C₀-C₆ alkylène)-(aryle ayant 6 à 12 chaînons), - (C₀-C₆ alkylène) -(hétéroaryle ayant 5 à 12 chaînons), -(C₂-C₆ alcénylène)-(aryle à 6 à 12 chaînons), -(C₂-C₆ alcénylène)-(hétéroaryle ayant 5 à 12 chaînons), -O-(C₀-C₆ alkylène)-(aryle ayant 12 chaînons), -O-(C₀-C₆ alkylène)-(hétéroaryle ayant 5 à 12 chaînons), -O-(C₂-C₆ alcénylène)-(aryle ayant 6 à 12 chaînons), -O-(C₂-C₆ alcénylène)-(hétéroaryle ayant 5 à 12 chaînons), -(C₀-C₆ alkylène)-O-(aryle ayant 6 à 12 chaînons), -(C₀-C₆ alkylène)-O-(hétéroaryle ayant 5 à 12 chaînons), -(C₂-C₆ alcénylène)-O-(aryle ayant 6 à 12 chaînons), ou -(C₂-C₆ alcénylène)-O-(hétéroaryle ayant 5 à 12 chaînons); ou pour l'aryle ayant 6 à 12 chaînons ou l'hétéroaryle ayant 5 à 12 chaînons mentionnés ci-dessus, lorsque le nombre de substituants est de 2, les deux substituants adjacents sont également éventuellement cyclisés l'un à l'autre en un carbocycle ou un hétérocycle saturé ou insaturé ayant 5 à 6 chaînons;
dans lequel l'aryle ayant 6 à 12 chaînons est de préférence un phényle; l'hétéroaryle ayant 5 à 12 chaînons est de préférence un pyridyle, un imidazolyle ou un pyrazolyle; ou pour l'aryle ayant 6 à 12 chaînons ou l'hétéroaryle ayant 5 à 12 chaînons mentionnés ci-dessus, lorsque le nombre de substituants est de 2, les deux substituants adjacents sont également éventuellement cyclisés l'un à l'autre en un carbocycle ou un hétérocycle saturé ou insaturé ayant 5 à 6 chaînons;

9. Le composé selon la revendition 8, ayant une structure de formule (VI), 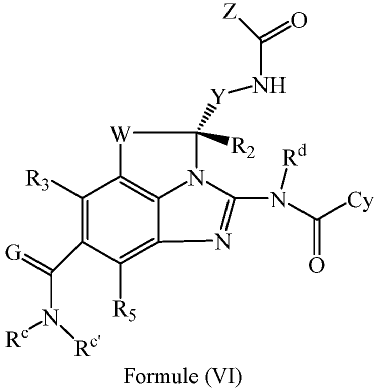 dans laquelle R₂ est choisi parmi l'hydrogène ou C₁-C₆ alkyle, et W, R₃, R₅, R^{c}, R^{c'}, R^{d}, G, Z, Y, et Cy sont tous tels que définis dans la revendication 8.

10. Le composé selon l'une quelconque des revendications 8 à 9, dans lequel G consiste en O ou NH;
dans laquelle Y consiste en les groupes suivants substitués par 0, 1, 2, 3 ou 4 substituants sélectionnés parmi hydroxy, halo et alkyle C1-C6: -alkylène C₁-C₆, -alcénylène C₂-C₆ ou - cycloalkyle C₃-C₆;
dans laquelle W consiste en -CR^{a}R^{a'}-O, -O-CR^{a}R^{a'}-, -C(O)-NR^{b}-, ou -NR^{b}-C(O)-, où R^{a}, R^{a'}, et R^{b} représentent chacun indépendamment hydrogène, C₁-C₆ alkyle ou C₃-C₆ cycloalkyle;
dans laquelle Z consiste en -O-(C₀-C₆ alkylène)-(aryle ayant 6 à 12 chaînons), -(C₀-C₆ alkylène)-(aryle ayant 6 à 12 chaînons), -(C₂-C₆ alcénylène)-(aryle ayant 6 à 12 chaînons), -(C₀-C₆ alkylène)-(hétéroaryle ayant 5 à 12 chaînons), -(C₂-C₆ alcénylène)-(hétéroaryle ayant 5 à 12 chaînons), -O-(C₂-C₆ alcénylène)-(aryle ayant 6 à 12 chaînons), -(C₀-C₆ alkylène)-O-(aryle ayant 6 à 12 chaînons), -O-(C₀-C₆ alkylène)-(hétéroaryle ayant 5 à 12 chaînons), -O-(C₂-C₆ alcénylène)-(hétéroaryle ayant 5 à 12 chaînons), ou -(C₀-C₆ alkylène)-O-(hétéroaryle ayant 5 à 12 chaînons), et éventuellement l'aryle ayant 6 à 12 chaînons (de préférence phényle) ou l'hétéroaryle ayant 5 à 12 chaînons (de préférence pyridyle) est chacun indépendamment substitué par 0, 1, 2, 3 ou 4 substituants choisis parmi les groupes suivants constitués de: halo, hydroxy, nitro, C₁-C₆ alkyle, halo(C₁-C₆ alkyle), amino, sulfonyle, cyano, C₁-C₆ alcoxy, C₁-C₆ alkylthio, C₁-C₆ amino et di(C₁-C₆ alkyle)amino;
dans laquelle R₂ représente hydrogène ou un C₁-C₆ alkyle;
dans laquelle R³ et R⁵ représentent chacun indépendamment hydrogène, halogène ou C₁-C₆ alkyle;
dans laquelle R^{c} et R^{c'} représentent chacun indépendamment hydrogène, halogène ou C₁-C₆ alkyle .
dans laquelle R^{d} représente hydrogène ou C₁-C₆ alkyle; ou
dans laquelle Cy représente pyrazolyle et est éventuellement substitué par 0, 1, 2 ou 3 C₁-C₆ alkyle.

11. Le composé selon la revendition 1, ayant une structure choisie parmi :
| No. | Structure du composé | No. | Structure du composé |
|---|---|---|---|
| 1 | 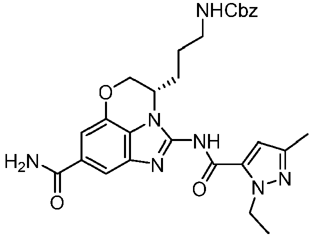 | 2 | |
| 3 | 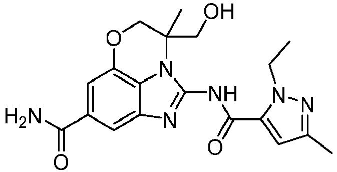 | 4 | 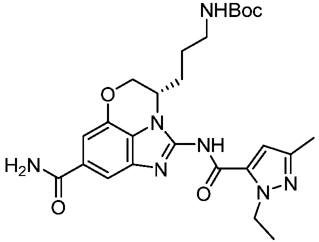 |
| 5 | 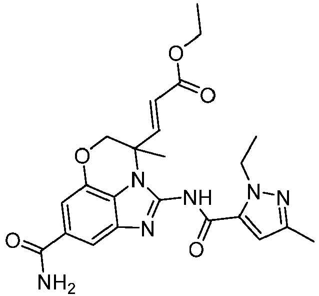 | 6 | 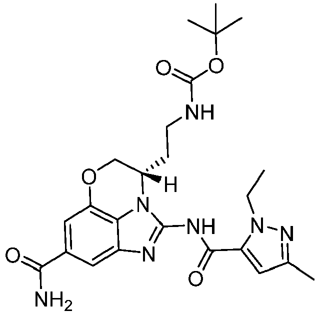 |
| 7 | 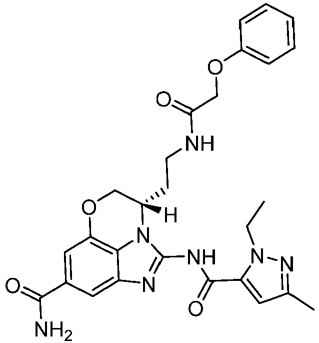 | 8 | 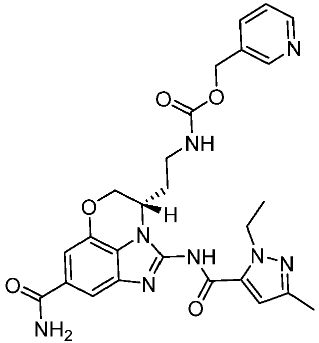 |
| 9 | 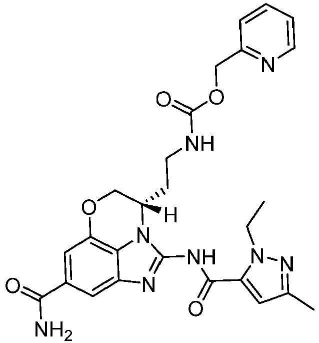 | 10 | 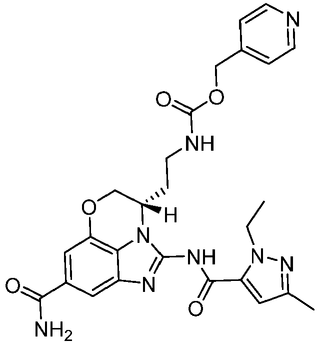 |
| 11 | 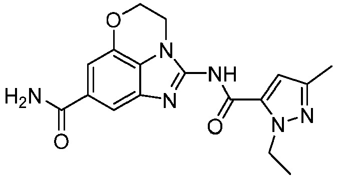 | 12 | 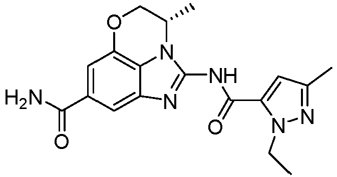 |
| 13 | 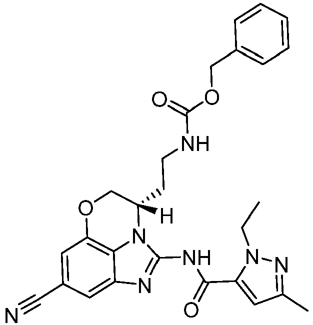 | 14 | 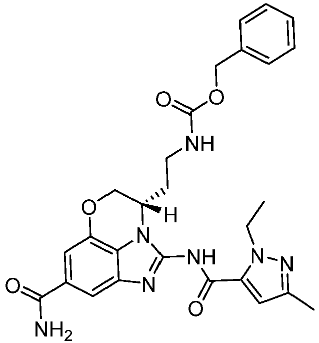 |
| 15 | 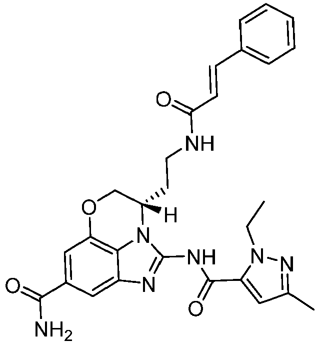 | 16 | 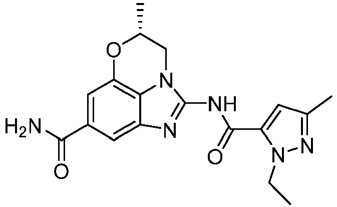 |
| 17 | 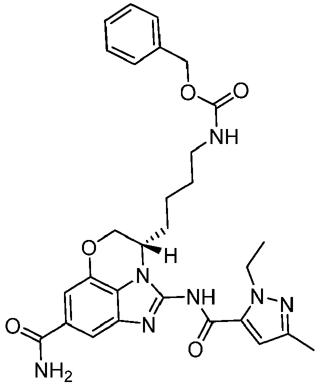 | 18 | 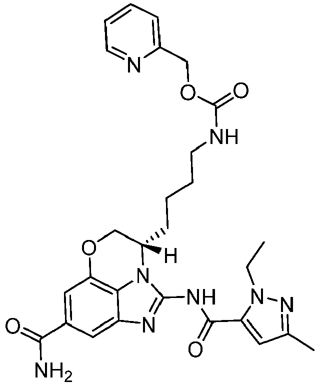 |
| 19 | 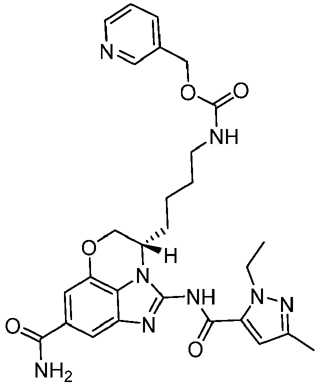 | 20 | 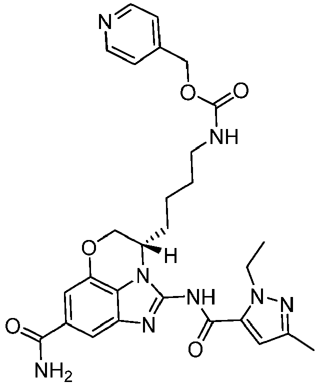 |
| 21 | 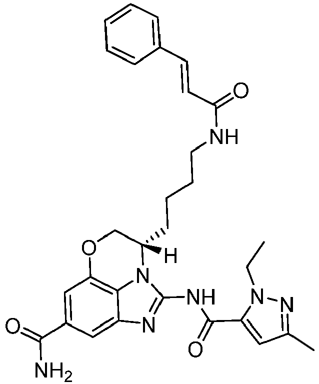 | 22 | 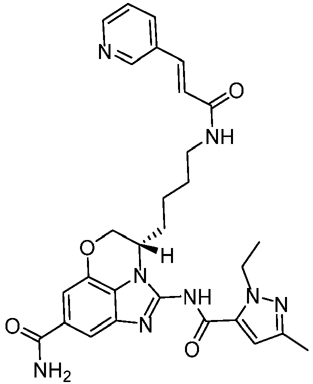 |
| 23 | 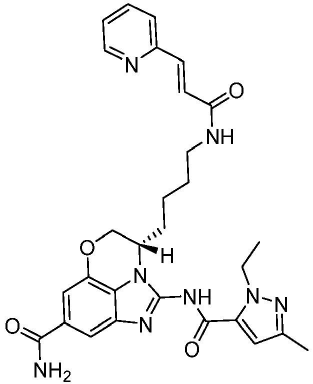 | 24 | 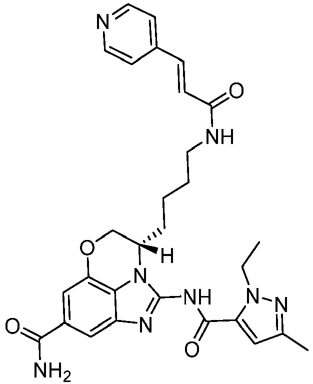 |
| 25 | 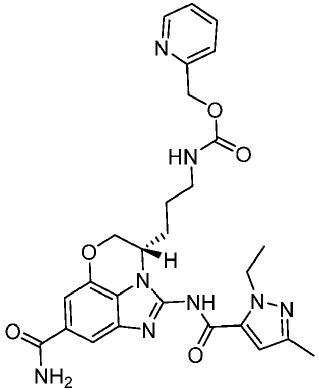 | 26 | 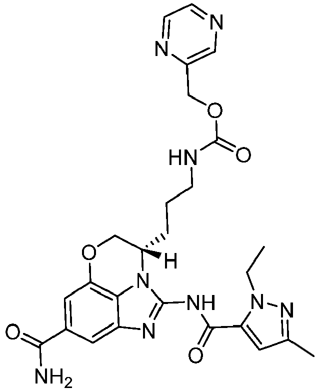 |
| 27 | 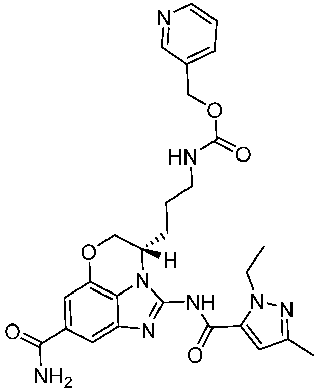 | 28 | 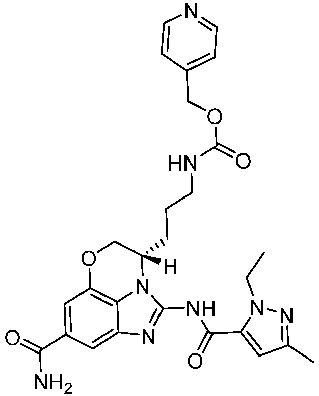 |
| 29 | 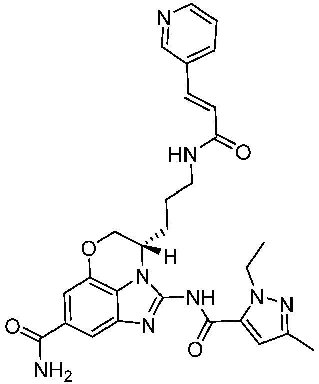 | 30 | 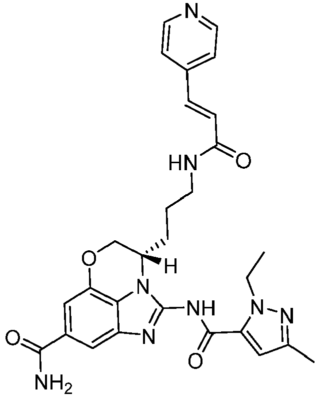 |
| 31 | 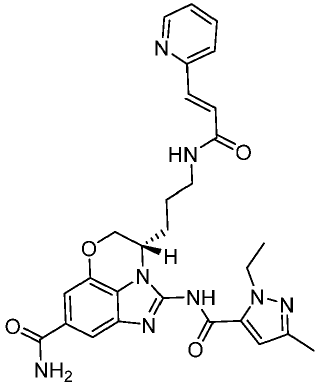 | 32 | 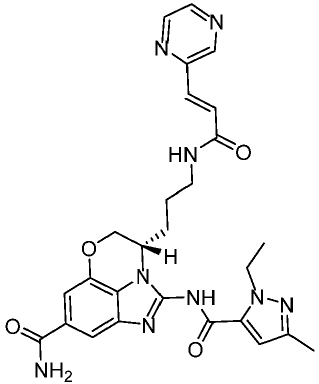 |
| 33 | 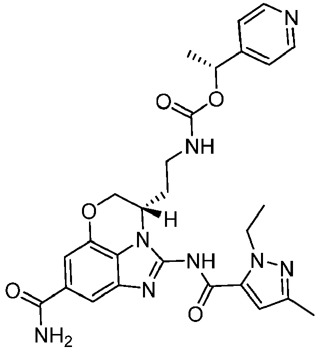 | 34 | 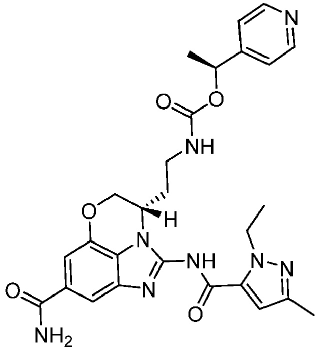 |
| 35 | 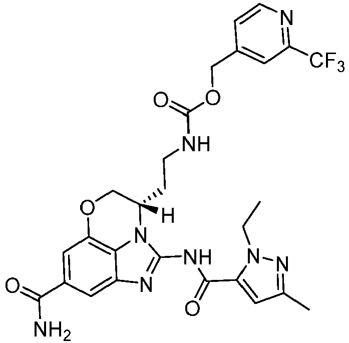 | 36 | 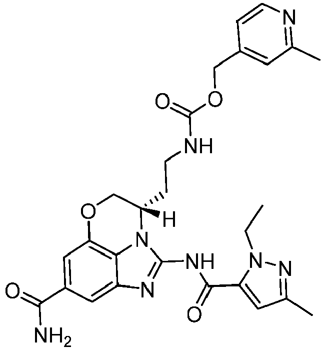 |
| 37 | 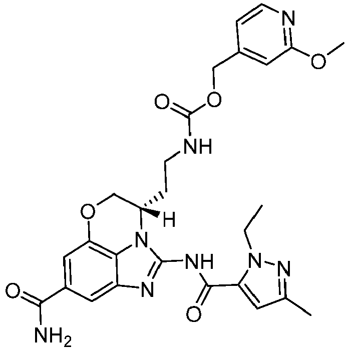 | 38 | 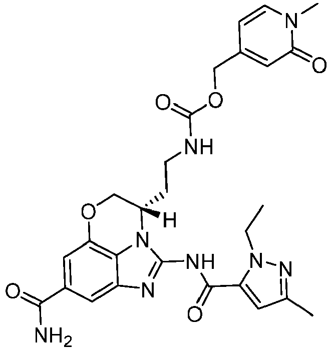 |
| 39 | 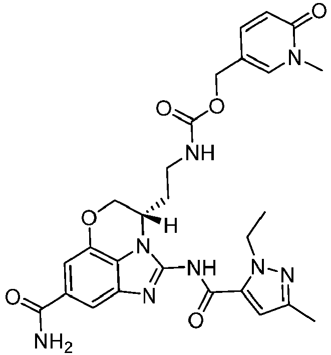 | 40 | 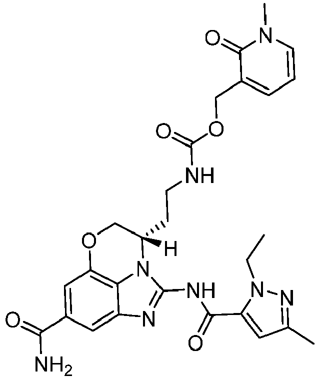 |
| 41 | 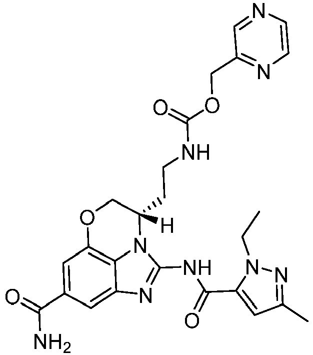 | 42 | 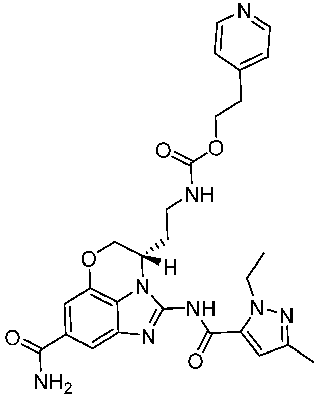 |
| 43 | 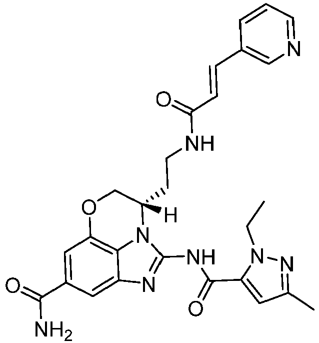 | 44 | 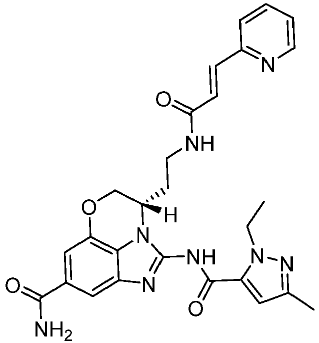 |
| 45 | 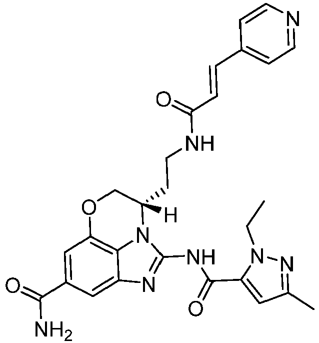 | 46 | 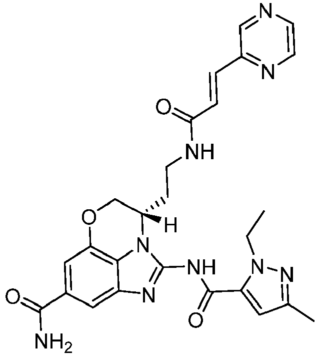 |
| 47 | 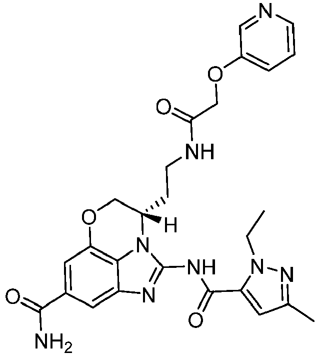 | 48 | 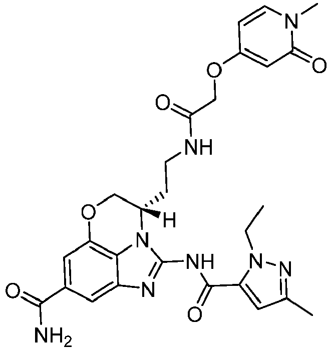 |
| 49 | 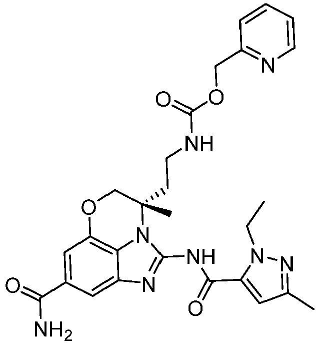 | 50 | 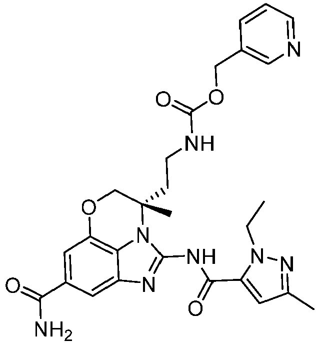 |
| 51 | 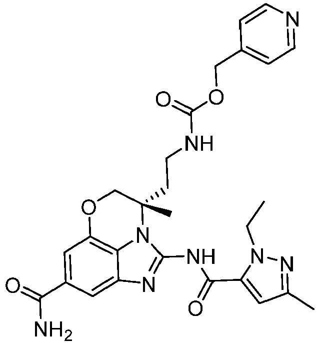 | 52 | 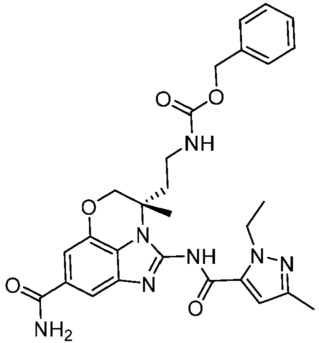 |
| 53 | 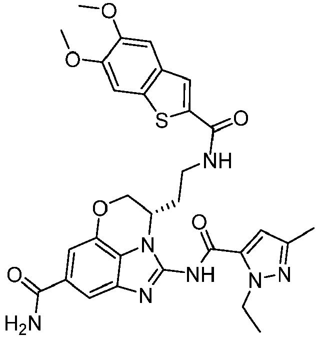 | 54 | 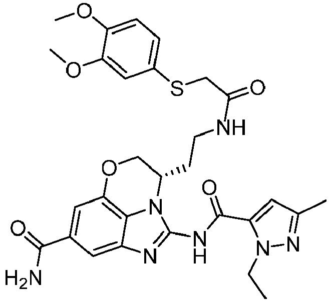 |
| 55 | 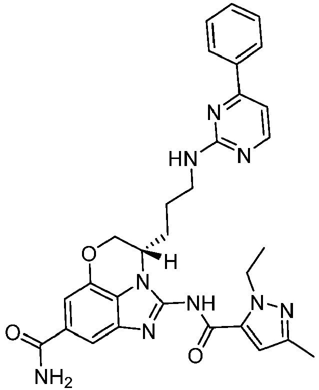 | 56 | 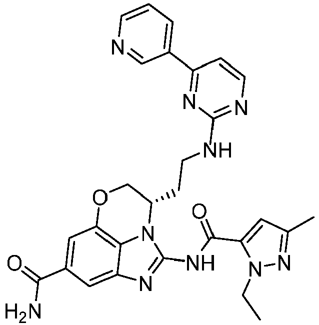 |
| 57 | 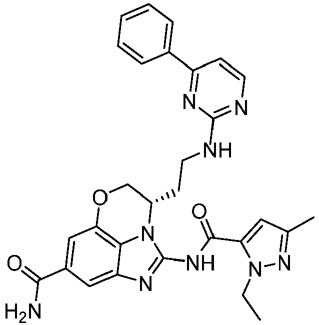 | 58 | 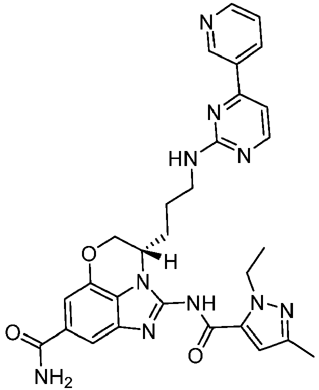 |
| 59 | 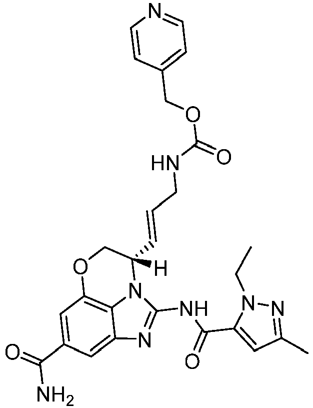 | 60 | 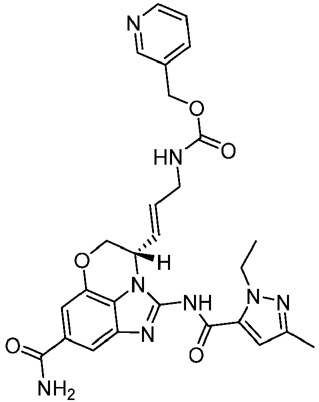 |
| 61 | 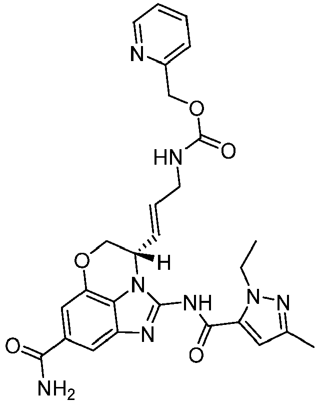 | 62 | 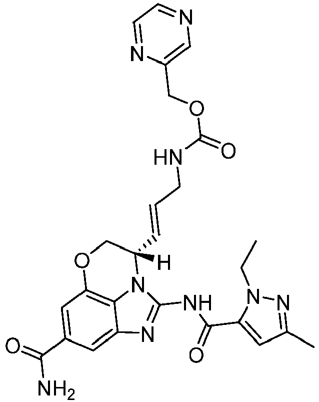 |
| 63 | 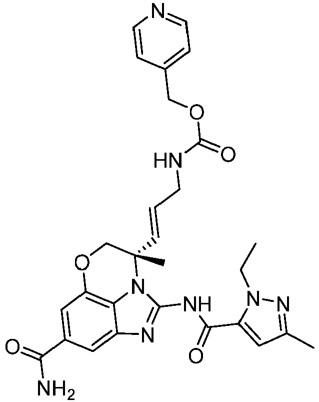 | 64 | 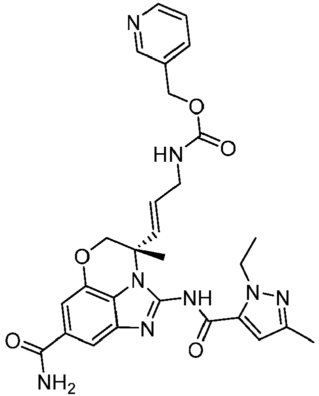 |
| 65 | 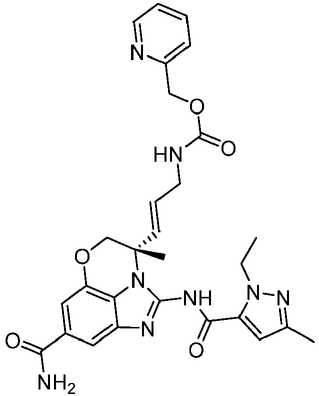 | 66 | 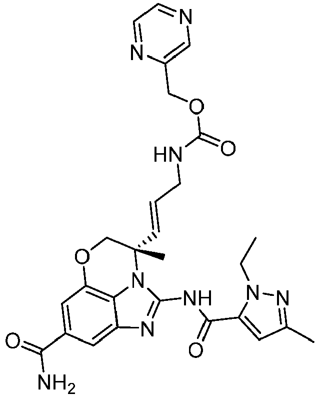 |
| 67 | 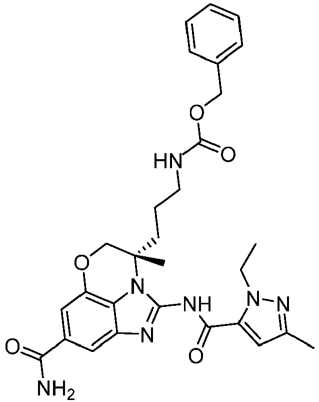 | 68 | 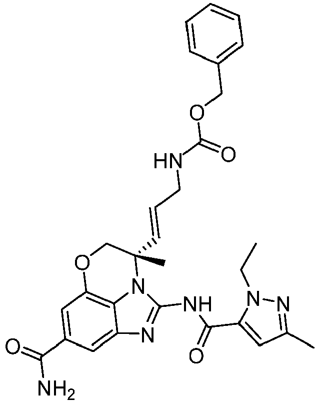 |
| 69 | 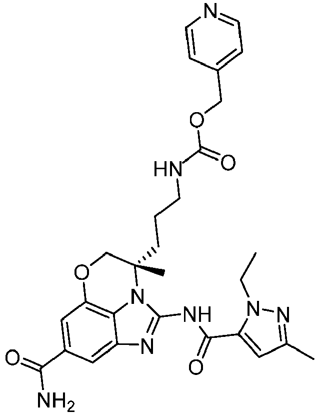 | 70 | 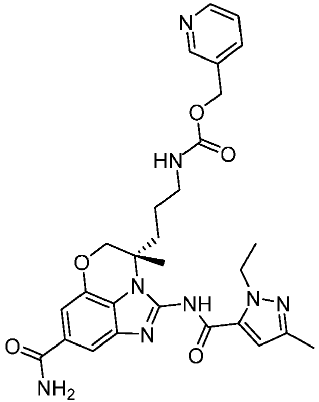 |
| 71 | 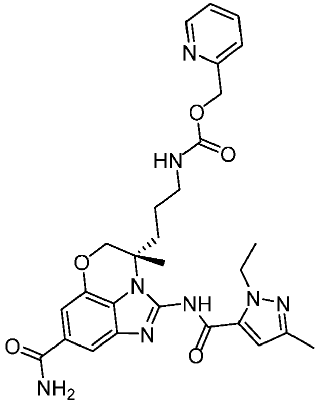 | 72 | 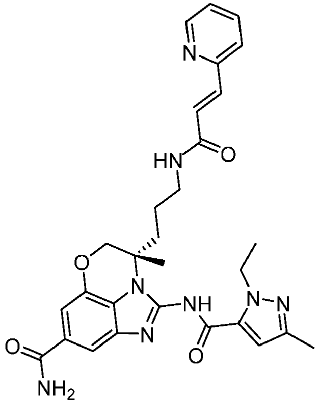 |
| 73 | 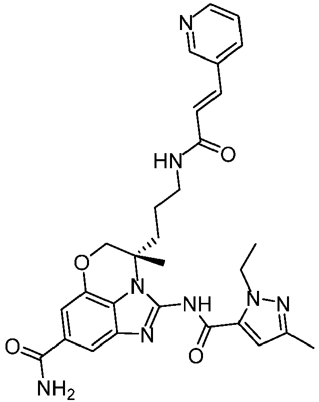 | 74 | 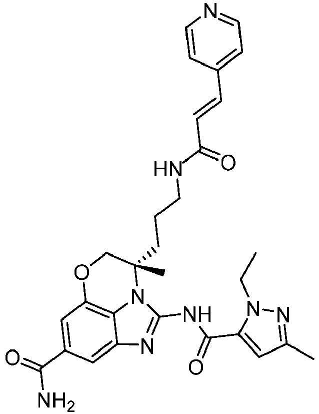 |
| 75 | 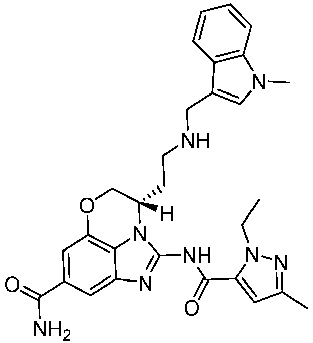 | 76 | 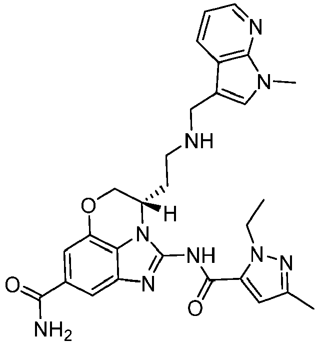 |
| 77 | 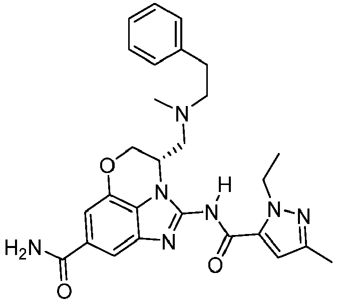 | 78 | 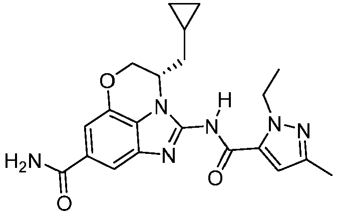 |
| 79 | 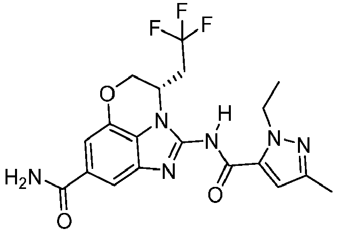 | 80 | 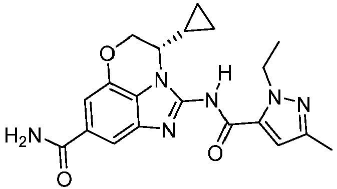 |
| 81 | 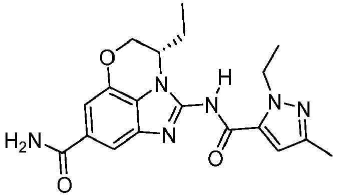 | 82 | 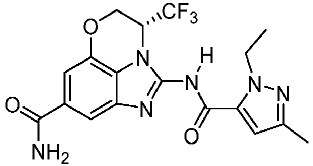 |
| 83 | 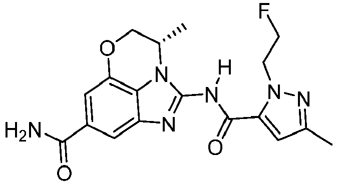 | 84 | 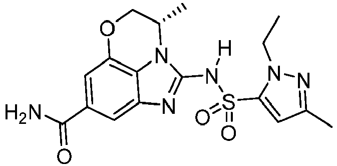 |
| 85 | 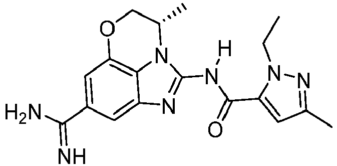 | 86 | 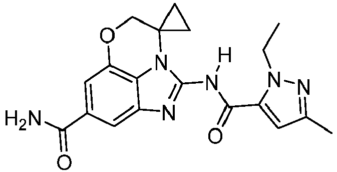 |
| 87 | 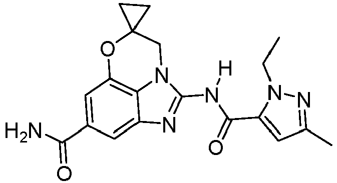 | 88 | 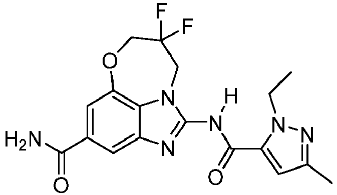 |
| 89 | 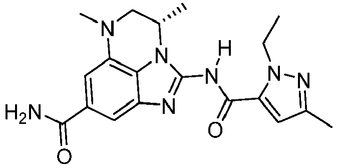 | 90 | 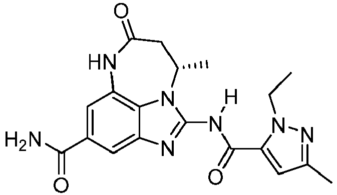 |
| 91 | 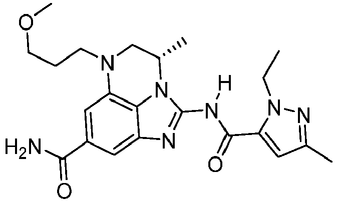 | 92 | 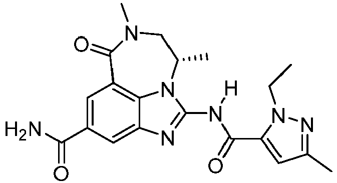 |
| 93 | 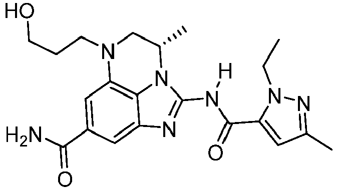 | 94 | 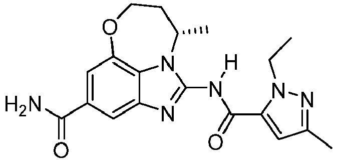 |
| 95 | 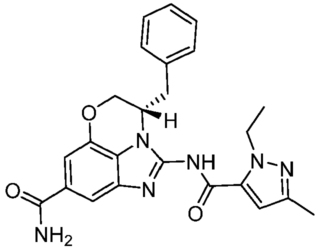 | 96 | 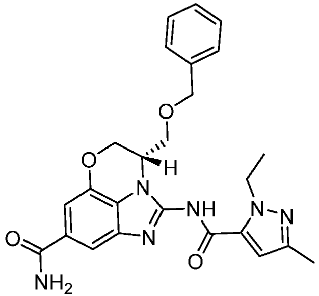 |
| 97 | 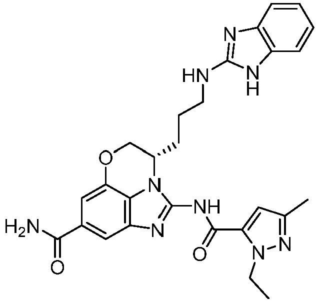 | 98 | 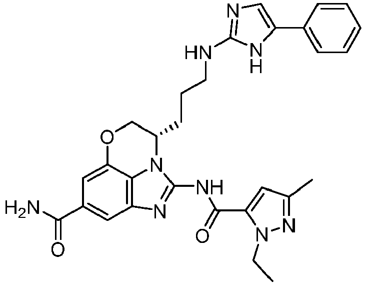 |
| 99 | 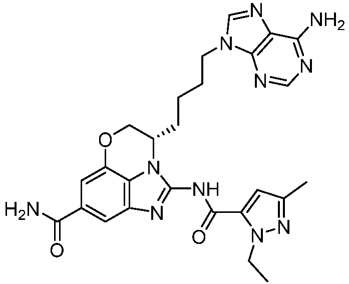 | 100 | 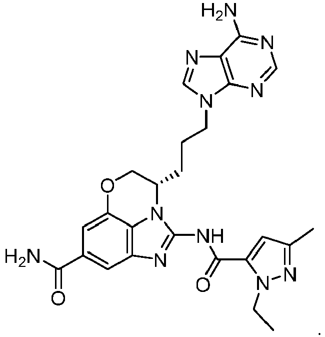 |
| 101 | 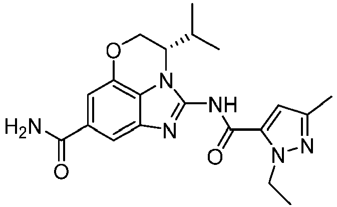 | 102 | 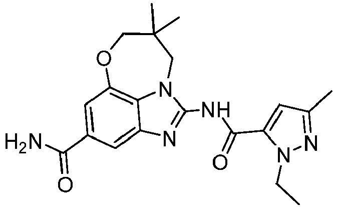 |
| 103 | 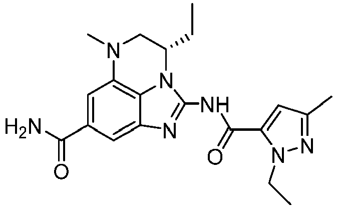 | 104 | 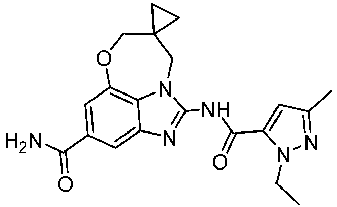 |
| 105 | 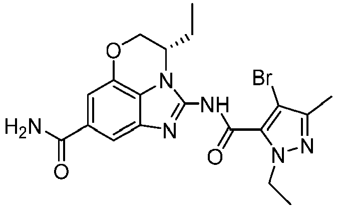 | 106 | 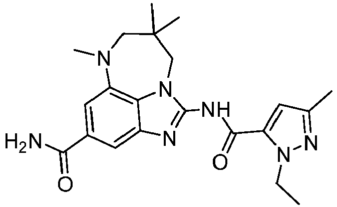 |
| 107 | 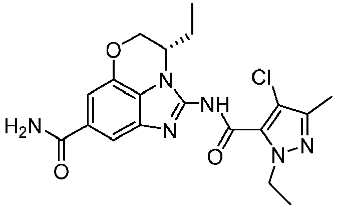 | 108 | 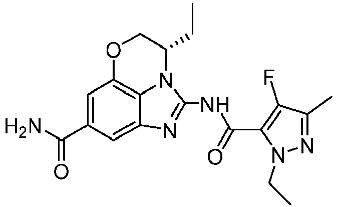 |
| 109 | 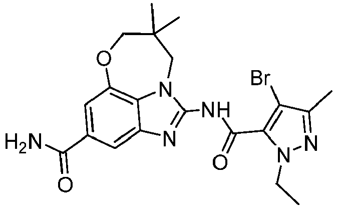 | 110 | 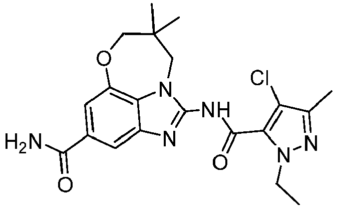 |
| 111 | 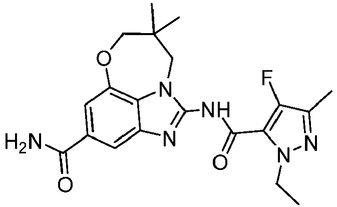 | 112 | 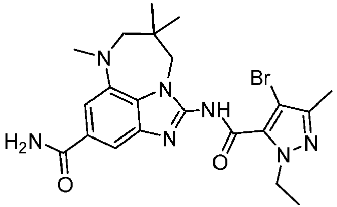 |
| 113 | 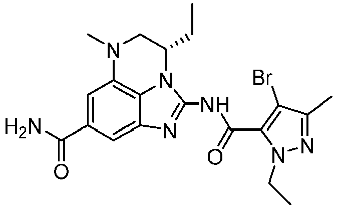 | 114 | 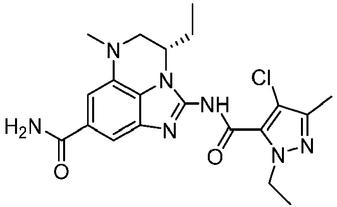 |
| 115 | 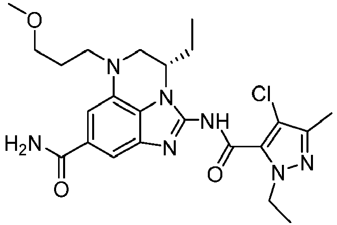 | 116 | 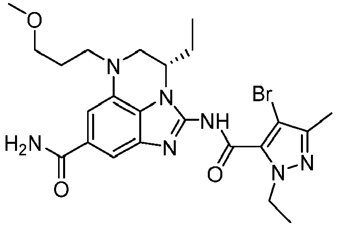 |
| 117 | 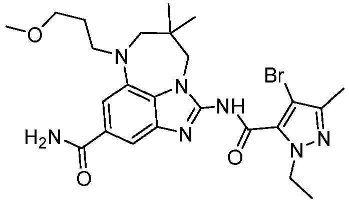 | 118 | 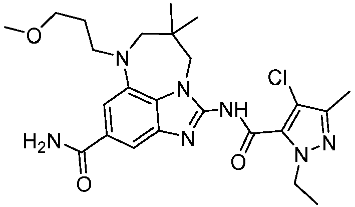 |
| 119 | 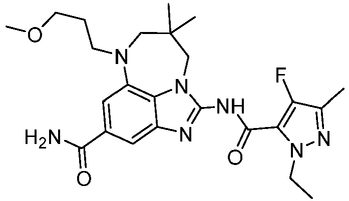 | 120 | 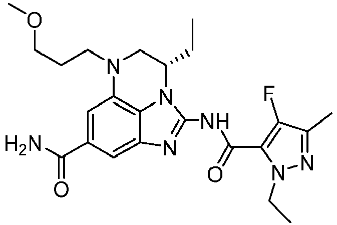 |
| 121 | 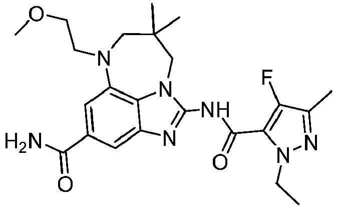 | 122 | 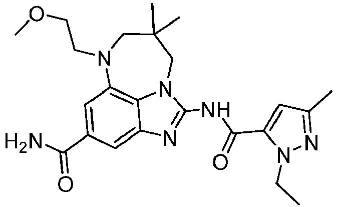 |
| 123 | 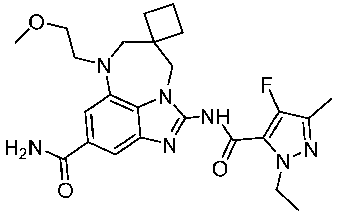 | 124 | 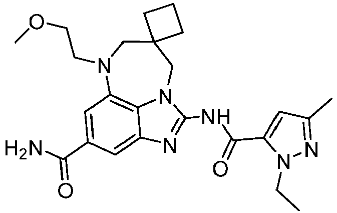 |
| 125 | 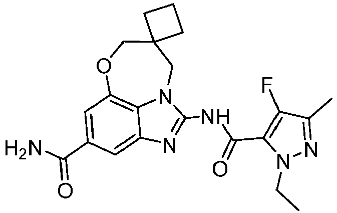 | 126 | 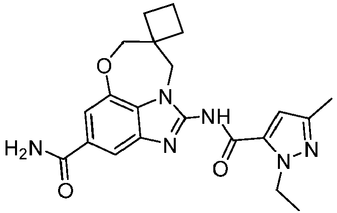 |
| 127 | 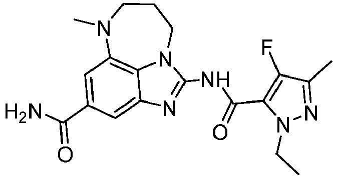 | 128 | 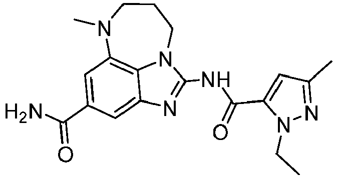 |
| 129 | 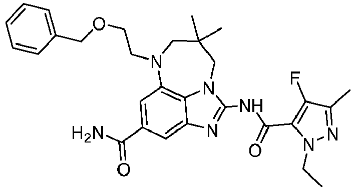 | 130 | 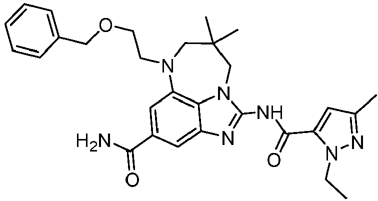 |
| 131 | 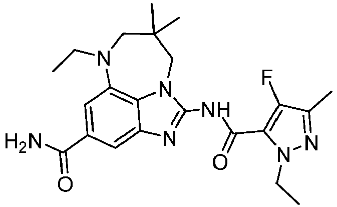 | 132 | 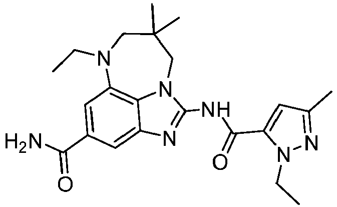 |
| 133 | 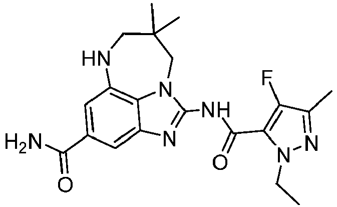 | 134 | 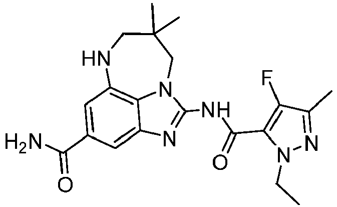 |
| 135 | 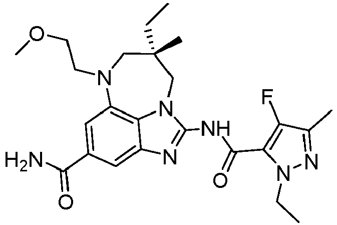 | 136 | 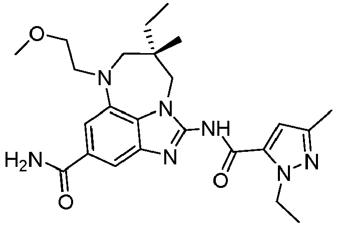 |
| 137 | 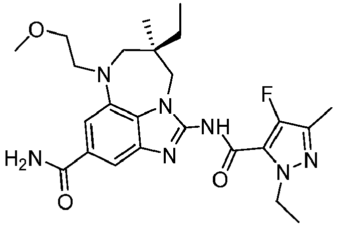 | 138 | 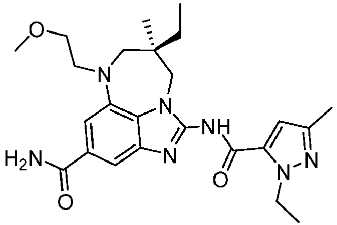 |
| 139 | 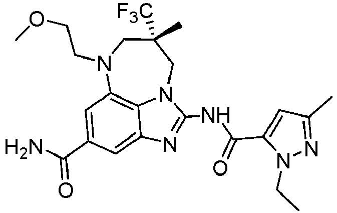 | 140 | 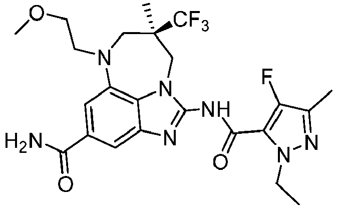 |
| 141 | 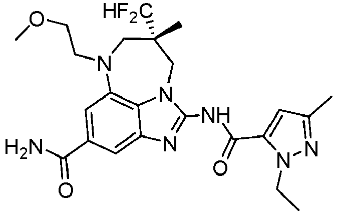 | 142 | 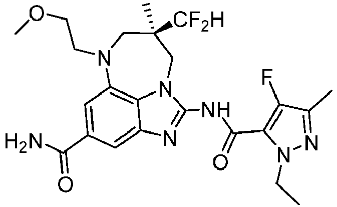 |
| 143 | 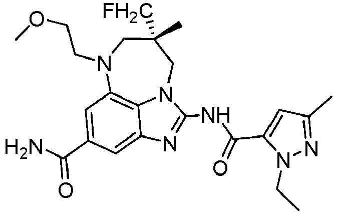 | 144 | 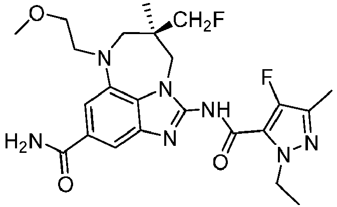 |
| 145 | 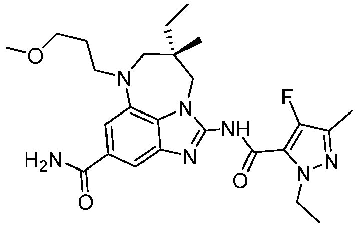 | 146 | 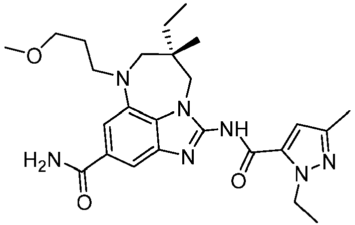 |
| 147 | 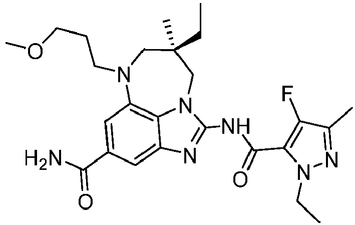 | 148 | 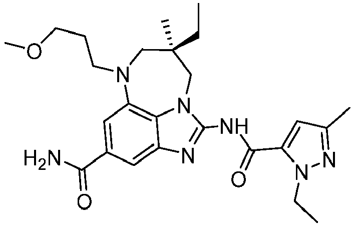 |
| 149 | 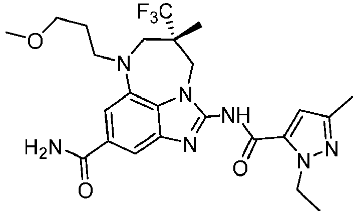 | 150 | 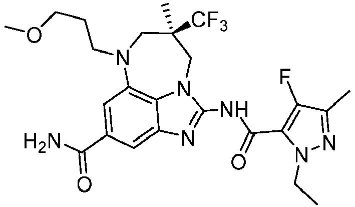 |
| 151 | 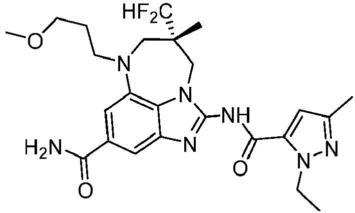 | 152 | 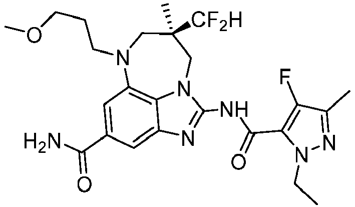 |
| 153 | 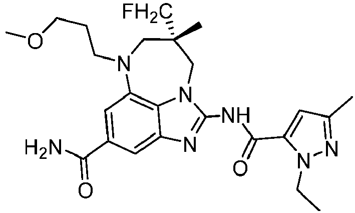 | 154 | 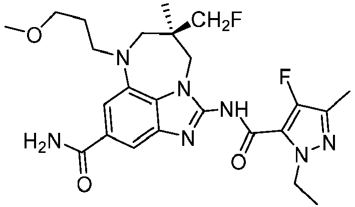 |
| 155 | 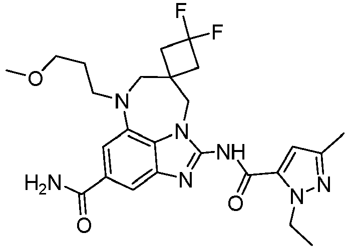 | 156 | 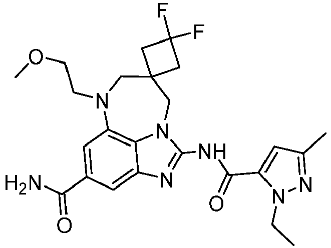 |
| 157 | 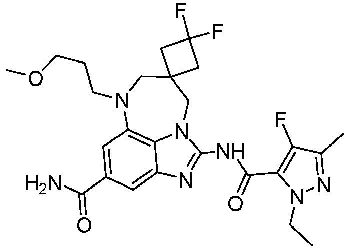 | 158 | 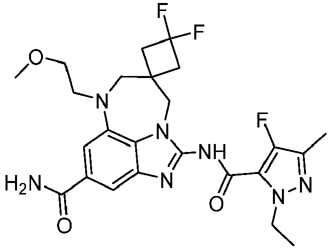 |

12. Une composition pharmaceutique comprenant un composé selon l'une quelconque des revendications 1 à 11 et un support pharmaceutiquement acceptable.

13. La composition pharmaceutique selon la revendication 12, comprenant en outre un agent thérapeutique supplémentaire et/ou un inhibiteur de point de contrôle immunitaire, dans laquelle l'agent thérapeutique supplémentaire est de préférence choisi parmi le Chlorambucil, Melphalan, Cyclophosphamide, Ifosfamide, Busulfan, Carmustine, Lomustine, Streptozotocine, Cisplatine, Carboplatine, Oxaliplatine, Dacarbazine, Témozolomide, Procarbazine, Méthotrexate, Fluorouracile, Cytarabine, Gemcitabine, Mercaptopurine, Fludarabine, Vinblastine, Vincristine, Vinorelbine, Paclitaxel, Docétaxel, Topotécan, Irinotécan, Étoposide, Trabectédine, Dactinomycine, Doxorubicine, Epirubicine, Daunorubicine, Mitoxantrone, Bléomycine, Mitomycine C, Ixabépilone, Tamoxifène, Flutamide, Analogues de Gonadoreline, Mégestrol, Prednisone, Dexaméthasone, Méthylprednisolone, Thalidomide, Interféron A, Folinate de Calcium, Sirolimus, Sirolimus Lipide, Everolimus, Afatinib, Alisertib, Amuvatinib, Apatinib, Axitinib, Bortezomib, Bosutinib, Brivanib, Cabozantinib, Cediranib, Crenolanib, Crizotinib, Dabrafenib, Dacomitinib, Danusertib, Dasatinib, Dovitinib, Erlotinib, Foretinib, Ganetespib, Gefitinib, Ibrutinib, Icotinib, Imatinib, Iniparib, Lapatinib, Lenvatinib, Linifanib, Linsitinib, Masitinib, Momelotinib, Motesanib, Neratinib Nilotinib, Niraparib, Oprozomib, Olaparib, Pazopanib, Pictiliisib, Ponatin ib, Quizartinib, Régorafénib, Rigosertib, Rucaparib, Ruxolitinib, Saracatinib, Saridegib, Sorafenib, Sunitinib, Telatinib, Tivantinib, Tivozanib, Tofacitinib, Trametinib, Vandetanib, Veliparib, Vemurafenib, Erivedge, Volasertib, Alemtuzumab, Bevacizumab, Brentuximab Vedotin, Catumaxomab, Cetuximab, Denosumab, Gemtuzumab, Ipilimumab, Nimotuzumab, Ofatumumab, Panitumumab, Rituximab, Tositumomab, Trastuzumab, inhibiteurs de PI3K, inhibiteurs du CSF1R, antagonistes des récepteurs A2A et/ou A2B, inhibiteurs de l'IDO, anticorps anti-PD-1, anticorps anti-PD-L1, anticorps LAG3, anticorps TIM-3 et anticorps anti-CTLA-4, ou toute combinaison de ceux-ci.

14. Le composé selon l'une quelconque des revendications 1 à 11 ou la composition pharmaceutique selon la revendication 12 ou 13 pour une utilisation dans la prévention et/ou le traitement des tumeurs, du cancer, des infections virales, du rejet de greffe d'organe, des maladies neurodégénératives, des maladies liées à l'attention, ou des maladies auto-immunes.

15. Le composé ou la composition pharmaceutique pour une utilisation selon la revendication 14, dans lequel la tumeur ou le cancer est choisi dans le groupe constitué du cancer de la peau, du cancer de la vessie, du cancer des ovaires, du cancer du sein, du cancer gastrique, du cancer du pancréas, du cancer de la prostate, du cancer du côlon, du cancer du poumon, du cancer des os, du cancer du cerveau, du neurocytome, du cancer rectal, du cancer du côlon, du cancer de la polypose adénomateuse familiale, du cancer colorectal héréditaire sans polypose, du cancer de l'oesophage, du cancer des lèvres, du cancer du larynx, du cancer de l'hypopharyngé, du cancer de la langue, du cancer des glandes salivaires, du cancer gastrique, de l'adénocarcinome, du cancer médullaire de la thyroïde, du cancer papillaire de la thyroïde, du cancer du rein, du carcinome du parenchyme rénal, du cancer de l'ovaire, du cancer du col de l'utérus, du carcinome du corps, cancer de l'endomètre, du choriocarcinome, du cancer du pancréas, du cancer de la prostate, du cancer des testicules, du carcinome du système urinaire, du mélanome, du tumeurs cérébrales telles que glioblastome, de l'astrocytome, du méningiome, du médulloblastome et tumeurs neuroectodermiques périphériques, du lymphome de Hodgkin, du lymphome non hodgkinien, du lymphome de Burkitt, du leucémie lymphoblastique aiguë (LAL), du leucémie lymphoïde chronique (LLC), du leucémie myéloïde aiguë (LAM), du leucémie myéloïde chronique (LMC), du lymphome leucémique à cellules T de l'adulte, du lymphome diffus à grandes cellules B (DLBCL), du carcinome hépatocellulaire, du carcinome de la vésicule biliaire, du carcinome bronchique, du carcinome du poumon à petites cellules, du carcinome du poumon non à petites cellules, du myélome multiple, du tumeur basocellulaire, du tératome, du rétinoblastome, du mélanome choroïdien, du séminome, du rhabdomyosarcome, du craniopharyngiome, de l'ostéosarcome, du chondrosarcome, du myosarcome, du liposarcome, du fibrosarcome, du sarcome d'Ewing ou du plasmocytome.
